# EUROPEAN PATENT SPECIFICATION

(11) **EP 2 444 503 B1**
(45) Date of publication and mention of the grant of the patent: **02.03.2016**
(21) Application number: 10306142.0
(22) Date of filing: 20.10.2010
(51) Int. Cl.: C12Q 1/68

(54) **Use of specific genes for the prognosis of lung cancer and the corresponding progonosis method**
Verwendung spezifischer Gene oder ihrer codierten Proteine für ein Prognoseverfahren für klassifizierten Lungenkrebs
Utilisation de gènes spécifiques ou de leurs protéines codées pour le pronostic du cancer du poumon classé

(43) Date of publication of application: 25.04.2012
(73) Proprietor: Université Joseph Fourier, 38041 Grenoble Cedex 09 (FR)
(72) Inventor: Pison-Rousseaux, Sophie, 38410 Saint Martin D'Uriage (FR); Khochbin, Saadi, 38240 Meylan (FR)
(74) Representative: Grosset-Fournier, Chantal Catherine

(56) References cited:
- EP-A1- 2 107 127
- WO-A1-2010/065944
- WO-A2-2006/029176
- US-A1- 2010 009 357
- DATABASE Geneseq [Online] 23 January 2003 (2003-01-23), "Group III cDNA cancer related clone SEQ ID NO:1567.", XP002621841, retrieved from EBI accession no. GSN:ABZ19141 Database accession no. ABZ19141 -& WO 02/078516 A2 (CORIXA CORP [US]; WANG TONGTONG [US]; WANG SIQING AKA STEVEN [US]; BAN) 10 October 2002 (2002-10-10)
- LIU LI ET AL: "[Design of oligonucleotide probes for cancer-testis genes for cancer diagnosis microarray].", SICHUAN DA XUE XUE BAO. YI XUE BAN = JOURNAL OF SICHUAN UNIVERSITY. MEDICAL SCIENCE EDITION MAR 2009 LNKD- PUBMED:19462920, vol. 40, no. 2, March 2009 (2009-03), pages 330-333 , 336, XP009143273, ISSN: 1672-173X
- GURE ALI O ET AL: "Cancer-testis genes are coordinately expressed and are markers of poor outcome in non-small cell lung cancer.", CLINICAL CANCER RESEARCH : AN OFFICIAL JOURNAL OF THE AMERICAN ASSOCIATION FOR CANCER RESEARCH 15 NOV 2005 LNKD- PUBMED:16299236, vol. 11, no. 22, 15 November 2005 (2005-11-15), pages 8055-8062, XP002621272, ISSN: 1078-0432
- CARON C ET AL: "Functional characterization of ATAD2 as a new cancer/testis factor and a predictor of poor prognosis in breast and lung cancers.", ONCOGENE 16 SEP 2010 LNKD- PUBMED:20581866, vol. 29, no. 37, 16 September 2010 (2010-09-16), pages 5171-5181, XP002621273, ISSN: 1476-5594
- LUCAS S ET AL: "MAGE-B5, MAGE-B6, MAGE-C2, and MAGE-C3: Four new members of the MAGE family with tumor-specific expression", INTERNATIONAL JOURNAL OF CANCER, JOHN WILEY & SONS, INC, UNITED STATES, SWITZERLAND, GERMANY, vol. 87, no. 1, 1 July 2000 (2000-07-01), pages 55-60, XP002471717, ISSN: 0020-7136, DOI: DOI:10.1002/1097-0215(20000701)87:1<55::AI D-IJC8>3.0.CO;2-J
- CHEN YAO-TSENG ET AL: "Identification of CT46/HORMAD1, an immunogenic cancer/testis antigen encoding a putative meiosis-related protein", CANCER IMMUNITY, ACADEMY OF CANCER IMMUNOLOGY, CH, vol. 5, 7 July 2005 (2005-07-07), page 9, XP002380625, ISSN: 1424-9634
- KWIATKOWSKI D J ET AL: "Molecular pathologic substaging in 244 stage I non-small-cell lung cancer patients: Clinical implications", JOURNAL OF CLINICAL ONCOLOGY, AMERICAN SOCIETY OF CLINICAL ONCOLOGY, US, vol. 16, no. 7, 1 January 1998 (1998-01-01), pages 2468-2477, XP009096353, ISSN: 0732-183X
- E. BRAMBILLA ET AL: "The new World Health Organization classification of lung tumours", EUROPEAN RESPIRATORY JOURNAL, vol. 18, no. 6, 1 December 2001 (2001-12-01), pages 1059-1068, XP055053635, ISSN: 0903-1936, DOI: 10.1183/09031936.01.00275301

## Description

The present invention relates to the use of specific genes for the prognosis of lung cancer.

Lung cancer is a disease of uncontrolled cell growth in tissues of the lung. This growth may lead to metastasis, which is the invasion of adjacent tissues and infiltration beyond the lungs. The vast majority of primary lung cancers are carcinomas of the lung, derived from epithelial cells. Lung cancer, the most common cause of cancer-related death in men and women, is responsible for 1.3 million deaths worldwide annually, as of 2004. The most common symptoms are shortness of breath, coughing (including coughing up blood), and weight loss.

Due to the high prevalence of this type of tumors, there is a need to efficiently diagnose lung cancer. Moreover, it is important to propose a prognosis method that allows the pathologist to determine, when a patient is afflicted by lung tumors, the survival rate during a short and a long period, and consequently to propose an adapted therapy.

Presently, several clinical and pathological parameters help defining the prognosis, including histological subtypes, TNM stages (tumour size, presence of tumour cells in lymph nodes, presence of distant metastasis).

Classically, prognosis and diagnosis methods intend to detect the variation of expression of genes between the sample from a patient and a healthy control sample. However, with these methods, false positive results are frequent and indistinguishable from real positive samples.

US patent US 2010/0009357 discloses a method to estimate the likelihood of lung cancer recurrence in a subject afflicted with NSCLC (Non-Small-Cell Lung Carcinoma) based on the determination of the expression of clusters of genes. However, this method is based on the generation of statistical trees using algorithms.

Cancer Testis (CT) genes are genes that are expressed in testis cells, but not expressed in somatic non pathologic cells. In cancers, CT genes are deregulated and are expressed ectopically in somatic cells. They appear as good candidate for cancer diagnosis.

Some works have intended to identify a "general" strategy for diagnosing lung cancer, by detecting cancer testis gene expression.

For instance, the international application WO 2009/121878 discloses the use of a minimal group of CT genes for identifying any somatic or ovarian cancer. However, even if specific genes, or combinations of genes, can be used for diagnosing cancer, there is no indication that these genes can be used to establish a reliable prognosis during a short or a long period.

Recently, Gure et al. (Gure at al. Clin Cancer reaserch, 2005, 11(22) p:8055-8061) have proposed that cancer testis genes are coordinatly expressed in non-small cell lung cancers, and are markers of poor outcome. The study suggests that X-linked CT genes can be associated with worse prognosis, either by their expression, or by their increased expression.

Therefore, there is a need to provide prognosis marker that can give specific evolution of tumoral progression, and patient survival, for instance by using CT genes.

One aim of the invention is to a simple, rapid, easy-to-use and effective method for giving a prognosis of lung cancer.

Another aim of the invention is to provide a general prognosis method of lung tumor. Another aim of the invention is to provide a kit for diagnosing lung cancer.

The present disclosure describes the use of
- at least 2 genes chosen among a set of 23 genes comprising or consisting of the nucleic acid sequences SEQ ID NO 1 to 23,
- or fragments of said genes,
- or complementary sequences of said genes,
- or sequences having at least 80% homology with said genes or fragment thereof,
- or protein coded by said genes,
- or fragments of said proteins,
- or antibodies directed against said proteins,
said at least 2 genes being such that
- at least one gene belongs to a first set A of 7 genes comprising or consisting of the nucleic acid sequences SEQ ID NO: 1-7
- at least one gene belongs to a second set B of 16 genes comprising or consisting of the nucleic acid sequences SEQ ID NO: 8-23
for the implementation of a prognosis method, preferably *in vitro*, of the survival rate of a patient afflicted by a lung cancer, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one set A or B is expressed, the patient survival rate during a period of time from 30 to 120 months after the diagnosis of said lung cancer is about from about 3% to about 70%.

The present invention relates to the use of- at least 2 genes chosen among a set of 23 genes comprising or consisting of the nucleic acid sequences SEQ ID NO 1 to 23,
- or complementary sequences of said at least 2 genes chosen among a set of 23 genes comprising or consisting of the nucleic acid sequences SEQ ID NO 1 to 23,
- or sequences having at least 80% homology with said genes,
- or proteins coded by said at least 2 genes chosen among a set of 23 genes comprising or consisting of the nucleic acid sequences SEQ ID NO 1 to 23, said
   ◆ at least 2 genes being such that
- at least one gene belongs to a first set A of 7 genes comprising or consisting of the nucleic acid sequences SEQ ID NO: 1-7
- at least one gene belongs to a second set B of 16 genes comprising or consisting of the nucleic acid sequences SEQ ID NO: 8-23,
   ◆ at least 2 proteins being such that
- at least one protein belongs to a first set AP of 7 proteins, each 7 proteins being coded by the genes comprising or consisting of the nucleic acid sequences SEQ ID NO: 1-7,
- at least one protein belongs to a second set BP of 16 proteins, each 16 proteins being coded by the genes comprising or consisting of the nucleic acid sequences SEQ ID NO: 8-23,
for an *in vitro* prognosis method of the survival rate of a patient afflicted by a lung cancer, said method comprising a step of measuring, in a lung cancer sample of said patient, the expression of said at least 2 genes, or said complementary sequences, or said sequences having at least 80% homology with said genes, or said at least 2 proteins, and said prognosis being such that:
either
▪ if none of the 23 genes of said set is expressed, the patient survival rate during a period of time from 30 to 120 months after the diagnosis of said lung cancer is from about 59% to about 78%, and
▪ if at least one gene of at least one set A or B is expressed, the patient survival rate during a period of time from 30 to 120 months after the diagnosis of said lung cancer is about from about 3% to about 70%,
or
▪ if none of the 23 proteins of said set is expressed, the patient survival rate during a period of time from 30 to 120 months after the diagnosis of said lung cancer is from about 59% to about 78%, and
▪ if at least one protein of at least one set AP or BP is expressed, the patient survival rate during a period of time from 30 to 120 months after the diagnosis of said lung cancer is about from about 3% to about 70%,
wherein said gene or protein is determined as being expressed when:
- either it is expressed in a sample of a patient afflicted by a lung cancer but not in a control sample of an healthy individual,
- or it is expressed above a threshold corresponding to a background signal observed in a series of reference healthy tissues for each detection method, and
   said gene or protein is determined as being not expressed when:
- it is neither expressed in a sample of a patient afflicted by a lung cancer nor in a control sample of an healthy individual,
- or it is expressed in a sample of a patient afflicted by a lung cancer at a level substantially equal or inferior to the level in a control sample of an healthy individual.

The present invention is based on the unexpected observation made by the Inventors that the expression of at least two genes of a group of 23 determined genes is sufficient to determine the survival rate of a patient afflicted by a lung cancer.

In other words, and as explained and exemplified hereafter, according to the invention the determination of the gene expression status on a ON/OFF basis of at least 2 genes chosen among a set of 23 genes allows to estimate at 30 months, or 60 months or 120 months after the diagnosis of a lung cancer, the probability of survival of an individual.

The diagnosis method proposed by the Inventors can also be carried out by detecting the expression of proteins expressed by at least two genes above mentioned, chosen among proteins coded by said 23 genes, also on a absence/presence basis.

Another aspect of this disclosure is that the diagnosis can also be carried out by determining the presence, of at least 2 specific antibodies specifically recognising at least 2 proteins coded by said at least 2 genes mentioned above. The above antibodies are specific of one protein, each protein being coded by one gene of the set of 23 genes.

A key aspect of the invention is the concept of ON/OFF for gene expression. The concept of ON/OFF expression can be extended to presence/absence of the proteins and antibodies detecting these proteins. This specific approach has the advantage of simplifying the analyses and making them independent of complex statistical tests to measure variations in expression levels applied to the majority of the existing tests.

The ON/OFF status of gene expression is established by determination of a threshold of gene expression allowing them to decide on the ON/OFF status of a gene such that:
- if a gene is expressed at a level lower than the threshold, the gene is considered as not expressed or weakly expressed (defined as OFF), and
- if a gene is expressed at a level upper to the threshold, the gene is considered as being expressed (defined as ON).

According to the invention, the prognosis is carried out as described hereafter:

The Inventors have identified 23 genes comprising or being constituted by the nucleic acid sequences SEQ ID NO 1 to 23, as being cancer testis genes (CT genes) that can be used to carry out the prognosis method according to the invention.

The above 23 genes have been identified as being liable to be "expressed" (form here by, "expressed" refers to the ON status and "not expressed" to the OFF status) in lung cancer cells, but not in healthy samples. In other words, the above 23 genes are such as
- they are not expressed, or weakly expressed in healthy lung cells, and
- they maybe expressed in lung tumor cells.

The difference between the absence of expression, or weak expression, and the expression determines its ON/OFF status, which is a key step of the invention. Indeed, the Inventors have identified that the ON status of the above 23 genes is a key step to determine the prognosis of lung cancer.

On microarrays, the expression level of the above mentioned genes is determined by the fact that a threshold of expression has been identified by the Inventors allowing to determine expression (ON) and non-expression (OFF) of said genes. The threshold determination is detailed hereafter, in the Example section.

For the microarrays, the threshold enabling to determine the expression status of a gene (ON versus OFF) is calculated by using the signal mean value and distribution obtained from transcriptomic data (in the same technology) with the corresponding probes in a large number of somatic tissues (which do not express the genes).

A similar strategy enables determining a threshold for the presence/absence of the encoded proteins or antibodies. For each protein or antibody, the mean value and distribution of the signal intensities obtained in an appropriate number of control somatic tissues serves as a basis for calculating the threshold.

By "not expressed" it is defined in the invention the fact that the transcription of a gene is either not carried out, or is not detectable by common techniques known in the art, such as Quantitative RT-PCR, Northern blot or when microarrays data are considered.

By "weakly expressed", it is defined in the invention that a gene is expressed at a low level, meaning that the values are within the range of those measured for healthy tissue samples by Q-RT-PCR and by Northern blots or below the threshold when microarray data are considered. These values are considered as false-positive expressions, due to probe cross hybridization for instance. All the expression falling in these categories are considered as "OFF"

By "expressed", the invention defined that the transcript of a gene is detectable by the above known techniques while it is not detectable in healthy tissues or determined as being above the threshold when microarrays data are considered.

The 23 genes according to the invention have been classified by the Inventors in two sets:
- a first set of 7 genes,
- and a second set of 16 genes.

The first set, also called in the invention set A, of 7 genes consists of the genes comprising or constituted by the nucleic acid sequences SEQ ID NO: 1, SEQ ID NO: 2, SEQ ID NO: 3, SEQ ID NO: 4, SEQ ID NO: 5, SEQ ID NO: 6 and SEQ ID NO: 7. The second set, also called in the invention set B, of 16 genes consists of the genes comprising or constituted by the nucleic acid sequences SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, and SEQ ID NO: 23.

The inventors have also defined subsets of each of the above sets A and B as follows: Set A is divided into 4 subsets A1, A5, A6 and A7, said subset being such that:
- subset A1 consists of the genes comprising or being constituted by the nucleic acid sequences SEQ ID NO: 1 and SEQ ID NO: 2,
- subset A5 consists of the gene comprising or being constituted by the nucleic acid sequence SEQ ID NO: 3,
- subset A6 consists of the genes comprising or being constituted by the nucleic acid sequence SEQ ID NO: 4, and SEQ ID NO: 5, and
- subset A7 consists of the genes comprising or being constituted by the nucleic acid sequences SEQ ID NO: 6 and SEQ ID NO: 7

Set A can also be divided into the following subsets:
- subset A1 consists of the genes comprising or being constituted by the nucleic acid sequences SEQ ID NO: 1 and SEQ ID NO: 2,
- subset A2 consists of the genes comprising or being constituted by the nucleic acid sequences SEQ ID NO: 1, SEQ ID NO: 2 and SEQ ID NO: 3,
- subset A3 consists of the genes comprising or being constituted by the nucleic acid sequences SEQ ID NO: 1, SEQ ID NO: 2, SEQ ID NO: 3, SEQ ID NO: 4 and SEQ ID NO: 5, and
- subset A4 consists of the genes comprising or being constituted by the nucleic acid sequences SEQ ID NO: 1, SEQ ID NO: 2, SEQ ID NO: 3, SEQ ID NO: 4, SEQ ID NO: 5, SEQ ID NO: 6 and SEQ ID NO: 7.

Set B is divided into 3 subsets B1, B4 and B5, said subset being such that:
- subset B1 consists of the genes comprising or being constituted by the nucleic acid sequences SEQ ID NO: 8, SEQ ID NO: 9, SEQ ID NO: 10, SEQ ID NO: 11, SEQ ID NO: 12, SEQ ID NO: 13, SEQ ID NO: 14 and SEQ ID NO: 15,
- subset B4 consists of the genes comprising or being constituted by the nucleic acid sequences SEQ ID NO: 16, SEQ ID NO: 17, SEQ ID NO: 18, SEQ ID NO: 19, SEQ ID NO: 20 and SEQ ID NO: 21, and
- subset B5 consists of the genes comprising or being constituted by the nucleic acid sequences SEQ ID NO: 22, and SEQ ID NO: 23.

Set B can also be divided into the following subsets:
- subset B 1 consists of the genes comprising or being constituted by the nucleic acid sequences SEQ ID NO: 8, SEQ ID NO: 9, SEQ ID NO: 10, SEQ ID NO: 11, SEQ ID NO: 12, SEQ ID NO: 13, SEQ ID NO: 14 and SEQ ID NO: 15,
- subset B2 consists of the genes comprising or being constituted by the nucleic acid sequences SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 and SEQ ID NO: 21, and
- subset B3 consists of the genes comprising or being constituted by the nucleic acid sequences SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 and SEQ ID NO: 21, SEQ ID NO: 22, and SEQ ID NO: 23.

Thus, the prognosis method according to the invention is such that, when determining the expression status of 2 genes on a ON/OFF basis, belonging to the set of 23 genes comprising or consisting of nucleic acid sequences SEQ ID NO : 1 to 23,
- if none of said at least 2 genes are expressed, the patient survival rate at 30 months, 60 months or 120 months following the diagnosis is from about 59% to about 78% or more, and
- if at least one of said at least two genes is expressed, according to the defined expression threshold, the patient survival rate at 30 months, 60 months or 120 months following the diagnosis is from about 3% to about 70%.

According to the invention, the above mentioned at least 2 genes are such that:
- at least one of said at least two genes belongs to a first set A of 7 genes comprising or consisting of nucleic acid sequences SEQ ID NO : 1 to 7, and
- at least one of said at least two genes belongs to a first set B of 16 genes comprising or consisting of nucleic acid sequences SEQ ID NO : 8 to 23.

Therefore, the prognosis method according to the invention can be carried out as by measuring at least the expression of the following 112 couples of genes:
SEQ ID NO: 1 + SEQ ID NO: 8, SEQ ID NO: 1 + SEQ ID NO: 9, SEQ ID NO: 1 + SEQ ID NO: 10, SEQ ID NO: 1 + SEQ ID NO: 11, SEQ ID NO: 1 + SEQ ID NO: 12, SEQ ID NO: 1 + SEQ ID NO: 13, SEQ ID NO: 1 + SEQ ID NO: 14, SEQ ID NO: 1 + SEQ ID NO: 15, SEQ ID NO: 1 + SEQ ID NO: 16, SEQ ID NO: 1 + SEQ ID NO: 17, SEQ ID NO: 1 + SEQ ID NO: 18, SEQ ID NO: 1 + SEQ ID NO: 19, SEQ ID NO: 1 + SEQ ID NO: 20, SEQ ID NO: 1 + SEQ ID NO: 21, SEQ ID NO: 1 + SEQ ID NO: 22, SEQ ID NO: 1 + SEQ ID NO: 23, SEQ ID NO: 2 + SEQ ID NO: 8, SEQ ID NO: 2 + SEQ ID NO: 9, SEQ ID NO: 2 + SEQ ID NO: 10, SEQ ID NO: 2 + SEQ ID NO: 11, SEQ ID NO: 2 + SEQ ID NO: 12, SEQ ID NO: 2 + SEQ ID NO: 13, SEQ ID NO: 2 + SEQ ID NO: 14, SEQ ID NO: 2 + SEQ ID NO: 15, SEQ ID NO: 2 + SEQ ID NO: 16, SEQ ID NO: 2 + SEQ ID NO: 17, SEQ ID NO: 2 + SEQ ID NO: 18, SEQ ID NO: 2 + SEQ ID NO: 19, SEQ ID NO: 2 + SEQ ID NO: 20, SEQ ID NO: 2 + SEQ ID NO: 21, SEQ ID NO: 2 + SEQ ID NO: 22, SEQ ID NO: 2 + SEQ ID NO: 23, SEQ ID NO: 3 + SEQ ID NO: 8, SEQ ID NO: 3 + SEQ ID NO: 9, SEQ ID NO: 3 + SEQ ID NO: 10, SEQ ID NO: 3 + SEQ ID NO: 11, SEQ ID NO: 3 + SEQ ID NO: 12, SEQ ID NO: 3 + SEQ ID NO: 13, SEQ ID NO: 3 + SEQ ID NO: 14, SEQ ID NO: 3 + SEQ ID NO: 15, SEQ ID NO: 3 + SEQ ID NO: 16, SEQ ID NO: 3 + SEQ ID NO: 17, SEQ ID NO: 3 + SEQ ID NO: 18, SEQ ID NO: 3 + SEQ ID NO: 19, SEQ ID NO: 3 + SEQ ID NO: 20, SEQ ID NO: 3 + SEQ ID NO: 21, SEQ ID NO: 3 + SEQ ID NO: 22, SEQ ID NO: 3 + SEQ ID NO: 23, SEQ ID NO: 4 + SEQ ID NO: 8, SEQ ID NO: 4 + SEQ ID NO: 9, SEQ ID NO: 4 + SEQ ID NO: 10, SEQ ID NO: 4 + SEQ ID NO: 11, SEQ ID NO: 4 + SEQ ID NO: 12, SEQ ID NO: 4 + SEQ ID NO: 13, SEQ ID NO: 4 + SEQ ID NO: 14, SEQ ID NO: 4 + SEQ ID NO: 15, SEQ ID NO: 4 + SEQ ID NO: 16, SEQ ID NO: 4 + SEQ ID NO: 17, SEQ ID NO: 4 + SEQ ID NO: 18, SEQ ID NO: 4 + SEQ ID NO: 19, SEQ ID NO: 4 + SEQ ID NO: 20, SEQ ID NO: 4 + SEQ ID NO: 21, SEQ ID NO: 4 + SEQ ID NO: 22, SEQ ID NO: 4 + SEQ ID NO: 23, SEQ ID NO: 5 + SEQ ID NO: 8, SEQ ID NO: 5 + SEQ ID NO: 9, SEQ ID NO: 5 + SEQ ID NO: 10, SEQ ID NO: 5 + SEQ ID NO: 11, SEQ ID NO: 5 + SEQ ID NO: 12, SEQ ID NO: 5 + SEQ ID NO: 13, SEQ ID NO: 5 + SEQ ID NO: 14, SEQ ID NO: 5 + SEQ ID NO: 15, SEQ ID NO: 5 + SEQ ID NO: 16, SEQ ID NO: 5 + SEQ ID NO: 17, SEQ ID NO: 5 + SEQ ID NO: 18, SEQ ID NO: 5 + SEQ ID NO: 19, SEQ ID NO: 5 + SEQ ID NO: 20, SEQ ID NO: 5 + SEQ ID NO: 21, SEQ ID NO: 5 + SEQ ID NO: 22, SEQ ID NO: 5 + SEQ ID NO: 23, SEQ ID NO: 6 + SEQ ID NO: 8, SEQ ID NO: 6 + SEQ ID NO: 9, SEQ ID NO: 6 + SEQ ID NO: 10, SEQ ID NO: 6 + SEQ ID NO: 11, SEQ ID NO: 6 + SEQ ID NO: 12, SEQ ID NO: 6 + SEQ ID NO: 13, SEQ ID NO: 6 + SEQ ID NO: 14, SEQ ID NO: 6 + SEQ ID NO: 15, SEQ ID NO: 6 + SEQ ID NO: 16, SEQ ID NO: 6 + SEQ ID NO: 17, SEQ ID NO: 6 + SEQ ID NO: 18, SEQ ID NO: 6 + SEQ ID NO: 19, SEQ ID NO: 6 + SEQ ID NO: 20, SEQ ID NO: 6 + SEQ ID NO: 21, SEQ ID NO: 6 + SEQ ID NO: 22, SEQ ID NO: 6 + SEQ ID NO: 23, SEQ ID NO: 7 + SEQ ID NO: 8, SEQ ID NO: 7 + SEQ ID NO: 9, SEQ ID NO: 7 + SEQ ID NO: 10, SEQ ID NO: 7 + SEQ ID NO: 11, SEQ ID NO: 7 + SEQ ID NO: 12, SEQ ID NO: 7 + SEQ ID NO: 13, SEQ ID NO: 7 + SEQ ID NO: 14, SEQ ID NO: 7 + SEQ ID NO: 15, SEQ ID NO: 7 + SEQ ID NO: 16, SEQ ID NO: 7 + SEQ ID NO: 17, SEQ ID NO: 7 + SEQ ID NO: 18, SEQ ID NO: 7 + SEQ ID NO: 19, SEQ ID NO: 7 + SEQ ID NO: 20, SEQ ID NO: 7 + SEQ ID NO: 21, SEQ ID NO: 7 + SEQ ID NO: 22 and SEQ ID NO: 7 + SEQ ID NO: 23.

For instance, the prognosis method according to the invention can be carried out by determining the expression status of the above couple SEQ ID NO: 1 + SEQ ID NO: 8, wherein
- if neither SEQ ID NO: 1 nor SEQ ID NO : 8 is expressed, the patient survival rate at 30 months, 60 months or 120 months following the diagnosis is from about 59% to about 78% or more, and
- if either SEQ ID NO: 1 or SEQ ID NO: 8 or both SEQ ID NO: 1+ SEQ ID NO: 8 is(are) expressed, the patient survival rate at 30 months, 60 months or 120 months following the diagnosis is from about 3% to about 70%.

According to the invention, the terms "about X%" means that the percentage of survival proposed for the prognosis method have to be considered with a standard deviation corresponding to individual variability. This standard deviation is about 5%.

By "at least 2 genes/proteins/antibodies chosen among a set of 23 genes/proteins/antibodies", it is defined in the present disclosure : 2 genes/proteins/antibodies, or 3 genes/proteins/antibodies, or 4 genes/proteins/antibodies, or 5 genes/proteins/antibodies, or 6 genes/proteins/antibodies, or 7 genes/proteins/antibodies, or 8 genes/proteins/antibodies, or 9 genes/proteins/antibodies, or 10 genes/proteins/antibodies, or 11 genes/proteins/antibodies, or 12 genes/proteins/antibodies, or 13 genes/proteins/antibodies, or 14 genes/proteins/antibodies, or 15 genes/proteins/antibodies, or 16 genes/proteins/antibodies, or 17 genes/proteins/antibodies, or 18 genes/proteins/antibodies, or 19 genes/proteins/antibodies, or 20 genes/proteins/antibodies, or 21 genes/proteins/antibodies, or 22 genes/proteins/antibodies, or 23 genes/proteins/antibodies.

By "at least 2 genes/proteins chosen among a set of 23 genes/proteins", it is defined in the invention : 2 genes/proteins, or 3 genes/proteins, or 4 genes/proteins, or 5 genes/proteins, or 6 genes/proteins, or 7 genes/proteins, or 8 genes/proteins, or 9 genes/proteins, or 10 genes/proteins, or 11 genes/proteins, or 12 genes/proteins, or 13 genes/proteins, or 14 genes/proteins, or 15 genes/proteins, or 16 genes/proteins, or 17 genes/proteins, or 18 genes/protein, or 19 genes/proteins, or 20 genes/proteins, or 21 genes/proteins, or 22 genes/proteins, or 23 genes/proteins.

The present disclosure describes the use of
- at least 2 genes chosen among a set of 23 genes comprising or consisting of the nucleic acid sequences SEQ ID NO 1 to 23,
- or fragments of said least 2 genes chosen among a set of 23 genes comprising or consisting of the nucleic acid sequences SEQ ID NO 1 to 23,
- or complementary sequences of said least 2 genes chosen among a set of 23 genes comprising or consisting of the nucleic acid sequences SEQ ID NO 1 to 23,
- or sequences having at least 80% homology with said genes or fragment thereof,
   - or proteins coded by said least 2 genes chosen among a set of 23 genes comprising or consisting of the nucleic acid sequences SEQ ID NO 1 to 23, said proteins comprising or consisting in amino acid sequences SEQ ID NO 24 to 46
   - or fragments of said proteins comprising or consisting in amino acid sequences SEQ ID NO 24 to 46,
   - or antibodies directed against said proteins comprising or consisting in amino acid sequences SEQ ID NO 24 to 46,
      said
      ◆ at least 2 genes being such that
   - at least one gene belongs to a first set A of 7 genes comprising or consisting of the nucleic acid sequences SEQ ID NO: 1-7
   - at least one gene belongs to a second set B of 16 genes comprising or consisting of the nucleic acid sequences SEQ ID NO: 8-23,
      ◆ at least 2 proteins being such that
   - at least one protein belongs to a first set AP of 7 proteins comprising or consisting of the amino acid sequences SEQ ID NO: 24-30,
   - at least one protein belongs to a second set BP of 16 proteins comprising or consisting of the amino acid sequences SEQ ID NO: 31-46,
      ◆ at least 2 antibodies directed against said 2 proteins being such that
   - at least one antibody specifically recognises one protein that belongs to a first set AP of 7 proteins comprising or consisting of the amino acid sequences SEQ ID NO: 24-30,
   - at least one antibody specifically recognises one protein that belongs to a second set BP of 16 proteins comprising or consisting of the amino acid sequences SEQ ID NO: 31-46,
   for the implementation of a prognosis method, preferably *in vitro*, of the survival rate of a patient afflicted by a lung cancer, said prognosis being such that: either
   ▪ if none of the 23 genes of said set is expressed, the patient survival rate during a period of time from 30 to 120 months after the diagnosis of said lung cancer is from about 59% to about 78% or more, and
   ▪ if at least one gene of at least one set A or B is expressed, the patient survival rate during a period of time from 30 to 120 months after the diagnosis of said lung cancer is about from about 3% to about 70%,
   or
   ▪ if none of the 23 proteins of said set is expressed, the patient survival rate during a period of time from 30 to 120 months after the diagnosis of said lung cancer is from about 59% to about 78% or more, and
   ▪ if at least one protein of at least one set AP or BP is expressed, the patient survival rate during a period of time from 30 to 120 months after the diagnosis of said lung cancer is about from about 3% to about 70%,
   or
   ▪ if none of the antibodies directed against said 23 proteins of said set is expressed, the patient survival rate during a period of time from 30 to 120 months after the diagnosis of said lung cancer is from about 59% to about 78% or more, and
   ▪ if at least one antibody directed against one protein of at least one set AP or BP is expressed, the patient survival rate during a period of time from 30 to 120 months after the diagnosis of said lung cancer is about from about 3% to about 70%,
wherein said gene, protein or antibody is determined as being expressed when:
- either it is expressed in a sample of a patient afflicted by a lung cancer but not in a control sample of an healthy individual,
- or it is expressed above a threshold corresponding to a background signal observed in a series of reference healthy tissues for each detection method, and
   said gene, protein or antibody is determined as being not expressed when:
- it is neither expressed in a sample of a patient afflicted by a lung cancer nor in a control sample of an healthy individual,
- or it is expressed in a sample of a patient afflicted by a lung cancer at a level substantially equal or inferior to the level in a control sample of an healthy individual.

To summarise, the invention relates to the use of
- at least 2 genes chosen among a set of 23 genes comprising or consisting of the nucleic acid sequences SEQ ID NO 1 to 23,
- or complementary sequences of said least 2 genes chosen among a set of 23 genes comprising or consisting of the nucleic acid sequences SEQ ID NO 1 to 23,
- or sequences having at least 80% homology with said genes,
- or proteins coded by said least 2 genes chosen among a set of 23 genes comprising or consisting of the nucleic acid sequences SEQ ID NO 1 to 23, said proteins comprising or consisting in amino acid sequences SEQ ID NO 24 to 46,
said
◆ at least 2 genes being such that
- at least one gene belongs to a first set A of 7 genes comprising or consisting of the nucleic acid sequences SEQ ID NO: 1-7
- at least one gene belongs to a second set B of 16 genes comprising or consisting of the nucleic acid sequences SEQ ID NO: 8-23,
◆ at least 2 proteins being such that
- at least one protein belongs to a first set AP of 7 proteins comprising or consisting of the amino acid sequences SEQ ID NO: 24-30,
- at least one protein belongs to a second set BP of 16 proteins comprising or consisting of the amino acid sequences SEQ ID NO: 31-46,
for the implementation of an *in vitro* prognosis method of the survival rate of a patient afflicted by a lung cancer, said method comprising a step of measuring, in a lung cancer sample of said patient, the expression of said at least 2 genes, or said complementary sequences, or said sequences having at least 80% homology with said genes, or said at least 2 proteins, and said prognosis being such that: either
▪ if none of the 23 genes of said set is expressed, the patient survival rate during a period of time from 30 to 120 months after the diagnosis of said lung cancer is from about 59% to about 78% or more, and
▪ if at least one gene of at least one set A or B is expressed, the patient survival rate during a period of time from 30 to 120 months after the diagnosis of said lung cancer is about from about 3% to about 70%,
or
▪ if none of the 23 proteins of said set is expressed, the patient survival rate during a period of time from 30 to 120 months after the diagnosis of said lung cancer is from about 59% to about 78% or more, and
▪ if at least one protein of at least one set AP or BP is expressed, the patient survival rate during a period of time from 30 to 120 months after the diagnosis of said lung cancer is about from about 3% to about 70%,
wherein said gene or protein is determined as being expressed when:
- either it is expressed in a sample of a patient afflicted by a lung cancer but not in a control sample of an healthy individual,
- or it is expressed above a threshold corresponding to a background signal observed in a series of reference healthy tissues for each detection method, and
   said gene or protein is determined as being not expressed when:
- it is neither expressed in a sample of a patient afflicted by a lung cancer nor in a control sample of an healthy individual,
- or it is expressed in a sample of a patient afflicted by a lung cancer at a level substantially equal or inferior to the level in a control sample of an healthy individual.

According to the invention, "a control sample of an healthy individual" corresponds to a somatic tissue in which the CT gene is not expressed or weakly expressed as defined above, said somatic tissue originating from a person not afflicted by cancer.

In the invention "is expressed at a level above a threshold corresponding to a background signal observed in a series of reference healthy tissues for each detection method" means that the threshold, which corresponds to the key step of the invention, is determined by measuring the background signal in negative control samples of healthy tissues, in which there is no expression of CT genes.

This background signal depends upon the method used to carry out the invention. However, it is easy for a skilled person to measure such threshold whatever the method used, i.e.:
- if the number of control samples is significantly statistically representative, e.g. at least 30 independent control samples, and the background signal of these control sample follows a normal distribution (Gaussian distribution), the threshold is determined by the mean + 2 standard deviations of the background signal measured in the control samples,
- If the number of control sample is not statistically representative, e.g. less than 30 independent control samples, or if the background signal of these control sample does not follow a normal distribution, the threshold is determined as being the maximal value of the background signal measured in the control samples.

According to the invention, the gene comprising or consisting of the nucleic acid sequence SEQ ID NO : 1 codes for the protein comprising or consisting of the amino acid sequence SEQ ID NO : 24, the gene comprising or consisting of the nucleic acid sequence SEQ ID NO : 2 codes for the protein comprising or consisting of the amino acid sequence SEQ ID NO : 25, the gene comprising or consisting of the nucleic acid sequence SEQ ID NO : 3 codes for the protein comprising or consisting of the amino acid sequence SEQ ID NO : 26, the gene comprising or consisting of the nucleic acid sequence SEQ ID NO : 4 codes for the protein comprising or consisting of the amino acid sequence SEQ ID NO : 27, the gene comprising or consisting of the nucleic acid sequence SEQ ID NO : 5 codes for the protein comprising or consisting of the amino acid sequence SEQ ID NO : 28, the gene comprising or consisting of the nucleic acid sequence SEQ ID NO : 6 codes for the protein comprising or consisting of the amino acid sequence SEQ ID NO : 29, the gene comprising or consisting of the nucleic acid sequence SEQ ID NO : 7 codes for the protein comprising or consisting of the amino acid sequence SEQ ID NO : 30, the gene comprising or consisting of the nucleic acid sequence SEQ ID NO : 8 codes for the protein comprising or consisting of the amino acid sequence SEQ ID NO : 31, the gene comprising or consisting of the nucleic acid sequence SEQ ID NO : 9 codes for the protein comprising or consisting of the amino acid sequence SEQ ID NO : 32, the gene comprising or consisting of the nucleic acid sequence SEQ ID NO : 10 codes for the protein comprising or consisting of the amino acid sequence SEQ ID NO : 33, the gene comprising or consisting of the nucleic acid sequence SEQ ID NO : 11 codes for the protein comprising or consisting of the amino acid sequence SEQ ID NO : 34, the gene comprising or consisting of the nucleic acid sequence SEQ ID NO : 12 codes for the protein comprising or consisting of the amino acid sequence SEQ ID NO : 35, the gene comprising or consisting of the nucleic acid sequence SEQ ID NO : 13 codes for the protein comprising or consisting of the amino acid sequence SEQ ID NO : 36, the gene comprising or consisting of the nucleic acid sequence SEQ ID NO : 14 codes for the protein comprising or consisting of the amino acid sequence SEQ ID NO : 37, the gene comprising or consisting of the nucleic acid sequence SEQ ID NO : 15 codes for the protein comprising or consisting of the amino acid sequence SEQ ID NO : 38, the gene comprising or consisting of the nucleic acid sequence SEQ ID NO : 16 codes for the protein comprising or consisting of the amino acid sequence SEQ ID NO : 39, the gene comprising or consisting of the nucleic acid sequence SEQ ID NO : 17 codes for the protein comprising or consisting of the amino acid sequence SEQ ID NO : 40, the gene comprising or consisting of the nucleic acid sequence SEQ ID NO : 18 codes for the protein comprising or consisting of the amino acid sequence SEQ ID NO : 41, the gene comprising or consisting of the nucleic acid sequence SEQ ID NO : 19 codes for the protein comprising or consisting of the amino acid sequence SEQ ID NO : 42, the gene comprising or consisting of the nucleic acid sequence SEQ ID NO : 20 codes for the protein comprising or consisting of the amino acid sequence SEQ ID NO : 43, the gene comprising or consisting of the nucleic acid sequence SEQ ID NO : 21 codes for the protein comprising or consisting of the amino acid sequence SEQ ID NO : 44, the gene comprising or consisting of the nucleic acid sequence SEQ ID NO : 22 codes for the protein comprising or consisting of the amino acid sequence SEQ ID NO : 45 and the gene comprising or consisting of the nucleic acid sequence SEQ ID NO : 23 codes for the protein comprising or consisting of the amino acid sequence SEQ ID NO : 46.

The 23 genes according to the invention code for 23 proteins. The 23 proteins have been classified by the Inventors in two sets:
- a first set of 7 proteins,
- and a second set of 16 proteins.

The first set, also called in the invention set AP, of 7 proteins consists of the proteins comprising or constituted by the amino acid sequences SEQ ID NO: 24, SEQ ID NO: 25, SEQ ID NO: 26, SEQ ID NO: 27, SEQ ID NO: 28, SEQ ID NO: 29 and SEQ ID NO: 30.

The second set, also called in the invention set BP, of 16 proteins consists of the proteins comprising or constituted by the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, and SEQ ID NO: 46.

The inventors have also defined subsets of each of the above sets AP and BP as follows:
Set AP is divided into 4 subsets AP1, AP5 A6 and AP7, said subset being such that:
   - subset AP1 consists of the proteins comprising or being constituted by the amino acid sequences SEQ ID NO: 24 and SEQ ID NO: 25,
   - subset AP5 consists of the protein comprising or being constituted by the amino acid sequence SEQ ID NO: 26,
   - subset AP6 consists of the proteins comprising or being constituted by the amino acid sequences SEQ ID NO: 27 and SEQ ID NO: 28, and
   - subset AP7 consists of the proteins comprising or being constituted by the amino acid sequences SEQ ID NO: 29 and SEQ ID NO: 30.

Set AP can also be divided into the following subsets :
- subset AP1 consists of the proteins comprising or being constituted by the amino acid sequences SEQ ID NO: 24 and SEQ ID NO: 25,
- subset AP2 consists of the proteins comprising or being constituted by the amino acid sequences SEQ ID NO: 24, SEQ ID NO: 25 and SEQ ID NO: 26,
- subset AP3 consists of the proteins comprising or being constituted by the amino acid sequences SEQ ID NO: 24, SEQ ID NO: 25, SEQ ID NO: 26, SEQ ID NO: 27 and SEQ ID NO: 28, and
- subset AP4 consists of the proteins comprising or being constituted by the amino acid sequences SEQ ID NO: 24, SEQ ID NO: 25, SEQ ID NO: 26, SEQ ID NO: 27, SEQ ID NO: 28, SEQ ID NO: 29 and SEQ ID NO: 30.

Set BP is divided into 3 subsets BP1, BP4 and BP5, said subset being such that:
- subset BP1 consists of the proteins comprising or being constituted by the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37 and SEQ ID NO: 38,
- subset BP4 consists of the proteins comprising or being constituted by the amino acid sequences SEQ ID NO: 39, SEQ ID NO: 40, SEQ ID NO: 41, SEQ ID NO: 42, SEQ ID NO: 43 and SEQ ID NO: 44, and
- subset BP5 consists of the proteins comprising or being constituted by the amino acid sequences SEQ ID NO: 45, and SEQ ID NO: 46.

Set BP can also be divided into the following subsets :
- subset BP1 consists of the proteins comprising or being constituted by the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37 and SEQ ID NO: 38,
- subset BP2 consists of the proteins comprising or being constituted by the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43 and SEQ ID NO: 44, and
- subset BP3 consists of the proteins comprising or being constituted by the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, and SEQ ID NO: 46.

The present disclosure describes the use of
- at least 2 genes chosen among a set of 23 genes comprising or consisting of the nucleic acid sequences SEQ ID NO 1 to 23,
- or fragments of said genes,
- or complementary sequences of said genes,
- or sequences having at least 80% homology with said genes or fragment thereof,
- or proteins coded by said genes,
- or fragments of said proteins,
- or antibodies directed against said proteins,
   said at least 2 genes being such that
- at least one gene belongs to a subset A1 of a first set A of 7 genes comprising or consisting of the nucleic acid sequences SEQ ID NO: 1-7, said subset A1 comprising or consisting of nucleic acid sequences SEQ ID NO : 1 or 2
- at least one gene belongs to a subset B1 of a second set B of 16 genes comprising or consisting of the nucleic acid sequences SEQ ID NO: 8-23, said subset B1 comprising or consisting of nucleic acid sequences SEQ ID NO: 8 to 15
for the implementation of a prognosis method, preferably *in vitro*, of the survival rate of a patient afflicted by a lung cancer, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A1 or B1 is expressed, the patient survival rate during a period of time from 30 to 120 months after the diagnosis of said lung cancer is about from about 3% to about 70%.

In one advantageous embodiment, the invention relates to the use of
- at least 2 genes chosen among a set of 23 genes comprising or consisting of the nucleic acid sequences SEQ ID NO 1 to 23,
- or complementary sequences of said genes,
- or sequences having at least 80% homology with said genes,
- or proteins coded by said genes,
   said at least 2 genes being such that
- at least one gene belongs to a subset A1 of a first set A of 7 genes comprising or consisting of the nucleic acid sequences SEQ ID NO: 1-7, said subset A1 comprising or consisting of nucleic acid sequences SEQ ID NO : 1 or 2
- at least one gene belongs to a subset B 1 of a second set B of 16 genes comprising or consisting of the nucleic acid sequences SEQ ID NO: 8-23, said subset B1 comprising or consisting of nucleic acid sequences SEQ ID NO: 8 to 15
for the implementation of an *in vitro* prognosis method of the survival rate of a patient afflicted by a lung cancer, said method comprising a step of measuring, in a lung cancer sample of said patient, the expression of said at least 2 genes, or said complementary sequences, or said sequences having at least 80% homology with said genes, or said at least 2 proteins, and said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A1 or B1 is expressed, the patient survival rate during a period of time from 30 to 120 months after the diagnosis of said lung cancer is about from about 3% to about 70%.

In this embodiment of the invention, the prognosis method is carried out by using at least one of the following couples of genes:
SEQ ID NO: 1 + SEQ ID NO: 8, SEQ ID NO: 1 + SEQ ID NO: 9, SEQ ID NO: 1 + SEQ ID NO: 10, SEQ ID NO: 1 + SEQ ID NO: 11, SEQ ID NO: 1 + SEQ ID NO: 12, SEQ ID NO: 1 + SEQ ID NO: 13, SEQ ID NO: 1 + SEQ ID NO: 14, SEQ ID NO: 1 + SEQ ID NO: 15, SEQ ID NO: 2 + SEQ ID NO: 8, SEQ ID NO: 2 + SEQ ID NO: 9, SEQ ID NO: 2 + SEQ ID NO: 10, SEQ ID NO: 2 + SEQ ID NO: 11, SEQ ID NO: 2 + SEQ ID NO: 12, SEQ ID NO: 2 + SEQ ID NO: 13, SEQ ID NO: 2 + SEQ ID NO: 14 and SEQ ID NO: 2 + SEQ ID NO: 15.

The above 16 couples are sufficient to define a significant prognosis over 120 month of lung cancer.

Any other supplementary genes belonging to the group of 23 genes according to the invention can be used in order to affine the prognosis according to the invention. The present disclosure describes the use of
- at least 2 genes chosen among a set of 23 genes comprising or consisting of the nucleic acid sequences SEQ ID NO 1 to 23,
- or fragments of said genes,
- or complementary sequences of said genes,
- or sequences having at least 80% homology with said genes or fragment thereof,
- or proteins coded by said genes,
- or fragments of said proteins,
- or antibodies directed against said proteins,
   said at least 2 genes being such that
- at least one gene belongs to a subset A2 of a first set A of 7 genes comprising or consisting of the nucleic acid sequences SEQ ID NO: 1-7, said subset A2 comprising or consisting of nucleic acid sequences SEQ ID NO : 1 to 3
- at least one gene belongs to a subset B2 of a second set B of 16 genes comprising or consisting of the nucleic acid sequences SEQ ID NO: 8-23, said subset B2 comprising or consisting of nucleic acid sequences SEQ ID NO : 8 to 21
for the implementation of a prognosis method, preferably *in vitro*, of the survival rate of a patient afflicted by a lung cancer, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A2 or B2 is expressed, the patient survival rate during a period of time from 30 to 120 months after the diagnosis of said lung cancer is about from about 3% to about 70%.

In one advantageous embodiment, the invention relates to the use of
- at least 2 genes chosen among a set of 23 genes comprising or consisting of the nucleic acid sequences SEQ ID NO 1 to 23,
- or complementary sequences of said genes,
- or sequences having at least 80% homology with said genes,
- or proteins coded by said genes,
said at least 2 genes being such that
- at least one gene belongs to a subset A2 of a first set A of 7 genes comprising or consisting of the nucleic acid sequences SEQ ID NO: 1-7, said subset A2 comprising or consisting of nucleic acid sequences SEQ ID NO : 1 to 3
- at least one gene belongs to a subset B2 of a second set B of 16 genes comprising or consisting of the nucleic acid sequences SEQ ID NO: 8-23, said subset B2 comprising or consisting of nucleic acid sequences SEQ ID NO: 8 to 21
for the implementation of a prognosis method, preferably *in vitro*, of the survival rate of a patient afflicted by a lung cancer, said method comprising a step of measuring, in a lung cancer sample of said patient, the expression of said at least 2 genes, or said complementary sequences, or said sequences having at least 80% homology with said genes, or said at least 2 proteins, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A2 or B2 is expressed, the patient survival rate during a period of time from 30 to 120 months after the diagnosis of said lung cancer is about from about 3% to about 70%.

In this embodiment of the invention, the prognosis method is carried out by using at least one of the following couples of genes:
SEQ ID NO: 1 + SEQ ID NO: 8, SEQ ID NO: 1 + SEQ ID NO: 9, SEQ ID NO: 1 + SEQ ID NO: 10, SEQ ID NO: 1 + SEQ ID NO: 11, SEQ ID NO: 1 + SEQ ID NO: 12, SEQ ID NO: 1 + SEQ ID NO: 13, SEQ ID NO: 1 + SEQ ID NO: 14, SEQ ID NO: 1 + SEQ ID NO: 15, SEQ ID NO: 1 + SEQ ID NO: 16, SEQ ID NO: 1 + SEQ ID NO: 17, SEQ ID NO: 1 + SEQ ID NO: 18, SEQ ID NO: 1 + SEQ ID NO: 19, SEQ ID NO: 1 + SEQ ID NO: 20, SEQ ID NO: 1 + SEQ ID NO: 21, SEQ ID NO: 2 + SEQ ID NO: 8, SEQ ID NO: 2 + SEQ ID NO: 9, SEQ ID NO: 2 + SEQ ID NO: 10, SEQ ID NO: 2 + SEQ ID NO: 11, SEQ ID NO: 2 + SEQ ID NO: 12, SEQ ID NO: 2 + SEQ ID NO: 13, SEQ ID NO: 2 + SEQ ID NO: 14, SEQ ID NO: 2 + SEQ ID NO: 15, SEQ ID NO: 2 + SEQ ID NO: 16, SEQ ID NO: 2 + SEQ ID NO: 17, SEQ ID NO: 2 + SEQ ID NO: 18, SEQ ID NO: 2 + SEQ ID NO: 19, SEQ ID NO: 2 + SEQ ID NO: 20, SEQ ID NO: 2 + SEQ ID NO: 21, SEQ ID NO: 3 + SEQ ID NO: 8, SEQ ID NO: 3 + SEQ ID NO: 9, SEQ ID NO: 3 + SEQ ID NO: 10, SEQ ID NO: 3 + SEQ ID NO: 11, SEQ ID NO: 3 + SEQ ID NO: 12, SEQ ID NO: 3 + SEQ ID NO: 13, SEQ ID NO: 3 + SEQ ID NO: 14, SEQ ID NO: 3 + SEQ ID NO: 15, SEQ ID NO: 3 + SEQ ID NO: 16, SEQ ID NO: 3 + SEQ ID NO: 17, SEQ ID NO: 3 + SEQ ID NO: 18, SEQ ID NO: 3 + SEQ ID NO: 19 and SEQ ID NO: 3 + SEQ ID NO: 20.

The present disclosure describes the use of
- at least 2 genes chosen among a set of 23 genes comprising or consisting of the nucleic acid sequences SEQ ID NO 1 to 23,
- or fragments of said genes,
- or complementary sequences of said genes,
- or sequences having at least 80% homology with said genes or fragment thereof,
- or proteins coded by said genes,
- or fragments of said proteins,
- or antibodies directed against said proteins,
   said at least 2 genes being such that
- at least one gene belongs to a subset A3 of a first set A of 7 genes comprising or consisting of the nucleic acid sequences SEQ ID NO: 1-7, said subset A3 comprising or consisting of nucleic acid sequences SEQ ID NO : 1 to 5
- at least one gene belongs to a subset B2 of a second set B of 16 genes comprising or consisting of the nucleic acid sequences SEQ ID NO: 8-23, said subset B2 comprising or consisting of nucleic acid sequences SEQ ID NO: 8 to 21
for the implementation of a prognosis method, preferably *in vitro*, of the survival rate of a patient afflicted by a lung cancer, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A3 or B2 is expressed, the patient survival rate during a period of time from 30 to 120 months after the diagnosis of said lung cancer is about from about 3% to about 70%.

In one advantageous embodiment, the invention relates to the use of
- at least 2 genes chosen among a set of 23 genes comprising or consisting of the nucleic acid sequences SEQ ID NO 1 to 23,
- or complementary sequences of said genes,
- or sequences having at least 80% homology with said genes,
- or proteins coded by said genes,
   said at least 2 genes being such that
- at least one gene belongs to a subset A3 of a first set A of 7 genes comprising or consisting of the nucleic acid sequences SEQ ID NO: 1-7, said subset A3 comprising or consisting of nucleic acid sequences SEQ ID NO : 1 to 5
- at least one gene belongs to a subset B2 of a second set B of 16 genes comprising or consisting of the nucleic acid sequences SEQ ID NO: 8-23, said subset B2 comprising or consisting of nucleic acid sequences SEQ ID NO : 8 to 21
for the implementation of an *in vitro* prognosis method of the survival rate of a patient afflicted by a lung cancer, said method comprising a step of measuring, in a lung cancer sample of said patient, the expression of said at least 2 genes, or said complementary sequences, or said sequences having at least 80% homology with said genes, or said at least 2 proteins, and said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A3 or B2 is expressed, the patient survival rate during a period of time from 30 to 120 months after the diagnosis of said lung cancer is about from about 3% to about 70%.

In this embodiment of the invention, the prognosis method is carried out by using at least one of the following couples of genes:
SEQ ID NO: 1 + SEQ ID NO: 8, SEQ ID NO: 1 + SEQ ID NO: 9, SEQ ID NO: 1 + SEQ ID NO: 10, SEQ ID NO: 1 + SEQ ID NO: 11, SEQ ID NO: 1 + SEQ ID NO: 12, SEQ ID NO: 1 + SEQ ID NO: 13, SEQ ID NO: 1 + SEQ ID NO: 14, SEQ ID NO: 1 + SEQ ID NO: 15, SEQ ID NO: 1 + SEQ ID NO: 16, SEQ ID NO: 1 + SEQ ID NO: 17, SEQ ID NO: 1 + SEQ ID NO: 18, SEQ ID NO: 1 + SEQ ID NO: 19, SEQ ID NO: 1 + SEQ ID NO: 20, SEQ ID NO: 1 + SEQ ID NO: 21, SEQ ID NO: 1 + SEQ ID NO: 22, SEQ ID NO: 1 + SEQ ID NO: 23, SEQ ID NO: 2 + SEQ ID NO: 8, SEQ ID NO: 2 + SEQ ID NO: 9, SEQ ID NO: 2 + SEQ ID NO: 10, SEQ ID NO: 2 + SEQ ID NO: 11, SEQ ID NO: 2 + SEQ ID NO: 12, SEQ ID NO: 2 + SEQ ID NO: 13, SEQ ID NO: 2 + SEQ ID NO: 14, SEQ ID NO: 2 + SEQ ID NO: 15, SEQ ID NO: 2 + SEQ ID NO: 16, SEQ ID NO: 2 + SEQ ID NO: 17, SEQ ID NO: 2 + SEQ ID NO: 18, SEQ ID NO: 2 + SEQ ID NO: 19, SEQ ID NO: 2 + SEQ ID NO: 20, SEQ ID NO: 2 + SEQ ID NO: 21, SEQ ID NO: 2 + SEQ ID NO: 22, SEQ ID NO: 2 + SEQ ID NO: 23, SEQ ID NO: 3 + SEQ ID NO: 8, SEQ ID NO: 3 + SEQ ID NO: 9, SEQ ID NO: 3 + SEQ ID NO: 10, SEQ ID NO: 3 + SEQ ID NO: 11, SEQ ID NO: 3 + SEQ ID NO: 12, SEQ ID NO: 3 + SEQ ID NO: 13, SEQ ID NO: 3 + SEQ ID NO: 14, SEQ ID NO: 3 + SEQ ID NO: 15, SEQ ID NO: 3 + SEQ ID NO: 16, SEQ ID NO: 3 + SEQ ID NO: 17, SEQ ID NO: 3 + SEQ ID NO: 18, SEQ ID NO: 3 + SEQ ID NO: 19, SEQ ID NO: 3 + SEQ ID NO: 20, SEQ ID NO: 3 + SEQ ID NO: 21, SEQ ID NO: 3 + SEQ ID NO: 22 and SEQ ID NO: 3 + SEQ ID NO: 23.

The present disclosure describes the use of
- at least 2 proteins chosen among a set of 23 proteins coded by said 23 genes, said 23 proteins comprising or consisting of SEQ ID NO : 24 to 46,
- or fragments of said proteins,
- or antibodies directed against said proteins,
   said at least 2 proteins being such that
- at least one protein belongs to a subset AP1 of a first set AP of 7 proteins comprising or consisting of the amino acid sequences SEQ ID NO: 24-30, said subset AP1 comprising or consisting of amino acid sequences SEQ ID NO : 24 or 25
- at least one protein belongs to a subset BP1 of a second set BP of 16 proteins comprising or consisting of the amino acid sequences SEQ ID NO: 31-46, said subset BP1 comprising or consisting of nucleic acid sequences SEQ ID NO : 31 to 38
for the implementation of a prognosis method, preferably *in vitro*, of the survival rate of a patient afflicted by a lung cancer, said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP1 or BP1 is expressed, the patient survival rate during a period of time from 30 to 120 months after the diagnosis of said lung cancer is about from about 3% to about 70%.

In one advantageous embodiment, the invention relates to the use of at least 2 proteins chosen among a set of 23 proteins coded by said 23 genes, said 23 proteins comprising or consisting of SEQ ID NO : 24 to 46, said at least 2 proteins being such that
- at least one protein belongs to a subset AP1 of a first set AP of 7 proteins comprising or consisting of the amino acid sequences SEQ ID NO: 24-30, said subset AP1 comprising or consisting of amino acid sequences SEQ ID NO : 24 or 25
- at least one protein belongs to a subset BP1 of a second set BP of 16 proteins comprising or consisting of the amino acid sequences SEQ ID NO: 31-46, said subset BP1 comprising or consisting of nucleic acid sequences SEQ ID NO : 31 to 38
for the implementation of an *in vitro* prognosis method of the survival rate of a patient afflicted by a lung cancer, said method comprising a step of measuring, in a lung cancer sample of said patient, the expression of said at least 2 proteins, and said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP1 or BP1 is expressed, the patient survival rate during a period of time from 30 to 120 months after the diagnosis of said lung cancer is about from about 3% to about 70%.

In this embodiment of the invention, the prognosis method is carried out by using at least one of the following couples of proteins:
SEQ ID NO: 24 + SEQ ID NO: 31, SEQ ID NO: 24 + SEQ ID NO: 32, SEQ ID NO: 24 + SEQ ID NO: 33, SEQ ID NO: 24 + SEQ ID NO: 34, SEQ ID NO: 24 + SEQ ID NO: 35, SEQ ID NO: 24 + SEQ ID NO: 36, SEQ ID NO: 24 + SEQ ID NO: 37, SEQ ID NO: 24 + SEQ ID NO: 38, SEQ ID NO: 25 + SEQ ID NO: 31, SEQ ID NO: 25 + SEQ ID NO: 32, SEQ ID NO: 25 + SEQ ID NO: 33, SEQ ID NO: 25 + SEQ ID NO: 34, SEQ ID NO: 25 + SEQ ID NO: 35, SEQ ID NO: 25 + SEQ ID NO: 36, SEQ ID NO: 25 + SEQ ID NO: 37 and SEQ ID NO: 25 + SEQ ID NO: 38.

The above 16 couples are sufficient to defined a significant prognosis over 120 month of lung cancer.

The present disclosure describes the use of
- or at least 2 proteins chosen among a set of 23 proteins coded by said 23 genes, said 23 proteins comprising or consisting of SEQ ID NO : 24 to 46,
- or fragments of said proteins,
- or antibodies directed against said proteins,
   said at least 2 proteins being such that
- at least one protein belongs to a subset AP2 of a first set AP of 7 proteins comprising or consisting of the amino acid sequences SEQ ID NO: 24-30, said subset AP2 comprising or consisting of amino acid sequences SEQ ID NO : 24 to 26
- at least one protein belongs to a subset BP2 of a second set BP of 16 proteins comprising or consisting of the amino acid sequences SEQ ID NO: 31-46, said subset BP2 comprising or consisting of nucleic acid sequences SEQ ID NO : 31 to 44
for the implementation of a prognosis method, preferably *in vitro*, of the survival rate of a patient afflicted by a lung cancer, said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP2 or BP2 is expressed, the patient survival rate during a period of time from 30 to 120 months after the diagnosis of said lung cancer is about from about 3% to about 70%.

In one advantageous embodiment, the invention relates to the use of
at least 2 proteins chosen among a set of 23 proteins coded by said 23 genes, said 23 proteins comprising or consisting of SEQ ID NO : 24 to 46, said at least 2 proteins being such that
- at least one protein belongs to a subset AP2 of a first set AP of 7 proteins comprising or consisting of the amino acid sequences SEQ ID NO: 24-30, said subset AP2 comprising or consisting of amino acid sequences SEQ ID NO : 24 to 26
- at least one protein belongs to a subset BP2 of a second set BP of 16 proteins comprising or consisting of the amino acid sequences SEQ ID NO: 31-46, said subset BP2 comprising or consisting of nucleic acid sequences SEQ ID NO : 31 to 44
for the implementation of an *in vitro* prognosis method of the survival rate of a patient afflicted by a lung cancer, said method comprising a step of measuring, in a lung cancer sample of said patient, the expression of said at least 2 proteins, and said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP2 or BP2 is expressed, the patient survival rate during a period of time from 30 to 120 months after the diagnosis of said lung cancer is about from about 3% to about 70%.

The present disclosure describes the use of
- or at least 2 proteins chosen among a set of 23 proteins coded by said 23 genes, said 23 proteins comprising or consisting of SEQ ID NO : 24 to 46,
- or fragments of said proteins,
- or antibodies directed against said proteins,
   said at least 2 proteins being such that
- at least one protein belongs to a subset AP3 of a first set AP of 7 proteins comprising or consisting of the amino acid sequences SEQ ID NO: 24-30, said subset AP3 comprising or consisting of amino acid sequences SEQ ID NO : 24 to 28
- at least one protein belongs to a subset BP2 of a second set BP of 16 proteins comprising or consisting of the amino acid sequences SEQ ID NO: 31-46, said subset BP2 comprising or consisting of nucleic acid sequences SEQ ID NO : 31 to 44
for the implementation of a prognosis method, preferably *in vitro*, of the survival rate of a patient afflicted by a lung cancer, said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP3 or BP2 is expressed, the patient survival rate during a period of time from 30 to 120 months after the diagnosis of said lung cancer is about from about 3% to about 70%.

In one advantageous embodiment, the invention relates to the use of
at least 2 proteins chosen among a set of 23 proteins coded by said 23 genes, said 23 proteins comprising or consisting of SEQ ID NO : 24 to 46, said at least 2 proteins being such that
- at least one protein belongs to a subset AP3 of a first set AP of 7 proteins comprising or consisting of the amino acid sequences SEQ ID NO: 24-30, said subset AP3 comprising or consisting of amino acid sequences SEQ ID NO : 24 to 28
- at least one protein belongs to a subset BP2 of a second set BP of 16 proteins comprising or consisting of the amino acid sequences SEQ ID NO: 31-46, said subset BP2 comprising or consisting of nucleic acid sequences SEQ ID NO : 31 to 44
for the implementation of an *in vitro* prognosis method of the survival rate of a patient afflicted by a lung cancer, said method comprising a step of measuring, in a lung cancer sample of said patient, and said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP3 or BP2 is expressed, the patient survival rate during a period of time from 30 to 120 months after the diagnosis of said lung cancer is about from about 3% to about 70%.

The present disclosure describes the use of
- at least 2 genes chosen among a set of 23 genes comprising or consisting of the nucleic acid sequences SEQ ID NO 1 to 23,
- or fragments of said genes,
- or complementary sequences of said genes,
- or sequences having at least 80% homology with said genes or fragment thereof,
- or proteins coded by said genes,
- or fragments of said proteins,
- or antibodies directed against said proteins,
   said at least 2 genes being such that
- at least one gene belongs to a subset A5 of a first set A of 7 genes comprising or consisting of the nucleic acid sequences SEQ ID NO: 1-7, said subset A5 comprising or consisting of nucleic acid sequences SEQ ID NO : 3,
- at least one gene belongs to a subset B4 of a second set B of 16 genes comprising or consisting of the nucleic acid sequences SEQ ID NO: 8-23, said subset B4comprising or consisting of nucleic acid sequences SEQ ID NO : 16 to 21,
for the implementation of a prognosis method, preferably *in vitro*, of the survival rate of a patient afflicted by a lung cancer, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A5 or B4 is expressed, the patient survival rate during a period of time from 30 to 120 months after the diagnosis of said lung cancer is about from about 3% to about 70%.

In one advantageous embodiment, the invention relates to the use of
- at least 2 genes chosen among a set of 23 genes comprising or consisting of the nucleic acid sequences SEQ ID NO 1 to 23,
- or complementary sequences of said genes,
- or sequences having at least 80% homology with said genes,
- or proteins coded by said genes,
   said at least 2 genes being such that
- at least one gene belongs to a subset A5 of a first set A of 7 genes comprising or consisting of the nucleic acid sequences SEQ ID NO: 1-7, said subset A5 comprising or consisting of nucleic acid sequences SEQ ID NO : 3,
- at least one gene belongs to a subset B4 of a second set B of 16 genes comprising or consisting of the nucleic acid sequences SEQ ID NO: 8-23, said subset B4 comprising or consisting of nucleic acid sequences SEQ ID NO: 16 to 21,
for the implementation of an *in vitro* prognosis method of the survival rate of a patient afflicted by a lung cancer, said method comprising a step of measuring, in a lung cancer sample of said patient, the expression of said at least 2 genes, or said complementary sequences, or said sequences having at least 80% homology with said genes, or said at least 2 proteins, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A5 or B4 is expressed, the patient survival rate during a period of time from 30 to 120 months after the diagnosis of said lung cancer is about from about 3% to about 70%.

The present disclosure describes the use of
- at least 2 proteins chosen among a set of 23 proteins coded by said 23 genes, said 23 proteins comprising or consisting of SEQ ID NO : 24 to 46,
- or fragments of said proteins,
- or antibodies directed against said proteins,
   said at least 2 proteins being such that
- at least one protein belongs to a subset AP5 of a first set AP of 7 proteins comprising or consisting of the amino acid sequences SEQ ID NO: 24-30, said subset AP5 comprising or consisting of amino acid sequences SEQ ID NO : 26
- at least one protein belongs to a subset BP4 of a second set BP of 16 proteins comprising or consisting of the amino acid sequences SEQ ID NO: 31-46, said subset BP4 comprising or consisting of nucleic acid sequences SEQ ID NO : 39 to 44
for the implementation of a prognosis method, preferably *in vitro*, of the survival rate of a patient afflicted by a lung cancer, said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP5 or BP4 is expressed, the patient survival rate during a period of time from 30 to 120 months after the diagnosis of said lung cancer is about from about 3% to about 70%.

In one advantageous embodiment, the invention relates to the use of
at least 2 proteins chosen among a set of 23 proteins coded by said 23 genes, said 23 proteins comprising or consisting of SEQ ID NO : 24 to 46, said at least 2 proteins being such that
- at least one protein belongs to a subset AP5 of a first set AP of 7 proteins comprising or consisting of the amino acid sequences SEQ ID NO: 24-30, said subset AP5 comprising or consisting of amino acid sequences SEQ ID NO : 26
- at least one protein belongs to a subset BP4 of a second set BP of 16 proteins comprising or consisting of the amino acid sequences SEQ ID NO: 31-46, said subset BP4 comprising or consisting of nucleic acid sequences SEQ ID NO : 39 to 44
for the implementation of an *in vitro* prognosis method of the survival rate of a patient afflicted by a lung cancer, said method comprising a step of measuring, in a lung cancer sample of said patient, the expression of said at least 2 proteins, and said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP5 or BP4 is expressed, the patient survival rate during a period of time from 30 to 120 months after the diagnosis of said lung cancer is about from about 3% to about 70%.

The present disclosure also describes the use as mentioned above, wherein said prognosis method is such that:
▪ if none of the 23 genes, or proteins, or antibodies, of said set is expressed, the patient survival rate during a period of time from 30 to 120 months after the diagnosis of said lung cancer is from about 59% to about 78% or more,
▪ if none of the genes or proteins of the set B or BP, or of the subsets B 1, BP1, B2, or BP2, or antibodies, is expressed and at least one gene or protein, or antibody, of the set A or AP, or of the subset A1, AP1, A2, AP2, A3 or AP3 is expressed, the patient survival rate during a period of time from 30 to 120 months after the diagnosis of said lung cancer is from about 27% to about 70%, and
▪ if at least one gene, or protein, of the set B or BP, or of the subsets B 1, BP1, B2 or BP2 or antibody, is expressed, the patient survival rate during a period of time from 30 to 120 months after the diagnosis of said lung cancer is from about 3% to about 55%.

In one other advantageous embodiment, the invention relates to the use as mentioned above, wherein said prognosis method is such that:
▪ if none of the 23 genes, or proteins, of said set is expressed, the patient survival rate during a period of time from 30 to 120 months after the diagnosis of said lung cancer is from about 59% to about 78% or more,
▪ if none of the genes or proteins of the set B or BP, or of the subsets B1, BP1, B2, or BP2, is expressed and at least one gene or protein, of the set A or AP, or of the subset A1, AP1, A2, AP2, A3 or AP3 is expressed, the patient survival rate during a period of time from 30 to 120 months after the diagnosis of said lung cancer is from about 27% to about 70%, and
▪ if at least one gene, or protein, of the set B or BP, or of the subsets B1, BP1, B2 or BP2, is expressed, the patient survival rate during a period of time from 30 to 120 months after the diagnosis of said lung cancer is from about 3% to about 55%.

In one other advantageous embodiment, the invention relates to the use as mentioned above, wherein said prognosis method is such that:
▪ if none of the 23 genes, of said set is expressed, the patient survival rate during a period of time from 30 to 120 months after the diagnosis of said lung cancer is from about 59% to about 78% or more,
▪ if none of the genes of the set B, or of the subsets B1 or B2 is expressed and at least one gene of the set A or of the subset A1, A2, or A3 is expressed, the survival rate during a period of time from 30 to 120 months after the diagnosis of said lung cancer is from about 27% to about 70%, and
▪ if at least one gene of the set B or of the subsets B1, or B2 is expressed, survival rate during a period of time from 30 to 120 months after the diagnosis of said lung cancer is from about 3% to about 55%.

In one other advantageous embodiment, the invention relates to the use as mentioned above, wherein said prognosis method is such that:
▪ if none of the 23 proteins, of said set is expressed, the patient survival rate during a period of time from 30 to 120 months after the diagnosis of said lung cancer is from about 59% to about 78% or more,
▪ if none of the proteins, of the BP, or of the subsets BP1or BP2, is expressed and at least one protein, of the set AP, or of the subset AP1, AP2, or AP3 is expressed, the patient survival rate during a period of time from 30 to 120 months after the diagnosis of said lung cancer is from about 27% to about 70%, and
▪ if at least one protein, of the set BP, or of the subsets BP1 or BP2 is expressed, the survival rate during a period of time from 30 to 120 months after the diagnosis of said lung cancer is from about 3% to about 55%.

The present disclosure also describes the above defined use, wherein said prognosis method is such that:
▪ if none of the 23 genes or proteins, or antibodies, of said set is expressed, the patient survival rate during a period of time from 30 to 120 months after the diagnosis of said lung cancer is from about 59% to about 78% or more,
▪ if
   ∘ none of the genes or proteins of the set B or BP, or of the subset B1, BP1, B2 or BP2, or antibodies, is expressed and at least 3 genes or proteins of the set A or AP, or of the subset A1, AP1, A2, AP2, A3 or AP3, or antibodies, is expressed, or
   ∘ at least 1 gene or protein of the set B or BP, or of the subset B1, BP1, B2 or BP2, or antibody, is expressed and from none to 2 genes or proteins of the set A or AP, or of the subset A1, AP1, A2, AP2, A3 or AP3, or antibodies, is expressed, or
   ∘ at least 2 genes or proteins of the set B or BP, or of the subset B1, BP1, B2 or BP2, or antibodies, is expressed and no gene or protein, of the set A or AP, or of the subset A1, AP1, A2, AP2, A3 or AP3, or antibodies, is expressed,
   the patient survival rate during a period of time from 30 to 120 months after the diagnosis of said lung cancer is from about 27% to about 55%, and
▪ if
   ∘ one gene or protein, of the genes or proteins of the set B or BP, or of the subset B1, BP1, B2 or BP2, or antibodies, is expressed and at least 3 genes or proteins of the set A or AP, or of the subset A1, AP1, A2, AP2, A3 or AP3, or antibodies, are expressed, or
   ∘ at least 2 genes or proteins of the set B or BP, or of the subset B1, BP1, B2 or BP2, or antibodies, are expressed and at least 1 gene or protein of the set A or AP, or of the subset A1, AP1, A2, AP2, A3 or AP3, or antibodies, are expressed,
   the patient survival rate during a period of time from 30 to 120 months after the diagnosis of said lung cancer is from about 3% to about 13%.

In one other advantageous embodiment, the invention relates to the above defined use, wherein said prognosis method is such that:
▪ if none of the 23 genes or proteins of said set is expressed, the patient survival rate during a period of time from 30 to 120 months after the diagnosis of said lung cancer is from about 59% to about 78% or more,
▪ if
   ∘ none of the genes or proteins of the set B or BP, or of the subset B1, BP1, B2 or BP2, is expressed and at least 3 genes or proteins of the set A or AP, or of the subset A1, AP1, A2, AP2, A3 or AP3, is expressed, or
   ∘ at least 1 gene or protein of the set B or BP, or of the subset B1, BP1, B2 or BP2, is expressed and from none to 2 genes or proteins of the set A or AP, or of the subset A1, AP1, A2, AP2, A3 or AP3, is expressed, or
   ∘ at least 2 genes or proteins of the set B or BP, or of the subset B1, BP1, B2 or BP2, is expressed and no gene or protein, of the set A or AP, or of the subset A1, AP1, A2, AP2, A3 or AP3, is expressed,
   the patient survival rate during a period of time from 30 to 120 months
after the diagnosis of said lung cancer is from about 27% to about 55%, and
▪ if
   ∘ one gene or protein, of the genes or proteins of the set B or BP, or of the subset B1, BP1, B2 or BP2, is expressed and at least 3 genes or proteins of the set A or AP, or of the subset A1, AP1, A2, AP2, A3 or AP3, are expressed, or
   ∘ at least 2 genes or proteins of the set B or BP, or of the subset B1, BP1, B2 or BP2, are expressed and at least 1 gene or protein of the set A or AP, or of the subset A1, AP1, A2, AP2, A3 or AP3 are expressed,
the patient survival rate during a period of time from 30 to 120 months after the diagnosis of said lung cancer is from about 3% to about 13%.

In the invention "from none to 2" corresponds to none, or one or two.
By analogy, in the invention, "from none to X", X varying from 3 to 23, corresponds to none, or one, or two, or three, or four, or five, or six, or seven... or twenty three.

In one other advantageous embodiment, the invention relates to the above defined use, wherein said prognosis method is such that:
▪ if none of the 23 genes of said set is expressed, the patient survival rate during a period of time from 30 to 120 months after the diagnosis of said lung cancer is from about 59% to about 78% or more,
▪ if
   ∘ none of the genes of the set B or of the subset B1 or B2, is expressed and at least 3 genes of the set A, or of the subset A1, A2 or A3 is expressed, or
   ∘ at least 1 gene of the set B, or of the subset B1 or B2, is expressed and from none to 2 genes of the set A or of the subset A1, A2, or A3, is expressed, or
   ∘ at least 2 genes of the set B or of the subset B1 or B2 is expressed and no gene of the set A or of the subset A1, A2, or A3, is expressed,
   the patient survival rate during a period of time from 30 to 120 months after the diagnosis of said lung cancer is from about 27% to about 55%, and
▪ if
   ∘ one gene of the genes of the set B or of the subset B 1 or B2, is expressed and at least 3 genes of the set A or of the subset A1, A2, or A3, are expressed, or
   ∘ at least 2 genes of the set B or of the subset B1, or B2 are expressed and at least 1 gene of the set A or of the subset A1, A2, or A3, are expressed,
   the patient survival rate during a period of time from 30 to 120 months after the diagnosis of said lung cancer is from about 3% to about 13%.

In one other advantageous embodiment, the invention relates to the above defined use, wherein said prognosis method is such that:
▪ if none of the 23 proteins, of said set is expressed, the patient survival rate during a period of time from 30 to 120 months after the diagnosis of said lung cancer is from about 59% to about 78% or more,
▪ if
   ∘ none of the proteins of the set BP, or of the subset BP1 or BP2, is expressed and at least 3 proteins, of the set AP, or of the subset AP1, AP2 or AP3, is expressed, or
   ∘ at least 1 protein of the set BP, or of the subset BP1 or BP2, is expressed and from none to 2 proteins, of the set AP, or of the subset AP1, AP2 or AP3, is expressed, or
   ∘ at least 2 proteins of the set BP, or of the subset BP1 or BP2, is expressed and no protein of the set AP, or of the subset AP1, AP2, or AP3, is expressed,
   the patient survival rate during a period of time from 30 to 120 months after the diagnosis of said lung cancer is from about 27% to about 55%, and
▪ if
   ∘ one protein of the proteins of the set BP, or of the subset BP1 or BP2, is expressed and at least 3 proteins of the set AP, or of the subset AP1, AP2 or AP3, are expressed, or
   ∘ at least 2 proteins of the set BP, or of the subset BP1 or BP2, are expressed and at least 1 protein of the set AP, or of the subset AP1, AP2 or AP3, are expressed,
   the patient survival rate during a period of time from 30 to 120 months after the diagnosis of said lung cancer is from about 3% to about 13%.

The present disclosure describes the use as defined above, wherein said prognosis method is such that:
▪ if none of the 23 genes or proteins of said set, or antibodies, is expressed the patient survival rate during a period of time from 30 to 120 months after the diagnosis of said lung cancer is from about 59% to about 78% or more,
▪ if none of the genes or proteins of the set B or BP, or of the subsets B1, BP1, B2 or BP2, or antibodies, is expressed and one or two genes or proteins of the set A or AP, or of the subsets A1, AP1, A2, AP2, A3 or AP3, or antibodies, is expressed, the patient survival rate during a period of time from 30 to 120 months after the diagnosis of said lung cancer is from about 38% to about 70%,
▪ if
   ∘ none of the genes or proteins of the set B or BP, or of the subsets B1, BP1, B2 or BP2, or antibodies, is expressed and at least 3 genes or proteins of the first set A or AP or of the subsets A1, AP1, A2, AP2, A3 or AP3, or antibodies, is expressed, or
   ∘ at least 1 gene or protein of the set B or BP, or of the subsets B1, BP1, B2 or BP2, or antibody, is expressed and from none to 2 genes or proteins of the set A or AP, or of the subsets A1, AP1, A2, AP2, A3 or AP3, or antibodies, is expressed, or
   ∘ at least 2 genes or proteins of the set B or BP, or of the subsets B1, BP1, B2 or BP2, or antibodies, is expressed and no gene or protein of the set A or AP, or of the subsets A1, AP1, A2, AP2, A3 or AP3, or antibody, is expressed,
   the patient survival rate during a period of time from 30 to 120 months after the diagnosis of said lung cancer is from about 27% to about 55%, and
▪ if
   ∘ one gene or protein of the genes or proteins, or antibodies, of the set B or BP, or of the subsets B1, BP1, B2 or BP2, or antibody, is expressed and at least 3 genes or proteins of the set A or AP, or of the subsets A1, AP1, A2, AP2, A3 or AP3, or antibodies, are expressed, or
   ∘ at least 2 genes or proteins of the set B or BP, or of the subsets B1, BP1, B2 or BP2, or antibodies, are expressed and at least 1 gene or protein of the set A or AP, or of the subsets A1, AP1, A2, AP2, A3 or AP3, or antibody, is expressed
   the patient survival rate during a period of time from 30 to 120 months after the diagnosis of said lung cancer is from about 3% to about 13%.

In one another advantageous embodiment, the invention relates to the use as defined above, wherein said prognosis method is such that:
▪ if none of the 23 genes or proteins of said set is expressed the patient survival rate during a period of time from 30 to 120 months after the diagnosis of said lung cancer is from about 59% to about 78% or more,
▪ if none of the genes or proteins of the set B or BP, or of the subsets B1, BP1, B2 or BP2, is expressed and one or two genes or proteins of the set A or AP, or of the subsets A1, AP1, A2, AP2, A3 or AP3, is expressed, the patient survival rate during a period of time from 30 to 120 months after the diagnosis of said lung cancer is from about 38% to about 70%,
▪ if
   ∘ none of the genes or proteins of the set B or BP, or of the subsets B1, BP1, B2 or BP2, is expressed and at least 3 genes or proteins of the first set A or AP or of the subsets A1, AP1, A2, AP2, A3 or AP3, is expressed, or
   ∘ at least 1 gene or protein of the set B or BP, or of the subsets B1, BP1, B2 or BP2, is expressed and from none to 2 genes or proteins of the set A or AP, or of the subsets A1, AP1, A2, AP2, A3 or AP3, is expressed, or
   ∘ at least 2 genes or proteins of the set B or BP, or of the subsets B1, BP1, B2 or BP2, is expressed and no gene or protein of the set A or AP, or of the subsets A1, AP1, A2, AP2, A3 or AP3, is expressed,
   the patient survival rate during a period of time from 30 to 120 months after the diagnosis of said lung cancer is from about 27% to about 55%, and
▪ if
   ∘ one gene or protein of the genes or proteins of the set B or BP, or of the subsets B1, BP1, B2 or BP2, is expressed and at least 3 genes or proteins of the set A or AP, or of the subsets A1, AP1, A2, AP2, A3 or AP3, are expressed, or
   ∘ at least 2 genes or proteins of the set B or BP, or of the subsets B1, BP1, B2 or BP2, are expressed and at least 1 gene or protein of the set A or AP, or of the subsets A1, AP1, A2, AP2, A3 or AP3, is expressed
the patient survival rate during a period of time from 30 to 120 months after the diagnosis of said lung cancer is from about 3% to about 13%.

In one another advantageous embodiment, the invention relates to the use as defined above, wherein said prognosis method is such that:
▪ if none of the 23 genes of said set is expressed, the patient survival rate during a period of time from 30 to 120 months after the diagnosis of said lung cancer is from about 59% to about 78% or more,
▪ if none of the genes of the set B or of the subsets B1 or B2, is expressed and one or two genes of the set A or of the subsets A1, A2 or A3, is expressed, the patient survival rate is from about 38% to about 70%,
▪ if
   ∘ none of the genes of the set B or of the subsets B 1 or B2, is expressed and at least 3 genes of the first set is expressed, or
   ∘ at least 1 gene of the set B or of the subsets B 1 or B2, is expressed and from none to 2 genes of the set A or of the subsets A1, A2 or A3, is expressed, or
   ∘ at least 2 genes of the set B or of the subsets B1 or B2, is expressed and no gene of the set A or of the subsets A1, A2 or A3, is expressed,
   the patient survival rate during a period of time from 30 to 120 months after the diagnosis of said lung cancer is from about 27% to about 55%, and
▪ if
   ∘ one gene of the genes of the set B or of the subsets B 1 or B2, is expressed and at least 3 genes of the set A or of the subsets A1, A2 or A3, are expressed, or
   ∘ at least 2 genes of the set B or of the subsets B1 or B2, are expressed and at least 1 gene of the set A or of the subsets A1, A2 or A3, are expressed
the patient survival rate during a period of time from 30 to 120 months after the diagnosis of said lung cancer is from about 3% to about 13%.

In one another advantageous embodiment, the invention relates to the use as defined above, wherein said prognosis method is such that:
▪ if none of the 23 proteins of said set is expressed, the patient survival rate during a period of time from 30 to 120 months after the diagnosis of said lung cancer is from about 59% to about 78% or more,
▪ if none of the proteins of the set BP or of the subsets BP1 or BP2, is expressed and one or two proteins of the set AP or of the subsets AP1, AP2 or AP3, is expressed, the patient survival rate during a period of time from 30 to 120 months after the diagnosis of said lung cancer is from about 38% to about 70%,
▪ if
   ∘ none of the proteins of the set BP or of the subsets BP1 or BP2, is expressed and at least 3 proteins of the first set is expressed, or
   ∘ at least 1 protein of the set BP or of the subsets BP1 or BP2, is expressed and from none to 2 proteins of the set AP or of the subsets AP1, AP2 or AP3, is expressed, or
   ∘ at least 2 proteins of the set BP or of the subsets BP1 or BP2, is expressed and no protein of the set AP or of the subsets AP1, AP2 or AP3, is expressed,
   the patient survival rate during a period of time from 30 to 120 months after the diagnosis of said lung cancer is from about 27% to about 55%, and
▪ if
   ∘ one protein of the proteins of the set BP or of the subsets BP1 or BP2, is expressed and at least 3 proteins of the set AP or of the subsets AP1, AP2 or AP3, are expressed, or
   ∘ at least 2 proteins of the set BP or of the subsets BP1 or BP2, are expressed and at least 1 protein of the set AP or of the subsets AP1, AP2 or AP3, are expressed
the patient survival rate during a period of time from 30 to 120 months after the diagnosis of said lung cancer is from about 3% to about 13%.

The present disclosure describes the use as defined above, wherein during a period of time of 120 months from the diagnosis of said lung cancer
▪ if none of the 23 genes or proteins of said set, or antibodies, is expressed, the patient survival rate is from about 59% or more, and
▪ if at least one gene or protein of the genes or proteins of said set A or AP or B or BP, or of the subsets A1, AP1, A2, AP2, A3, AP3 or B1, BP1, B2 or BP2, or antibody, is expressed, the patient survival rate is about from about 3% to about 38%.

In another embodiment, the invention relates to the use as defined above, wherein during a period of time of 120 months from the diagnosis of said lung cancer
▪ if none of the 23 genes or proteins of said set is expressed, the patient survival rate is from about 59% or more, and
▪ if at least one gene or protein of the genes or proteins of said set A or AP or B or BP, or of the subsets A1, AP1, A2, AP2, A3, AP3 or B1, BP1, B2 or BP2, is expressed, the patient survival rate is about from about 3% to about 38%.

In another embodiment, the invention relates to the use as defined above, wherein during a period of time of 120 months from the diagnosis of said lung cancer
▪ if none of the 23 genes of said set is expressed, the patient survival rate is from about 59% or more, and
▪ if at least one gene of the genes of said set A or B, or of the subsets A1, A2, A3 or B 1 or B2 is expressed, the patient survival rate is about from about 3% to about 38%.

In another embodiment, the invention relates to the use as defined above, wherein during a period of time of 120 months from the diagnosis of said lung cancer
▪ if none of the 23 proteins of said set is expressed, the patient survival rate is from about 59% or more, and
▪ if at least one protein of the proteins of said set AP or BP, or of the subsets AP1, AP2, AP3 or BP1, BP2 is expressed, the patient survival rate is about from about 3% to about 38%.

The present disclosure describes the use as previously defined, wherein during a period of time of 120 months from the diagnosis of said lung cancer
▪ if none of the 23 genes or proteins of said set, or antibodies, is expressed, the patient survival rate is about 59% or more,
▪ if none of the genes or proteins of the set B or BP, or of the subsets B1, BP1, B2 or BP2, or antibodies, is expressed and one or two genes or proteins of the set A or AP, or of subsets A1, AP1, A2, AP2, A3, or AP3, or antibodies, is expressed, the patient survival rate is about 38%, and
▪ if at least one gene or protein of the set B or BP, or of the subset B1, BP1, B2 or BP2, or antibody, is expressed, the patient survival rate is about 3% to about 27%.

In an advantageous embodiment, the invention relates to the use as previously defined, wherein during a period of time of 120 months from the diagnosis of said lung cancer
▪ if none of the 23 genes or proteins of said set is expressed, the patient survival rate is about 59% or more,
▪ if none of the genes or proteins of the set B or BP, or of the subsets B1, BP1, B2 or BP2, is expressed and one or two genes or proteins of the set A or AP, or of subsets A1, AP1, A2, AP2, A3, or AP3, is expressed, the patient survival rate is about 38%, and
▪ if at least one gene or protein of the set B or BP, or of the subset B1, BP1, B2 or BP2, is expressed, the patient survival rate is about 3% to about 27%.

In an advantageous embodiment, the invention relates to the use as previously defined, wherein during a period of time of 120 months from the diagnosis of said lung cancer
▪ if none of the 23 genes of said set is expressed, the patient survival rate is about 59% or more, ▪ if none of the genes of the set B, or of the subsets B1, or B2 is expressed and one or two genes of the set A, or of subsets A1, A2, or A3, is expressed, the patient survival rate is about 38%, and
▪ if at least one gene of the set B, or of the subset B1, or B2, is expressed, the patient survival rate is about 3% to about 27%.

In an advantageous embodiment, the invention relates to the use as previously defined, wherein during a period of time of 120 months from the diagnosis of said lung cancer
▪ if none of the 23 proteins of said set is expressed, the patient survival rate is about 59% or more,
▪ if none of the proteins of the set BP, or of the subsets BP1 or BP2, is expressed and one or two proteins of the set AP, or of subsets AP1, AP2 or AP3 is expressed, the patient survival rate is about 38%, and
▪ if at least one protein of the set B or BP, or of the subset B1, BP1, B2 or BP2, is expressed, the patient survival rate is about 3% to about 27%.

The present disclosure describes the use as previously defined, wherein during a period of time of 120 months from the diagnosis of said lung cancer
▪ if none of the 23 genes or proteins of said set, or antibodies, is expressed, the patient survival rate is about 59% or more,
▪ if
   ∘ none of the genes or proteins of the set B or BP, or of subsets B1, BP1, B2 or BP2, or antibodies, is expressed and at least 3 genes or proteins of the set A or AP, or of subsets A1, AP1, A2, AP2, A3 or AP3, or antibodies, is expressed, or
   ∘ at least 1 gene or protein of the set B or BP, or of subsets B1, BP1, B2 or BP2, or antibody, is expressed and from none to 2 genes or proteins of the set A or AP, or of subsets A1, AP1, A2, AP2, A3 or AP3, or antibodies, is expressed, or
   ∘ at least 2 genes or proteins of the set B or BP, or of subsets B1, BP1, B2 or BP2, or antibodies, is expressed and no gene or protein of the set A or AP, or of subsets A1, AP1, A2, AP2, A3 or AP3, or antibody, is expressed,
   the patient survival rate is about 27%, and
▪ if
   ∘ one gene or protein of the genes or proteins, or antibodies, of the set B or BP, or of subsets B1, BP1, B2 or BP2, or antibody, is expressed and at least 3 genes or proteins of the set A or AP or of subsets A1, AP1, A2, AP2, A3 or AP3, or antibodies, are expressed, or
   ∘ at least 2 genes or proteins, of the set B or BP, or of subsets B1, BP1, B2 or BP2, or antibodies are expressed and at least 1 gene or protein of the set A or AP, or of subsets A1, AP1, A2, AP2, A3 or AP3, or antibody, are expressed
   the patient survival rate is about 3%.

In an advantageous embodiment, the invention relates to the use as previously defined, wherein during a period of time of 120 months from the diagnosis of said lung cancer
▪ if none of the 23 genes or proteins of said set is expressed, the patient survival rate is about 59% or more,
▪ if
   ∘ none of the genes or proteins of the set B or BP, or of subsets B1, BP1, B2 or BP2, is expressed and at least 3 genes or proteins of the set A or AP, or of subsets A1, AP1, A2, AP2, A3 or AP3, is expressed, or
   ∘ at least 1 gene or protein of the set B or BP, or of subsets B1, BP1, B2 or BP2 is expressed and from none to 2 genes or proteins of the set A or AP, or of subsets A1, AP1, A2, AP2, A3 or AP3, is expressed, or
   ∘ at least 2 genes or proteins of the set B or BP, or of subsets B1, BP1, B2 or BP2, is expressed and no gene or protein of the set A or AP, or of subsets A1, AP1, A2, AP2, A3 or AP3, is expressed,
   the patient survival rate is about 27%, and
▪ if
   ∘ one gene or protein of the genes or proteins of the set B or BP, or of subsets B1, BP1, B2 or BP2, is expressed and at least 3 genes or proteins of the set A or AP or of subsets A1, AP1, A2, AP2, A3 or AP3, are expressed, or
   ∘ at least 2 genes or proteins of the set B or BP, or of subsets B1, BP1, B2 or BP2, are expressed and at least 1 gene or protein of the set A or AP, or of subsets A1, AP1, A2, AP2, A3 or AP3, are expressed
   the patient survival rate is about 3%.

In an advantageous embodiment, the invention relates to the use as previously defined, wherein during a period of time of 120 months from the diagnosis of said lung cancer
▪ if none of the 23 genes of said set is expressed, the patient survival rate is about 59% or more,
▪ if
   ∘ none of the genes of the set B, or of subsets B1 or B2, is expressed and at least 3 genes of the set A, or of subsets A1, A2 or A3, is expressed, or
   ∘ at least 1 gene of the set B, or of subsets B1 or B2, is expressed and from none to 2 genes of the set A, or of subsets A1, A2 or A3, is expressed, or
   ∘ at least 2 genes of the set B, or of subsets B1 or B2, is expressed and no gene of the set A, or of subsets A1, A2, or A3, is expressed,
   the patient survival rate is about 27%, and
▪ if
   ∘ one gene of the genes of the set B, or of subsets B 1 or B2, is expressed and at least 3 genes of the set A, or of subsets A1, A2, or A3, are expressed, or
   ∘ at least 2 genes of the set B, or of subsets B1, or B2, are expressed and at least 1 gene of the set A, or of subsets A1, A2, or A3, are expressed the patient survival rate is about 3%.

In an advantageous embodiment, the invention relates to the use as previously defined, wherein during a period of time of 120 months from the diagnosis of said lung cancer
▪ if none of the 23 proteins of said set is expressed, the patient survival rate is about 59% or more,
▪ if
   ∘ none of the proteins of the set BP, or of subsets BP1 or BP2, is expressed and at least 3 proteins of the set AP, or of subsets AP1, AP2 or AP3, is expressed, or
   ∘ at least 1 protein of the set BP, or of subsets BP1 or BP2, is expressed and from none to 2 proteins of the set AP, or of subsets AP1, AP2 or AP3, is expressed, or
   ∘ at least 2 proteins of the set BP, or of subsets BP1 or BP2, is expressed and no protein of the set AP, or of subsets AP1, AP2, or AP3, is expressed,
   the patient survival rate is about 27%, and
▪ if
   ∘ one protein of the proteins of the set BP, or of subsets BP1 or BP2, is expressed and at least 3 proteins of the set AP, or of subsets AP1, AP2, or AP3, are expressed, or
   ∘ at least 2 proteins of the set BP, or of subsets BP1, or BP2, are expressed and at least 1 protein of the set AP, or of subsets AP1, AP2, or AP3, are expressed
   the patient survival rate is about 3%.

The present disclosure describes the use as previously defined, wherein said prognosis method is such that:
▪ if none of the 23 genes or proteins of said set, or antibodies, is expressed, the patient survival rate is about 59% or more,
▪ if none of the genes or proteins of the set B or BP, or of subsets B1, BP1, B2 or BP2, or antibodies, is expressed and one or two genes or proteins of the set A or AP, or of subsets A1, AP1, A2, AP2, A3 or AP3, or antibodies, is expressed, the patient survival rate is about 38%,
▪ if
   ∘ none of the genes or proteins of the set B or BP, or of subsets B1, BP1, B2 or BP2, or antibodies, is expressed and at least 3 genes or proteins of the set A or AP, or of subsets A1, AP1, A2, AP2, A3 or AP3, or antibodies, is expressed, or
   ∘ at least 1 gene or protein of the set B or BP, or of subsets B1, BP1, B2 or BP2, or antibody, is expressed and from none to 2 genes or proteins of the set A or AP, or of subsets A1, AP1, A2, AP2, A3 or AP3, or antibodies, is expressed, or
   ∘ at least 2 genes or proteins of the set B or BP, or of subsets B1, BP1, B2 or BP2, or antibodies, is expressed and no gene or protein of the set A or AP, or of subsets A1, AP1, A2, AP2, A3 or AP3, or antibody, is expressed,
   the patient survival rate is about 27%, and
▪ if
   ∘ one gene or protein of the genes or proteins, or antibodies, of the set B or BP, or of subsets B1, BP1, B2 or BP2, or antibody, is expressed and at least 3 genes or proteins of the set A or AP, or of subsets A1, AP1, A2, AP2, A3 or AP3, or antibodies, are expressed, or
   ∘ at least 2 genes or proteins of the set B or BP, or of subsets B1, BP1, B2 or BP2, or antibodies, are expressed and at least 1 gene or protein of the set A or AP, or of subsets A1, AP1, A2, AP2, A3 or AP3, or antibody, is expressed
   the patient survival rate is about 3%.

In an advantageous embodiment, the invention relates to the use as previously defined, wherein said prognosis method is such that:
▪ if none of the 23 genes or proteins of said set is expressed, the patient survival rate is about 59% or more,
▪ if none of the genes or proteins of the set B or BP, or of subsets B1, BP1, B2 or BP2, is expressed and one or two genes or proteins of the set A or AP, or of subsets A1, AP1, A2, AP2, A3 or AP3, is expressed, the patient survival rate is about 38%,
▪ if
   ∘ none of the genes or proteins of the set B or BP, or of subsets B1, BP1, B2 or BP2, is expressed and at least 3 genes or proteins of the set A or AP, or of subsets A1, AP1, A2, AP2, A3 or AP3, is expressed, or
   ∘ at least 1 gene or protein of the set B or BP, or of subsets B1, BP1, B2 or BP2, is expressed and from none to 2 genes or proteins of the set A or AP, or of subsets A1, AP1, A2, AP2, A3 or AP3, is expressed, or
   ∘ at least 2 genes or proteins of the set B or BP, or of subsets B1, BP1, B2 or BP2, is expressed and no gene or protein of the set A or AP, or of subsets A1, AP1, A2, AP2, A3 or AP3, is expressed,
   the patient survival rate is about 27%, and
▪ if
   ∘ one gene or protein of the genes or proteins of the set B or BP, or of subsets B1, BP1, B2 or BP2, is expressed and at least 3 genes or proteins of the set A or AP, or of subsets A1, AP1, A2, AP2, A3 or AP3, are expressed, or
   ∘ at least 2 genes or proteins of the set B or BP, or of subsets B1, BP1, B2 or BP2, are expressed and at least 1 gene or protein of the set A or AP, or of subsets A1, AP1, A2, AP2, A3 or AP3, is expressed
   the patient survival rate is about 3%.

In an advantageous embodiment, the invention relates to the use as previously defines, wherein said prognosis method is such that:
▪ if none of the 23 genes of said set is expressed, the patient survival rate is about 59% or more,
▪ if none of the genes of the set B, or of subsets B1 or B2, is expressed and one or two genes of the set A, or of subsets A1, A2 or A3, is expressed, the patient survival rate is about 38%,
▪ if
   ∘ none of the genes of the set B, or of subsets B1 or B2, is expressed and at least 3 genes of the set A, or of subsets A1, A2, or A3, is expressed, or
   ∘ at least 1 gene of the set B, or of subsets B1 or B2, is expressed and from none to 2 genes of the set A, or of subsets A1, A2, or A3 is expressed, or
   ∘ at least 2 genes of the set B, or of subsets B1, or B2, is expressed and no gene of the set A, or of subsets A1 A2, or A3, is expressed,
   the patient survival rate is about 27%, and
▪ if
   ∘ one gene of the genes of the set B, or of subsets B 1 or B2, is expressed and at least 3 genes of the set A, or of subsets A1, A2, or A3, are expressed, or
   ∘ at least 2 genes of the set B, or of subsets B1 or B2, are expressed and at least 1 gene of the set A, or of subsets A1, A2, A3, is expressed
   the patient survival rate is about 3%.

In an advantageous embodiment, the invention relates to the use as previously defines, wherein said prognosis method is such that:
▪ if none of the 23 proteins of said set is expressed, the patient survival rate is about 59% or more,
▪ if none of the proteins of the set BP, or of subsets BP1 or BP2, is expressed and one or two proteins of the set AP, or of subsets AP1, AP2, or AP3, is expressed, the patient survival rate is about 38%,
▪ if
   ∘ none of the proteins of the set BP, or of subsets BP1 or BP2, is expressed and at least 3 proteins of the set AP, or of subsets AP1, AP2 or AP3, is expressed, or
   ∘ at least 1 protein of the set BP, or of subsets BP1 or BP2, is expressed and from none to 2 proteins, of the set AP, or of subsets AP1, AP2 or AP3 is expressed, or
   ∘ at least 2 proteins of the set BP, or of subsets BP1 or BP2, is expressed and no protein, of the set AP, or of subsets AP1, AP2 or AP3, is expressed,
   the patient survival rate is about 27%, and
▪ if
   ∘ one protein of the proteins of the set BP, or of subsets BP1 or BP2, is expressed and at least 3 proteins of the set AP, or of subsets AP1, AP2 or AP3, are expressed, or
   ∘ at least 2 proteins of the set BP, or of subsets BP 1 or BP2, are expressed and at least 1 protein of the set AP, or of subsets AP1, AP2 or AP3, is expressed
   the patient survival rate is about 3%.

The present disclosure describes the use as defined above, wherein during a period of time of 60 months from the diagnosis of said lung cancer
▪ if none of the 23 genes or proteins of said set, or antibodies, is expressed, the patient survival rate is from about 66% or more, and
▪ if at least one gene or protein of the genes or proteins of said set A or AP or B or BP, or of the subsets A1, AP1, A2, AP2, A3, AP3 or B1, BP1, B2 or BP2, or antibody, is expressed, the patient survival rate is about from about 3% to about 54%.

In another embodiment, the invention relates to the use as defined above, wherein during a period of time of 60 months from the diagnosis of said lung cancer
▪ if none of the 23 genes or proteins of said set is expressed, the patient survival rate is from about 66% or more, and
▪ if at least one gene or protein of the genes or proteins of said set A or AP or B or BP, or of the subsets A1, AP1, A2, AP2, A3, AP3 or B1, BP1, B2 or BP2, is expressed, the patient survival rate is about from about 3% to about 54%.

In another embodiment, the invention relates to the use as defined above, wherein during a period of time of 60 months from the diagnosis of said lung cancer
▪ if none of the 23 genes of said set is expressed, the patient survival rate is from about 66% or more, and
▪ if at least one gene of the genes of said set A or B, or of the subsets AP1, AP2, AP3 or BP1 or BP2 is expressed, the patient survival rate is about from about 3% to about 54%.

In another embodiment, the invention relates to the use as defined above, wherein during a period of time of 60 months from the diagnosis of said lung cancer
▪ if none of the 23 proteins of said set is expressed, the patient survival rate is from about 66% or more, and
▪ if at least one protein of the proteins of said set AP or BP, or of the subsets AP1, AP2, AP3 or BP1, BP2 is expressed, the patient survival rate is about from about 3% to about 54%.

The present disclosure describes the use as previously defined, wherein during a period of time of 60 months from the diagnosis of said lung cancer
▪ if none of the 23 genes or proteins of said set, or antibodies, is expressed, the patient survival rate is about 66% or more,
▪ if none of the genes or proteins of the set B or BP, or of the subsets B1, BP1, B2 or BP2, or antibodies, is expressed and one or two genes or proteins of the set A or AP, or of subsets A1, AP1, A2, AP2, A3, or AP3, or antibodies, is expressed, the patient survival rate is about 54%, and
▪ if at least one gene or protein of the set B or BP, or of the subset B1, BP1, B2 or BP2, or antibody, is expressed, the patient survival rate is about 3% to about 36%.

In an advantageous embodiment, the invention relates to the use as previously defined, wherein during a period of time of 60 months from the diagnosis of said lung cancer
▪ if none of the 23 genes or proteins of said set is expressed, the patient survival rate is about 66% or more,
▪ if none of the genes or proteins of the set B or BP, or of the subsets B1, BP1, B2 or BP2, is expressed and one or two genes or proteins of the set A or AP, or of subsets A1, AP1, A2, AP2, A3, or AP3, is expressed, the patient survival rate is about 54%, and
▪ if at least one gene or protein of the set B or BP, or of the subset B1, BP1, B2 or BP2, is expressed, the patient survival rate is about 3% to about 36%.

In an advantageous embodiment, the invention relates to the use as previously defined, wherein during a period of time of 60 months from the diagnosis of said lung cancer
▪ if none of the 23 genes of said set is expressed, the patient survival rate is about 66% or more,
▪ if none of the genes of the set B, or of the subsets B1, or B2 is expressed and one or two genes of the set A, or of subsets A1, A2, or A3, is expressed, the patient survival rate is about 54%, and
▪ if at least one gene of the set B, or of the subset B1, or B2, is expressed, the patient survival rate is about 3% to about 36%.

In an advantageous embodiment, the invention relates to the use as previously defined, wherein during a period of time of 60 months from the diagnosis of said lung cancer
▪ if none of the 23 proteins of said set is expressed, the patient survival rate is about 66% or more,
▪ if none of the proteins of the set BP, or of the subsets BP1 or BP2, is expressed and one or two proteins of the set AP, or of subsets AP1, AP2 or AP3 is expressed, the patient survival rate is about 54%, and
▪ if at least one protein of the set B or BP, or of the subset B1, BP1, B2 or BP2, is expressed, the patient survival rate is about 3% to about 36%.

The present disclosure describes the use as previously defined, wherein during a period of time of 60 months from the diagnosis of said lung cancer
▪ if none of the 23 genes or proteins of said set is expressed, or antibodies, the patient survival rate is about 66% or more,
▪ if
   ∘ none of the genes or proteins of the set B or BP, or of subsets B1, BP1, B2 or BP2, or antibodies, is expressed and at least 3 genes or proteins of the set A or AP, or of subsets A1, AP1, A2, AP2, A3 or AP3, or antibodies, is expressed, or
   ∘ at least 1 gene or protein of the set B or BP, or of subsets B1, BP1, B2 or BP2, or antibody, is expressed and from none to 2 genes or proteins of the set A or AP, or of subsets A1, AP1, A2, AP2, A3 or AP3, or antibodies, is expressed, or
   ∘ at least 2 genes or proteins of the set B or BP, or of subsets B1, BP1, B2 or BP2, or antibodies, is expressed and no gene or protein of the set A or AP, or of subsets A1, AP1, A2, AP2, A3 or AP3, or antibody, is expressed,
   the patient survival rate is about 36%, and
▪ if
   ∘ one gene or protein of the genes or proteins of the set B or BP, or of subsets B1, BP1, B2 or BP2, or antibody, is expressed and at least 3 genes or proteins of the set A or AP or of subsets A1, AP1, A2, AP2, A3 or AP3, or antibodies, are expressed, or
   ∘ at least 2 genes or proteins of the set B or BP, or of subsets B1, BP1, B2 or BP2, or antibodies, are expressed and at least 1 gene or protein of the set A or AP, or of subsets A1, AP1, A2, AP2, A3 or AP3, or antibody, are expressed
   the patient survival rate is about 3%.

In an advantageous embodiment, the invention relates to the use as previously defined, wherein during a period of time of 60 months from the diagnosis of said lung cancer
▪ if none of the 23 genes or proteins of said set is expressed, the patient survival rate is about 66% or more,
- if
   ∘ none of the genes or proteins of the set B or BP, or of subsets B1, BP1, B2 or BP2, is expressed and at least 3 genes or proteins of the set A or AP, or of subsets A1, AP1, A2, AP2, A3 or AP3, is expressed, or
   ∘ at least 1 gene or protein of the set B or BP, or of subsets B 1, BP1, B2 or BP2, is expressed and from none to 2 genes or proteins of the set A or AP, or of subsets A1, AP1, A2, AP2, A3 or AP3, is expressed, or
   ∘ at least 2 genes or proteins of the set B or BP, or of subsets B1, BP1, B2 or BP2, is expressed and no gene or protein of the set A or AP, or of subsets A1, AP1, A2, AP2, A3 or AP3, is expressed,
   the patient survival rate is about 36%, and
▪ if
   ∘ one gene or protein of the genes or proteins of the set B or BP, or of subsets B1, BP1, B2 or BP2, is expressed and at least 3 genes or proteins of the set A or AP or of subsets A1, AP1, A2, AP2, A3 or AP3, are expressed, or
   ∘ at least 2 genes or proteins of the set B or BP, or of subsets B1, BP1, B2 or BP2, are expressed and at least 1 gene or protein of the set A or AP, or of subsets A1, AP1, A2, AP2, A3 or AP3, are expressed
   the patient survival rate is about 3%.

In an advantageous embodiment, the invention relates to the use as previously defined, wherein during a period of time of 60 months from the diagnosis of said lung cancer
▪ if none of the 23 genes of said set is expressed, the patient survival rate is about 66% or more,
- if
   ∘ none of the genes of the set B, or of subsets B1 or B2, is expressed and at least 3 genes of the set A, or of subsets A1, A2 or A3, is expressed, or
   ∘ at least 1 gene of the set B, or of subsets B 1 or B2, is expressed and from none to 2 genes of the set A, or of subsets A1, A2 or A3, is expressed, or
   ∘ at least 2 genes of the set B, or of subsets B1 or B2, is expressed and no gene of the set A, or of subsets A1, A2, or A3, is expressed,
   the patient survival rate is about 36%, and
▪ if
   ∘ one gene of the genes of the set B, or of subsets B1 or B2, is expressed and at least 3 genes of the set A, or of subsets A1, A2, or A3, are expressed, or
   ∘ at least 2 genes of the set B, or of subsets B1, or B2, are expressed and at least 1 gene of the set A, or of subsets A1, A2, or A3, are expressed
   the patient survival rate is about 3%.

In an advantageous embodiment, the invention relates to the use as previously defined, wherein during a period of time of 60 months from the diagnosis of said lung cancer
▪ if none of the 23 proteins of said set is expressed, the patient survival rate is about 66% or more,
- if
   ∘ none of the proteins of the set BP, or of subsets BP1 or BP2, is expressed and at least 3 proteins of the set AP, or of subsets AP1, AP2 or AP3, is expressed, or
   ∘ at least 1 protein of the set BP, or of subsets BP1 or BP2, is expressed and from none to 2 proteins of the set AP, or of subsets AP1, AP2 or AP3, is expressed, or
   ∘ at least 2 proteins of the set BP, or of subsets BP1 or BP2, is expressed and no protein of the set AP, or of subsets AP1, AP2, or AP3, is expressed,
   the patient survival rate is about 36%, and
▪ if
   ∘ one protein of the proteins of the set BP, or of subsets BP1 or BP2, is expressed and at least 3 proteins of the set AP, or of subsets AP1, AP2, or AP3, are expressed, or
   ∘ at least 2 proteins of the set BP, or of subsets BP1, or BP2, are expressed and at least 1 protein of the set AP, or of subsets AP1, AP2, or AP3, are expressed
   the patient survival rate is about 3%.

The present disclosure describes the use as previously defined, wherein said prognosis method is such that:
▪ if none of the 23 genes or proteins of said set, or antibodies, is expressed, the patient survival rate is about 66% or more,
▪ if none of the genes or proteins of the set B or BP, or of subsets B1, BP1, B2 or BP2, or antibodies, is expressed and one or two genes or proteins of the set A or AP, or of subsets A1, AP1, A2, AP2, A3 or AP3, or antibodies is expressed, the patient survival rate is about 54%,
▪ if
   ∘ none of the genes or proteins of the set B or BP, or of subsets B1, BP1, B2 or BP2, or antibodies, is expressed and at least 3 genes or proteins of the set A or AP, or of subsets A1, AP1, A2, AP2, A3 or AP3, or antibodies, is expressed, or
   ∘ at least 1 gene or protein of the set B or BP, or of subsets B1, BP1, B2 or BP2, or antibody, is expressed and from none to 2 genes or proteins of the set A or AP, or of subsets A1, AP1, A2, AP2, A3 or AP3, or antibodies, is expressed, or
   ∘ at least 2 genes or proteins of the set B or BP, or of subsets B1, BP1, B2 or BP2, or antibodies, is expressed and no gene or protein of the set A or AP, or of subsets A1, AP1, A2, AP2, A3 or AP3, or antibody, is expressed,
   the patient survival rate is about 36%, and
▪ if
   ∘ one gene or protein of the genes or proteins of the set B or BP, or of subsets B1, BP1, B2 or BP2, or antibody, is expressed and at least 3 genes or proteins of the set A or AP, or of subsets A1, AP1, A2, AP2, A3 or AP3, or antibodies, are expressed, or
   ∘ at least 2 genes or proteins of the set B or BP, or of subsets B1, BP1, B2 or BP2, or antibodies, are expressed and at least 1 gene or protein of the set A or AP, or of subsets A1, AP1, A2, AP2, A3 or AP3, or antibody, is expressed
   the patient survival rate is about 3%.

In an advantageous embodiment, the invention relates to the use as previously defined, wherein said prognosis method is such that:
▪ if none of the 23 genes or proteins of said set is expressed, the patient survival rate is about 66% or more,
▪ if none of the genes or proteins of the set B or BP, or of subsets B1, BP1, B2 or BP2, is expressed and one or two genes or proteins of the set A or AP, or of subsets A1, AP1, A2, AP2, A3 or AP3, is expressed, the patient survival rate is about 54%,
▪ if
   ∘ none of the genes or proteins of the set B or BP, or of subsets B1, BP1, B2 or BP2, is expressed and at least 3 genes or proteins of the set A or AP, or of subsets A1, AP1, A2, AP2, A3 or AP3, is expressed, or
   ∘ at least 1 gene or protein of the set B or BP, or of subsets B1, BP1, B2 or BP2, is expressed and from none to 2 genes or proteins of the set A or AP, or of subsets A1, AP1, A2, AP2, A3 or AP3, is expressed, or
   ∘ at least 2 genes or proteins of the set B or BP, or of subsets B1, BP1, B2 or BP2, is expressed and no gene or protein of the set A or AP, or of subsets A1, AP1, A2, AP2, A3 or AP3, is expressed,
   the patient survival rate is about 36%, and
▪ if
   ∘ one gene or protein of the genes or proteins of the set B or BP, or of subsets B1, BP1, B2 or BP2 is expressed and at least 3 genes or proteins of the set A or AP, or of subsets A1, AP1, A2, AP2, A3 or AP3 are expressed, or
   ∘ at least 2 genes or proteins of the set B or BP, or of subsets B1, BP1, B2 or BP2, are expressed and at least 1 gene or protein of the set A or AP, or of subsets A1, AP1, A2, AP2, A3 or AP3 is expressed
   the patient survival rate is about 3%.

In an advantageous embodiment, the invention relates to the use as previously defined, wherein said prognosis method is such that:
▪ if none of the 23 genes of said set is expressed, the patient survival rate is about 66% or more,
▪ if none of the genes of the set B, or of subsets B 1 or B2, is expressed and one or two genes of the set A, or of subsets A1, A2 or A3, is expressed, the patient survival rate is about 54%,
▪ if
   ∘ none of the genes of the set B, or of subsets B 1 or B2, is expressed and at least 3 genes of the set A, or of subsets A1, A2, or A3, is expressed, or
   ∘ at least 1 gene of the set B, or of subsets B1 or B2, is expressed and from none to 2 genes of the set A, or of subsets A1, A2, or A3 is expressed, or
   ∘ at least 2 genes of the set B, or of subsets B1, or B2, is expressed and no gene of the set A, or of subsets A1 A2, or A3, is expressed,
   the patient survival rate is about 36%, and
▪ if
   ∘ one gene of the genes of the set B, or of subsets B 1 or B2, is expressed and at least 3 genes of the set A, or of subsets A1, A2, or A3, are expressed, or
   ∘ at least 2 genes of the set B, or of subsets B 1 or B2, are expressed and at least 1 gene of the set A, or of subsets A1, A2, A3, is expressed
   the patient survival rate is about 3%.

In an advantageous embodiment, the invention relates to the use as previously defined, wherein said prognosis method is such that:
▪ if none of the 23 proteins of said set is expressed, the patient survival rate is about 66% or more,
▪ if none of the proteins of the set BP, or of subsets BP1 or BP2, is expressed and one or two proteins of the set AP, or of subsets AP1, AP2, or AP3, is expressed, the patient survival rate is about 54%,
▪ if
   ∘ none of the proteins of the set BP, or of subsets BP1 or BP2, is expressed and at least 3 proteins of the set AP, or of subsets AP1, AP2 or AP3, is expressed, or
   ∘ at least 1 protein of the set BP, or of subsets BP1 or BP2, is expressed and from none to 2 proteins, of the set AP, or of subsets AP1, AP2 or AP3 is expressed, or
   ∘ at least 2 proteins of the set BP, or of subsets BP1 or BP2, is expressed and no protein, of the set AP, or of subsets AP1, AP2 or AP3, is expressed,
   the patient survival rate is about 36%, and
▪ if
   ∘ one protein of the proteins of the set BP, or of subsets BP1 or BP2, is expressed and at least 3 proteins of the set AP, or of subsets AP1, AP2 or AP3, are expressed, or
   ∘ at least 2 proteins of the set BP, or of subsets BP 1 or BP2, are expressed and at least 1 protein of the set AP, or of subsets AP1, AP2 or AP3, is expressed
   the patient survival rate is about 3%.

The present disclosure describes the use as defined above, wherein during a period of time of 30 months from the diagnosis of said lung cancer
▪ if none of the 23 genes or proteins of said set, or antibodies, is expressed, the patient survival rate is from about 78% or more, and
▪ if at least one gene or protein of the genes or proteins of said set A or AP or B or BP, or of the subsets A1, AP1, A2, AP2, A3, AP3 or B1, BP1, B2 or BP2, or antibody, is expressed, the patient survival rate is about from about 13% to about 70%.

In another embodiment, the invention relates to the use as defined above, wherein during a period of time of 30 months from the diagnosis of said lung cancer
▪ if none of the 23 genes or proteins of said set is expressed, the patient survival rate is from about 78% or more, and
▪ if at least one gene or protein of the genes or proteins of said set A or AP or B or BP, or of the subsets A1, AP1, A2, AP2, A3, AP3 or B1, BP1, B2 or BP2, is expressed, the patient survival rate is about from about 13% to about 70%.

In another embodiment, the invention relates to the use as defined above, wherein during a period of time of 30 months from the diagnosis of said lung cancer
▪ if none of the 23 genes of said set is expressed, the patient survival rate is from about 78% or more, and
▪ if at least one gene of the genes of said set A or B, or of the subsets AP1, AP2, AP3 or BP1 or BP2 is expressed, the patient survival rate is about from about 13% to about 70%.

In another embodiment, the invention relates to the use as defined above, wherein during a period of time of 30 months from the diagnosis of said lung cancer
▪ if none of the 23 proteins of said set is expressed, the patient survival rate is from about 78% or more, and
▪ if at least one protein of the proteins of said set AP or BP, or of the subsets AP1, AP2, AP3 or BP1, BP2 is expressed, the patient survival rate is about from about 13% to about 70%.

The present disclosure describes the use as previously defined, wherein during a period of time of 30 months from the diagnosis of said lung cancer
▪ if none of the 23 genes or proteins of said set, or antibodies, is expressed, the patient survival rate is about 78% or more,
▪ if none of the genes or proteins of the set B or BP, or of the subsets B1, BP1, B2 or BP2, or antibodies, is expressed and one or two genes or proteins of the set A or AP, or of subsets A1, AP1, A2, AP2, A3, or AP3, or antibodies, is expressed, the patient survival rate is about 70%, and
▪ if at least one gene or protein of the set B or BP, or of the subset B1, BP1, B2 or BP2, or antibody, is expressed, the patient survival rate is about 13% to about 55%.

In an advantageous embodiment, the invention relates to the use as previously defined, wherein during a period of time of 30 months from the diagnosis of said lung cancer
▪ if none of the 23 genes or proteins of said set is expressed, the patient survival rate is about 78% or more,
▪ if none of the genes or proteins of the set B or BP, or of the subsets B 1, BP1, B2 or BP2, is expressed and one or two genes or proteins of the set A or AP, or of subsets A1, AP1, A2, AP2, A3, or AP3, is expressed, the patient survival rate is about 70%, and
▪ if at least one gene or protein of the set B or BP, or of the subset B1, BP1, B2 or BP2, is expressed, the patient survival rate is about 13% to about 55%.

In an advantageous embodiment, the invention relates to the use as previously defined, wherein during a period of time of 30 months from the diagnosis of said lung cancer
▪ if none of the 23 genes of said set is expressed, the patient survival rate is about 78% or more,
▪ if none of the genes of the set B, or of the subsets B1, or B2 is expressed and one or two genes of the set A, or of subsets A1, A2, or A3, is expressed, the patient survival rate is about 70%, and
▪ if at least one gene of the set B, or of the subset B1, or B2, is expressed, the patient survival rate is about 13% to about 55%.

In an advantageous embodiment, the invention relates to the use as previously defines, wherein during a period of time of 30 months from the diagnosis of said lung cancer
▪ if none of the 23 proteins of said set is expressed, the patient survival rate is about 78% or more,
▪ if none of the proteins of the set BP, or of the subsets BP1 or BP2, is expressed and one or two proteins of the set AP, or of subsets AP1, AP2 or AP3 is expressed, the patient survival rate is about 70%, and
▪ if at least one protein of the set BP, or of the subset BP1 or BP2, is expressed, the patient survival rate is about 13% to about 55%.

The present disclosure describes the use as previously defined, wherein during a period of time of 30 months from the diagnosis of said lung cancer
▪ if none of the 23 genes or proteins of said set, or antibodies, is expressed, the patient survival rate is about 78% or more,
▪ if
   o none of the genes or proteins of the set B or BP, or of subsets B1, BP1, B2 or BP2, or antibodies, is expressed and at least 3 genes or proteins of the set A or AP, or of subsets A1, AP1, A2, AP2, A3 or AP3, or antibodies, is expressed, or
   ∘ at least 1 gene or protein of the set B or BP, or of subsets B1, BP1, B2 or BP2, or antibody, is expressed and from none to 2 genes or proteins of the set A or AP, or of subsets A1, AP1, A2, AP2, A3 or AP3, or antibodies, is expressed, or
   ∘ at least 2 genes or proteins of the set B or BP, or of subsets B1, BP1, B2 or BP2, or antibodies, is expressed and no gene or protein of the set A or AP, or of subsets A1, AP1, A2, AP2, A3 or AP3, or antibody, is expressed,
   the patient survival rate is about 55%, and
▪ if
   ∘ one gene or protein of the genes or proteins, or antibodies, of the set B or BP, or of subsets B1, BP1, B2 or BP2, or antibody, is expressed and at least 3 genes or proteins of the set A or AP or of subsets A1, AP1, A2, AP2, A3 or AP3, or antibodies, are expressed, or
   ∘ at least 2 genes or proteins, of the set B or BP, or of subsets B1, BP1, B2 or BP2, or antibodies are expressed and at least 1 gene or protein of the set A or AP, or of subsets A1, AP1, A2, AP2, A3 or AP3, or antibody, are expressed
   the patient survival rate is about 13%.

In an advantageous embodiment, the invention relates to the use as previously defined, wherein during a period of time of 30 months from the diagnosis of said lung cancer
▪ if none of the 23 genes or proteins of said set is expressed, the patient survival rate is about 78% or more,
▪ if
   ∘ none of the genes or proteins of the set B or BP, or of subsets B1, BP1, B2 or BP2, is expressed and at least 3 genes or proteins of the set A or AP, or of subsets A1, AP1, A2, AP2, A3 or AP3, is expressed, or
   ∘ at least 1 gene or protein of the set B or BP, or of subsets B1, BP1, B2 or BP2, is expressed and from none to 2 genes or proteins of the set A or AP, or of subsets A1, AP1, A2, AP2, A3 or AP3, is expressed, or
   ∘ at least 2 genes or proteins of the set B or BP, or of subsets B1, BP1, B2 or BP2, is expressed and no gene or protein of the set A or AP, or of subsets A1, AP1, A2, AP2, A3 or AP3, is expressed,
   the patient survival rate is about 55%, and
▪ if
   ∘ one gene or protein of the genes or proteins of the set B or BP, or of subsets B1, BP1, B2 or BP2, is expressed and at least 3 genes or proteins of the set A or AP or of subsets A1, AP1, A2, AP2, A3 or AP3, are expressed, or
   ∘ at least 2 genes or proteins, of the set B or BP, or of subsets B1, BP1, B2 or BP2, are expressed and at least 1 gene or protein of the set A or AP, or of subsets A1, AP1, A2, AP2, A3 or AP3, are expressed
   the patient survival rate is about 13%.

In an advantageous embodiment, the invention relates to the use as previously defined, wherein during a period of time of 30 months from the diagnosis of said lung cancer
▪ if none of the 23 genes of said set is expressed, the patient survival rate is about 78% or more,
▪ if
   ∘ none of the genes of the set B, or of subsets B 1 or B2, is expressed and at least 3 genes of the set A, or of subsets A1, A2 or A3, is expressed, or
   ∘ at least 1 gene of the set B, or of subsets B1 or B2, is expressed and from none to 2 genes of the set A, or of subsets A1, A2 or A3, is expressed, or
   ∘ at least 2 genes of the set B, or of subsets B1 or B2, is expressed and no gene of the set A, or of subsets A1, A2, or A3, is expressed,
   the patient survival rate is about 55%, and
▪ if
   ∘ one gene of the genes of the set B, or of subsets B 1 or B2, is expressed and at least 3 genes of the set A, or of subsets A1, A2, or A3, are expressed, or
   ∘ at least 2 genes of the set B, or of subsets B1, or B2, are expressed and at least 1 gene of the set A, or of subsets A1, A2, or A3, are expressed
   the patient survival rate is about 13%.

In an advantageous embodiment, the invention relates to the use as previously defined, wherein during a period of time of 30 months from the diagnosis of said lung cancer
▪ if none of the 23 proteins of said set is expressed, the patient survival rate is about 78% or more,
▪ if
   ∘ none of the proteins of the set BP, or of subsets BP1 or BP2, is expressed and at least 3 proteins of the set AP, or of subsets AP1, AP2 or AP3, is expressed, or
   ∘ at least 1 protein of the set BP, or of subsets BP 1 or BP2, is expressed and from none to 2 proteins of the set AP, or of subsets AP1, AP2 or AP3, is expressed, or
   ∘ at least 2 proteins of the set BP, or of subsets BP1 or BP2, is expressed and no protein of the set AP, or of subsets AP1, AP2, or AP3, is expressed,
   the patient survival rate is about 55%, and
▪ if
   ∘ one protein of the proteins of the set BP, or of subsets BP1 or BP2, is expressed and at least 3 proteins of the set AP, or of subsets AP1, AP2, or AP3, are expressed, or
   ∘ at least 2 proteins of the set BP, or of subsets BP1, or BP2, are expressed and at least 1 protein of the set AP, or of subsets AP1, AP2, or AP3, are expressed
   the patient survival rate is about 13%.

The present disclosure describes the use as previously defined, wherein said prognosis method is such that:
▪ if none of the 23 genes or proteins of said set, or antibodies, is expressed, the patient survival rate is about 78% or more,
▪ if none of the genes or proteins of the set B or BP, or of subsets B1, BP1, B2 or BP2, or antibodies, is expressed and one or two genes or proteins of the set A or AP, or of subsets A1, AP1, A2, AP2, A3 or AP3, or antibodies, is expressed, the patient survival rate is about 70%,
▪ if
   ∘ none of the genes or proteins of the set B or BP, or of subsets B1, BP1, B2 or BP2, or antibodies, is expressed and at least 3 genes or proteins, of the set A or AP, or of subsets A1, AP1, A2, AP2, A3 or AP3, or antibodies, is expressed, or
   ∘ at least 1 gene or protein of the set B or BP, or of subsets B1, BP1, B2 or BP2, or antibody, is expressed and from none to 2 genes or proteins of the set A or AP, or of subsets A1, AP1, A2, AP2, A3 or AP3, or antibodies, is expressed, or
   ∘ at least 2 genes or proteins of the set B or BP, or of subsets B1, BP1, B2 or BP2, or antibodies, is expressed and no gene or protein of the set A or AP, or of subsets A1, AP1, A2, AP2, A3 or AP3, or antibody, is expressed,
   the patient survival rate is about 55%, and
▪ if
   ∘ one gene or protein of the genes or proteins, or antibodies, of the set B or BP, or of subsets B1, BP1, B2 or BP2, or antibody, is expressed and at least 3 genes or proteins of the set A or AP, or of subsets A1, AP1, A2, AP2, A3 or AP3, or antibodies, are expressed, or
   ∘ at least 2 genes or proteins of the set B or BP, or of subsets B1, BP1, B2 or BP2, or antibodies, are expressed and at least 1 gene or protein of the set A or AP, or of subsets A1, AP1, A2, AP2, A3 or AP3, or antibody, is expressed,
   the patient survival rate is about 13%.

In an advantageous embodiment, the invention relates to the use as previously defined, wherein said prognosis method is such that:
▪ if none of the 23 genes or proteins of said set is expressed, the patient survival rate is about 78% or more,
▪ if none of the genes or proteins of the set B or BP, or of subsets B1, BP1, B2 or BP2, is expressed and one or two genes or proteins of the set A or AP, or of subsets A1, AP1, A2, AP2, A3 or AP3, is expressed, the patient survival rate is about 70%,
▪ if
   ∘ none of the genes or proteins of the set B or BP, or of subsets B1, BP1, B2 or BP2, is expressed and at least 3 genes or proteins, of the set A or AP, or of subsets A1, AP1, A2, AP2, A3 or AP3, is expressed, or
   ∘ at least 1 gene or protein of the set B or BP, or of subsets B1, BP1, B2 or BP2, is expressed and from none to 2 genes or proteins of the set A or AP, or of subsets A1, AP1, A2, AP2, A3 or AP3, is expressed, or
   ∘ at least 2 genes or proteins of the set B or BP, or of subsets B1, BP1, B2 or BP2, is expressed and no gene or protein of the set A or AP, or of subsets A1, AP1, A2, AP2, A3 or AP3, is expressed,
   the patient survival rate is about 55%, and
▪ if
   ∘ one gene or protein of the genes or proteins of the set B or BP, or of subsets B1, BP1, B2 or BP2, is expressed and at least 3 genes or proteins of the set A or AP, or of subsets A1, AP1, A2, AP2, A3 or AP3, are expressed, or
   ∘ at least 2 genes or proteins of the set B or BP, or of subsets B1, BP1, B2 or BP2, are expressed and at least 1 gene or protein of the set A or AP, or of subsets A1, AP1, A2, AP2, A3 or AP3, is expressed,
the patient survival rate is about 13%.

In an advantageous embodiment, the invention relates to the use as previously defined, wherein said prognosis method is such that:
▪ if none of the 23 genes of said set is expressed, the patient survival rate is about 78% or more,
▪ if none of the genes of the set B, or of subsets B 1 or B2, is expressed and one or two genes of the set A, or of subsets A1, A2 or A3, is expressed, the patient survival rate is about 70%,
▪ if
   ∘ none of the genes of the set B, or of subsets B1 or B2, is expressed and at least 3 genes of the set A, or of subsets A1, A2, or A3, is expressed, or
   ∘ at least 1 gene of the set B, or of subsets B 1 or B2, is expressed and from none to 2 genes of the set A, or of subsets A1, A2, or A3 is expressed, or
   ∘ at least 2 genes of the set B, or of subsets B1, or B2, is expressed and no gene of the set A, or of subsets A1 A2, or A3, is expressed,
   the patient survival rate is about 55%, and
▪ if
   ∘ one gene of the genes of the set B, or of subsets B1 or B2, is expressed and at least 3 genes of the set A, or of subsets A1, A2, or A3, are expressed, or
   ∘ at least 2 genes of the set B, or of subsets B 1 or B2, are expressed and at least 1 gene of the set A, or of subsets A1, A2, A3, is expressed
the patient survival rate is about 13%.

In an advantageous embodiment, the invention relates to the use as previously defined, wherein said prognosis method is such that:
▪ if none of the 23 proteins of said set is expressed, the patient survival rate is about 78% or more,
▪ if none of the proteins of the set BP, or of subsets BP1 or BP2, is expressed and one or two proteins of the set AP, or of subsets AP1, AP2, or AP3, is expressed, the patient survival rate is about 70%,
▪ if
   ∘ none of the proteins of the set BP, or of subsets BP1 or BP2, is expressed and at least 3 proteins of the set AP, or of subsets AP1, AP2 or AP3, is expressed, or
   ∘ at least 1 protein of the set BP, or of subsets BP1 or BP2, is expressed and from none to 2 proteins, of the set AP, or of subsets AP1, AP2 or AP3 is expressed, or
   ∘ at least 2 proteins of the set BP, or of subsets BP1 or BP2, is expressed and no protein, of the set AP, or of subsets AP1, AP2 or AP3, is expressed,
   the patient survival rate is about 55%, and
▪ if
   ∘ one protein of the proteins of the set BP, or of subsets BP1 or BP2, is expressed and at least 3 proteins of the set AP, or of subsets AP1, AP2 or AP3, are expressed, or
   ∘ at least 2 proteins of the set BP, or of subsets BP 1 or BP2, are expressed and at least 1 protein of the set AP, or of subsets AP1, AP2 or AP3, is expressed
   the patient survival rate is about 13%.

In one another advantageous embodiment, the invention relates to the use as defined above, wherein said lung tumor has been previously histologically classified.

In one another advantageous embodiment, the invention relates to the use as defined above, wherein said histologically classified tumor belongs to the set consisting of :
ADK, SQC, BAS, and LCNE, wherein ADK corresponds to adenocarcinoma, SQC corresponds to Squamous cell carcinoma, BAS corresponds to Basaloid tumours and LCNE corresponds to Large Cell Neuroendocrine.

The present disclosure also describes a prognosis method, preferably *in vitro*, of the survival rate of a patient afflicted by a lung tumour, from a biological sample containing said lung tumor, at a time from 30 to 120 months after the diagnosis of said lung cancer, as defined above,
said method comprising a step of measuring, in said biological sample, the expression of
- at least 2 genes chosen among a set of 23 genes comprising or consisting of the nucleic acid sequences SEQ ID NO 1 to 23,
- or fragments of said least 2 genes chosen among a set of 23 genes comprising or consisting of the nucleic acid sequences SEQ ID NO 1 to 23,
- or complementary sequences of said least 2 genes chosen among a set of 23 genes comprising or consisting of the nucleic acid sequences SEQ ID NO 1 to 23,
- or sequences having at least 80% homology with said genes or fragment thereof,
- or proteins coded by said least 2 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
   said
   ◆ at least 2 genes being such that
- at least one gene belongs to a first set A of 7 genes comprising or consisting of the nucleic acid sequences SEQ ID NO: 1-7
- at least one gene belongs to a second set B of 16 genes comprising or consisting of the nucleic acid sequences SEQ ID NO: 8-23,
   ◆ at least 2 proteins being such that
- at least one protein belongs to a first set AP of 7 proteins comprising or consisting of the amino acid sequences SEQ ID NO: 24-30,
- at least one protein belongs to a second set BP of 16 proteins comprising or consisting of the amino acid sequences SEQ ID NO: 31-46,
   ◆ at least 2 antibodies directed against said 2 proteins being such that
- at least one antibody specifically recognises one protein that belongs to a first set AP of 7 proteins comprising or consisting of the amino acid sequences SEQ ID NO: 24-30,
- at least one antibody specifically recognises one protein that belongs to a second set BP of 16 proteins comprising or consisting of the amino acid sequences SEQ ID NO: 31-46,
said method being such that:
either
▪ if none of the 23 genes of said set is expressed, the patient survival rate during a period of time from 30 to 120 months after the diagnosis of said lung cancer is from about 59% to about 78% or more, and
▪ if at least one gene of at least one set A or B is expressed, the patient survival rate during a period of time from 30 to 120 months after the diagnosis of said lung cancer is about from about 3% to about 70%,
or
▪ if none of the 23 proteins of said set is expressed, the patient survival rate during a period of time from 30 to 120 months after the diagnosis of said lung cancer is from about 59% to about 78% or more, and
▪ if at least one protein of at least one set AP or BP is expressed, the patient survival rate during a period of time from 30 to 120 months after the diagnosis of said lung cancer is about from about 3% to about 70%,
or
▪ if none of the antibodies directed against said 23 proteins of said set is expressed, the patient survival rate during a period of time from 30 to 120 months after the diagnosis of said lung cancer is from about 59% to about 78% or more, and
▪ if at least one antibody directed against one protein of at least one set AP or BP is expressed, the patient survival rate during a period of time from 30 to 120 months after the diagnosis of said lung cancer is about from about 3% to about 70%.

The invention also relates to an *in vitro* prognosis method, of the survival rate of a patient afflicted by a lung tumour, from a lung cancer sample, at a time from 30 to 120 months after the diagnosis of said lung cancer, as defined above, said method comprising a step of measuring, in said lung cancer sample, the expression of
- at least 2 genes chosen among a set of 23 genes comprising or consisting of the nucleic acid sequences SEQ ID NO 1 to 23,
- or complementary sequences of said least 2 genes chosen among a set of 23 genes comprising or consisting of the nucleic acid sequences SEQ ID NO 1 to 23,
- or sequences having at least 80% homology with said genes,
- or proteins coded by said least 2 genes chosen among a set of 23 genes comprising or consisting of the nucleic acid sequences SEQ ID NO 1 to 23, said proteins comprising or consisting in amino acid sequences SEQ ID NO 24 to 46
   said
   ◆ at least 2 genes being such that
- at least one gene belongs to a first set A of 7 genes comprising or consisting of the nucleic acid sequences SEQ ID NO: 1-7
- at least one gene belongs to a second set B of 16 genes comprising or consisting of the nucleic acid sequences SEQ ID NO: 8-23,
   ◆ at least 2 proteins being such that
- at least one protein belongs to a first set AP of 7 proteins comprising or consisting of the amino acid sequences SEQ ID NO: 24-30,
- at least one protein belongs to a second set BP of 16 proteins comprising or consisting of the amino acid sequences SEQ ID NO: 31-46,
said method being such that:
either
▪ if none of the 23 genes of said set is expressed, the patient survival rate during a period of time from 30 to 120 months after the diagnosis of said lung cancer is from about 59% to about 78% or more, and
▪ if at least one gene of at least one set A or B is expressed, the patient survival rate during a period of time from 30 to 120 months after the diagnosis of said lung cancer is about from about 3% to about 70%,
or
▪ if none of the 23 proteins of said set is expressed, the patient survival rate during a period of time from 30 to 120 months after the diagnosis of said lung cancer is from about 59% to about 78% or more, and
▪ if at least one protein of at least one set AP or BP is expressed, the patient survival rate during a period of time from 30 to 120 months after the diagnosis of said lung cancer is about from about 3% to about 70%.

The present disclosure also describes a prognosis method, as defined above,
said method comprising a step of measuring, in said biological sample, the expression of
- at least 2 genes chosen among a set of 23 genes comprising or consisting of the nucleic acid sequences SEQ ID NO 1 to 23,
- or fragments of said genes
- or complementary sequences of said genes
- or sequences having at least 80% homology with said genes or fragment thereof,
- or protein coded by said genes,
- or fragments of said proteins,
- or antibodies directed against said proteins,
   said at least 2 genes being such that
- at least one gene belongs to a first set A of 7 genes, or of a subset A1, A2 or A3 as defined above, said set comprising or consisting of the nucleic acid sequences SEQ ID NO: 1-7
- at least one gene belongs to a second set B of 16 genes, or of a subset B1 or B2 as defined above, said set comprising or consisting of the nucleic acid sequences SEQ ID NO: 8-23
said prognosis method being such that
▪ if none of the 23 genes of said set is expressed, the patient survival rate is from about 59% to about 78%, or more, and
▪ if at least one gene of said set A or B, or of subsets A1, A2, A3, B1 or B2 is expressed, the patient survival rate from about 3% to about 70%.

In one advantageous embodiment, the invention relates to a prognosis method, as defined above,
said method comprising a step of measuring, in said lung cancer sample, the expression of
- at least 2 genes chosen among a set of 23 genes comprising or consisting of the nucleic acid sequences SEQ ID NO 1 to 23,
- or complementary sequences of said genes,
- or sequences having at least 80% homology with said genes,
- or protein coded by said genes,
   said at least 2 genes being such that
- at least one gene belongs to a first set A of 7 genes, or of a subset A1, A2 or A3 as defined above, said set comprising or consisting of the nucleic acid sequences SEQ ID NO: 1-7
- at least one gene belongs to a second set B of 16 genes, or of a subset B1 or B2 as defined above, said set comprising or consisting of the nucleic acid sequences SEQ ID NO: 8-23
said prognosis method being such that
▪ if none of the 23 genes of said set is expressed, the patient survival rate is from about 59% to about 78%, or more, and
▪ if at least one gene of said set A or B, or of subsets A1, A2, A3, B1 or B2 is expressed, the patient survival rate from about 3% to about 70%.

In another advantageous embodiment, the invention relates to a prognosis method previously defined, wherein the step of measuring is carried out by using a technique chosen among the set consisting of:
- Quantitative PCR,
- DNA CHIP, and
- Northern blot.

In another advantageous embodiment, the invention relates to a prognosis method as previously defined, wherein the step of measuring is carried out by using nucleic acid molecules consisting of from 15 to 100 nucleotides molecules being complementary to said at least 2 genes.

In one advantageous embodiment, the invention relates to a prognosis method as defined above, wherein the step of measuring is carried out by DNA CHIP using
- at least one nucleic acid probe comprising or being constituted by the nucleic acid sequences SEQ ID NO: 47 to SEQ ID NO: 53, and
- at least one nucleic acid probe comprising or being constituted by the nucleic acid sequences SEQ ID NO: 54 to SEQ ID NO: 69.

In another advantageous embodiment, the invention relates to the method as defined above, wherein the step of measuring is carried out by DNA CHIP using at least 2, preferably at least 3 nucleic acid probes as defined above.

An advantageous embodiment of the invention relates to the above method wherein the nucleic acid probes comprising or being constituted by the nucleic acid sequences SEQ ID NO: 47 to 69 are used, together.

In the invention the correspondence between the genes and the nucleic acid probes are as follows: SEQ ID NO:1 is able to be detected by the nucleic acid probe comprising or being constituted by the nucleic acid sequence SEQ ID NO: 47, SEQ ID NO:2 is able to be detected by the nucleic acid probe comprising or being constituted by the nucleic acid sequence SEQ ID NO: 48, SEQ ID NO:3 is able to be detected by the nucleic acid probe comprising or being constituted by the nucleic acid sequence SEQ ID NO: 49, SEQ ID NO:4 is able to be detected by the nucleic acid probe comprising or being constituted by the nucleic acid sequence SEQ ID NO: 50, SEQ ID NO:5 is able to be detected by the nucleic acid probe comprising or being constituted by the nucleic acid sequence SEQ ID NO: 51, SEQ ID NO:6 is able to be detected by the nucleic acid probe comprising or being constituted by the nucleic acid sequence SEQ ID NO: 52, SEQ ID NO:7 is able to be detected by the nucleic acid probe comprising or being constituted by the nucleic acid sequence SEQ ID NO: 53, SEQ ID NO:8 is able to be detected by the nucleic acid probe comprising or being constituted by the nucleic acid sequence SEQ ID NO: 54, SEQ ID NO:9 is able to be detected by the nucleic acid probe comprising or being constituted by the nucleic acid sequence SEQ ID NO: 55, SEQ ID NO:10 is able to be detected by the nucleic acid probe comprising or being constituted by the nucleic acid sequence SEQ ID NO: 56, SEQ ID NO: 11 is able to be detected by the nucleic acid probe comprising or being constituted by the nucleic acid sequence SEQ ID NO: 57, SEQ ID NO:12 is able to be detected by the nucleic acid probe comprising or being constituted by the nucleic acid sequence SEQ ID NO: 58, SEQ ID NO: 13 is able to be detected by the nucleic acid probe comprising or being constituted by the nucleic acid sequence SEQ ID NO: 59, SEQ ID NO:14 is able to be detected by the nucleic acid probe comprising or being constituted by the nucleic acid sequence SEQ ID NO: 60, SEQ ID NO:15 is able to be detected by the nucleic acid probe comprising or being constituted by the nucleic acid sequence SEQ ID NO: 61, SEQ ID NO:16 is able to be detected by the nucleic acid probe comprising or being constituted by the nucleic acid sequence SEQ ID NO: 62, SEQ ID NO: 17 is able to be detected by the nucleic acid probe comprising or being constituted by the nucleic acid sequence SEQ ID NO: 63, SEQ ID NO: 18 is able to be detected by the nucleic acid probe comprising or being constituted by the nucleic acid sequence SEQ ID NO: 64, SEQ ID NO: 19 is able to be detected by the nucleic acid probe comprising or being constituted by the nucleic acid sequence SEQ ID NO: 65, SEQ ID NO:20 is able to be detected by the nucleic acid probe comprising or being constituted by the nucleic acid sequence SEQ ID NO: 66, SEQ ID NO:21 is able to be detected by the nucleic acid probe comprising or being constituted by the nucleic acid sequence SEQ ID NO: 67, SEQ ID NO:22 is able to be detected by the nucleic acid probe comprising or being constituted by the nucleic acid sequence SEQ ID NO: 68, SEQ ID NO:23 is able to be detected by the nucleic acid probe comprising or being constituted by the nucleic acid sequence SEQ ID NO: 69.

The present disclosure describes a prognosis method, preferably *in vitro*, of the survival rate of a patient afflicted by a lung tumour, from a biological sample containing said lung tumor, at a time from 30 to 120 months after the diagnosis of said lung cancer,
said method comprising a step of measuring, in said biological sample, the expression of
- at least 2 proteins chosen among a set of 23 proteins coded by 23 genes comprising or consisting of the nucleic acid sequences SEQ ID NO 1 to 23,
- or fragments of said proteins,
   said at least 2 proteins being such that
- at least one protein belongs to a first set AP of 7 proteins, each 7 proteins being coded by one of 7 genes comprising or consisting of the nucleic acid sequences SEQ ID NO: 1-7
- at least one protein belongs to a second set BP of 16 proteins, each 16 proteins being coded by one of 16 genes comprising or consisting of the nucleic acid sequences SEQ ID NO: 8-23
said prognosis method being such that
▪ if none of the 23 proteins is expressed, the patient survival rate is from about 59% to about 78%, or more, and
▪ if at least one protein of said set AP or BP is expressed, the patient survival rate from about 3% to about 70%.

In another advantageous embodiment, the invention relates to an *in vitro* prognosis method of the survival rate of a patient afflicted by a lung tumour, from a lung cancer sample, at a time from 30 to 120 months after the diagnosis of said lung cancer, said method comprising a step of measuring, in said lung cancer sample, the expression of
at least 2 proteins chosen among a set of 23 proteins coded by 23 genes comprising or consisting of the nucleic acid sequences SEQ ID NO 1 to 23, said at least 2 proteins being such that
- at least one protein belongs to a first set AP of 7 proteins, each 7 proteins being coded by one of 7 genes comprising or consisting of the nucleic acid sequences SEQ ID NO: 1-7
- at least one protein belongs to a second set BP of 16 proteins, each 16 proteins being coded by one of 16 genes comprising or consisting of the nucleic acid sequences SEQ ID NO: 8-23
said prognosis method being such that
▪ if none of the 23 proteins is expressed, the patient survival rate is from about 59% to about 78%, or more, and
▪ if at least one protein of said set AP or BP is expressed, the patient survival rate is from about 3% to about 70%.

In one another advantageous embodiment, the invention relates to a prognosis method, wherein the step of measuring is carried out by using a technique chosen among the set consisting of:
- western Blot,
- ELISA,
- Immunofluorescence, and
- Immunohistochemistry.

In one another advantageous embodiment, the invention relates to a prognosis method, wherein the step of measuring is carried out by using antibodies directed against said at least 2 proteins coded by said at least two genes.

In one another advantageous embodiment, the invention relates to a prognosis method, further comprising a step of comparison of said measured expression to the expression in at least one control sample.

According to this disclosure, the above mentioned gene, proteins or antibody expression can be compared with the expression level of the same genes, proteins and antibodies measured in
- a control sample corresponding to a sample originating from an healthy individual, and/or
- a positive sample corresponding to a sample of an individual expressing the above mentioned gene, protein, or antibodies.

According to the invention, the above mentioned gene or protein expression can be compared with the expression level of the same genes or proteins measured in
- a control sample corresponding to a sample originating from an healthy individual, and/or
- a positive sample corresponding to a sample of an individual expressing the above mentioned gene or protein.

The invention also concerns a kit comprising a DNA CHIP comprising at least the nucleic acid probes comprising or being constituted by the nucleic acid sequences SEQ ID NO : 47 to 69.

The invention is illustrated by the following 40 figures and examples.

### Figures

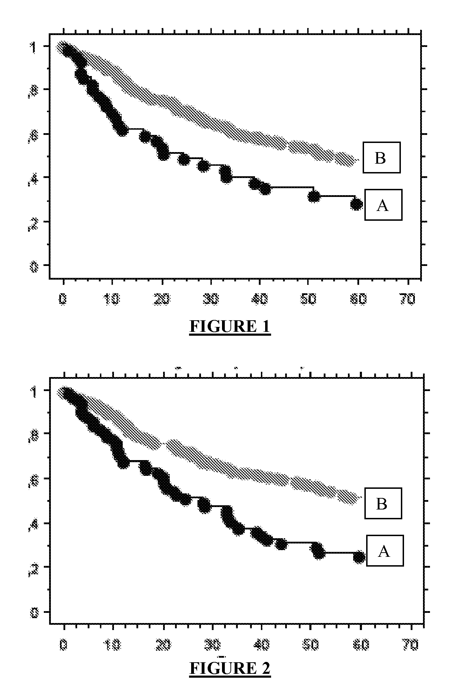

**Figure 1** represents the cumulative survival rate (Kaplan Mayer curve) over 60 months of patients expressing (A) or not (B) the gene SEQ ID NO: 4 (gene 1161). Y-axis represents cumulative survival in %, X-axis represents time in months.
**Figure 2** represents the cumulative survival rate (Kaplan Mayer curve) over 60 months of patients expressing (A) or not (B) the gene SEQ ID NO: 4 (gene 1161) and or the gene SEQ ID NO: 6 (gene 391). Y- axis represents cumulative survival in %, X-axis represents time in months.

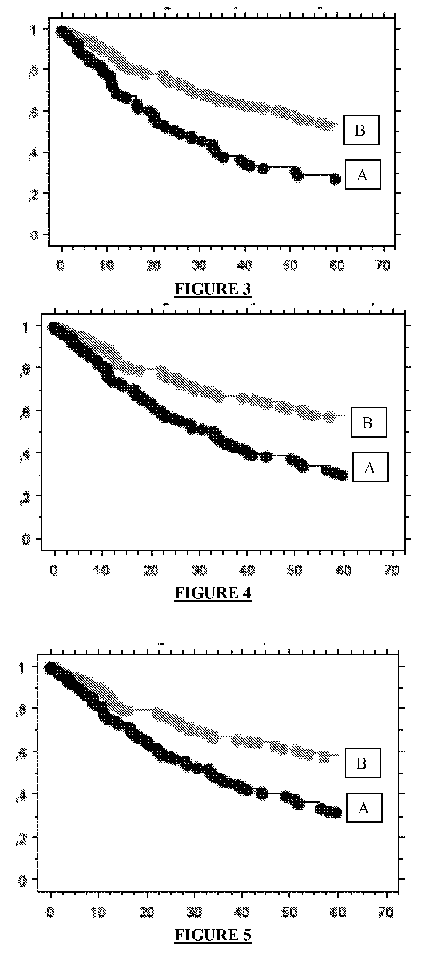

**Figure 3** represents the cumulative survival rate (Kaplan Mayer curve) over 60 months of patients expressing (A) or not (B) the gene SEQ ID NO: 4 (gene 1161) and/or the gene SEQ ID NO: 6 (gene 391) and/or the gene SEQ ID NO: 2 (gene 35). Y- axis represents cumulative survival in %, X-axis represents time in months.
**Figure 4** represents the cumulative survival rate (Kaplan Mayer curve) over 60 months of patients expressing (A) or not (B) at least one of the following genes: SEQ ID NO: 4 (gene 1161), SEQ ID NO: 6 (gene 391), SEQ ID NO: 2 (gene 35) and SEQ ID NO: 1 (gene 442). Y- axis represents cumulative survival in %, X-axis represents time in months.
**Figure 5** represents the cumulative survival rate (Kaplan Mayer curve) over 60 months of patients expressing (A) or not (B) at least one of the following genes: SEQ ID NO: 4 (gene 1161), SEQ ID NO: 6 (gene 391), SEQ ID NO: 2 (gene 35), SEQ ID NO: 1 (gene 442) and SEQ ID NO 5 (gene 102). Y- axis represents cumulative survival in %, X-axis represents time in months.

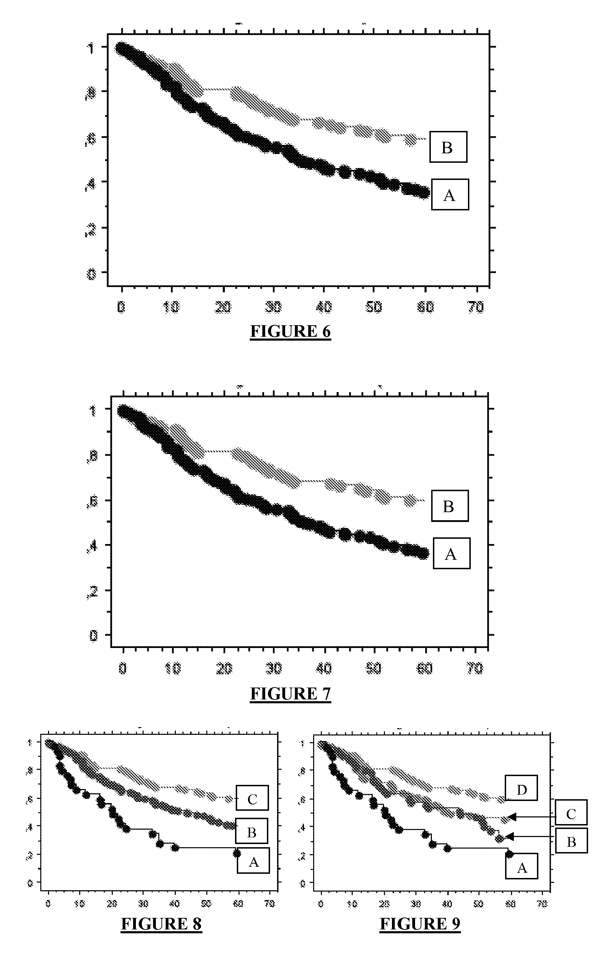

**Figure 6** represents the cumulative survival rate (Kaplan Mayer curve) over 60 months of patients expressing (A) or not (B) at least one of the following genes: SEQ ID NO: 4 (gene 1161), SEQ ID NO: 6 (gene 391), SEQ ID NO: 2 (gene 35), SEQ ID NO: 1 (gene 442), SEQ ID NO 5 (gene 102) and SEQ ID NO:7 (gene 390). Y- axis represents cumulative survival in %, X-axis represents time in months.
**Figure 7** represents the cumulative survival rate (Kaplan Mayer curve) over 60 months of patients expressing (A) or not (B) at least one of the genes: SEQ ID NO: 1 to 7. Y- axis represents cumulative survival in %, X-axis represents time in months.
**Figure 8** represents the cumulative survival rate (Kaplan Mayer curve) over 60 months of patients expressing one or two (B), or three or more (A) or no (C) genes: SEQ ID NO: 1 to 7. Y- axis represents cumulative survival in %, X-axis represents time in months.
**Figure 9** represents the cumulative survival rate (Kaplan Mayer curve) over 60 months of patients expressing one (C) or two (B), or three or more (A) or no (D) genes: SEQ ID NO: 1 to 7. Y- axis represents cumulative survival in %, X-axis represents time in months.

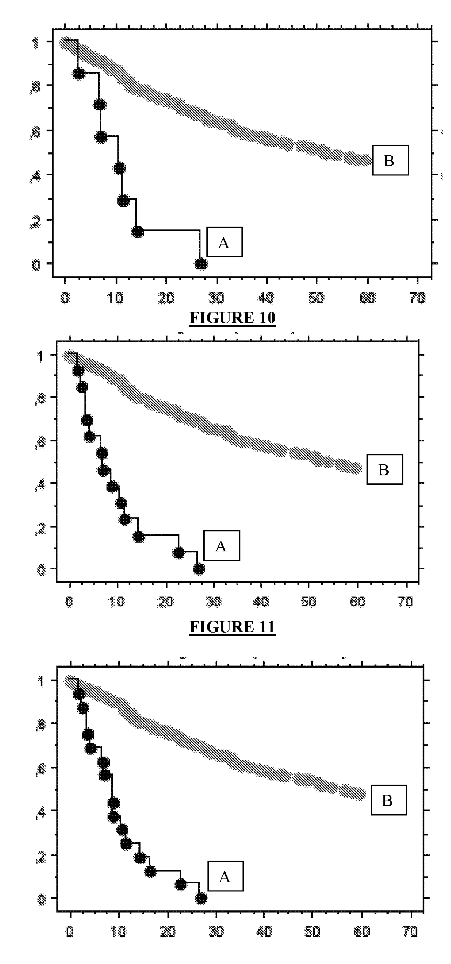

**Figure 10** represents the cumulative survival rate (Kaplan Mayer curve) over 60 months of patients expressing (A) or not (B) the gene SEQ ID NO: 16 (gene 125). Y-axis represents cumulative survival in %, X-axis represents time in months.
**Figure 11** represents the cumulative survival rate (Kaplan Mayer curve) over 60 months of patients expressing (A) or not (B) at least one of the following genes: SEQ ID NO: 16 (gene 125) and SEQ ID NO: 22 (gene 117). Y- axis represents cumulative survival in %, X-axis represents time in months.

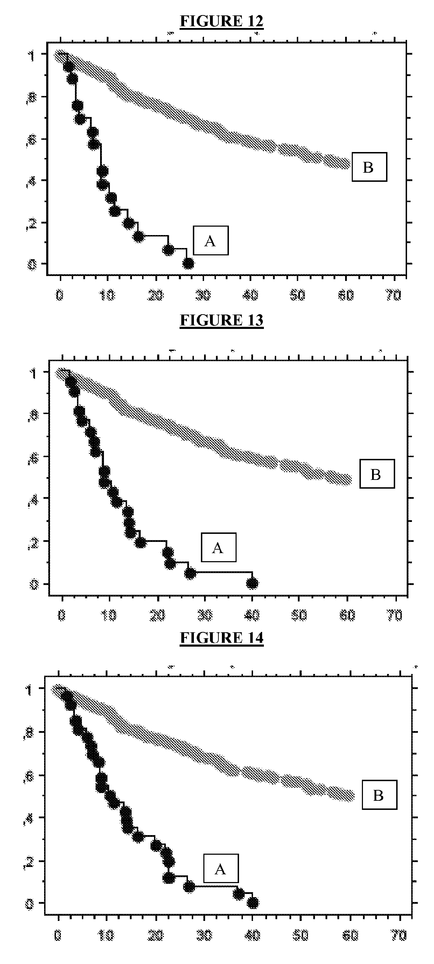

**Figure 12** represents the cumulative survival rate (Kaplan Mayer curve) over 60 months of patients expressing (A) or not (B) at least one of the following genes: SEQ ID NO: 16 (gene 125), SEQ ID NO: 22 (gene 117) and SEQ ID NO:19 (766). Y- axis represents cumulative survival in %, X-axis represents time in months.
**Figure 13** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766) and SEQ ID NO: 17(gene 144). Y- axis represents cumulative survival in %, X-axis represents time in months.
**Figure 14** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144) and SEQ ID NO: 12 (gene 108). Y- axis represents cumulative survival in %, X-axis represents time in months.

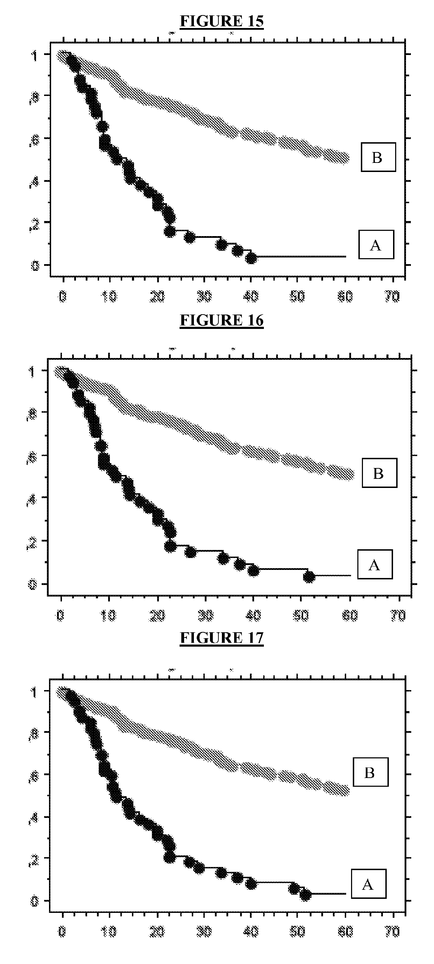

**Figure 15** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108) and SEQ ID NO: 8 (gene 222). Y-axis represents cumulative survival in %, X-axis represents time in months.
**Figure 16** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222) and SEQ ID NO: 17 (gene 72). Y- axis represents cumulative survival in %, X-axis represents time in months.
**Figure 17** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72) and SEQ ID NO: 10 (gene 1165). Y- axis represents cumulative survival in %, X-axis represents time in months.

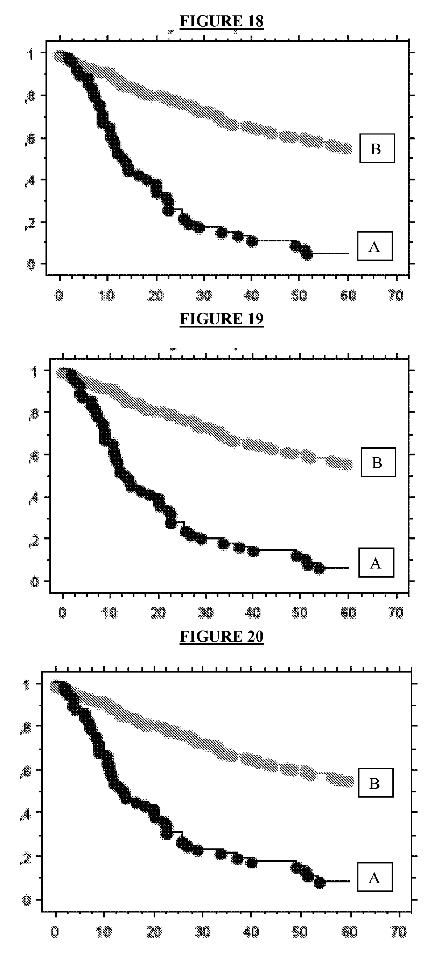

**Figure 18** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165) and SEQ ID NO: 21 (gene 487). Y-axis represents cumulative survival in %, X-axis represents time in months.
**Figure 19** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487) and SEQ ID NO: 9(gene 1261). Y- axis represents cumulative survival in %, X-axis represents time in months.
**Figure 20** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261) and SEQ ID N NO: 13 (gene 205). Y- axis represents cumulative survival in %, X-axis represents time in months.

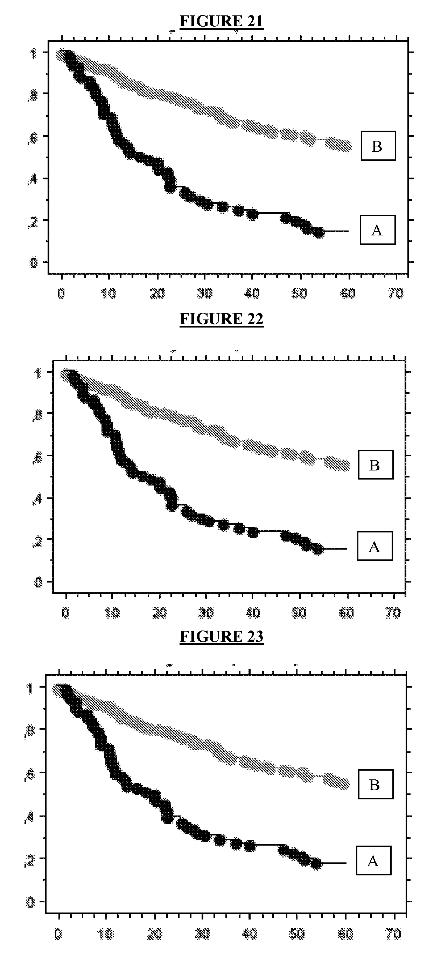

**Figure 21** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261), SEQ ID N NO: 13 (gene 205) and SEQ ID NO: 18 (gene 437). Y-axis represents cumulative survival in %, X-axis represents time in months.
**Figure 22** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261), SEQ ID N NO: 13 (gene 205), SEQ ID NO: 18 (gene 437) and SEQ ID NO:15 (gene 1328). Y- axis represents cumulative survival in %, X-axis represents time in months.
**Figure 23** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261), SEQ ID N NO: 13 (gene 205), SEQ ID NO: 18 (gene 437), SEQ ID NO:15 (gene 1328) and SEQ ID NO: 14 (gene 1188). Y- axis represents cumulative survival in %, X-axis represents time in months.

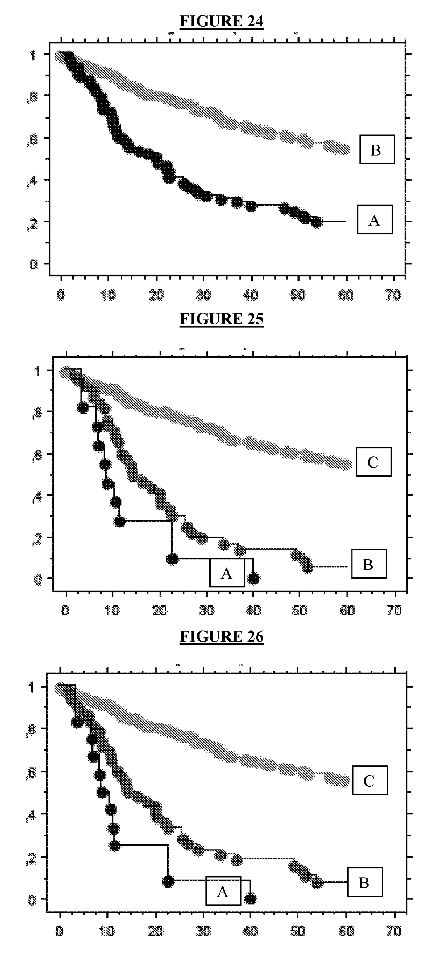

**Figure 24** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261), SEQ ID N NO: 13 (gene 205), SEQ ID NO: 18 (gene 437), SEQ ID NO:15 (gene 1328), SEQ ID NO: 14 (gene 1188) and SEQ ID NO: 20 (gene 436). Y- axis represents cumulative survival in %, X-axis represents time in months.
**Figure 25** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261), SEQ ID N NO: 13 (gene 205), SEQ ID NO: 18 (gene 437), SEQ ID NO:15 (gene 1328), SEQ ID NO: 14 (gene 1188), SEQ ID NO: 20 (gene 436) and SEQ ID NO: 23 (gene 135). Y- axis represents cumulative survival in %, X-axis represents time in months.
**Figure 26** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487) and SEQ ID NO: 9 (gene 1261). Y- axis represents cumulative survival in %, X-axis represents time in months.

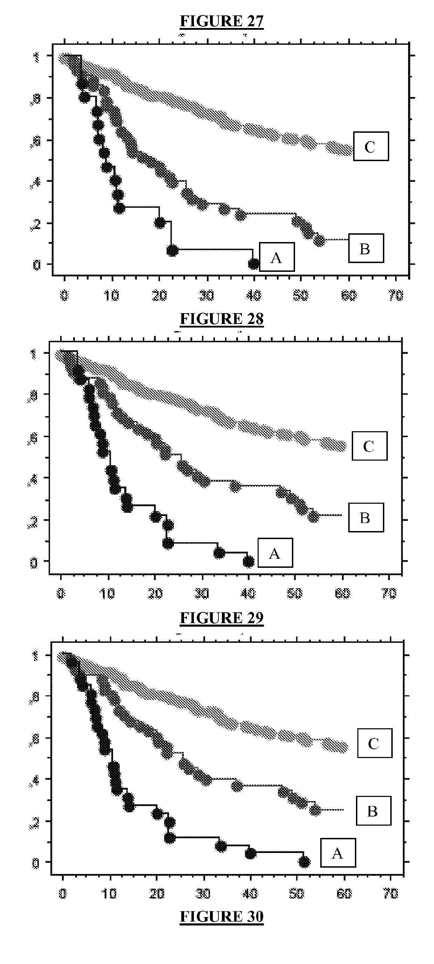

**Figure 27** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261) and SEQ ID N NO: 13 (gene 205). Y- axis represents cumulative survival in %, X-axis represents time in months.
**Figure 28** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205) and SEQ ID NO: 18 (gene 437). Y- axis represents cumulative survival in %, X-axis represents time in months.
**Figure 29** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437) and SEQ ID NO: 15 (gene 1328). Y- axis represents cumulative survival in %, X-axis represents time in months.
**Figure 30** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437), SEQ ID NO:15 (gene 1328) and SEQ ID NO: 14 (gene 1188). Y- axis represents cumulative survival in %, X-axis represents time in months.

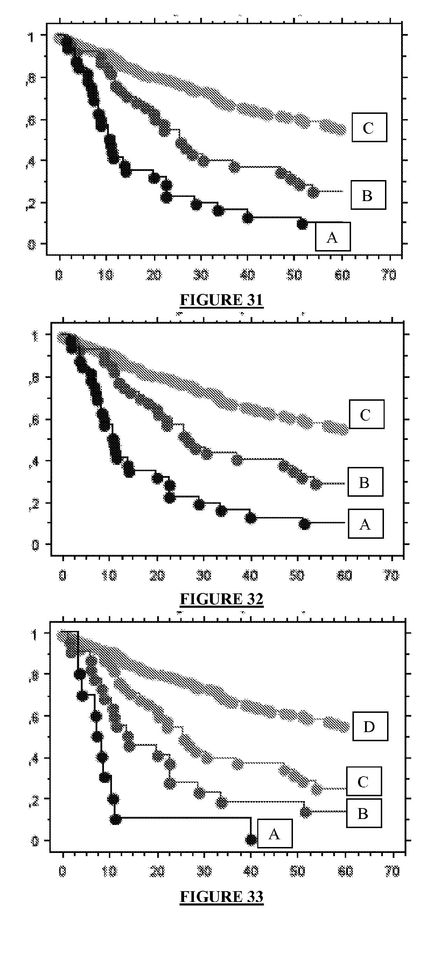

**Figure 31** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437), SEQ ID NO:15 (gene 1328), SEQ ID NO: 14 (gene 1188) and SEQ ID NO: 20 (gene 436). Y- axis represents cumulative survival in %, X-axis represents time in months.
**Figure 32** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437), SEQ ID NO:15 (gene 1328), SEQ ID NO: 14 (gene 1188), SEQ ID NO: 20 (gene 436) and SEQ ID NO: 23 (gene 135). Y- axis represents cumulative survival in %, X-axis represents time in months.
**Figure 33** represents the cumulative survival rate (Kaplan Mayer curve) over 60 months of patients expressing one (D), or two (B) or three or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437), SEQ ID NO:15 (gene 1328), SEQ ID NO: 14 (gene 1188) and SEQ ID NO: 20 (gene 436). Y- axis represents cumulative survival in %, X-axis represents time in months.

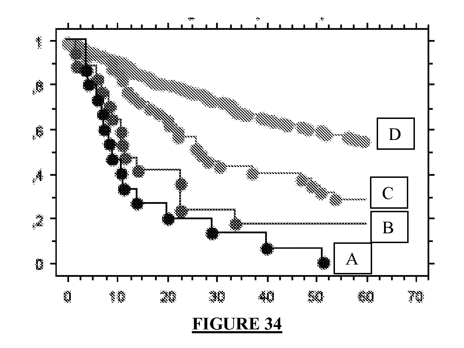

**Figure 34** represents the cumulative survival rate (Kaplan Mayer curve) over 60 months of patients expressing one (C), or two (B) or three or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437), SEQ ID NO:15 (gene 1328), SEQ ID NO: 14 (gene 1188), SEQ ID NO: 20 (gene 436) and SEQ ID NO: 23 (gene 135). Y- axis represents cumulative survival in %, X-axis represents time in months.

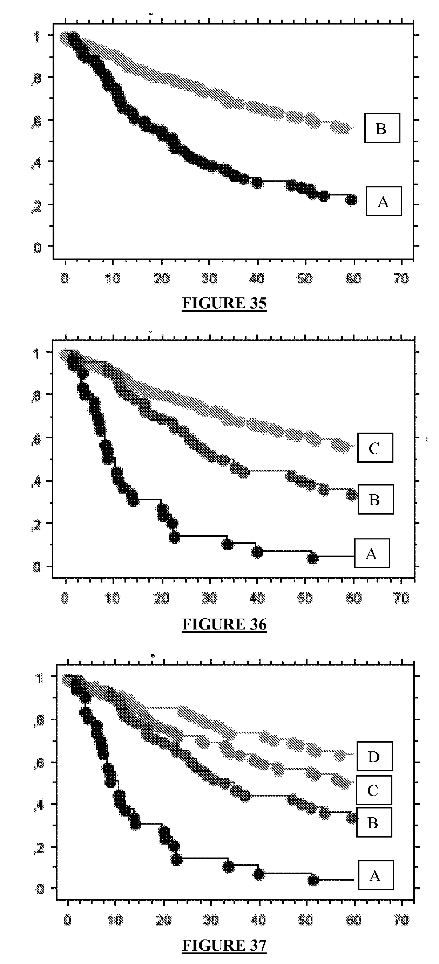

**Figure 35** represents the cumulative survival rate (Kaplan Mayer curve) over 60 months of patients expressing no (B), or at least one gene SEQ ID NO:1-23. Y- axis represents cumulative survival in %, X-axis represents time in months.
**Figure 36** represents the cumulative survival rate (Kaplan Mayer curve) over 60 months of patients expressing no (C), or none of the genes SEQ ID NO: 8-23 and at least 3 genes SEQ ID NO: 1-7 are expressed expressed, or at least 1 gene SEQ ID NO: 8-23 is expressed and from none to 2 genes SEQ ID NO: 1-7 is expressed, or at least 2 genes SEQ ID NO: 8-23 are expressed and no gene SEQ ID NO: 1-7 is expressed (B),
   or one gene SEQ ID NO: 8-23 is expressed and at least 3 genes SEQ ID NO: 1-7 are expressed, or at least 2 genes SEQ ID NO: 8-23 are expressed and at least 1 gene SEQ ID NO: 1-7 is expressed (A).
   Y- axis represents cumulative survival in %, X-axis represents time in months.
**Figure 37** represents the cumulative survival rate (Kaplan Mayer curve) over 60 months of patients expressing no (D),
   or if none of the genes SEQ ID NO: 8-23 is expressed and one or two genes SEQ ID NO: 1-7 is expressed (C)
   or none of the genes SEQ ID NO: 8-23 and at least 3 genes SEQ ID NO: 1-7 are expressed, or at least 1 gene SEQ ID NO: 8-23 is expressed and from none to 2 genes SEQ ID NO: 1-7 is expressed, or at least 2 genes SEQ ID NO: 8-23 are expressed and no gene SEQ ID NO: 1-7 is expressed (B),
   or one gene SEQ ID NO: 8-23 is expressed and at least 3 genes SEQ ID NO: 1-7 are expressed, or at least 2 genes SEQ ID NO: 8-23 are expressed and at least 1 gene SEQ ID NO: 1-7 is expressed (A).
   Y- axis represents cumulative survival in %, X-axis represents time in months.
**Figure 38** represents the cumulative survival rate (Kaplan Mayer curve) over 60 months of patients expressing no genes SEQ ID NO:1-23 (A) or expressing LDHC gene (B).
**Figure 39** represents the cumulative survival rate (Kaplan Mayer curve) over 60 months of patients expressing no genes SEQ ID NO:1-23 (A) or expressing MAGEA5 gene (B).
**Figure 40** represents the cumulative survival rate (Kaplan Mayer curve) over 60 months of patients expressing no genes SEQ ID NO:1-23 (A) or expressing MAGEB18 gene (B).

### EXAMPLES:

The invention describes a group of **23 genes,** which can be used to establish the **survival prognosis of lung tumour patients.** All these genes are actively repressed and silent in normal adult somatic cells, since their expression is strictly restricted to placenta or male germinal cells. The inventors have demonstrated that the aberrant expression in malignant cells of at least one of these genes is associated with significantly poorer prognosis for lung cancer patients. Moreover, the detection of the expression of several combinations of these genes allows predicting prognosis in lung tumour patients with higher significance and accuracy than with individual genes.

The invention has led to the identification of 23 genes, whose aberrant expression was found associated with poor prognosis in lung tumour patients.

According to their expression and prognosis value in lung cancer patients, these 23 genes were divided into two groups
- A group of 7 genes, whose aberrant expression in lung cancer is relatively frequent (>7% of cases of our series). The expression of each individual one of these seven genes is associated with a significantly reduced survival probability (global or disease free survival over five years significantly reduced, logrank test p<0.07) of all lung cancer patients (without considering histological subtypes).
- A group of 16 genes, whose aberrant expression in lung cancer is relatively rare (<7% of cases of our series). The expression of each individual one of these seven genes is associated with a significantly reduced survival probability (global or disease free survival over five years significantly reduced, logrank test p<0.07) of all lung cancer patients (without considering histological subtypes).

The lists of these genes and their individual association with survival rates in patients from a series of 271 lung tumours are shown in the following **table 1.**

**Table 1**

| **GeneID:** | **SEQ ID NO gene** | **SEQ ID NO protein** | **Gene name** | **Logrank p value** | **Nb lung cancer** |
|---|---|---|---|---|---|
| **442** | **1** | **24** | SOX30 | 0,047 | 61 |
| **35** | **2** | **25** | SPATA22 | 0,02 | 22 |
| **295** | **3** | **26** | MAEL | 0,069 | 27 |
| **1161** | **4** | **27** | COX8C | 0,004 | 40 |
| **102** | **5** | **28** | TKTL1 | 0,059 | 40 |
| **391** | **6** | **29** | RBM46 | 0,007 | 29 |
| **390** | **7** | **30** | MAGEB6 | 0,062 | 68 |
| **222** | **8** | **31** | NBPF4 | 0,0008 | 8 |
| **1261** | **9** | **32** | C12orf37 | 0,013 | 9 |
| **1165** | **10** | **33** | TPTE2P3 | 0,003 | 5 |
| **72** | **11** | **34** | DPEP3 | 0,002 | 9 |
| **108** | **12** | **35** | C10orf82 | 0,0005 | 6 |
| **205** | **13** | **36** | LOC440896 | 0,021 | 9 |
| **1188** | **14** | **37** | CDNA clone IMAGE:5265646 | 0,04 | 6 |
| **1328** | **15** | **38** | HIST1H3C | 0,029 | 16 |
| **125** | **16** | **39** | PIWIL1 | <0,0001 | 7 |
| **144** | **17** | **40** | C19orf41 | <0,0001 | 4 |
| **437** | **18** | **41** | RNF17 | 0,026 | 7 |
| **766** | **19** | **42** | GALNTL5 | <0,0001 | 5 |
| **436** | **20** | **43** | RFX4 | 0,06 | 15 |
| **487** | **21** | **44** | LGALS14 | 0,009 | 5 |
| **117** | **22** | **45** | IGFBP1 | <0,0001 | 6 |
| **135** | **23** | **46** | TUBA3C | 0,077 | 9 |

The correspondence between the gene ID number and the corresponding SEQ ID is represented as follows:

Gene Num ID : 442 corresponds to SEQ ID NO: 1, Gene Num ID : 35 corresponds to SEQ ID NO: 2, Gene Num ID : 295 corresponds to SEQ ID NO: 3, Gene Num ID : 1161 corresponds to SEQ ID NO: 4, Gene Num ID : 102 corresponds to SEQ ID NO: 5, Gene Num ID : 391 corresponds to SEQ ID NO: 6, Gene Num ID : 390 corresponds to SEQ ID NO: 7, Gene Num ID : 222 corresponds to SEQ ID NO: 8, Gene Num ID : 1261 corresponds to SEQ ID NO: 9, Gene Num ID : 1165 corresponds to SEQ ID NO: 10, Gene Num ID : 72 corresponds to SEQ ID NO: 11, Gene Num ID : 108 corresponds to SEQ ID NO: 12, Gene Num ID : 205 corresponds to SEQ ID NO: 13, Gene Num ID : 1188 corresponds to SEQ ID NO: 14, Gene Num ID : 1328 corresponds to SEQ ID NO: 15, Gene Num ID : 125 corresponds to SEQ ID NO: 16, Gene Num ID : 144 corresponds to SEQ ID NO: 17, Gene Num ID : 437 corresponds to SEQ ID NO: 18, Gene Num ID : 766 corresponds to SEQ ID NO: 19, Gene Num ID : 436 corresponds to SEQ ID NO: 20, Gene Num ID : 487 corresponds to SEQ ID NO: 21, Gene Num ID : 117 corresponds to SEQ ID NO: 22 and Gene Num ID : 135 corresponds to SEQ ID NO: 23.

Logrank p value corresponds to the significance of difference in cumulative global survival probabilities over 5 years between patients expressing the gene and those not expressing the gene.
Nb lung cancer corresponds to the number of lung cancer patients expressing the gene (/271).

Each of these genes can be used individually to establish a prognosis in lung cancer patients: any patient expressing any one of the 23 genes (of the group of 7 or the group of 16) has significantly lower chances of survival compared to the patients not expressing the gene (see **table 2a**).

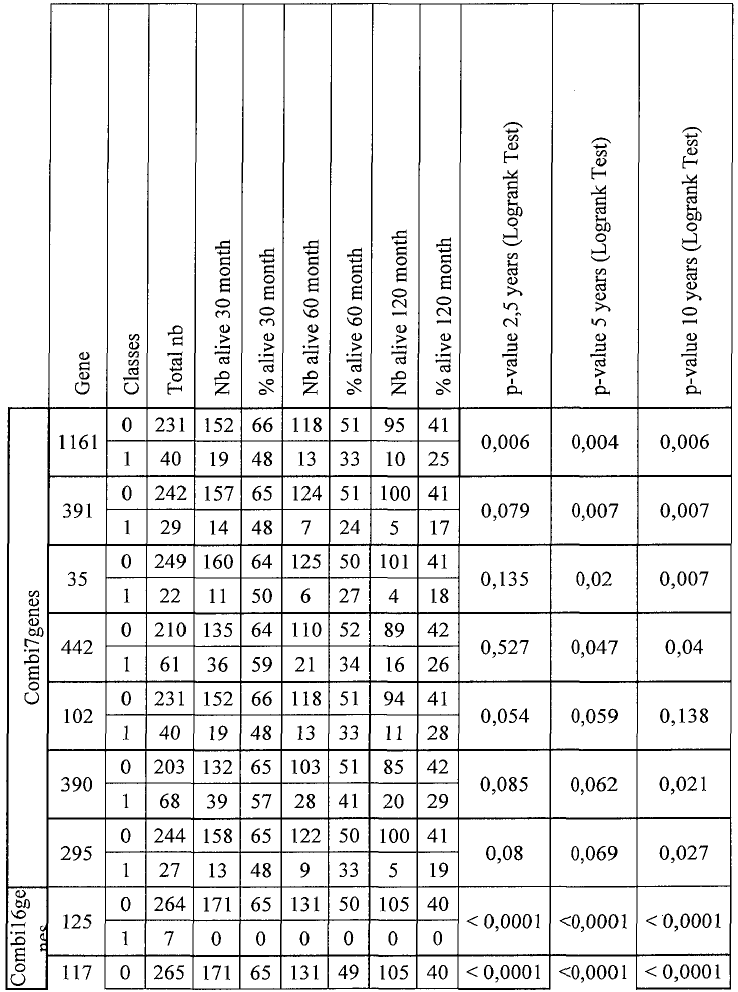

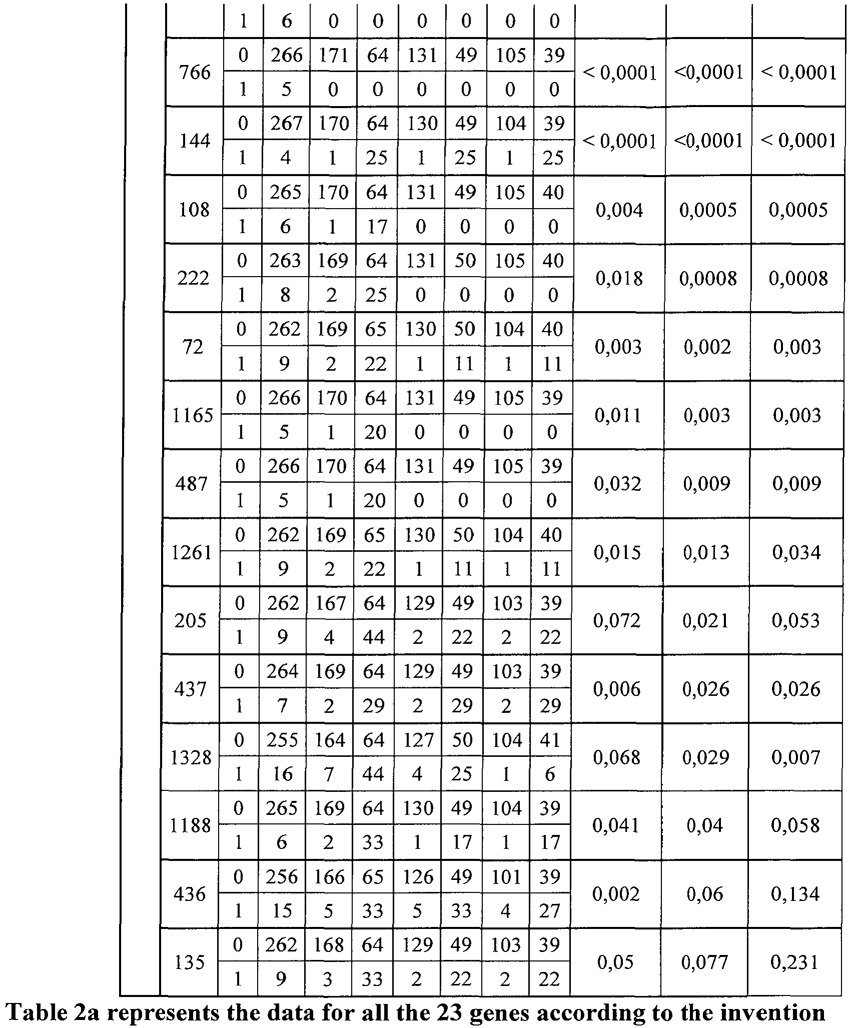

- Gene = gene identifier(s) of individual genes or combinations of genes
- Classes = Lung Kc patient not expressing (=0) or expressing (=1) the gene;

In the case of combinations of several genes, some patients could express one gene only or at least one gene of the combination (=1), 2 genes only or 2 genes or more of the combination (=2) etc...
- Total Nb = total number of patients of this class (considering the whole Brambilla study with 271 cases, including all histological types)
- % or nb alive 30, 60, 120 month = % or number of patients of this class alive after 30, 60, 120 month
- p-value corresponding to the significance of the difference in cumulative global survival probabilities between the different classes of patients over 2.5, 5 and 10 years (Logrank Test, considering survival curves of all the classes of patients).

The inventors have also shown that using combinations of these genes allows a more accurate prognosis. Examples of these combinations and their use to establish a prognosis are shown below.

In order to establish a prognosis in lung cancer patients, the aberrant expression of these genes can be used as follows:
- Combinations of all or several of the genes of the group of 7 genes and/or of the group of 16 genes (examples of combinations, see tables 2b and 2c)
- Any one gene of the group of 7 genes (preferably) or of the group of 16 genes (see table 2a)

**Table 2b**

| genes | Classes | Total Nb | Nb alive 30 m | % alive 30 m | Nb alive 60 m | % alive 60 m | Nb alive 120 m | % alive 120 m | p-value 2,5 years (Logrank Test) | p-value 5 years (Logrank Test) | p-value 10 years (Logrank Test) | Corresponding figure |
|---|---|---|---|---|---|---|---|---|---|---|---|---|
| 1161 | 0 | 231 | 152 | 66 | 118 | 51 | 95 | 41 | 0,006 | 0,0038 | 0,006 | Figure 1 |
| | 1 | 40 | 19 | 48 | 13 | 33 | 10 | 25 | | | | |
| 1161 +391 | 0 | 209 | 141 | 67 | 114 | 55 | 92 | 44 | 0,003 | <0,0001 | 0,0001 | Figure 2 |
| | >=1 | 62 | 30 | 48 | 17 | 27 | 13 | 21 | | | | |
| 1161 +39 1+35 | 0 | 193 | 134 | 69 | 108 | 56 | 88 | 46 | 0,0002 | <0,0001 | <0,0001 | Figure 3 |
| | >=1 | 78 | 37 | 47 | 23 | 29 | 17 | 22 | | | | |
| 1161 +391 +35 +442 | 0 | 155 | 110 | 71 | 93 | 60 | 76 | 49 | 0,002 | <0,0001 | <0,0001 | Figure 4 |
| | >=1 | 116 | 61 | 53 | 38 | 33 | 29 | 25 | | | | |
| | 0 | 155 | 110 | 71 | 93 | 60 | 76 | 49 | 0,008 | <0,0001 | <0,0001 | |
| | 1 | 86 | 45 | 52 | 29 | 34 | 23 | 27 | | | | |
| | >=2 | 30 | 16 | 53 | 9 | 30 | 6 | 20 | | | | |
| 1161 +391 +35 +442 + 102 | 0 | 145 | 103 | 71 | 88 | 61 | 72 | 50 | 0,005 | <0,0001 | <0,0001 | Figure 5 |
| | >=1 | 126 | 68 | 54 | 43 | 34 | 33 | 26 | | | | |
| | 0 | 145 | 103 | 71 | 88 | 61 | 72 | 50 | 0,006 | 0,0001 | 0,0002 | |
| | 1 | 74 | 44 | 59 | 28 | 38 | 21 | 28 | | | | |
| | >=2 | 52 | 24 | 46 | 15 | 29 | 12 | 23 | | | | |
| | 0 | 113 | 81 | 72 | 69 | 61 | 59 | 52 | 0,009 | 0,0006 | 0,0001 | Figure 6 |
| 1161 +391 +35 +442 + 102 +390 | >=1 | 158 | 90 | 57 | 62 | 39 | 46 | 29 | | | | |
| | 0 | 113 | 81 | 72 | 69 | 61 | 59 | 52 | 0,007 | 0,0002 | 0,0001 | |
| | 1 | 84 | 53 | 63 | 40 | 48 | 29 | 35 | | | | |
| | >=2 | 74 | 37 | 50 | 22 | 30 | 27 | 36 | | | | |
| | 0 | 113 | 81 | 72 | 69 | 61 | 59 | 52 | 0,004 | <0,0001 | <0,0001 | |
| | 1 | 84 | 53 | 63 | 40 | 48 | 29 | 35 | | | | |
| | 2 | 53 | 29 | 55 | 18 | 34 | 14 | 26 | | | | |
| | >=3 | 21 | 8 | 38 | 4 | 19 | 3 | 14 | | | | |
| | 0 | 113 | 81 | 72 | 69 | 61 | 59 | 52 | 0,002 | <0,0001 | <0,0001 | |
| | 1&2 | 137 | 82 | 60 | 58 | 42 | 43 | 31 | | | | |
| | >=3 | 21 | 8 | 38 | 4 | 19 | 3 | 14 | | | | |
| | 0 | 105 | 76 | 72 | 65 | 62 | 57 | 54 | 0,008 | 0,0005 | <0,0001 | Figure 7 |
| | >=1 | 166 | 95 | 57 | 66 | 40 | 48 | 29 | | | | |
| | 0 | 105 | 76 | 72 | 65 | 62 | 57 | 54 | 0,008 | 0,0002 | <0,0001 | |
| 1161 +391 +35 +442 + 102 +390 +295 | 1 | 87 | 55 | 63 | 42 | 48 | 29 | 33 | | | | |
| | >=2 | 79 | 40 | 51 | 24 | 30 | 19 | 24 | | | | |
| | 0 | 105 | 76 | 72 | 65 | 62 | 57 | 54 | 0,002 | <0,0001 | <0,0001 | Figure 9 |
| | 1 | 87 | 55 | 63 | 42 | 48 | 29 | 33 | | | | |
| | 2 | 50 | 29 | 58 | 18 | 36 | 15 | 30 | | | | |
| | >=3 | 29 | 11 | 38 | 6 | 21 | 4 | 14 | | | | |
| | 0 | 105 | 76 | 72 | 65 | 62 | 57 | 54 | 0,0006 | <0,0001 | <0,0001 | Figure 8 |
| | 1&2 | 137 | 84 | 61 | 60 | 44 | 44 | 32 | | | | |
| | >=3 | 29 | 11 | 38 | 6 | 21 | 4 | 14 | | | | |

**Table 2c**

| Genes | Classes | Total Nb | Nb alive 30 m | % alive 30 m | Nb alive 60 m | % alive 60 m | Nb alive 120 m | % alive 120 m | p-value 2,5 years (Logrank Test) | p-value 5 years (Logrank Test) | p-value 10 years (Logrank Test) | Corresponding figure |
|---|---|---|---|---|---|---|---|---|---|---|---|---|
| 125 | 0 | 264 | 171 | 65 | 131 | 50 | 105 | 40 | <0,0001 | <0,0001 | <0,0001 | Figure 10 |
| | 1 | 7 | 0 | 0 | 0 | 0 | 0 | 0 | | | | |
| 125 +117 | 0 | 258 | 171 | 66 | 131 | 51 | 105 | 41 | <0,0001 | <0,0001 | <0,0001 | Figure 11 |
| | >=1 | 13 | 0 | 0 | 0 | 0 | 0 | 0 | | | | |
| 125 +117 +766 | 0 | 255 | 171 | 67 | 131 | 51 | 105 | 41 | <0,0001 | <0,0001 | <0,0001 | Figure 12 |
| | >=1 | 16 | 0 | 0 | 0 | 0 | 0 | 0 | | | | |
| 125 +117 +766 + 144 | 0 | 254 | 170 | 67 | 130 | 51 | 104 | 41 | <0,0001 | <0,0001 | <0,0001 | Figure 13 |
| | >=1 | 17 | 1 | 6 | 1 | 6 | 0 | 0 | | | | |
| 125 +117 +766 + 144 +108 | 0 | 249 | 169 | 68 | 130 | 52 | 104 | 42 | <0,0001 | <0,0001 | <0,0001 | Figure 14 |
| | >=1 | 22 | 2 | 9 | 1 | 5 | 0 | 0 | | | | |
| 125 +117 +766 +144 +108 +222 | 0 | 244 | 168 | 69 | 130 | 53 | 104 | 43 | <0,0001 | <0,0001 | <0,0001 | Figure 15 |
| | >=1 | 27 | 3 | 11 | 1 | 4 | 0 | 0 | | | | |
| 125 +117 +766+144 +108 + 222 +72 | 0 | 238 | 166 | 70 | 129 | 54 | 103 | 43 | <0,0001 | <0,0001 | <0,0001 | Figure 16 |
| | >=1 | 33 | 5 | 15 | 2 | 6 | 2 | 6 | | | | |
| 125 +117 +766 +144 +108 + 222 +72 +1165 | 0 | 236 | 165 | 70 | 129 | 55 | 103 | 44 | <0,0001 | <0,0001 | <0,0001 | Figure 17 |
| | >=1 | 35 | 6 | 17 | 2 | 6 | 2 | 6 | | | | |
| 125 +117 +766 +144 +108 +222 +72 +1165 + 487 | 0 | 231 | 164 | 71 | 129 | 56 | 103 | 45 | <0,0001 | <0,0001 | <0,0001 | Figure 18 |
| | >=1 | 40 | 7 | 18 | 2 | 5 | 2 | 5 | | | | |
| 125 +117 +766 +144 +108 +222 +72 +1165 +487 +1261 | 0 | 222 | 162 | 73 | 128 | 58 | 102 | 46 | <0,0001 | <0,0001 | <0,0001 | Figure 19 |
| | >=1 | 49 | 9 | 18 | 3 | 6 | 3 | 6 | | | | |
| | 0 | 222 | 162 | 73 | 128 | 58 | 102 | 46 | <0,0001 | <0,0001 | <0,0001 | Figure 26 |
| | 1 | 38 | 8 | 21 | 3 | 8 | 3 | 8 | | | | |
| | >=2 | 11 | 1 | 9 | 0 | 0 | 0 | 0 | | | | |
| 125 +117 +766 +144 +108 +222 +72 +1165 +487 +1261 +205 | 0 | 216 | 159 | 74 | 126 | 58 | 100 | 46 | <0,0001 | <0,0001 | <0,0001 | Figure 20 |
| | >=1 | 55 | 12 | 22 | 5 | 9 | 5 | 9 | | | | |
| | 0 | 216 | 159 | 74 | 126 | 58 | 100 | 46 | <0,0001 | <0,0001 | <0,0001 | Figure 27 |
| | 1 | 43 | 11 | 26 | 5 | 12 | 5 | 12 | | | | |
| | >=2 | 12 | 1 | 8 | 0 | 0 | 0 | 0 | | | | |
| 125 +117 +766 +144 +108 +222 +72 +1165 +487 +1261 +205 +437 | 0 | 214 | 157 | 73 | 124 | 58 | 98 | 46 | <0,0001 | <0,0001 | <0,0001 | Figure 21 |
| | >=1 | 57 | 14 | 25 | 7 | 12 | 7 | 12 | | | | |
| | 0 | 214 | 157 | 73 | 124 | 58 | 98 | 46 | <0,0001 | <0,0001 | <0,0001 | Figure 28 |
| | 1 | 42 | 13 | 31 | 7 | 17 | 7 | 17 | | | | |
| | >=2 | 15 | 1 | 7 | 0 | 0 | 0 | 0 | | | | |
| 125 +117 +766 +144 +108 +222 +72 +1165 +487 +1261 +205+437 +1328 | 0 | 206 | 151 | 73 | 120 | 58 | 97 | 47 | <0,0001 | <0,0001 | <0,0001 | Figure 22 |
| | >=1 | 65 | 20 | 31 | 11 | 17 | 8 | 12 | | | | |
| | 0 | 206 | 151 | 73 | 120 | 58 | 97 | 47 | <0,0001 | <0,0001 | <0,0001 | Figure 29 |
| | 1 | 42 | 18 | 43 | 11 | 26 | 8 | 19 | | | | |
| | >=2 | 23 | 2 | 9 | 0 | 0 | 0 | 0 | | | | |
| 125 +117 +766 +144 +108 +222 +72 +1165 +487 +1261 +205 +437 +1328 +1188 | 0 | 204 | 150 | 74 | 119 | 58 | 96 | 47 | <0,0001 | <0,0001 | <0,0001 | Figure 23 |
| | >=1 | 67 | 21 | 31 | 12 | 18 | 9 | 13 | | | | |
| | 0 | 204 | 150 | 74 | 119 | 58 | 96 | 47 | <0,0001 | <0,0001 | <0,0001 | Figure 30 |
| | 1 | 41 | 18 | 44 | 12 | 29 | 9 | 22 | | | | |
| | >=2 | 26 | 3 | 12 | 0 | 0 | 0 | 0 | | | | |
| 125 +117 +766 +144 +108 +222 +72 +1165 +487 +1261 +205 +437 +1328 +1188 +436 | 0 | 201 | 148 | 74 | 117 | 58 | 94 | 47 | <0,0001 | <0,0001 | <0,0001 | Figure 24 |
| | >=1 | 70 | 23 | 33 | 14 | 20 | 11 | 16 | | | | |
| | 0 | 201 | 148 | 74 | 117 | 58 | 94 | 47 | <0,0001 | <0,0001 | <0,0001 | Figure 31 |
| | 1 | 38 | 17 | 45 | 11 | 29 | 9 | 24 | | | | |
| | >=2 | 32 | 6 | 19 | 3 | 9 | 2 | 6 | | | | |
| | 0 | 201 | 148 | 74 | 117 | 58 | 94 | 47 | <0,0001 | <0,0001 | <0,0001 | Figure 33 |
| | 1 | 38 | 17 | 45 | 11 | 29 | 9 | 24 | | | | |
| | 2 | 22 | 5 | 23 | 3 | 14 | 2 | 9 | | | | |
| | >=3 | 10 | 1 | 10 | 0 | 0 | 0 | 0 | | | | |
| 125 +117 +766 +144 +108 +222 +72 +1165 +487 +1261 +205 +437 +1328 +1188 +436 +135 | 0 | 199 | 146 | 73 | 115 | 58 | 92 | 46 | <0,0001 | <0,0001 | <0,0001 | Figure 25 |
| | >=1 | 72 | 25 | 35 | 16 | 22 | 13 | 18 | | | | |
| | 0 | 199 | 146 | 73 | 115 | 58 | 92 | 46 | <0,0001 | <0,0001 | <0,0001 | Figure 32 |
| | 1 | 40 | 19 | 48 | 13 | 33 | 11 | 28 | | | | |
| | >=2 | 32 | 6 | 19 | 3 | 9 | 2 | 6 | | | | |
| | 0 | 199 | 146 | 73 | 115 | 58 | 92 | 46 | <0,0001 | <0,0001 | <0,0001 | Figure 34 |
| | 1 | 40 | 19 | 48 | 13 | 33 | 11 | 28 | | | | |
| | 2 | 17 | 4 | 24 | 3 | 18 | 2 | 12 | | | | |
| | >=3 | 15 | 2 | 13 | 0 | 0 | 0 | 0 | | | | |

### Detailed procedure and examples

### I- Methodological approach

The following procedure allowed identifying the genes according to the invention.

### Overview

The expression of the 497 testis- and placenta- specific genes of our list was studied in a series of 271 lung cancer samples by extracting the corresponding expression data from genome-wide transcriptomic data (the latter were obtained by C. and E. Brambilla supported by the Ligue CIT program (Carte d'Identite des Tumeurs)).

For each gene, the patients were divided into two groups, those expressing the gene (calculated as described below), and those not expressing the gene (following the procedure for the determination of the ON/OFF gene expression status).

For each of the 497 genes of the list, the global and disease free survival probabilities were compared between the patients expressing the gene (ON) and those not expressing the gene (OFF). This was done considering the whole period of the study, as well as for 10 years (120 months), 5 years (60 months) and 2.5 years (30 months) of follow-up. This was performed considering all patients of the study (n=271), as well as each of the main histological subtypes of this population (ADK=adenocarcinoma; BAS= basaloid; LCNE=Large cell neuroendocrine; SQC=squamous cell tumour). The genes whose ON status allowed discriminating the patients with good or bad prognosis with a significance corresponding to a p value =<0.07 (Logrank p value, obtained when comparing cumulative global survival and/or disease free survival over 5 years between patients expressing or not expressing the gene) were selected as candidate prognosis markers.

For all these genes, the correlation between their expression (ON status) and prognosis was validated in at least one of the two following lung published cancer transcriptomic studies with survival clinical data using the same Affymetrix technology (website GEO: http://www.ncbi.nlm.nih.gov/geo, respectively GSE4576 and GSE8894).

These studies were selected as external populations of lung cancer patients in order to validate our survival data obtained by analysing the transcriptomic data of the Brambilla study.

### Detailed procedure

### 1- Establishment of a list of placenta and testis restricted genes and analysis of their aberrant expression in lung cancer patients

1a- A list of 497 human genes whose expression was restricted to placenta or male germ cells was established as mentioned in the international application WO/2009/121878.
These genes are never expressed in normal adult somatic tissues (adult somatic tissues comprise all tissues except germinal cells, foetal tissues and placenta).

1b- Expression data were extracted from a series of 112 normal adult somatic tissues randomly selected from a genome wide study of normal human tissues (GSE3526 on GEO, this study was chosen because it uses the same probes and measurement technology to detect gene expression as the Brambilla study: Affymetrix Human Genome U133 Plus 2.0 Array). The CEL files (raw data) from the control samples were downloaded from GEO. They were entered in the Genespring software and normalized (RMA algorithm) simultaneously with the CEL files from the Brambilla study.

1c- For each of the 497 genes/probes, the mean hybridization intensity signal value of the 112 control samples + 2sd was defined as the threshold for expression.
This threshold was used to distinguish between the cancer samples expressing the gene ON) and those not expressing the gene (OFF).
The measurement of expression of the genes using Affymetrix microarrays involves the hybridization of fluorescence labeled cDNAs from each tissue sample on microarrays containing gene-specific probes, the fluorescence intensity signal corresponding to each probe of the microarray is measured and changed into a raw value. The absolute value of the fluorescence intensity signal is highly variable and probe-dependent (different probes corresponding to the same gene can give different intensities of fluorescence). Therefore, on the basis of these absolute fluorescence intensity values it is generally not possible to determine whether a gene is expressed or not, and commonly people use this technique to assess variations of expression between samples (see below for more details).

In the invention, the definition of a precise threshold for expression was possible because the selected 497 genes are NOT expressed in any normal adult somatic tissue (according to the original criteria for their selection). Therefore the signal values obtained in the 112 normal adult somatic control samples give a high confidence set of values corresponding to the background noise signal, which allow further analyses.
A threshold signal value for expression could not have been defined for genes, which do not have a restricted expression pattern. Indeed in all these types of transcriptomic experiments the background noise signal value is highly dependent on the sequence of the probe. For instance several probes representative of the same gene generally give different signal values (although these signal values should normally vary between samples in the same direction). In the case of non-restricted genes (most genes have a pattern of expression, which is not restricted to germinal cells or placenta), it is therefore impossible to use these signal values as "absolute" indicators of the presence or absence of expression. However, one can compare expression levels between two groups of tissues (= expression in group of tissues A is significantly higher/lower than expression in group of tissues B). Therefore, in this particular study, since we have previously demonstrated that all the studied 497 genes are NOT expressed in normal adult somatic tissues, we were able to define a threshold differentiating expression (ON) and non-expression (OFF). This is a specific key feature of our approach.
1d- Based on this threshold, the expression of each of the 497 genes in each of the samples was defined as negative (OFF) or positive (ON) as follow. In each cancer sample, if the normalised signal value was above this threshold, the gene was considered as aberrantly expressed in this sample (gene ON), if it was under this threshold, it was considered as not expressed (gene OFF).

1e- From the Brambilla study (271 cases of lung cancer), the Inventors found that **130** of the 497 genes were aberrantly expressed in at least 1% of these lung cancer cases.

**2- Correlation between the expression of each individual gene (of the list of 130 genes) and the prognosis for survival in the lung cancer patients, and selection of 23 genes individually associated to the prognosis of all lung cancer cases (without considering histological subtypes; named after "global prognosis genes").**

2a- As a first step, using each of the 130 genes individually, we compared the global survival over a period of five years between the groups of patients expressing the gene (yes) versus those not expressing the gene (no). A Logrank Mantel-Cox test was performed and a p value was calculated. This analysis was performed first with the whole population of lung cancer patients of the Brambilla study (n=271), second with each one of the following populations: ADK cases (n=91), BAS cases (n=46), LCNE cases (n=47), SQC cases (n=62).

**2b- A total of 23 genes were selected, whose individual expression was significantly associated with a poorer prognosis (as measured by the cumulative global survival and/or disease-free survival over five years; p<0.05) in the Brambilla lung cancer study, as well as in at least one of the external validation populations.**

The expression of any one of 23 genes in lung cancer is significantly associated with a poor prognosis when considering all histological subtypes.

2c- A detailed **quantitative evaluation of the prognosis** is given in **table 2a,** using each of the 23 genes associated with poor prognosis in all lung cancer types. The Kaplan Meyer survival curves obtained using each of these genes can be visualized by clicking on the link in the last column of the table.

2d- These 23 genes were then divided into two groups
- A group of 7 genes, whose aberrant expression in lung cancer is relatively frequent (>10% of cases of our series). The expression of each individual one of these seven genes is associated with a significantly reduced survival probability (global or disease free survival over five years significantly reduced, logrank test p<0.07) of all lung cancer patients (without considering histological subtypes).
- A group of 16 genes, whose aberrant expression in lung cancer is relatively rare (<10% of cases of our series). The expression of each individual one of these seven genes is associated with a significantly reduced survival probability (global or disease free survival over five years significantly reduced, logrank test p<0.07) of all lung cancer patients (without considering histological subtypes).

**3- Association of several or all of 23 genes of the groups of 7 genes or 16 genes allows a more accurate prognosis in lung cancer patients than the use of each of them**

3a- Different associations of these 23 genes were tested for the correlation between the expression of at least one gene of a given association of genes and the prognosis.

3b- The **7 genes more frequently expressed** were classified by increasing Logrank p value as follows: 1161; 391; 35; 442; 102; 390; 295. Following the same order, **subgroups of the 1rst of these genes, the 1rst + 2^{nd} of these genes, the 1rst + 2^{nd} + 3^{rd} of these genes, etc... and finally the seven genes**, were respectively tested for their prognosis prediction value. The distribution of patients according to the number of the genes of the group aberrantly expressed was studied, and relevant groups of patients were compared for their survival probability. The detailed quantitative evaluation of the prognosis using these subgroups of the seven genes is given in **table 2b.**

3c- Similarly, the **16 genes rarely expressed** were classified by increasing p values as follow:125; 117; 766; 144; 108; 222; 72; 1165; 487; 1261; 205; 437; 1328; 1188; 436; 135. Following the same order, **subgroups of the 1rst of these genes, the 1rst + 2^{nd} of these genes, the 1rst + 2^{nd} + 3^{rd} of these genes, etc... and finally the sixteen genes,** were respectively tested for their prognosis prediction value. The distribution of patients according to the number of the genes of the group aberrantly expressed was studied, and relevant groups of patients were compared for their survival probability. The detailed quantitative evaluation of the prognosis using these subgroups of the sixteen genes is given **table 2c.**

3d- Using the expression data of **all 23 genes,** the distribution of the 271 lung cancer patients according to the number of genes aberrantly expressed **from the group of 7 genes and from the group of 16 genes respectively,** was studied. Nine groups of patients were constituted according to these criteria, and the survival Kaplan Meyer curves were compared between these nine groups of patients. Finally the 271 lung cancer patients were classified into three/four prognosis subgroups: P1a, P1b, P2 and P3. The P1a, P1b, P2 and P3 definition is indicated in the Table 4 below.

### II- Example of the combination using all the genes of the group of 7 genes and of the group of 16 genes to establish the prognosis of lung cancer:

Number of lung cancer patients distributed among the different groups according to the number of genes expressed from the "group7genes" (combination of 7 genes) and the "group16genes" (combination of 16 genes) (Table 3).

The groups P1a, P1b, P2 and P3 are defined as follows:
P1A corresponds to patient samples in which no gene of the group of 7 genes or of 16 genes are expressed.
P2B corresponds to patient samples in which 1 or 2 genes of the group of 7 genes are expressed but no genes of the group of 16 genes are expressed.
P2 corresponds to patient samples in which
   - either 3 or more genes of the group of 7 genes are expressed, but no genes of the group of 16 genes are expressed,
   - or at least 1 gene of the group of 16 genes is expressed but no genes of the group of 7 genes is expressed,
   - or one gene of the group of 16 gene is expressed, and 1 or 2 genes of the group of 7 genes is expressed.
P3 corresponds to patient samples in which
   - either 2 or more genes of the group of 16 genes are expressed, and 1 or 2 genes of the group of 7 genes are expressed,
   - or at least 3 genes of the group of 7 genes is expressed and at least 1 gene of the group of 16 genes are expressed.

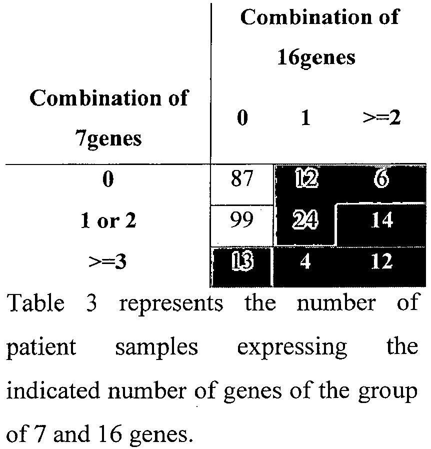

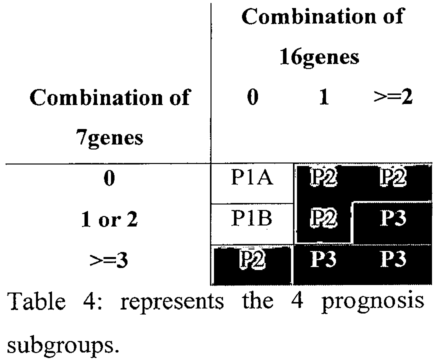

The following table 5 recapitulates the data of table 3 and table 4.

| | | Nb of patients |
|---|---|---|
| P1A | Combi7genes : 0 and Combi16genes : 0 | 87 |
| **Total P1A** | | **87** |
| P1B | Combi7genes : 1&2 and Combi16genes : 0 | 99 |
| **Total P1B** | | **99** |
| P2 | Combi7genes : 0 and Combi16genes : 1 | 12 |
| P2 | Combi7genes : 0 and Combi16genes : >=2 | 6 |
| P2 | Combi7genes : 1&2 and Combi16genes : 1 | 24 |
| P2 | Combi7genes : >=3 and Combi16genes : 0 | 13 |
| **Total P2** | | **55** |
| P3 | Combi7genes : >=3 and Combi16genes : >=2 | 12 |
| P3 | Combi7genes : >=3 and Combi16genes : 1 | 4 |
| P3 | Combi7genes : 1&2 and Combi16genes : >=2 | 14 |
| **Total P3** | | **30** |
| **All groups** | | **271** |

### III- Examples of CT genes whose aberrant expression in cancer is not correlated with prognosis

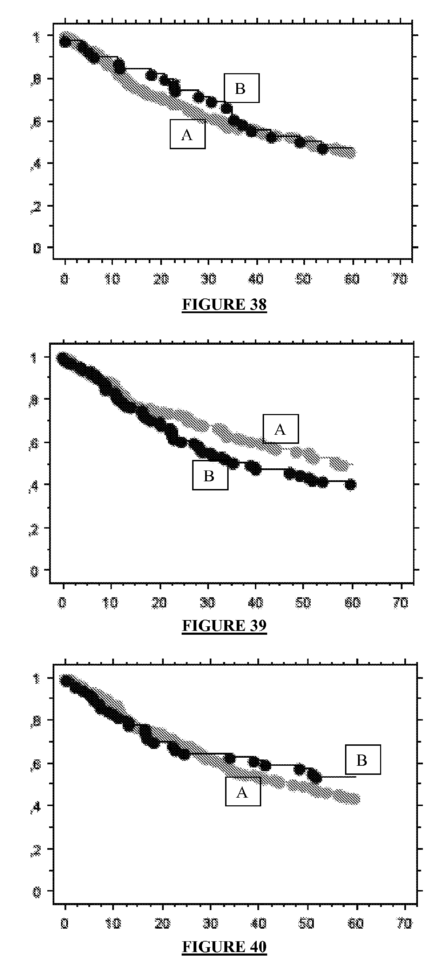

To enforce the specificity of the present prognosis method, the Inventors have evaluated the prognosis impact of 3 CT genes, identified as cancer marker.
The results are shown in figures 38-40.
These results demonstrate that 3 genes, well known as cancer markers, do not give any significant information about the survival rate of patients afflicted by lung tumors.

Therefore, the combination of 7 and 16 genes (i.e. 23 genes) according to the invention provides a very specific and useful method for prognosis lung tumors.

## Claims

1. Use of
- at least 2 genes chosen among a set of 23 genes comprising or consisting of the nucleic acid sequences SEQ ID NO 1 to 23,
- or complementary sequences of said at least 2 genes chosen among a set of 23 genes comprising or consisting of the nucleic acid sequences SEQ ID NO 1 to 23,
- or sequences having at least 80% homology with said genes,
- or proteins coded by said at least 2 genes chosen among a set of 23 genes comprising or consisting of the nucleic acid sequences SEQ ID NO 1 to 23, said
◆ at least 2 genes being such that
- at least one gene belongs to a first set A of 7 genes comprising or consisting of the nucleic acid sequences SEQ ID NO: 1-7
- at least one gene belongs to a second set B of 16 genes comprising or consisting of the nucleic acid sequences SEQ ID NO: 8-23,
◆ at least 2 proteins being such that
- at least one protein belongs to a first set AP of 7 proteins, each 7 proteins being coded by the genes comprising or consisting of the nucleic acid sequences SEQ ID NO: 1-7,
- at least one protein belongs to a second set BP of 16 proteins, each 16 proteins being coded by the genes comprising or consisting of the nucleic acid sequences SEQ ID NO: 8-23,
for an *in vitro* prognosis method of the survival rate of a patient afflicted by a lung cancer, said method comprising a step of measuring, in a lung cancer sample of said patient, the expression of said at least 2 genes, or said complementary sequences, or said sequences having at least 80% homology with said genes, or said at least 2 proteins, and said prognosis being such that:
either
▪ if none of the 23 genes of said set is expressed, the patient survival rate during a period of time from 30 to 120 months after the diagnosis of said lung cancer is from about 59% to about 78%, and
▪ if at least one gene of at least one set A or B is expressed, the patient survival rate during a period of time from 30 to 120 months after the diagnosis of said lung cancer is about from about 3% to about 70%,
or
▪ if none of the 23 proteins of said set is expressed, the patient survival rate during a period of time from 30 to 120 months after the diagnosis of said lung cancer is from about 59% to about 78%, and
▪ if at least one protein of at least one set AP or BP is expressed, the patient survival rate during a period of time from 30 to 120 months after the diagnosis of said lung cancer is about from about 3% to about 70%,
wherein said gene or protein is determined as being expressed when:
- either it is expressed in a sample of a patient afflicted by a lung cancer but not in a control sample of an healthy individual,
- or it is expressed above a threshold corresponding to a background signal observed in a series of reference healthy tissues for each detection method, and
said gene or protein is determined as being not expressed when:
- it is neither expressed in a sample of a patient afflicted by a lung cancer nor in a control sample of an healthy individual,
- or it is expressed in a sample of a patient afflicted by a lung cancer at a level substantially equal or inferior to the level in a control sample of an healthy individual.

2. Use according to claim 1, wherein said prognosis method is such that:
▪ if none of the 23 genes of said set is expressed, the patient survival rate is from about 59% to about 78%,
▪ if none of the genes of the set B is expressed and at least one gene of the set A is expressed, the patient survival rate is from about 27% to about 70%, and
▪ if at least one gene of the set B is expressed, the patient survival rate is from about 3% to about 55%.

3. Use according to claim 1 or 2, wherein said prognosis method is such that :
▪ if none of the 23 genes of said set is expressed, the patient survival rate is from about 59% to about 78%,
▪ if
∘ none of the genes of the set B is expressed and at least 3 genes of the set A is expressed, or
∘ at least 1 gene of the set B is expressed and from none to 2 genes of the set A is expressed, or
∘ at least 2 genes of the set B is expressed and no gene of the set A is expressed,
the patient survival rate is from about 27% to about 55%, and
▪ if
∘ one gene of the genes of the set B is expressed and at least 3 genes of the set A are expressed, or
∘ at least 2 genes of the set B are expressed and at least 1 gene of the set A are expressed
the patient survival rate is about from about 3% to about 13%.

4. Use according to anyone of claims 1 to 3, wherein said prognosis method is such that:
▪ if none of the 23 genes of said set is expressed, the patient survival rate is from about 59% to about 78%,
▪ if none of the genes of the set B is expressed and one or two genes of the set A is expressed, the patient survival rate is from about 38% to about 70%,
▪ if
∘ none of the genes of the set B is expressed and at least 3 genes of the first set is expressed, or
∘ at least 1 gene of the set B is expressed and from none to 2 genes of the set A is expressed, or
∘ at least 2 genes of the set B is expressed and no gene of the set A is expressed,
the patient survival rate is from about 27% to about 55%, and
▪ if
∘ one gene of the genes of the set B is expressed and at least 3 genes of the set A are expressed, or
∘ at least 2 genes of the set B are expressed and at least 1 gene of the set A are expressed
the patient survival rate is from about 3% to about 13%.

5. Use according to anyone of claims 1 to 4, wherein said lung tumor has been previously histologically classified.

6. Use according to claim 5, wherein said histologically classified tumor belongs to the set consisting of : adenocarcinoma, squamous cell carcinoma, basaloid tumours, and large cell neuroendocrine.

7. Use according to anyone of claims 1 to 6, wherein the determination of said at least two genes as being expressed is carried out by using a technique chosen among the set consisting of:
- Quantitative PCR,
- DNA CHIP, and
- Northern blot.

8. Use according to anyone of claims 1 to 7, wherein the determination of said at least two genes as being expressed is carried out by using nucleic acid molecules consisting of from 15 to 100 nucleotides molecules being complementary to said at least 2 genes.

9. Use according to anyone of claims 1 to 8, wherein the determination of said at least two genes as being expressed is carried out by DNA CHIP using
- at least one nucleic acid probe comprising or being constituted by the nucleic acid sequence SEQ ID NO: 47 to SEQ ID NO: 53, and
- at least one nucleic acid probe comprising or being constituted by the nucleic acid sequence SEQ ID NO: 54 to SEQ ID NO: 69.

10. Use according to claim 7, wherein the determination of said at least 2 proteins as being expressed is carried out by using a technique chosen among the set consisting of:
- western Blot,
- ELISA,
- Immunofluorescence, and
- Immunohistochemistry.

11. Use according to claims 1 to 6, wherein the determination of said at least 2 proteins as being expressed is carried out by using antibodies directed against said at least 2 proteins coded by said at least two genes.

## Patentansprüche

1. Verwendung von
- mindestens 2 Genen, ausgewählt aus einem Satz von 23 Genen, die die Nukleinsäuresequenzen SEQ ID NO: 1 bis 23 umfassen oder aus ihnen bestehen,
- oder komplementären Sequenzen der mindestens 2 Gene, ausgewählt aus einem Satz von 23 Genen, die die Nukleinsäuresequenzen SEQ ID NO: 1 bis 23 umfassen oder aus ihnen bestehen,
- oder Sequenzen mit mindestens 80 % Homologie mit den Genen,
- oder Proteinen, die von den mindestens 2 Genen codierten werden, ausgewählt aus einem Satz von 23 Genen, die die Nukleinsäuresequenzen SEQ ID NO: 1 bis 23 umfassen oder aus ihnen bestehen,
wobei
◆ mindestens 2 Gene derart ausgebildet sind, dass
- mindestens ein Gen zu einem ersten Satz A von 7 Genen gehört, die die Nukleinsäuresequenzen SEQ ID NO: 1 bis 7 umfassen oder aus ihnen bestehen,
- mindestens ein Gen zu einem zweiten Satz B von 16 Genen gehört, die die Nukleinsäuresequenzen SEQ ID NO: 8 bis 23 umfassen oder aus ihnen bestehen,
◆ mindestens 2 Proteine derart ausgebildet sind, dass
- mindestens ein Protein zu einem ersten Satz AP von 7 Proteinen gehört, wobei jedes der 7 Proteine von den Genen codiert wird, die die Nukleinsäuresequenzen SEQ ID NO: 1 bis 7 umfassen oder aus ihnen bestehen,
- mindestens ein Protein zu einem zweiten Satz BP von 16 Proteinen gehört, wobei jedes der 16 Proteine von den Genen codiert wird, die die Nukleinsäuresequenzen SEQ ID NO: 8 bis 23 umfassen oder aus ihnen bestehen,
für ein in vitro-Prognoseverfahren für die Überlebensrate eines mit einem Lungenkrebs befallenen Patienten, wobei das Verfahren einen Schritt des Messens der Expression der mindestens 2 Gene oder der komplementären Sequenzen oder der Sequenzen mit mindestens 80 % Homologie mit den Genen oder der mindestens 2 Proteine in einer Lungenkrebsprobe des Patienten umfasst, wobei das Prognoseverfahren derart ausgebildet ist, dass:
entweder,
▪ falls keines der 23 Gene des Satzes exprimiert wird, die Überlebensrate des Patienten während eines Zeitraums von 30 bis 120 Monaten nach der Diagnose des Lungenkrebses etwa 59 % bis etwa 78 % ist, und
▪ falls mindestens ein Gen von mindestens einem Satz A oder B exprimiert wird, die Überlebensrate des Patienten während eines Zeitraums von 30 bis 120 Monaten nach der Diagnose des Lungenkrebses etwa 3 % bis etwa 70 % ist,
oder,
▪ falls keines der 23 Proteine des Satzes exprimiert wird, die Überlebensrate des Patienten während eines Zeitraums von 30 bis 120 Monaten nach der Diagnose des Lungenkrebses etwa 59 % bis etwa 78 % ist, und
▪ falls mindestens ein Protein von mindestens einem Satz AP oder BP exprimiert wird, die Überlebensrate des Patienten während eines Zeitraums von 30 bis 120 Monaten nach der Diagnose des Lungenkrebses etwa 3 % bis etwa 70 % ist,
wobei das Gen oder Protein als exprimiert festgestellt wird, wenn:
- es entweder in einer Probe eines mit einem Lungenkrebs befallenen Patienten exprimiert wird, aber nicht in einer Kontrollprobe eines gesunden Patienten,
- oder es oberhalb einer Schwelle entsprechend einem Hintergrundsignal exprimiert wird, das in einer Reihe von gesunden Referenzgeweben für jedes Nachweisverfahren beobachtet wird, und
das Gen oder Protein als nicht exprimiert festgestellt wird, wenn:
- es weder in einer Probe eines mit einem Lungenkrebs befallenen Patienten noch in einer Kontrollprobe eines gesunden Patienten exprimiert wird,
- oder es in einer Probe eines mit einem Lungenkrebs befallenen Patienten mit einem Niveau exprimiert wird, das im Wesentlichen gleich oder niedriger als das Niveau in einer Kontrollprobe eines gesunden Patienten ist.

2. Verwendung nach Anspruch 1, wobei das Prognoseverfahren derart ausgebildet ist, dass,
▪ falls keines der 23 Gene des Satzes exprimiert wird, die Überlebensrate des Patienten etwa 59 % bis etwa 78 % ist,
▪ falls keines der Gene des Satzes B und mindestens ein Gen des Satzes A exprimiert wird, die Überlebensrate des Patienten etwa 27 % bis etwa 70 % ist, und
▪ falls mindestens ein Gen des Satzes B exprimiert wird, die Überlebensrate des Patienten etwa 3 % bis etwa 55 % ist.

3. Verwendung nach Anspruch 1 oder 2, wobei das Prognoseverfahren derart ausgebildet ist, dass,
▪ falls keines der 23 Gene des Satzes exprimiert wird, die Überlebensrate des Patienten etwa 59 % bis etwa 78 % ist,
▪ falls
∘ keines der Gene des Satzes B und mindestens 3 Gene des Satzes A exprimiert werden, oder
∘ mindestens 1 Gen des Satzes B und 0 bis 2 Gene des Satzes A exprimiert werden, oder
∘ mindestens 2 Gene des Satzes B und kein Gen des Satzes A exprimiert werden,
die Überlebensrate des Patienten etwa 27% bis etwa 55 % ist, und,
▪ falls
∘ ein Gen der Gene des Satzes B und mindestens 3 Gene des Satzes A exprimiert werden, oder
∘ mindestens 2 Gene des Satzes B und mindestens 1 Gen des Satzes A exprimiert werden,
die Überlebensrate des Patienten etwa 3 % bis etwa 13 % ist.

4. Verwendung nach einem der Ansprüche 1 bis 3, wobei das Prognoseverfahren derart ausgebildet ist, dass,
▪ falls keines der 23 Gene des Satzes exprimiert wird, die Überlebensrate des Patienten etwa 59 % bis etwa 78 % ist,
▪ falls keines der Gene des Satzes B und ein oder zwei Gene des Satzes A exprimiert werden, die Überlebensrate des Patienten etwa 38 % bis etwa 70 % ist,
▪ falls
∘ keines der Gene des Satzes B und mindestens 3 Gene des ersten Satzes exprimiert werden, oder
∘ mindestens 1 Gen des Satzes B und von 0 bis 2 Gene des Satzes A exprimiert werden, oder
∘ mindestens 2 Gene des Satzes B und kein Gen des Satzes A exprimiert werden,
die Überlebensrate des Patienten etwa 27 % bis etwa 55 % ist, und,
▪ falls
∘ ein Gen der Gene des Satzes B und mindestens 3 Gene des Satzes A exprimiert werden, oder
∘ mindestens 2 Gene des Satzes B und mindestens ein Gen des Satzes A exprimiert werden,
die Überlebensrate des Patienten etwa 3 % bis etwa 13 % ist.

5. Verwendung nach einem der Ansprüche 1 bis 4, wobei der Lungentumor vorab histologisch klassifiziert wurde.

6. Verwendung nach Anspruch 5, wobei der histologisch klassifizierte Lungentumor zu dem Satz gehört, der aus Folgendem besteht: Adenokarzinom, Plattenepithelkarzinom, basaloides Karzinom und großzelliges neuroendokrines Karzinom.

7. Verwendung nach einem der Ansprüche 1 bis 6, wobei die Bestimmung der mindestens zwei Gene als exprimiert durch eine Technik ausgeführt wird, die aus dem Satz ausgewählt wird, der aus Folgendem besteht:
- Quantitative PCR,
- DNA-CHIP und
- Northern-Blot.

8. Verwendung nach einem der Ansprüche 1 bis 7, wobei die Bestimmung der mindestens zwei Gene als exprimiert unter Verwendung von Nukleinsäuremolekülen ausgeführt wird, die aus 15 bis 100 Nukleotiden bestehen, die zu den mindestens 2 Genen komplementär sind.

9. Verwendung nach einem der Ansprüche 1 bis 8, wobei die Bestimmung der mindestens zwei Gene als exprimiert durch DNA-CHIP ausgeführt wird, wobei Folgendes verwendet wird:
- mindestens eine Nukleinsäureprobe, die die Nukleinsäuresequenz SEQ ID NO: 47 bis SEQ ID NO: 53 umfasst oder aus dieser besteht, und
- mindestens eine Nukleinsäureprobe, die die Nukleinsäuresequenz SEQ ID NO: 54 bis SEQ ID NO: 69 umfasst oder aus dieser besteht.

10. Verwendung nach Anspruch 7, wobei die Bestimmung der mindestens zwei Proteine als exprimiert durch eine Technik ausgeführt wird, die aus dem Satz ausgewählt wird, der aus Folgendem besteht:
- Western-Blot,
- ELISA,
- Immunfluoreszenz und
- Immunhistochemie.

11. Verwendung nach den Ansprüchen 1 bis 6, wobei die Bestimmung der mindestens zwei Proteine als exprimiert durch Verwendung von Antikörpern gegen die mindestens 2 Proteine, die durch die mindestens zwei Gene codiert werden, ausgeführt wird.

## Revendications

1. Utilisation de
- au moins 2 gènes choisis parmi un ensemble de 23 gènes comprenant ou consistant en les séquences, d'acide nucléique SEQ ID NO 1 à 23,
- ou séquences complémentaires desdits au moins 2 gènes choisis parmi un ensemble de 23 gènes comprenant ou consistant en les séquences d'acide nucléique SEQ ID NO 1 à 23,
- ou séquences ayant au moins 80% d'homologie avec lesdits gènes,
- ou protéines codées par lesdits au moins 2 gènes choisis parmi un ensemble de 23 gènes comprenant ou consistant en les séquences d'acides nucléiques SEQ ID NO 1 à 23,
lesdits
❖ au moins 2 gènes étant tels que
- au moins un gène appartient à un premier ensemble A de 7 gènes comprenant ou consistant en les séquences d'acide nucléique SEQ ID NO 1-7
- au moins un gène appartient à un second ensemble B de 16 gènes comprenant ou consistant en les séquences d'acide nucléique SEQ ID NO 8-23,
❖ au moins 2 protéines étant telles que
- au moins une protéine appartient à un premier ensemble AP de 7 protéines, chacune des 7 protéines étant codées par les gènes comprenant ou consistant en les séquences d'acide nucléique SEQ ID NO : 1-7,
- au moins une protéine appartient à un second ensemble BP de 16 protéines, chacune des 16 protéines étant codées par les gènes comprenant ou consistant en les séquences d'acide nucléique SEQ ID NO : 8-23,
pour une méthode de pronostic *in vitro* du taux de survie d'un patient atteint d'un cancer du poumon, ladite méthode comprenant une étape de mesure, dans un échantillon de cancer du poumon dudit patient, de l'expression desdits au moins 2 gènes, or desdites séquences complémentaires, ou desdites séquences ayant au moins 80% d'homologie avec lesdits gènes, ou desdites au moins 2 protéines, et ledit pronostic étant tel que :
soit
▪ si aucun des 23 gènes dudit ensemble n'est exprimé, le taux de survie du patient pendant une période de temps de 30 à 120 mois après le diagnostic dudit cancer du poumon est d'environ 59% à environ 78%, et
▪ si au moins un gène d'au moins un ensemble A ou B est exprimé, le taux de survie du patient pendant une période de temps de 30 à 120 mois après le diagnostic dudit cancer du poumon est environ d'environ 3% à environ 70%,
▪ si aucune des 23 protéines dudit ensemble n'est exprimée, le taux de survie du patient pendant une période de temps de 30 à 120 mois après le diagnostic dudit cancer du poumon est d'environ 59% à environ 78%, et
▪ si au moins une protéine d'au moins un ensemble AP ou BP est exprimé, le taux de survie du patient pendant une période de temps de 30 à 120 mois après le diagnostic dudit cancer du poumon est environ d'environ 3% à environ 70%,
dans laquelle ledit gène ou ladite protéine est déterminé comme étant exprimé quand :
- soit il est exprimé dans un échantillon d'un patient atteint d'un cancer du poumon mais pas dans un échantillon contrôle d'un individu sain,
- soit il est exprimé au-dessus d'un seuil correspondant à un signal de fond observé chez une série de tissus sains de référence pour chaque méthode de détection, et
ledit gène ou ladite protéine est déterminé comme n'étant pas exprimée quand :
- il n'est ni exprimé dans un échantillon d'un patient atteint d'un cancer du poumon ni dans un échantillon contrôle d'un individu sain,
- ou il est exprimé dans un échantillon d'un patient atteint d'un cancer du poumon à un niveau substantiellement égal ou inférieur au niveau dans un échantillon contrôle d'un individu sain.

2. Utilisation selon la revendication 1, dans laquelle ladite méthode de pronostic est telle que :
▪ si aucun des 23 gènes dudit ensemble n'est exprimé, le taux de survie du patient est d'environ 59% à environ 78%,
▪ si aucun des gènes de l'ensemble B n'est exprimé et au moins un gène de l'ensemble A est exprimé, le taux de survie du patient est d'environ 27% à environ 70%, et
▪ si au moins un gène de l'ensemble B est exprimé, le taux de survie du patient est d'environ 3% à environ 55%.

3. Utilisation selon la revendication 1 ou 2, dans laquelle ladite méthode de pronostic est telle que :
▪ si aucun des 23 gènes dudit ensemble n'est exprimé, le taux de survie du patient est d'environ 59% à environ 78%,
▪ si
∘ aucun des gènes de l'ensemble B n'est exprimé et au moins 3 gènes de l'ensemble A sont exprimés, ou
∘ au moins 1 gène de l'ensemble B est exprimé et de zéro à 2 gènes de l'ensemble A sont exprimés, ou
∘ au moins 2 gènes de l'ensemble B sont exprimés et aucun gène de l'ensemble A n'est exprimé,
le taux de survie du patient est d'environ 27ù à environ 55%, et
▪ si
∘ un gène des gènes de l'ensemble B est exprimé et au moins 3 gènes de l'ensemble A sont exprimés, ou
∘ au moins 2 gènes de l'ensemble B sont exprimés et au moins 1 gène de l'ensemble A est exprimé
le taux de survie du patient est environ d'environ 3% à environ 13%.

4. Utilisation selon l'une des revendications 1 à 3, dans laquelle ladite méthode de pronostic est telle que :
▪ si aucun des 23 gènes dudit ensemble n'est exprimé, le taux de survie du patient est d'environ 59% à environ 78%,
▪ si aucun des gènes de l'ensemble B n'est exprimé et un ou deux gènes de l'ensemble A sont exprimés, le taux de survie du patient est d'environ 38% à environ 70%,
▪ si
∘ aucun des gènes de l'ensemble B n'est exprimé et au moins 3 gènes du premier ensemble sont exprimés, ou
∘ au moins un gène de l'ensemble B est exprimé et de zéro à 2 gènes de l'ensemble A sont exprimés, ou
∘ au moins 2 gènes de l'ensemble B sont exprimés et aucun gène de l'ensemble A n'est exprimé,
le taux de survie du patient est d'environ 27% à environ 55%, et
▪ si
∘ un gène des gènes de l'ensemble B est exprimé et au moins 3 gènes de l'ensemble A sont exprimés, ou
∘ au moins 2 gènes de l'ensemble B sont exprimés et au moins 1 gène de l'ensemble A est exprimé
le taux de survie du patient est.d'environ 3% à environ 13%.

5. Utilisation selon l'une des revendications 1 à 4, dans laquelle ladite tumeur du poumon a été auparavant classée histologiquement.

6. Utilisation selon la revendication 5, dans laquelle ladite tumeur classée histologiquement appartient à l'ensemble consistant en : l'adénocarcinome, le carcinome épidermoïde, les tumeurs basaloïdes, et les tumeurs neuroendocrines à grandes cellules.

7. Utilisation selon l'une des revendications 1 à 6, dans laquelle la détermination desdits au moins deux gènes comme étant exprimés est réalisée en utilisant une technique choisie parmi l'ensemble consistant en :
- PCR quantitative,
- Puce à ADN, et
- Northern blot.

8. Utilisation selon l'une des revendications 1 à 7, dans laquelle la détermination desdits au moins deux gènes comme étant exprimés est réalisée en utilisant des molécules d'acide nucléique consistant en des molécules de 15 à 100 nucléotides complémentaires desdits au moins 2 gènes.

9. Utilisation selon l'une des revendications 1 à 8, dans laquelle la détermination desdits au moins deux gènes comme étant exprimés est réalisée par puce à ADN utilisant
▪ au moins une sonde d'acide nucléique comprenant ou étant constituée par la séquence d'acide nucléique SEQ ID NO : 47 à SEQ ID NO : 53, et
▪ au moins une sonde d'acide nucléique comprenant ou étant constituée par la séquence d'acide nucléique SEQ ID NO : 54 à SEQ ID NO : 69.

10. Utilisation selon la revendication 7, dans laquelle la détermination desdites au moins deux protéines comme étant exprimées est réalisée en utilisant une technique choisie parmi l'ensemble consistant en :
- werstern Blot,
- ELISA,
- Immunofluorescence, et
- Immunohistochimie.

11. Utilisation selon les revendications 1 à 6, dans laquelle la détermination desdites au moins deux protéines comme étant exprimées est réalisée en utilisant des anticorps dirigés contre lesdites au moins 2 protéines codées par lesdits au moins deux gènes.


### SEQUENCE LISTING

<110> Université Joseph Fourier
<120> USE OF SPECIFIC GENES FOR THE PROGNOSIS OF LUNG CANCER AND THE CORRESPONDING PROGONOSIS METHOD
<130> IOB 09 CF UJF LUNG
<160> 69
<170> PatentIn version 3.5
<210> 1
   <211> 3280
   <212> DNA
   <213> Homo sapiens
<400> 1
   gggggaggga tgactaaaga caacggctgt aagagaactc cacaagagag tcagaacgaa 60
   aatgtgcaac aagtgcggcg gctcctacca tggcaggtga ttcgaggact cagcacgagg 120
   cgagagtagg gaccaggaag agccggaaaa cccgcctgtg attggccgtc cacgggtatc 180
   ggtcgttgtg attgggtgag gcccagacag acagctgcgt tttgaaccgc gtagggttct 240
   gggtagcaaa ggccttgcaa ggctcttaac cgaaaggggg agggggaagg tcgccaacaa 300
   acggctgagc tcacaatcct ggccggggcg tccccctccc ccatggagag agccagaccc 360
   gagccgccgc ctcagccgcg cccgttgcgt cccgctccgc ccccgctgcc ggtcgagggc 420
   acctcctttt gggcagcagc catggagccc cctccgtcgt ctcccacact gagcgcggca 480
   gccagtgcga ccttggcctc gtcgtgcggg gaggcagtgg cgtccggctt acagcccgcg 540
   gtgcggcggc tgctgcaggt gaagccagag caggtgttgc tgctaccaca gcctcaggcc 600
   cagaacgagg aagccgctgc ctcgtccgcg caggcgcggc tgttgcagtt caggcccgac 660
   ctgcggctcc tgcagccgcc gacagcgtca gacggcgcca cctccaggcc cgagttgcac 720
   ccggtgcagc ccctggcgct gcatgtcaag gccaagaagc agaagctggg gcccagcctg 780
   gatcagtcag tggggcctcg aggggccgtc gaaaccggtc ctagagcctc cagggtggtc 840
   aagttggaag gccccgggcc ggccctcggc tacttccgag gggacgagaa gggcaagctg 900
   gaggcggagg aggtcatgag agactcgatg caaggcgggg caggcaaaag cccggcagcc 960
   atccgagaag gtgtgatcaa aacggaggaa cccgagagac tcctcgagga ctgcaggctc 1020
   ggcgcggagc ccgcgtccaa tggcctggtt catggcagcg cggaggtcat cttggcccca 1080
   acgtccggtg cctttgggcc gcaccagcaa gaccttagga tccctttgac gctccacacg 1140
   gtcccccctg gggcccggat ccagtttcag ggagctccgc cttcagagct gataagattg 1200
   accaaggtcc ccctgacacc agtgcctact aaaatgcagt ccctactgga gccttctgta 1260
   aaaattgaaa ccaaagatgt cccgctcacc gtgttgccct cagatgcagg cataccagat     1320
   actcccttca gtaaggacag aaatggtcat gtgaagcgac ccatgaacgc atttatggtt     1380
   tgggcaagga tccaccgacc agcactagcc aaagctaacc cagcagccaa caatgcagaa     1440
   atcagtgtcc agcttgggtt agagtggaac aaacttagtg aagaacaaaa gaaaccctat     1500
   tacgatgaag cacaaaagat taaggaaaag cacagagagg aatttcctgg ttgggtttat     1560
   cagcctcgtc cagggaagcg aaaacgattc cctctaagtg tttccaatgt attttctggt     1620
   accacacaga atatcatctc tacaaatcct acaacagttt atccttaccg ctcacctacg     1680
   tactctgtgg taattcccag cctacagaat cccatcactc atccagttgg tgaaacctca     1740
   cctgctatcc agctgcccac acctgcagtc cagagcccaa gccctgtcac acttttccag     1800
   cccagcgtct ccagtgctgc tcaggtggct gtccaggatc caagtctacc tgtctatcca     1860
   gcactcccac cccaacgctt tactgggcct tcccaaacag acactcatca gctgcattct     1920
   gaagccactc acactgtgaa gcaacccact cctgtctctc tagagagcgc caacaggatt     1980
   tcaagtagtg caagtactgc ccatgccaga tttgcaactt cgaccatcca acctcctagg     2040
   gagtattcca gcgtttcccc ttgtcccaga agtgctccaa tcccccaggc ttctcccatt     2100
   ccacacccac atgtctacca gccccctccc cttggccatc cagccacact gttcgggaca     2160
   ccaccaagat tctcttttca tcacccttac ttcctacccg gacctcacta cttcccatca     2220
   agtacatgcc cttacagtcg gcctcccttt ggctatggaa attttccgag ttcaatgcca     2280
   gaatgcctta gttattatga agacaggtac ccaaaacatg agggtatctt ttcaacttta     2340
   aatagagact attcttttag agactactca agtgaatgca cacacagtga aaattctcgg     2400
   agttgtgaga acatgaatgg aacttcttac tataacagtc atagccacag tggggaagaa     2460
   aacttaaacc ctgtgcctca gctggacatt ggaaccttgg agaatgtctt cacagccccg     2520
   acatcaactc cttctagcat ccagcaagtc aatgtcaccg acagtgatga ggaggaagaa     2580
   gaaaaagtgc tcagggattt ataattttaa aacaaatatg cacagaaaat aaacatttct     2640
   taaaatatat tctgggtcag ttggtatgag aaaaaaaaaa gcctagaatt ctttgttgaa     2700
   agttttcagt cgtgatttga ggagttaaaa ccaaatgcaa tttatgtctt cataaaattt     2760
   tgattagtga aactagagtc tggatgtttc attgtaggaa tatttaagtt attaagtagt     2820
   ttaattttaa tggctgaaat ttgcatcaac atgtattatt attactttat cctggaacat     2880
   gcaaaatact gaagcctcac agttgtatgt gaggggaaag gggaaataaa tctagcatag      2940
   tgtgattttt attttatctc aggatacatt ttttaaatga ttttttgttt gctttttatg     3000
   taatacttat ggatgttgtc aatttttgat gtaacatttt gaaagtattt tgacaactcc     3060
   tagtgaactt ggacttggtt gctaaattta acttacacta ataaccaatt ataagttcca     3120
   aatgtgtttt aatggcacct gggtgattct tcagctaaat ttagtcattt ctgtttctaa 3180
   atatttttat cattttaaaa tatttttttt ccatttggca tacatcgttc tttgttgtaa 3240
   ttaaataaac atagataaaa ttgttaaaaa aaaaaaaaaa 3280
<210> 2
   <211> 1479
   <212> DNA
   <213> Homo sapiens
<400> 2
   aggcgaggga aactgagggc gaaagttgtg tgtcgtgttg gcaggagggc ctagaaggga      60
   aagactgtgc gttgatacca aactgccccc actccgtggg cgggtcagga gaggcctttg     120
   gaagagcgtc tcaactcgga ctggagcctc ttctctccca ccgcggtcta gtgggacaat     180
   gtcatattat aaatttggaa tgctgaatag aaaattatag attttgatat tgaaggaaat     240
   gaagcgaagc ctaaatgaaa attcagctcg aagtacagca ggctgtttgc ctgttccgtt     300
   gttcaatcag aaaaagagga acagacagcc attaacttct aatccactta aagatgattc     360
   aggtatcagt accccttctg acaattatga ttttcctcct ctacctacag attgggcctg     420
   ggaagctgtg aatccagagt tggctcctgt aatgaaaaca gtggacaccg ggcaaatacc     480
   acattcagtt tctcgtcctc tgagaagtca agattctgtc tttaactcta ttcaatcaaa     540
   tactggaaga agccagggtg gttggagcta cagagatggt aacaaaaata ccagcttgaa     600
   aacttggaat aaaaatgatt ttaagcctca atgtaaacga acaaacttag tggcaaatga     660
   tggaaaaaat tcttgtccag tgagttcggg agctcaacaa caaaaacaat taagaatacc     720
   tgaacctcct aacttatctc gcaacaaaga aaccgagcta ctcagacaaa cacattcatc     780
   aaaaatatct ggctgcacaa tgagagggct agacaaaaac agtgcactac agacacttaa     840
   gcccaatttt caacaaaatc aatataagaa acaaatgttg gatgatattc cagaagacaa     900
   caccctgaag gaaacctcat tgtatcagtt acagtttaag gaaaaagcta gttctttaag     960
   aattatttct gcagttattg aaagcatgaa gtattggcgt gaacatgcac agaaaactgt    1020
   acttcttttt gaagtattag ctgttcttga ttcagctgtt acacctggcc catattattc    1080
   gaagactttt cttatgaggg atgggaaaaa tactctgcct tgtgtctttt atgaaatcga    1140
   tcgtgaactt ccgagactga ttagaggccg agttcataga tgtgttggca actatgacca    1200
   gaaaaagaac attttccaat gtgtttctgt cagaccggcg tctgtttctg aacaaaaaac    1260
   tttccaggca tttgtcaaaa ttgcagatgt tgagatgcag tattatatta atgtgatgaa    1320
   tgaaacttaa gtagtgataa aaggaagttt agcataaatt atagcagttt tctgttattg    1380
   cttaatttac catctccata gttttatagc tactattgta tttcacttgt tgaattaaag    1440
   tatttgaatt cttttaaatg tggaaaaaaa aaaaaaaaa                            1479
<210> 3
   <211> 1731
   <212> DNA
   <213> Homo sapiens
<400> 3
   ttagggcggg agcccggcga gggcgccggt gctttgttct gtctgaggcc aggaagtttg      60
   accgcgctgc catgccgaac cgtaaggcca gccggaatgc ttactatttc ttcgtgcagg     120
   agaagatccc cgaactacgg cgacgaggcc tgcctgtggc tcgcgttgct gatgccatcc     180
   cttactgctc ctcagactgg gcgcttctga gggaggaaga aaaggagaaa tacgcagaaa     240
   tggctcgaga atggagggcc gctcagggaa aggaccctgg gccctcagag aagcagaaac     300
   ctgttttcac accactgagg aggccaggca tgcttgtacc aaagcagaat gtttcacctc     360
   cagatatgtc agctttgtct ttaaaaggtg atcaagctct ccttggaggc attttttatt     420
   ttttgaacat ttttagccat ggcgagctac ctcctcattg tgaacagcgc ttcctccctt    480
   gtgaaattgg ctgtgttaag tattctctcc aagaaggtat tatggcagat ttccacagtt     540
   ttataaatcc tggtgaaatt ccacgaggat ttcgatttca ttgtcaggct gcaagtgatt     600
   ctagtcacaa gattcctatt tcaaattttg aacgtgggca taaccaagca actgtgttac     660
   aaaaccttta tagatttatt catcccaacc cagggaactg gccacctatc tactgcaagt    720
   ctgatgatag aaccagagtc aactggtgtt tgaagcatat ggcaaaggca tcagaaatca     780
   ggcaagatct acaacttctc actgtagagg accttgtagt ggggatctac caacaaaaat     840
   ttctcaagga gccctctaag acttggattc gaagcctcct agatgtggcc atgtgggatt     900
   attctagcaa cacaaggtgc aagtggcatg aagaaaatga tattctcttc tgtgctttag     960
   ctgtttgcaa gaagattgcg tactgcatca gtaattctct ggccactctc tttggaatcc    1020
   agctcacaga ggctcatgta ccactacaag attatgaggc cagcaatagt gtgacaccca    1080
   aaatggttgt attggatgca gggcgttacc agaagctaag ggttgggagt tcaggattct    1140
   ctcatttcaa ctcttctaat gaggaacaaa gatcaaacac acccattggt gactacccat    1200
   ctagggcaaa aatttctggc caaaacagca gcgttcgggg aagaggaatt acccgcttac    1260
   tagagagcat ttccaattct tccagcaata tccacaaatt ctccaactgt gacacttcac    1320
   tctcacctta catgtcccaa aaagatggat acaaatcttt ctcttcctta tcttaatgat    1380
   ggtactcttt tcaatttctg aaaacagtaa caggcccaac ttccttctta ctacagtcat    1440
   attaaacaga tcacatcaat gacaaatgtc actactataa aaactactta atttgtaagg    1500
   aaattgtttc atagatttaa aaaaattgtg gttggagagc atcttggcat ttgtgctttt    1560
   tttcttgagg gattgttctg cttcctggct gtatgatggg tatatcatta aagtttggag    1620
   tcctatatga acaaaactga catttttaga gttgtacttt tgggaatgtt atagattgat    1680
   cattctttct cctgataata aaggtattga atatctgtta tgaaaggttc t             1731
<210> 4
   <211> 531
   <212> DNA
   <213> Homo sapiens
<400> 4
   acgagcactg gagcttgcgt tacttggcct cacctcacct gtgctgtcca cgcctggctt      60
   tgtctcacct gacgcgatat gcctctcctg cgtgggcgct gtcctgcccg ccgccactac     120
   cgccgcttgg ccctgctcgg cctgcagccc gctccccgct tcgcccactc ggggcccccg     180
   cgccagcggc ccctgtctgc cgcggaaatg gctgttggac ttgtggtgtt ttttacgacc     240
   ttcttaacac cagctgcata tgtgctaggc aacctgaagc agttcagaag gaattagatg     300
   gaagatgatg ttgaacagct gttaacgtcc aaaaaacttt cagaaaaagc tgtgtttttg     360
   ttaacgagca aaattgccta gttgagttga tgcaaccatt gtggtattca ctttcctcat     420
   gtttatgatg aatattttgc acttttttag tactgtgcat tatatagatg tatagtcaaa     480
   aatgttctgc ttaagtgtta aataaaacgg aaacacttat tcgtgcttgg t              531
<210> 5
   <211> 2652
   <212> DNA
   <213> Homo sapiens
<400> 5
   tcccttgact tgcttgcgga gggagcggcc ggcggaggga gcggcaggtg gagggagtgg      60
   cacgaggcat gcggagggag ctgcaccgac atcacataaa cgcactgggc agctcgcagg     120
   cgccattcgc tcttcagacg ccggagacgt aggagtgggt cttcagactc caaaggggtt     180
   ggactaatgg cggatgctga ggcgagggct gagttcccgg aggaggccag acctgacagg     240
   ggcaccttgc aggtgttgca agatatggcc agccgcttgc gaatccattc catcagggcc     300
   acatgctcca cgagctccgg ccaccctaca tcatgtagca gttcttctga gatcatgtct     360
   gtgctgttct tctacatcat gaggtacaag cagtcagatc cagagaatcc ggacaacgac     420
   cgatttgtcc tcgcaaagag actgtcgttt gtggatgtgg caacaggatg gctcggacaa     480
   ggactgggag ttgcatgtgg aatggcatat actggcaagt acttcgacag ggccagctac     540
   cgggtgttct gcctcatgag tgatggcgag tcctcagaag gctctgtctg ggaggcaatg     600
   gcctttgctt cctactacag tctggacaat cttgtggcaa tctttgatgt gaaccgcctg     660
   ggacacagtg gtgcattgcc cgccgagcac tgcataaaca tctatcagag gcgctgcgaa      720
   gcctttgggt ggaacactta tgtggtggac ggccgggacg tggaggcact gtgccaggta      780
   ttctggcagg cttctcaggt gaagcacaag cccactgctg tggtggccaa gaccttcaag      840
   ggccggggca ccccaagtat tgaggatgca gaaagttggc atgcaaagcc aatgccgaga      900
   gaaagagcag atgccattat caaattaatt gagagccaga tacagaccag caggaatctt      960
   gacccacagc cccccattga ggactcacct gaagtcaaca tcacagatgt aaggatgacc     1020
   tctccacctg attacagagt tggtgacaag atagctactc ggaaagcatg cggtctggct     1080
   ctggctaagc tgggctacgc gaacaacaga gtcgttgtgc tggatggtga caccaggtac     1140
   tctactttct ctgagatatt caacaaggag taccctgagc gcttcatcga gtgctttatg     1200
   gctgaacaaa acatggtgag cgtggctctg ggctgtgcct cccgtggacg gaccattgct     1260
   tttgctagca cctttgctgc ctttctgact cgagcatttg atcacatccg gataggaggc     1320
   ctcgctgaga gcaacatcaa cattattggt tcccactgtg gggtatctgt tggtgacgat      1380
   ggtgcttccc agatggccct ggaggatata gccatgttcc gaaccattcc caagtgcacg     1440
   atcttctacc caactgatgc cgtctccacg gagcatgctg ttgctctggc agccaatgcc     1500
   aaggggatgt gcttcattcg gaccacccga ccagaaacta tggttattta caccccacaa     1560
   gaacgctttg agatcggaca ggccaaggtc ctccgccact gtgtcagtga caaggtcaca     1620
   gttattggag ctggaattac tgtgtatgaa gccttagcag ctgctgatga gctttcgaaa     1680
   caagatattt ttatccgtgt catcgacctg tttaccatta aacctctgga tgtcgccacc     1740
   atcgtctcca gtgcaaaagc cacagagggc cggatcatta cagtggagga tcactacccg     1800
   caaggtggca tcggggaagc tgtctgcgca gccgtctcca tggatcctga cattcaggtt     1860
   cattcgctgg cagtgtcggg agtgccccag agtgggaagt ccgaggaatt gctggatatg     1920
   tatggaatta gtgccagaca tatcatagtg gccgtgaaat gcatgttgct gaactaaaat     1980
   agctgttagc tttggtcttt tggcctcttt accctgtgtt tatgtttgtt ccaaaaccat     2040
   catttaaatc tctactgtca cattttgttt cttaaaagca aagccagcta acaccttcat     2100
   tcatccctag ttcggaaatt caagctaact acttaccctt taaactgtca ctgcatatgc     2160
   aagtaccgct ctaatttttg gatcattaaa gggagttaca caacttttaa gtgaaaaaaa     2220
   taggtaacaa aacaaccacc tgatagtaag ttttctgata agactataga taagtggtag     2280
   aggtaatcaa ttcttccgaa gtgtttcctt cgtgaataac tggtagaggt aatagttttt     2340
   tcaatgtatt tccttcatga gtaaagaaaa tgtggattga agtatagatt ccagtagcct     2400
   agtttccaca gcacgataac accatgacgc ctactgctgt tcccaccttg ggattctgtg     2460
   tgctgccatc ccacctgcag ctgccctgga attcccttcg ctgtttgcct tcatctccct     2520
   ccacgtttga gaggctgtca ggcagcagcg aaagcttgtt aggatgtcct gtgctgcttg     2580
   tgatgagagc ctccacactg tactgttcaa gtcaatgtta ataaagcatt tcaaaaccag     2640
   ctgctttatt ca                                                         2652
<210> 6
   <211> 2521
   <212> DNA
   <213> Homo sapiens
<400> 6
   aaacgagtgg agacacgagg accagcgcga gcggtcccgg tgggctaccc tccccctgcg      60
   acgacccccc ctcgctctga ccgactggtc ccctaaacgg tggcggcggt ttttggtcgt     120
   tgggccccgg gatttaggac caacatttga agacccgaag gggaactgca accatgaatg     180
   aagaaaatat agatggaaca aatggatgca gtaaagttcg aactggtatt cagaatgaag     240
   cagcattact tgctttgatg gaaaagactg gttacaacat ggttcaggaa aatggacaaa     300
   ggaaatttgg cggtcctcct ccaggttggg aaggtccacc tccacctaga ggctgtgaag     360
   tttttgtagg aaaaatacct cgtgatatgt atgaagatga gttagttcct gtatttgaaa     420
   gagctgggaa gatatatgaa tttcgactta tgatggaatt tagtggtgaa aatcgaggtt     480
   atgcttttgt gatgtacact acaaaagaag aagcccaatt agccatcaga attcttaata     540
   attatgaaat tcgaccaggg aagtttattg gtgtgtgtgt aagcctggat aattgtagat     600
   tatttattgg agctattccc aaggaaaaga agaaagaaga aattttagat gaaatgaaga     660
   aagttacaga aggagttgta gatgtcattg tttatccaag tgcaactgat aagaccaaaa     720
   atcgtggttt tgcatttgtg gaatatgaat ctcacagagc tgctgctatg gcaaggagga     780
   aactaattcc aggaacattc caactatggg gccacaccat tcaggtagat tgggctgacc     840
   cagagaaaga ggtggatgag gaaaccatgc agagagttaa agttctttat gtaagaaatt     900
   taatgatctc aactacagag gaaacaatta aagcagaatt caataaattt aagcctggtg     960
   cagttgaacg ggtaaagaaa cttagagatt atgcttttgt tcactttttc aaccgagaag    1020
   atgcagtggc tgccatgtct gttatgaatg gaaaatgcat tgatggagca agtattgagg    1080
   taacactagc taaaccagta aataaagaaa acacttggag acagcatctt aatggtcaga    1140
   ttagtccaaa ttctgaaaat ctgattgtgt ttgctaacaa agaagagagc cacccaaaaa    1200
   ctctaggcaa gctgccaact cttcctgctc gtctcaatgg tcagcatagc ccaagtccgc    1260
   ctgaagttga aagatgcact tacccttttt atcctggaac aaagcttact ccaattagta    1320
   tgtattcttt aaaatccaat cattttaatt ctgcagtaat gcatttggat tattactgca    1380
   acaaaaataa ctgggcacca ccagaatatt atttatattc aacaacaagt caagatggga    1440
   aagtactctt ggtgtataag atagttattc ctgctattgc aaatggatcc cagagttact     1500
   tcatgccaga caaactctgt actacgttag aagatgcaaa ggaactggca gcccagttta     1560
   cattacttca tttggactac aatttccatc gcagctcaat aaatagtctt tcccctgtta     1620
   gtgctaccct ctcttctggg actcccagcg tgcttcctta tacttcaagg ccttattctt     1680
   atccaggcta tcctttgtca ccaacaatat cacttgctaa tggcagccat gttggacagc     1740
   ggctatgtat ctccaatcag gcctccttct tctgaagaaa atactaacat tagtatgaaa     1800
   atttgtgtaa atttgtagta tgaaaacttg caaattaaaa tattgtttta ttttagaatc     1860
   gggtttgcat atttggtttt aaaaaggtat ttattccaaa gtactaaaca tcagctataa     1920
   ttcagaataa catggagttg tagaatttat aaaaatgcaa agtttaaaaa gttattcagt     1980
   ggtttctctt gataaaggta cagcaaacta ctattctttt taaacttcta ggattttctt     2040
   ctactttctg agtgggcaat agaacctagt catttatgtt tttttttttt tttgcataat     2100
   tttactaaat agtatttcac aaatattaaa gcacttgaag acaatggtta tagtagattt     2160
   gattaccaag gatcactatc tgtactggag attagaacaa ttatatgacc agaagcatct     2220
   aaccattatg taaaaagaaa tgatgagaca aaaagattaa gatacaaatt ttgtgcagta     2280
   ctaaagaaaa agcagtctac cattgtggtc cttgaaaata actatagata tttttgttat     2340
   ttgttagaca caaattataa ttttgttgtt aatgtattta agcattttat agttatgctt     2400
   tgtgtttttg atattctttg tattgttaat aacaagtgtt atgggttttt aatgttgaaa     2460
   tcatgtgtta atttttgtac ttgaattcaa attttttgac attaaatatg tgatgcttct     2520
   a                                                                     2521
<210> 7
   <211> 1949
   <212> DNA
   <213> Homo sapiens
<400> 7
   aataaagggg tctgagccgg tcgcctgagc ctgaaaagtg ctgtcacgtc agcggaagga      60
   ggcgtcccag atcttctcag ctgtcttggt gccagccttc ctagtcttcc tacccacact     120
   cctacctgct gtcacaggcc acagccatca tgcctcgggg tcacaagagt aagctccgta     180
   cctgtgagaa acgccaagag accaatggtc agccacaggg tctcacgggt ccccaggcca     240
   ctgcagagaa gcaggaagag tcccactctt cctcatcctc ttctcgcgct tgtctgggtg     300
   attgtcgtag gtcttctgat gcctccattc ctcaggagtc tcagggagtg tcacccactg     360
   ggtctcctga tgcagttgtt tcatattcaa aatccgatgt ggctgccaac ggccaagatg     420
   agaaaagtcc aagcacctcc cgtgatgcct ccgttcctca ggagtctcag ggagcttcac     480
   ccactggctc tcctgatgca ggtgtttcag gctcaaaata tgatgtggct gccaacggcc 540
   aagatgagaa aagtccaagc acttcccatg atgtctccgt tcctcaggag tctcagggag 600
   cttcacccac tggctcgcct gatgcaggtg tttcaggctc aaaatatgat gtggctgccg 660
   agggtgaaga tgaggaaagt gtaagcgcct cacagaaagc catcattttt aagcgcttaa 720
   gcaaagatgc tgtaaagaag aaggcgtgca cgttggcgca attcctgcag aagaagtttg 780
   agaagaaaga gtccattttg aaggcagaca tgctgaagtg tgtccgcaga gagtacaagc 840
   cctacttccc tcagatcctc aacagaacct cccaacattt ggtggtggcc tttggcgttg 900
   aattgaaaga aatggattcc agcggcgagt cctacaccct tgtcagcaag ctaggcctcc 960
   ccagtgaagg aattctgagt ggtgataatg cgctgccgaa gtcgggtctc ctgatgtcgc 1020
   tcctggttgt gatcttcatg aacggcaact gtgccactga agaggaggtc tgggagttcc 1080
   tgggtctgtt ggggatatat gatgggatcc tgcattcaat ctatggggat gctcggaaga 1140
   tcattactga agatttggtg caagataagt acgtggttta ccggcaggtg tgcaacagtg 1200
   atcctccatg ctatgagttc ctgtggggtc cacgagccta tgctgaaacc accaagatga 1260
   gagtcctgcg tgttttggcc gacagcagta acaccagtcc cggtttatac ccacatctgt 1320
   atgaagacgc tttgatagat gaggtagaga gagcattgag actgagagct taaggcaggg 1380
   ctggcactat ttccttggcc agggtacctt atggggccat atcctacaga tcctcccatt 1440
   tctagggagg tctgaagtag aattttcact ttatgttaga agagagtagt gagctttcta 1500
   agtagtgcag tatagtagag gctggaggga acaagatatg tatctttctt ttgttacaca 1560
   tgagtaactt gcagatttat gttttatctc tgtcagttat caacattgtt cctgttaagt 1620
   gaaggtttat tttgcttcag attatacaat tatcaataac atagctctca cattcatggc 1680
   tgtttaacca atctgaaagt tacggtttgg gaattaataa aacaaagtca tacaacacat 1740
   tttctttgta attgagaact agataacatg gtaacagaga attgattttc atatgaatct 1800
   taactccaca gtaaaatagt tgacatcata atatgaagag aaagaaaagg aaaaacagaa 1860
   atgtaaaagt tgtttaattc ttggtttgcc taattcgttt tcctatttct tttcatacaa 1920
   ataaaggata cctggattta tttaggtta 1949
<210> 8
   <211> 2499
   <212> DNA
   <213> Homo sapiens
<400> 8
   cttctaattc tgttattgca actgcagacc gttacctggt acgctggctg ctacctccct 60
   cactcttgtc agagtcggag ctacaggcag tgccttcagc tctgagctca ggcatcccgg 120
   tccctgtttt tgcggttaag gactctaaag tgttgtgtcg tgttcatcaa ctttttctca      180
   acttccctgg ctctacctct tctgccacaa acgtcagcat ggtggtatct gccgaccctt      240
   tgtccagcga gagggcagag atgaacatcc tagaaatcaa ccaggaattg cgctcgcagc      300
   tggcagagag caatcagcag ttccgagacc tcaaagagaa attccttata actcaagcta      360
   ctgcctactc cctggccaac cagctgaaga aatacaagtg tgaagagtac aaagacatca      420
   tagactctgt gctgagggat gaactgcagt ccatggagaa gctggcagag aagctcaggc      480
   aagctgagga gctcaggcag tataaagccc tggttcactc tcaggcaaaa gagctgaccc      540
   agttacggga gaagttacgg gaagggagag atgcctcccg ctggctgaac aagcatctga      600
   aaaccctcct cactcctgat gaccctgaca agtcccaggg tcaggacctc cgagagcagc      660
   tggctgaggg gcacaggctg gcagagcacc ttgttcacaa gctgagccca gaaaatgatg      720
   aagatgaaga tgaggatgaa gacgacaaag acgaggaggt tgagaaagta caggaatcac      780
   ctgcccccag agaggtgcag aagactgaag aaaaggaagt ccctcaggac tcactggagg      840
   aatgtgctgt cacttgttca aatagtcaca acccttctaa ctccaaccag cctcacagga      900
   gcaccaaaat cacatttaag gaacacgaag tcgactctgc tctggttgta gagagtgaac      960
   accctcatga tgaagaggag gaagctctaa acattccccc agaaaatcaa aatgaccatg     1020
   aggaggagga ggggaaagcg ccagtgcccc ccagacacca tgacaagtcc aactcttacc     1080
   ggcatcgtga agtctctttc ttggcattgg atgaacagaa agtttgctcc gctcaggatg     1140
   ttgccaggga ttactccaat cccaaatggg atgaaacctc acttggcttc ctcgaaaagc     1200
   aaagtgatct tgaagaggtg aaaggacaag aaacagttgc tcccaggctc agcaggggac     1260
   cgctgagagt ggacaagcat gaaatccccc aggagtcact ggatggatgt tgcttgactc     1320
   cttccatcct tcctgacctg actccctcct accaccctta ttggagcact ttgtactctt     1380
   ttgaagacaa gcaagtcagc ttggctcttg tagacaaaat taaaaaggat caagaggaga     1440
   tagaagacca aagcccacca tgccccaggc tcagccagga gctgccagag gtgaaggagc     1500
   aggaagtccc agaggactct gtgaatgaag tttacttgac tccctcagtt caccatgacg     1560
   tgtctgactg ccaccagcct tatagcagca ccttgtcctc attggaggat cagcttgcct     1620
   gctctgctct ggatgtagcc tcccccaccg aggcggcctg tccccaaggg acttggagtg     1680
   gagacttgag ccaccaccag tcagaggtgc aagtttcaca ggcacagctg gaaccaagca     1740
   ccctggtgcc cagttgtctg cgactacagc tggatcaagg gttccactgt gggaacggct     1800
   tggcccagcg gggcctttcc tccaccacct gcagcttctc agccaatgct gattctggga     1860
   accaatggcc cttccaagag ctggttttag agccctctct ggggatgaag aaccctcccc     1920
   agctggaaga tgatgcactt gaaggctcag caagcaacac acaagggcgt caagtcactg 1980
   gccggattcg tgcctccctt gtcctgatac tgaagaccat cagaagaaga ctcccgttca 2040
   gcaagtggag actggcattc agattcgctg gcccgcatgc tgagagcgca gagataccaa 2100
   atactgctgg aaggacgcaa aggatggcag gatgaaagaa tgtcacaaaa agcagctttt 2160
   ccacttgata aaaacaacta aaacagcaaa gcaagtttaa gtccaaacac aatactgcag 2220
   gggtccttca ctgaggattg aatttcagac acagaatact cttgatgact tcaagccact 2280
   atgctccttt gatttgagaa gccacattcc atccccctcc aattgtgatc aatacctagg 2340
   gagaccaatg cccagatgga caaatagcat tgaccggcgt tagccctgtt tctcaattcc 2400
   catcgtgtag agaacaggag tccgcagctg ctggcaggag acagcatgtc agccgggact 2460
   ctgccagggc agagtatgag caatgccatg ttcttgctg 2499
<210> 9
   <211> 1405
   <212> DNA
   <213> Homo sapiens
<400> 9
   atcttaagag gcgttccttt ttgcatagtt cccatgagca tgagagaaga agcaatgcac        60
   gctccggcag attcctagga accaaatacc tctgaggagc accagatttc agcttatggg       120
   atgctttgat tgctctgtgg ctgcatttag gagaaggaag ctgcagtcat gcgtcatcac       180
   tgccagcctc acatctcttg acagttaaag ccttagggtg gagcaaggga aaatttaaaa       240
   taacaaatga agcaaaagca agaggtgatg ttccaaagca gaggaaggct aagtttatat       300
   atacaaatgt caagtgtgta tagtgcaaaa ctaggaccag ttggtggaat ctgtggtcaa       360
   aaacaaaagc cttccttttt ttttttcaag gcccagtccc aagacgcaag accacttgcg        420
   ccagcagcgt gcatcagcaa gatagcaaaa gcaggacgag agctgcccgg aagacatcta       480
   cctggccaga agacacctac cctggccgga agacatgtac ccctgaagat agagaaagag       540
   gccatcgtgt actacgtagc agtcatgtca gactgggaca cttcctgttt acagaggact       600
   ataaaacccc tgtcctgtcc tcacttgggg ctgacgccat cttaggcctc agcccgcctg       660
   cagccaggcg ttcgttaaaa cagcatgttg ctccacaccg ccttgtattg tttgttggtc       720
   ccactctctg ggctcgaacc aatacaagca cctttcaagc agtatattct tcagtgtctt       780
   gatcctccaa ataactctct tctaattcct cctgaccaca aaaagcactt atactctagg       840
   atgactgatt ccagcccagt ggcctggcaa gggtgaatta caccttgcat atcacactct       900
   tgacatttgt gtgcgctagc ataagaatta taattgaaac agggatttaa gtatctcctc       960
   tctaggtgcc taccctcctt ggactcaggt caaatttatt aaaggaagtt ttgtttctag      1020
   ataggttgtt tgaaataaaa taacagaatg ttcaagtaac acagtgtacc tacagctttt      1080
   aacaaaattg aggacttggg tctcgaaaca atttcctttg attttcaggt attttatcta      1140
   taaaaaggga gataaagcat tagttcatag gacagttata tgtttaaatg tgataatgta      1200
   tattaaccac cttgcatgta ttcaaatgtg ttttgaaatc taacgtctac attttgatag      1260
   tttaactctt ctacataagt gacttacaac aggcattaaa tattgtttgg cattttcata      1320
   tatctgtaac tgtatcttaa tctacaatga gcttaatttt aagtgtagca taaaacagaa      1380
   ccttcaataa agtggtaata ttagg                                            1405
<210> 10
   <211> 3848
   <212> DNA
   <213> Homo sapiens
<400> 10
   agcgttgctg ctgccttgca gtttgatctc agactgctgt gctagcaatc agcgagactc 60
   cgtgggcgta ggaccctcca agccaggtga aagagcttat gatcctaagc acttccataa 120
   tagggtcagt agaatcatga tcgatgatca taatgtcccc actctacggg agatggtagc 180
   attctccaag gaagtgttgg agtggatggc tcaagattct gaaaacatcg tagtgattca 240
   ctgtaaagga ggcaaagaat agatatgttg gatattttgc acaagtgaaa catagctaca 300
   actggaatct ccctccaaga aaaacactgt ttataaaaag attcgttatt tattcgattc 360
   atggtgttgg aacaggcgat ggatatgatc taaaagtcca aatagtaatg aagaaaaaga 420
   ttgtcttttc ctgtacttcc ttaaagaatt gtcgggtatt tcatgacact gaaacagaca 480
   gggtaataac tgatgtgttc aactgtccac ctctgtatga tgatgtgaaa gtgcaagctt 540
   cctcttcaag agaagagggc agcacacctc gcagggctaa ctggaagggg gagccatcca 600
   ggagacctgt gctcaactga tgggtgggga gcaatagcga gaacgaggga gggacctgag 660
   agtggaagcc tttattggga tgtaaggtgt tacctgagca ggtttcctac ggggaggtct 720
   aactggtgga tttaatgcaa gcagtcatga gttccatgga gtcatgctgt gactgagagg 780
   tggtcattga tatatccaca tggtccatgc agagtacggg ggtctgtagg gaggttatat 840
   ctagctgtcc cataatgaag tagtcaccaa cagaaggttg tataaggcag atactgggat 900
   cagtcacatt gagaaacctg gaggaggtga actggaaact gtcaagggtg actgaaccct 960
   gcttctgata tcagaaagtc caatttatat ttgaaaggga tgctgaggca caaaaaaatt 1020
   gtaagaattc actacaaaaa tacttggcta tatataagca taggtcctta gtagattctg 1080
   tttagcacta tctaaaccag attcaaattt cagcatttaa attaaatatc tatcatggaa 1140
   aataaactat tccttgaaaa ttttggtaga aacagcaaga gaaagcaata gcattttctt 1200
   aagcctcctc ctctgtgtct tgagtgtgtt attatagaat gcagagtgct acctattgaa 1260
   tggttataat tatttgataa atatataaag gaataaagga aggaactttg atttctttgg 1320
   aatgatagtt cttggcatca attttacttt taaaatattt ttttttcttt ttaggatttt 1380
   cctaaatact atcacaacta cccttttttc ttctggttta acacatcttt aatacaaaat 1440
   aacgggtatg gatataatat caacccatag aaacaaccta atcttcaatg tctatgtata 1500
   agatgtaatg gcaagtcttt tgctggttgt cataagctta atttatagaa aacaaaaaat 1560
   ccttgagcca ccattgttca ttgccttact ccttttacgt tggctatttt aaaaatacag 1620
   ttgttcttga gacccccagt tgcagtatcc tcaaggtcca tgccatagga ctgtgttatg 1680
   agctcaaaag tattataatc agatcttaag tgtggaagta aattcctccc agagaagttc 1740
   aatatgaatc tgctcagtac cttcaacatg tcaggtcctc agtaggtgct gatttaccaa 1800
   tgacgaacca ccaccaaatt ttgtgctaaa gtaagggagg acctagggaa gcttcagcta 1860
   gctgaaaagc tgactgacac acttatatct aggagaagtt acaagacaca gtaagtatta 1920
   agaaatacag ctaaaaaatc attaaaattg gtagtctccc atttaaacat gggtttctaa 1980
   taactgaatt gggaaaactt tcttaaaaac tattaattgg aggctgggtg tggtggctca 2040
   tgcctgtaat cctagcactt tgggaggctg aggcgggcgg atcacctaag gttggaagtt 2100
   cgagactagc ctggccaaca tggtaaaact ccgtctctac taaaaataca aaaatcagcc 2160
   aggcgtggtg gcacatgcct gtaatcccag ctactcagga ggctgagcca gtagaatcgc 2220
   ttgaacccag gaggcagatt gcagtgagcc gagatcgcac cactacactc cagcctgggc 2280
   gacagagtga gactctgtct aaagaaaaaa gcaaaaaaac agaacaacta ttacttggat 2340
   ttggagatta ttgttcccag aaaaccttct gccatatttg gaaacttatt tctcagtcta 2400
   gaagttctcc actttaagta gcatttgttc tgtgctggtg aaaaactgag atttttttgt 2460
   attaaccata ctcttcaata caaaaggaga aaatattttt aaaatgcttc aggtcacagt 2520
   tgaggcagtt gctatgattg catgtggcat gaattggtag ttattgttac aaccagttct 2580
   agtcttttct tcaaatctga gctggatcta ataactcctt aagtccagca aggcaacagt 2640
   aaattaaacc tctggtctac acacttgcaa tacatacaca tttaatagat tttgatagag 2700
   tgaactttgg attggatgga aattttttaa aaatttgttt cttggatgca tacaaacaat 2760
   aagctttgac tcctaacatg agcaaagtcc ctcaattgtg agagctgggt ggagcttcat 2820
   ttgttgctgc tcctcaaatt gattcttggt aaaggataca gatttttcct ttgaaacacc 2880
   atgttcattt tggggaagca ataagttaga tcacctttat tttcactttt atataaattt 2940
   ctaaagattt ctgtaatatt taaatttata tactattggt aaagctgttt ttcttagttg 3000
   tgaaattgtt gtttagccaa aaatgccaac ttctgtcttt tagaacacta ggcataaatg 3060
   ggttaaccaa tttatgccta gtgttccatt attggaatgc taagcatgtg ggatttattt     3120
   atatcctact gctcaaggtc atcgccaagg gctgtttgca aaaattcaaa aaattgcaac     3180
   ctcaggcata aattaaaaga gatatagtat tttattattg ggttttgata catgtctaat     3240
   cagactgatt tctgtcacat atagaaattt agatactgta ttaaacctgg atgtcattaa     3300
   ttccataaaa agcaacgtta aaagaatcag tagcatgtgt tactgatgtg ttgctgaaga     3360
   ttaagatatt tttaagtctc accgaaaagg tagaaggagc caactgagac acaaaaaggg     3420
   gctgaggttc tattcatggt gagcaagtct ttttttttgt ttgtttcttc aagctctaac     3480
   aagggtgcct actacatggc ttttcagtta gccccaaaat aagatgtaac aatttttttt     3540
   tctattctta ggctttatct acaaagaaat gaattggata atcttcataa acaaaaaaca     3600
   tggaaaattt atcaaccaga atatgcagta gagatatatt ttaatgagaa atgacttaag     3660
   ttatgttgta actggtagct gattaagtat agttccctgc accccttctg ggaaagaatt     3720
   atgttctttc taaccctgcc acatagttat atgttctaaa tcttccttgc tggtacatct     3780
   atattgatat atgtatacac atgttcttta taaatctatt aaatatatac agaaaaaaaa     3840
   aaaaaaaa                                                              3848
<210> 11
   <211> 1756
   <212> DNA
   <213> Homo sapiens
<400> 11
   gcttgggggc ggaaaagccg tggcgccccc ttgcgtggcg cgtcggtctc agagtcgcgt      60
   gacttcaacc ccctcttcgg gaggctgggt cgtcatgatc cggaccccat tgtcggcctc     120
   tgcccatcgc ctgctcctcc caggctcccg cggccgaccc ccgcgcaaca tgcagcccac     180
   gggccgcgag ggttcccgcg cgctcagccg gcggtatctg cggcgtctgc tgctcctgct     240
   actgctgctg ctgctgcggc agcccgtaac ccgcgcggag accacgccgg gcgcccccag     300
   agccctctcc acgctgggct cccccagcct cttcaccacg ccgggtgtcc ccagcgccct     360
   cactacccca ggcctcacta cgccaggcac ccccaaaacc ctggaccttc ggggtcgcgc     420
   gcaggccctg atgcggagtt tcccactcgt ggacggccac aatgacctgc cccaggtcct     480
   gagacagcgt tacaagaatg tgcttcagga tgttaacctg cgaaatttca gccatggtca     540
   gaccagcctg gacaggctta gagacggcct cgtgggtgcc cagttctggt cagcctccgt     600
   ctcatgccag tcccaggacc agactgccgt gcgcctcgcc ctggagcaga ttgacctcat     660
   tcaccgcatg tgtgcctcct actctgaact cgagcttgtg acctcagctg aaggtctgaa     720
   cagctctcaa aagctggcct gcctcattgg cgtggagggt ggtcactcac tggacagcag     780
   cctctctgtg ctgcgcagtt tctatgtgct gggggtgcgc tacctgacac ttaccttcac     840
   ctgcagtaca ccatgggcag agagttccac caagttcaga caccacatgt acaccaacgt     900
   cagcggattg acaagctttg gtgagaaagt agtagaggag ttgaaccgcc tgggcatgat     960
   gatagatttg tcctatgcat cggacacctt gataagaagg gtcctggaag tgtctcaggc    1020
   tcctgtgatc ttctcccact cagctgccag agctgtgtgt gacaatttgt tgaatgttcc    1080
   cgatgatatc ctgcagcttc tgaagaagaa cggtggcatc gtgatggtga cactgtccat    1140
   gggggtgctg cagtgcaacc tgcttgctaa cgtgtccact gtggcagatc actttgacca    1200
   catcagggca gtcattggat ctgagttcat cgggattggt ggaaattatg acgggactgg    1260
   ccggttccct caggggctgg aggatgtgtc cacataccca gtcctgatag aggagttgct    1320
   gagtcgtagc tggagcgagg aagagcttca aggtgtcctt cgtggaaacc tgctgcgggt    1380
   cttcagacaa gtggaaaagg tgagagagga gagcagggcg cagagccccg tggaggctga    1440
   gtttccatat gggcaactga gcacatcctg ccactcccac ctcgtgcctc agaatggaca    1500
   ccaggctact catctggagg tgaccaagca gccaaccaat cgggtcccct ggaggtcctc    1560
   aaatgcctcc ccataccttg ttccaggcct tgtggctgct gccaccatcc caaccttcac    1620
   ccagtggctc tgctgacaca gtcggtcccc gcagaggtca ctgtggcaaa gcctcacaaa    1680
   gccccctctc ctagttcatt cacaagcata tgctgagaat aaacatgtta cacatggaaa    1740
   aaaaaaaaaa aaaaaa                                                   1756
<210> 12
   <211> 1009
   <212> DNA
   <213> Homo sapiens
<400> 12
   gggcagaggc caagtgggca ccggatagcg ccagccccgc ccagagagcg aaatcatgga      60
   gccttccaag accttcatga gaaacctgcc aatcacacca ggctatagcg gctttgtgcc     120
   attcctcagc tgccaaggaa tgtccaagga ggatgacatg aaccactgtg tgaaaacctt     180
   ccaggagaaa acacagcgct ataaagaaca gctgcgggaa ttgtgctgcg cagtggccac     240
   tgccccgaaa ctgaaacctg tcaactccga ggagacggtc ctgcaggccc tgcaccagta     300
   caatctgcag taccaccccc tgatcctgga atgcaaatat gtaaagaaac ctctccagga     360
   gcccccgatc cctggctggg caggctacct gccgagagcc aaggtcactg aatttggctg     420
   tggcacgaga tacactgtca tggccaaaaa ctgctacaag gacttcctgg agatcacgga     480
   gagggccaag aaggcacatc tgaaaccata tgaagagtga ggagaaatgt ctctttcctt     540
   cctactaccg ttttaaaaag gggatgaaat gtttgcagtg gcctttctgc ttagctgggc     600
   cagctccctg caactcacac ggacggttcc tctcctagat ggaagctgcc ctgcccttgg     660
   aaggcccctg agagaggacc ccaaaactcc gctgacatgt ggctgtgctc agaggccaag     720
   tataccatgc agtgggaaga tgtatctaga gccactgtcc tccgcaaagt atgcagaagg     780
   ctagaagcgc agagtctccc aaggaggtga actttaagtg gggcttccaa aacctgccat     840
   tctcatgttg gaatcacgcc cagtgagcaa taaagaaatt tagtaacaag aattttttaa    900
   ctgccgcctg catcctgagt ggttgacggt tgcatgtcat taatgataaa gaccgttttt     960
   tgtcatgtgg gaataaagag gctgcttctc cgcaaaaaaa aaaaaaaaa               1009
<210> 13
   <211> 3185
   <212> DNA
   <213> Homo sapiens
<400> 13
   aaaaaagttt gggattccca gtctacaaaa ccccacagct cctaggaatt ctctcaccac     60
   ccttgtgcct ttaggcttcg gtagattgca aatgacctgc tttctttcgg atcccgggct    120
   gctttcggac acctgtcgaa tagtaaatcc caagtaaggt acctgcggtc gtcggcagat    180
   ctgaattttc ttcttggaca cctaataccc acagtcctcc agagaccgag aggttgatgt    240
   cactcccaat atcggaggaa gtatacaccc ccgtgtgaga tggtccttaa taatattcca    300
   cggcggaggg ggtgatatga ctacatacat ggcagaaagt ggaaacctcc cagggatatt    360
   gttcccacga tcctggaggg aagaagatga tgttactttc aatatgacag aaggtggatg    420
   aagtggtgga ctgcccctcc acacctgtgg acacgccccc actgatattc cttctaattg    480
   cagcgtggga gaggaggata tgacacgcga tatcgcaggg agtagaaaca cccctgtgat    540
   actgttctta atattcaggg aggatgagga tgatattact cccaatacag acgggtgtac    600
   accctctgta caccgagggt gtacacctgt ctgtgaaaga gttcgtaatc tccagagggg    660
   gagatgatat tactcacaat atggtaaaga ggctgtgagt ccacggagga tcctcagagc     720
   cagcggggga agaggggctg gctctcagtc cccgcctcgc gggggtgact ccccccagtg      780
   cgatgggggt cctaagagcc agtgggggaa gaggggctgg ctctgagacc ccgcctcgtg    840
   gggggtgcct ccccgccctg tgatgggggt ccgaagagcc agaaggctta gaggggctgg    900
   ctctcagtcc ccgcctcgcg gggggtgcct cctccccctg cgatgggggt cgtaagagcc    960
   actgggggaa taggggcttg ctctcagtca ccgcctcgcg gggggtgcct cctttccttt   1020
   ctacatagac acagtgacag tctgatctct ctttcttttc cctacagatg gacacgcccc   1080
   cactgatatt gtttttaatg cagcgtggga ggggaggata tgacacgcga tatcgcaggt   1140
   agtagaaaca cccctttgat agtgttctta atattcaggg aggaagaaga tgatgttact   1200
   cccaatacag acggatgtac accctctgta caccgagggt gtacacccgt ctgtgaagga 1260
   gttcgtaatc tccagaggtg gagatgatat tactcacaat atggtaaaca ggctgtgagt 1320
   ccatcgcgga tcctcagagc caggtgggga agaggggctg gctgtcagtc ccctcctcgc 1380
   ggggggtgcc tcccccactg ctatggggat cccaagagcc agtgggggaa gaggtgctgg 1440
   ctctcagtct ccgcctcgcg aggtgcctct ccaccctgcg atcggggtcc gaagagccag 1500
   gggggaagag gggctggctc tcttcgtgga tgattctttt tccattctca ggcagttttc 1560
   ttttttcttt ctttcttttt tttttttttt gagactgagt cttgctctgt tgcccatgct 1620
   ttgctcgatc tcgggtgact gcaaccactg cctcccaggt tcaagagatt ctcctgcctc 1680
   agcctcctga gtagctggga ctagaggcgt gtgtcaccac acccagctaa tttttgtatt 1740
   tttagtagag atggggtttc accatgtttg ccaggatggt ctctatctcc tgcccatgtg 1800
   atccacccac ctcagcctcc caaagtgctg ggattgcagg tgcgagccac cgggtccagc 1860
   ctctcaggcg attttcatac ctgcatactc tggtcactac tctgttaaac agtcaaggag 1920
   ggtaagtatt atcttcagat ttccagagct ctgtctctgt acagccctct cctcctcaat 1980
   attctgccct atgaattcta gccacattgg ccttcccagg ctcacagttc tgtcttctca 2040
   actcaggaag atctctgagt tccatctgca ttctttcttc ctgtgctgtg gcctggaaag 2100
   ttttctaagg tgttagggag gtcaattgtg gggctagcct catttgtttc tcatctcttg 2160
   aggatcactg ccctttgatg cttgattcca gtgattgatt ccctttgttg cttgagggcc 2220
   atagtttcat atattttgtc cagtagtttt gttgttttag gtcagaaagt aattttggtc 2280
   tctgttactc tatcttggcc agaagtgtaa gacctaagca tttacacatc aaaatactgc 2340
   acacataatt ttagtttaag ctacttttta aaaaatctcc ttcattttcc atttagcatt 2400
   ctatttaggg tattacattg gtttttttga aattctgtta ttggcagttt ctattgccta 2460
   tcaatcccat ttaaagatag tgcatagggt attctaaaat agctgttaag caaagagaaa 2520
   attgggcctg atagggtgag aatcacagct ctaataccta gagtgacctt ataatgtatt 2580
   gtccaaagga gatatttttg acagtgaaag agggtgttgt tagtaattat atcaggacca 2640
   tggcctaaac caggactatc ccaggaagcc tgggacatat ttgtacccca tctctattta 2700
   atgcctttat acaattcttt acttaattct accagccttt attgagcctg ctttctttgt 2760
   ctagctgagt gccacgtgct gacgtcacta agatcaatac agcaaactct gaaagatgga 2820
   cagagagaca ggagatggtc ctttataatg cagtgtgatc tgtgctgcaa tagagttttg 2880
   gaagctagaa gttcaaaacg aggtgttggc agcaccatgc tctctctgaa gatgctagga 2940
   agaatctgct ccatgccttt ccattcgctt ctggggtttc ctgcaagccc tgacattcct 3000
   tggcttgtag atgcatcacc ccagtttccg cccccatcat cacatggcct cctctctgtg 3060
   tgtgcctctg cgttccctct attcttcttc taaggacacc gacaccagtc atagtggatt 3120
   aagtgtccac ccctaaccaa ttacatctgc aacaacccta tttccaaata aagtcacatt 3180
   ctaag 3185
<210> 14
   <211> 2106
   <212> DNA
   <213> Homo sapiens
<400> 14
   agcggatcgt ctcttgccgc tgccatgaaa ggagtgcttt ttgcctccca ccatgattct      60
   gaggcctccc cagccatgta gaattagcca gcctgttttt gatgaagagc aactgtactg     120
   gagagaaagc ctgtattcat attcatcgtc atcaggcaaa tcgaggagct gacttgctta     180
   ggatcatctg ctggttgaat ccatggatgc agaaccccag attcggaggg ctgaatgtac     240
   tttcgatagg ttcctgagca gcagtgatta tagtcacagc agactggaca gtttggtgag     300
   caagttcagg ggctggctta gtgattcata gttctttctt cctcctcccc cagcagtttt     360
   gtttcagcag tgatttggtc aaatgtgaac cacaaaccag cagcaaaagc attttctagg     420
   aacttgttaa aactggaagg cccaaggttg cactcaggct tactgagtca gtcactccaa     480
   gtggacccca tccctaaacc ttgagactca ctgctcctct tttgatctgg gagagaaaca     540
   gaaaaaagaa ggaaaaaaaa tccccaatgt gagtatgctg gaaagtaaat ttgaatagct     600
   ttttatattg ggtacttact gtgggtaatt tacaaactat tggaaggaac cacaaattca     660
   aacacagcca aatttaaggt ctctttagca gaaatcaatc ttacatcatg gcagttctcc     720
   cattctccag tctttcactt gatagatcac ttcctcatta ggatgaccta cagcaattct     780
   acatgaactg ctctttacat ttaaacagtg ctttctgtcc taacatgtca aaatgcttga     840
   caagcaatca aaaggtagct cactaatttt ttcaactcat ggcagtaaat agcctgcatc     900
   taccatcact gctgtttact ggtgatttct tggcagggaa actttaaatg ccttagcaat     960
   gtctagaaag tcaatgacta aagaaaatat tcattttggc ttccataggg ctgtatttga    1020
   tgaactgctc actaacttct attattacgt ctcaagaata gataatgata ctcagcaagg    1080
   gcaaatccca aagaatagtc tatgctttcc ttgattgaat ctgatttttg agaaattaca    1140
   tggcccaatc tacatttaat atgatacaaa tgctacttaa ctccctgtta tttctggttg    1200
   catataattg atactacttg aagagtaaga agcagagtct cactacactg gggacccttt    1260
   gagagccaag tctgtgtctt agtcatcttt gatgccttag tgaccagtac catacctgga    1320
   atagtatatg tgatcagtaa ctcaaatgta tgtatttctt gaatgtatgg tgcatgaatg    1380
   agtcaagata cctttagcgt tggaagcaat ctgtcatgga gtaggaggag aaaaagtagt 1440
   gaaaaaactt ctaaattctc aaaacctagt gctgaactga atgctgataa taacttgaca 1500
   acgtcctttg gcattgcaga tgttgagata ccaacatcta atccgccaag aaagaatttc 1560
   agaattaaaa tttgggtgtg ttcttgcgct ggggatttct ctgacctacc ttctgataga 1620
   actttgaacc cgagtcaata gtcaatatac tagtttttta cttaaaactg taacatttta 1680
   atctaatttt ggggacgtga aataaaacta atgggaaact cttgaaattt ttatcactgg 1740
   aataacctaa ttttgaaaac actgatggtt agtttcttga aatattaata ttactacaag 1800
   tcataagtaa aagcattcta tcttaagtga gaaactataa agttggataa ttactatttg 1860
   agtttgtggc ttggtttgaa taaacacttg cttgttttaa gtaaaagttc agctgaagtg 1920
   acaatcaacc tttaatcttg taaagcttct gtgttagata ttttctatct ctaacatgcc 1980
   aaacatgcat attaaactga gtttttttgc atgcaaaaaa aaaaaaaaaa aaaaaaaaaa 2040
   aaaaaaaaaa aaaaaaaaaa aaaaaaaaaa aaaaaaaaaa aaaaaaaaaa aaaaaaaaaa 2100
   aaaaaa 2106
<210> 15
   <211> 459
   <212> DNA
   <213> Homo sapiens
<400> 15
   atggctcgta cgaagcaaac agctcgcaag tctaccggcg gcaaagctcc gcgcaagcag 60
   cttgctacta aagcagcccg taagagcgct ccggccaccg gtggcgtgaa gaaacctcat 120
   cgctaccgcc cgggcaccgt ggccttgcgc gaaatccgtc gctaccagaa gtccaccgag 180
   ctgctgatcc ggaagctgcc gttccagcgc ctggtgcgag aaatcgccca ggacttcaaa 240
   accgacctgc gtttccagag ctctgcggtg atggcgctgc aggaggcttg tgaggcctac 300
   ctggtgggac tcttcgaaga caccaatctg tgcgctattc acgctaaacg cgtcaccatc 360
   atgcccaaag atatccagct ggcacgtcgc atccgtgggg aaagggcata agtctgcccg 420
   tttcttcctc attgaaaagg ctcttttcag agccactca 459
<210> 16
   <211> 3591
   <212> DNA
   <213> Homo sapiens
<400> 16
   ttactgggcg tatggcgtac agacacgagg ccggcgcccg ggaggcggtg ttcatccgcc      60
   cgggaaaaga gcgcctgttg ctcgctgccc gcgtgtccct ggctctctcg ggaacccagc     120
   gccgaaggcg aggtgggcgc gggccgaagg aggtcctggg aggtcggcgg cgcggaggga     180
   tctccgcggg agccgttggg gctgttggcc tcgggctgag gtgcaaggac caggactagg      240
   gcgagggcag cggtccaaga aatagaaaac aatgactggg agagcccgag ccagagccag      300
   aggaagggcc cgcggtcagg agacagcgca gctggtgggc tccactgcca gtcagcaacc      360
   tggttatatt cagcctaggc ctcagccgcc accagcagag ggggaattat ttggccgtgg      420
   acggcagaga ggaacagcag gaggaacagc caagtcacaa ggactccaga tatctgctgg      480
   atttcaggag ttatcgttag cagagagagg aggtcgtcgt agagattttc atgatcttgg      540
   tgtgaataca aggcagaacc tagaccatgt taaagaatca aaaacaggtt cttcaggcat      600
   tatagtaagg ttaagcacta accatttccg gctgacatcc cgtccccagt gggccttata      660
   tcagtatcac attgactata acccactgat ggaagccaga agactccgtt cagctcttct      720
   ttttcaacac gaagatctaa ttggaaagtg tcatgctttt gatggaacga tattattttt      780
   acctaaaaga ctacagcaaa aggttactga agtttttagt aagacccgga atggagagga      840
   tgtgaggata acgatcactt taacaaatga acttccacct acatcaccaa cttgtttgca      900
   gttctataat attattttca ggaggctttt gaaaatcatg aatttgcaac aaattggacg      960
   aaattattat aacccaaatg acccaattga tattccaagt cacaggttgg tgatttggcc     1020
   tggcttcact acttccatcc ttcagtatga aaacagcatc atgctctgca ctgacgttag     1080
   ccataaagtc cttcgaagtg agactgtttt ggatttcatg ttcaactttt atcatcagac     1140
   agaagaacat aaatttcaag aacaagtttc caaagaacta ataggtttag ttgttcttac     1200
   caagtataac aataagacat acagagtgga tgatattgac tgggaccaga atcccaagag     1260
   cacctttaag aaagccgacg gctctgaagt cagcttctta gaatactaca ggaagcaata     1320
   caaccaagag atcaccgact tgaagcagcc tgtcttggtc agccagccca agagaaggcg     1380
   gggccctggg gggacactgc cagggcctgc catgctcatt cctgagctct gctatcttac     1440
   aggtctaact gataaaatgc gtaatgattt taacgtgatg aaagacttag ccgttcatac     1500
   aagactaact ccagagcaaa ggcagcgtga agtgggacga ctcattgatt acattcataa     1560
   aaacgataat gttcaaaggg agcttcgaga ctggggtttg agctttgatt ccaacttact     1620
   gtccttctca ggaagaattt tgcaaacaga aaagattcac caaggtggaa aaacatttga     1680
   ttacaatcca caatttgcag attggtccaa agaaacaaga ggtgcaccat taattagtgt     1740
   taagccacta gataactggc tgttgatcta tacgcgaaga aattatgaag cagccaattc     1800
   attgatacaa aatctattta aagttacacc agccatgggc atgcaaatga gaaaagcaat     1860
   aatgattgaa gtggatgaca gaactgaagc ctacttaaga gtcttacagc aaaaggtcac     1920
   agcagacacc cagatagttg tctgtctgtt gtcaagtaat cggaaggaca aatacgatgc     1980
   tattaaaaaa tacctgtgta cagattgccc taccccaagt cagtgtgtgg tggcccgaac 2040
   cttaggcaaa cagcaaactg tcatggccat tgctacaaag attgccctac agatgaactg 2100
   caagatggga ggagagctct ggagggtgga catccccctg aagctcgtga tgatcgttgg 2160
   catcgattgt taccatgaca tgacagctgg gcggaggtca atcgcaggat ttgttgccag 2220
   catcaatgaa gggatgaccc gctggttctc acgctgcata tttcaggata gaggacagga 2280
   gctggtagat gggctcaaag tctgcctgca agcggctctg agggcttgga atagctgcaa 2340
   tgagtacatg cccagccgga tcatcgtgta ccgcgatggc gtaggagacg gccagctgaa 2400
   aacactggtg aactacgaag tgccacagtt tttggattgt ctaaaatcca ttggtagagg 2460
   ttacaaccct agactaacgg taattgtggt gaagaaaaga gtgaacacca gattttttgc 2520
   tcagtctgga ggaagacttc agaatccact tcctggaaca gttattgatg tagaggttac 2580
   cagaccagaa tggtatgact tttttatcgt gagccaggct gtgagaagtg gtagtgtttc 2640
   tcccacacat tacaatgtca tctatgacaa cagcggcctg aagccagacc acatacagcg 2700
   cttgacctac aagctgtgcc acatctatta caactggcca ggtgtcattc gtgttcctgc 2760
   tccttgccag tacgcccaca agctggcttt tcttgttggc cagagtattc acagagagcc 2820
   aaatctgtca ctgtcaaacc gcctttacta cctctaacct gcagaagacg atgcagccgc 2880
   ttttcttttt gaaatgactt tgggattttt ttaagctttt atttactttt tttttaactg 2940
   ttatctttct ggatgaaact tgggaagggg attaggagat ctagcatttt atttctagca 3000
   ttgctattca ccggcttcct tattttatac gtaaaaatta agattttata ttttatcttc 3060
   ttgtttctca tagatatttt gtgagcattt ttttgtttat tttgaagaaa tgtggataag 3120
   atacttggta gtataaaaca gactctctga gagtatttga aatgtgtttg gagatttact 3180
   taaacgtact ttcaggagtg agcaagtcct acttataaac ctatattaac tttatttttg 3240
   agatacctgt tttgaattta aaggagataa gaggcgtaaa gtaggatgct cactacaacc 3300
   ataggtgggg tttcagctca tatcttaaag ataaaaggta ctattatata acctatacac 3360
   aagatacagg agaaaatatg cttgattttt atttggcagg ggggctaggt tgtatgggag 3420
   taaaaaaaac attgaaaatt tttaaattgt ccaaagaaac attttaagac tctttaacaa 3480
   aaaaggccat gagtaaatct ctatattaac attactattt attttgtttt ggaactggga 3540
   catgattcta tttgttataa aataaaattg atgtgattgt caccttattt g 3591
<210> 17
   <211> 819
   <212> DNA
   <213> Homo sapiens
<400> 17
   gtgatgctcc ctgggcctcc tgaccgcgcc ctcgcctggg aggcggggcg ggccgggttc      60
   tctctgtgac gtcacaaagg ccccgccatg cctctggctt tgacccttct gctgctctcg     120
   ggcttgggcg cccccggagg ctggggctgc ctgcagtgcg accccttggt gctggaggcc     180
   ctgggtcacc tgcgctccgc cctcatcccc agtcgcttcc agttggagca gctgcaggcg     240
   cgcgccgggg ccgtgctgat gggcatggag gggcctttct tccgggacta cgcgctgaac     300
   gtgtttgtgg ggaaagtgga gacaaatcaa ctggaccttg tggcgtcctt tgtcaagaac     360
   caaacgcagc acttaatggg taactctctg aaagatgagc ctctgctgga agagctggtg     420
   accctcaggg cgaatgtgat caaggaattc aagaaagttt taatttcata tgaattaaaa     480
   gcctgcaacc ccaaactttg ccgcttgcta aaagaagagg tgttggactg tttacattgc     540
   cagaggatca ctcccaagtg tatccacaaa aagtactgct ttgtcgaccg gcaaccccgc     600
   gtggccctgc agtaccagat ggacagcaaa tacccgagga accaggcgct gttgggcatc     660
   ctcatttctg tgtctctggc tgtctttgtc ttcgtggtca tcgtggtctc ggcttgtaca     720
   tacagacaaa accgaaaact cctgctgcag taggacggtg gtttgggggt aaggagaaag     780
   gaaaataaat ttaataaaat tggtgacaaa tccaaaaaa                            819
<210> 18
   <211> 5120
   <212> DNA
   <213> Homo sapiens
<400> 18
   ggactcgcac tcggcggttg ttccagaaga aagagacagc gatggcggca gaggcttcga      60
   agactgggcc ttctaggtct tcctaccagc gaatggggag gaagagtcag ccctggggtg     120
   ccgctgaaat ccagtgcacc aggtgtggaa ggagggtatc cagatcatcc ggtcaccatt     180
   gtgaacttca atgtggacat gctttttgtg aactatgctt gttaatgact gaagaatgca     240
   ccacaattat atgccctgat tgtgaggttg ctacagctgt aaatactaga caacgctact     300
   acccaatggc tggatatatt aaggaagact ccataatgga aaaactgcag cctaagacga     360
   taaagaattg ttctcaggac tttaagaaga ctgctgatca gctaactact ggtttagaac     420
   gttcagcctc cacagacaag actcttttga actcatcagc tgtaatgttg gacactaata     480
   ctgcagaaga aattgatgaa gcattgaata cagcacacca tagtttcgaa cagttaagca     540
   ttgctggaaa agcacttgaa cacatgcaga agcaaacgat agaggaaaga gaaagagtta     600
   tagaagttgt ggagaaacag tttgaccaac ttttggcttt ttttgattcc aggaaaaaga     660
   acctgtgtga agaatttgca agaactactg atgattatct atcaaattta ataaaggcta     720
   aaagctacat tgaagagaaa aaaaataatt tgaatgcagc tatgaacata gcaagagcat     780
   tacaattatc gccttctcta agaacatact gtgacctgaa tcagattatc cggactttgc     840
   agttaacttc agatagtgaa ttagcacaag ttagttctcc acaactaagg aaccctccca     900
   ggttgagtgt gaattgcagt gagatcatct gtatgttcaa caatatggga aagattgaat     960
   ttagggactc aacaaaatgt tatccccaag aaaatgaaat tagacagaat gttcaaaaga    1020
   aatataataa caaaaaggaa ctttcttgtt acgatacata cccaccgcta gaaaagaaaa    1080
   aggttgacat gtctgtccta accagtgaag caccaccacc tcctttgcaa cctgagacaa    1140
   atgatgtaca tttagaagca aaaaacttcc agccacagaa agacgttgca acagcatccc    1200
   ctaaaaccat tgctgtgtta cctcagatgg gatctagccc tgatgtgata attgaagaaa    1260
   ttattgaaga caacgtggaa agttctgcag agctagtttt tgtaagccat gtaatagatc    1320
   cttgccattt ctacattcgg aagtattcac aaataaaaga cgccaaagta ctggagaaga    1380
   aggtgaatga attttgcaat aggagttcac accttgatcc ttcagacatt ttggaactag    1440
   gtgcaagaat atttgtcagc agtattaaaa atggaatgtg gtgtcgagga actatcacag    1500
   aattaattcc aatagagggt agaaatacca gaaaaccttg tagtccaacc agattatttg    1560
   tccatgaagt tgcactaata caaatattca tggtagattt tggaaattct gaagtcctga    1620
   ttgtcactgg agttgttgat acccatgtga gaccagaaca ctctgctaag caacatattg    1680
   cactaaatga tttatgtctg gttctaagga aatctgaacc atatactgaa gggctgctaa    1740
   aagacatcca gccattagca caaccatgct cattgaaaga cattgttcca cagaattcaa    1800
   atgaaggctg ggaagaggaa gctaaagtgg aatttttgaa aatggtaaat aacaaggctg    1860
   tttcaatgaa agtttttaga gaagaagatg gtgtgcttat tgtagatctg caaaaaccac    1920
   caccgaataa aataagcagt gatatgcctg tgtctcttag agatgcgcta gtttttatgg    1980
   aactagcaaa gtttaagtca caatcactaa gaagtcactt tgaaaaaaat actactttac    2040
   actatcatcc acctattttg cctaaagaaa tgacagatgt ttcagtaacg gtttgtcata    2100
   taaatagtcc tggagatttc tatcttcagt tgatagaggg cctggatatt ttatttctat    2160
   taaagacaat cgaggaattc tataaaagtg aagatggaga aaatctggaa atcctctgtc    2220
   cagttcaaga tcaagcctgt gtagctaaat ttgaagatgg aatttggtac cgagcaaaag    2280
   ttatcggatt gcctggacat caggaagttg aagttaaata tgtggacttt ggtaatactg    2340
   caaaaataac aatcaaagac gtgcgtaaaa taaaggatga gtttctgaat gccccagaga    2400
   aggcaattaa atgtaagttg gcctatattg aaccatataa aaggacaatg cagtggtcca    2460
   aagaagctaa agaaaaattt gaagaaaagg ctcaagataa atttatgaca tgttcagtta    2520
   tcaaaattct ggaagataat gtgctcttag ttgagctttt cgattctctt ggtgctcctg    2580
   aaatgactac tactagtatt aatgaccagc tagttaaaga gggcctagca tcttatgaaa    2640
   taggatacat cctcaaagat aattctcaaa agcatattga agtttgggat ccttctccag     2700
   aagaaattat ttcaaatgaa gtacacaact taaatcctgt gtctgcaaaa tctctaccta     2760
   atgagaattt tcagtcactt tataataagg aattgcctgt gcatatctgt aatgtaatat     2820
   ctcctgagaa gatttatgtt cagtggttgt taactgaaaa cttacttaat agtttagaag     2880
   aaaagatgat agctgcttat gaaaactcaa aatgggaacc tgttaaatgg gaaaatgata     2940
   tgcactgtgc tgttaagatc caagataaaa atcagtggcg aagaggccag atcatcagaa     3000
   tggttacaga cacattggta gaggtcttgc tgtatgatgt gggtgttgaa ctagtagtga     3060
   atgttgactg tttaagaaaa cttgaagaaa atctaaagac aatgggaaga ctctctttgg     3120
   aatgttctct ggttgacata agaccagctg gtgggagtga caagtggaca gcaacagctt     3180
   gtgactgtct ttcattgtac ctgactggag ctgtagcaac tataatctta caggtggata     3240
   gtgaggaaaa caacacaaca tggccattac ctgtgaaaat tttctgcaga gatgaaaaag     3300
   gagagcgtgt tgatgtttct aaatatttga ttaaaaaggg tttggctttg agagaaagga     3360
   gaattaataa cttagataac agccattcat tatctgagaa gtctctggaa gtccccctgg     3420
   aacaggaaga ttcagtagtt actaactgta ttaaaactaa ctttgaccct gacaagaaaa     3480
   ctgctgacat aatcagtgaa cagaaagtgt ctgaatttca ggagaaaatt ctagaaccaa     3540
   gaaccactag agggtataag ccaccagcta ttcctaacat gaacgtattt gaggcaacag     3600
   tcagctgtgt tggtgatgat ggaactatat ttgtagtacc taaactatca gaatttgagc     3660
   taataaaaat gacaaatgaa attcaaagta atttaaaatg ccttggtctt ttggagcctt     3720
   atttctggaa aaaaggagaa gcatgtgcag taagaggatc cgatactctg tggtatcgtg     3780
   gcaaggtgat ggaggttgta ggtggcgctg tcagagtaca atatttagat catggattca     3840
   ctgaaaagat tccgcagtgc catctttacc ctattttgct gtatcctgat ataccccagt     3900
   tttgtattcc ttgtcagctc cataatacca cacctgttgg gaatgtctgg caaccagatg     3960
   caatagaagt tcttcaacaa ctgctttcaa agagacaggt ggacattcac attatggagt     4020
   tacctaaaaa tccatgggag aaattgtcta ttcacctcta ttttgatgga atgtcacttt     4080
   cttattttat ggcatactat aaatactgta cttctgaaca tactgaggag atgttgaaag     4140
   aaaaaccaag atcagatcat gataaaaagt atgaagagga acaatgggaa ataaggtttg     4200
   aggaattgct ttcggctgaa acagacactc ctcttttacc accatatttg tcttcatctc     4260
   tgccttcccc aggagaactc tatgctgttc aagttaagca cgttgtctca cctaatgaag     4320
   tgtatatttg ccttgattct atagaaactt ctaaccagtc taaccagcat agtgacacag     4380
   atgatagtgg agtcagcggg gaatcagaat ccgagagcct tgatgaagca ctgcagaggg     4440
   ttaataagaa ggtagaggcg cttcctcctc tgacggattt tagaacagaa atgccttgcc 4500
   ttgcagaata tgatgatggc ttatggtata gagcgaagat tgttgccatt aaagaattta 4560
   atcctttatc tatcttagta caatttgttg attatggatc aactgcaaag ctgacattaa 4620
   acagactgtg ccaaattcct tctcatctta tgcggtatcc agctcgagcc ataaaggttc 4680
   tcttggcagg gtttaaacct cccttaaggg atctagggga gacaagaata ccatattgtc 4740
   ccaaatggag catggaggca ctgtgggcta tgatagactg tcttcaagga aaacaactct 4800
   atgctgtgtc catggctcca gcaccagaac agatagtgac attatatgac gatgaacagc 4860
   atccagttca tatgccgttg gtagaaatgg ggcttgcaga taaagatgaa taagtgccta 4920
   agtgtataca gtgagagcat ctatagaagc ctagaagaat tctgttatgt ttagactatg 4980
   tcttatcttt agactatttc aggcttaatt ttcctaactt gttcagcact agtgctttac 5040
   ctctcatttt taattgaact gttaggaatt gtgtggggaa aaaaagtaaa taaatgttcg 5100
   cttccaaaaa aaaaaaaaaa 5120
<210> 19
   <211> 1738
   <212> DNA
   <213> Homo sapiens
<400> 19
   gatgtcatca ggctttgtga gggggactgt actgcccttt gagtactact gtgtggcccc      60
   gcaacccagc acaaccaggt atctgcttgg aacccagcca ccataaagcc tgctagctaa     120
   aaaaaatttt acatctctca gttcattcgg cacagacccc tgcctcattc agctgtgact     180
   ctgcttggaa aattcatcag ttacaaagca gccaatgcaa ttatctcaag ggaaattgaa     240
   aaatggacct ttgaaaatgc tagatttaca atgagaaatg ccataattca aggtttattc     300
   tatgggtcct tgacatttgg gatctggaca gctctgttat tcatatattt gcaccataat     360
   catgtgagca gctggcagaa gaaaagccag gagcctctgt cagcttggtc ccctggaaaa     420
   aaagtgcatc agcaaattat ctatggctca gagcaaatac caaaacctca tgtaatagtc     480
   aaaaggactg atgaagataa agcaaagtct atgttaggta cagattttaa ccatacaaac     540
   ccagaacttc ataaagaact tttaaaatat ggatttaatg tgattatcag tagaagcttg     600
   ggcatcgaaa gagaagtgcc agataccagg agtaaaatgt gtcttcaaaa acattaccca     660
   gcccgcctcc cgactgccag cattgtcatt tgcttctata atgaagaatg taatgccttg     720
   tttcagacca tgtccagtgt cacgaacctc acgccacact attttcttga agaaattatt     780
   ttggtagatg acatgagcaa agttgatgat ttgaaagaaa aactagacta tcacctggaa     840
   acttttcggg gaaaggttaa aataataaga aacaaaaaga gagaggggct gattcgagca     900
   aggctgattg gagcttctca tgcttcaggg gatgttctgg tgttcctgga cagccactgt     960
   gaggtgaaca gagtatggct ggagcccctg ctgcatgcca ttgccaagga ccccaaaatg    1020
   gtggtgtgcc ccctgataga tgtcattgat gatagaactc tggagtataa gccctctcct    1080
   cttgtaaggg gaacttttga ttggaaccta caatttaaat gggataatgt tttctcttat    1140
   gagatggatg gaccagaagg atctactaaa ccaatccggt cacctgcaat gtctggagga    1200
   atttttgcta tacgtcggca ttattttaat gaaattggac agtatgacaa ggatatggat    1260
   ttttggggaa gagaaaattt ggaactttca ctaaggatct ggatgtgtgg aggccaactc    1320
   tttataatcc cctgctctcg agtaggacat atcagtaaga aacaaactgg aaaaccttct    1380
   acaatcatca gtgctatgac acataactac ctaagactgg tgcacgtttg gctggatgaa    1440
   tataaggagc agttttttct tcgaaagcct ggtctgaaat atgtcaccta cggaaatatt    1500
   cgcgagcgtg ttgagttaag gaaacgactg ggttgcaagt catttcagtg gtatttggat    1560
   aatgtcttcc cagagttgga ggcatctgtg aacagcctgt gaaaggaaaa caaatcactt    1620
   tcattaataa agggttaaaa gtctcctagt cattcaacat agtgtcacaa gagtgtaagt    1680
   ttggaacatc gtggaattac gtgaaatgca attaaaaaaa tatgaccaga cgtgaaaa      1738
<210> 20
   <211> 3623
   <212> DNA
   <213> Homo sapiens
<400> 20
   tctagcacag gggatcccca aacatcagga cttttggggg gcgcctgtgc tgtccatggg      60
   aagagcatgc attgtgggtt actggaggaa cccgacatgg attccacaga gagctggatt     120
   gaaagatgtc tcaacgaaag tgaaaacaaa cgttattcca gccacacatc tctggggaat     180
   gtttctaatg atgaaaatga ggaaaaagaa aataatagag catccaagcc ccactccact     240
   cctgctactc tgcaatggct ggaggagaac tatgagattg cagagggggt ctgcatccct     300
   cgcagtgccc tctatatgca ttacctggat ttctgcgaga agaatgatac ccaacctgtc     360
   aatgctgcca gctttggaaa gatcataagg cagcagtttc ctcagttaac caccagaaga     420
   ctcgggaccc gaggacagtc aaagtaccat tactatggca ttgcagtgaa agaaagctcc     480
   caatattatg atgtgatgta ttccaagaaa ggagctgcct gggtgagtga gacgggcaag     540
   aaagaagtga gcaaacagac agtggcatat tcaccccggt ccaaactcgg aacactgctg     600
   ccagaatttc ccaatgtcaa agatctaaat ctgccagcca gcctgcctga ggagaaggtt     660
   tctaccttta ttatgatgta cagaacacac tgtcagagaa tactggacac tgtaataaga     720
   gccaactttg atgaggttca aagtttcctt ctgcactttt ggcaaggaat gccgccccac     780
   atgctgcctg tgctgggctc ctccacggtg gtgaacattg tcggcgtgtg tgactccatc      840
   ctctacaaag ctatctccgg ggtgctgatg cccactgtgc tgcaggcatt acctgacagc      900
   ttaactcagg tgattcgaaa gtttgccaag caactggatg agtggctaaa agtggctctc      960
   cacgacctcc cagaaaactt gcgaaacatc aagttcgaat tgtcgagaag gttctcccaa     1020
   attctgagac ggcaaacatc actaaatcat ctctgccagg catctcgaac agtgatccac     1080
   agtgcagaca tcacgttcca aatgctggaa gactggagga acgtggacct gaacagcatc     1140
   accaagcaaa ccctttacac catggaagac tctcgcgatg agcaccggaa actcatcacc     1200
   caattatatc aggagtttga ccatctcttg gaggagcagt ctcccatcga gtcctacatt     1260
   gagtggctgg ataccatggt tgaccgctgt gttgtgaagg tggctgccaa gagacaaggg     1320
   tccttgaaga aagtggccca gcagttcctc ttgatgtggt cctgtttcgg cacaagggtg     1380
   atccgggaca tgaccttgca cagcgccccc agcttcgggt cttttcacct aattcactta     1440
   atgtttgatg actacgtgct ctacctgtta gaatctctgc actgtcagga gcgggccaat     1500
   gagctcatgc gagccatgaa gggagaagga agcactgcag aagtccgaga agagatcatc     1560
   ttgacagagg ctgccgcacc aaccccttca ccagtgccat cgttttctcc agcaaaatct     1620
   gccacatctg tggaagtgcc acctccctct tcccctgtta gcaatccttc ccctgagtac     1680
   actggcctca gcactacagg agcaatgcag tcttacacgt ggtctctaac atacacagtg     1740
   acgacggctg ctgggtcccc agctgagaac tcccaacagc tgccctgtat gaggaacact     1800
   catgtgcctt cttcctccgt cacacacagg ataccagttt atccccacag agaggaacat     1860
   ggatacacgg gaagctataa ctatgggagc tatggcaacc agcatcctca ccccatgcag     1920
   agccagtatc cggccctccc tcatgacaca gctatctctg ggccactcca ctatgcccct     1980
   taccacagga gctctgcaca gtaccctttt aatagcccca cttcccggat ggaaccttgt    2040
   ttgatgagca gtactcccag actgcatcct accccagtca ctccccgctg gccagaggtg     2100
   ccctcagcca acacgtgcta cacaagcccg tctgtgcatt ctgcgaggta cggaaactct     2160
   agtgacatgt atacacctct gacaacgcgc aggaattctg aatatgagca catgcaacac     2220
   tttcctggct ttgcttacat caacggagag gcctctacag gatgggctaa atgactgcta     2280
   tcataggcat ccatatttaa tattaataat aataattaat aataataata aacccaacac     2340
   ccatccccca gaagacttta tctctataca ttgtaactca tgggctattc ctaagtgccc     2400
   attttcctaa tgaacatgag gatgggatca atgtgggatg aataaacttt agttcagaaa     2460
   caggacttac taaaagtcag tgggactggg tttctgtagc caagccagac ttgactgttt     2520
   ctgtagagca ctatctcggg caggccattc tgtgcctttt ccctctgttc catgactttg     2580
   ctttgtgttg gcaaccactt ctagtaagct actgattttc ctgttgacaa aatctcttta     2640
   gtcttgaagg atggatactg gagacagaat ctggtttgtg ttcttggatg ggcacataat     2700
   ttaccaagag cattcacctt gccatctgtc ttgtcattgt actgtacaag gaacagccct     2760
   cagacgtgtt ctgcacatcc cttcttcctg gtggtaccat ccctatttcc tggagcacca     2820
   gggctaaatg gggagctatc tggaaactct agattttctg tcatacccac atctgtcaca     2880
   gtacctgcat tgtcttggaa tgtaagcact gtcttgaggg aaggaagagg tctgttctgt     2940
   attgccttaa gttgattgag gtttgtagga gactggttct tctacataca aggatttgtc     3000
   ttaagtttgc acaatggcta gtgtcagcaa aaggcaggag agggtttttg tttttttttt     3060
   aagttctatg agaatgtgga tttatggcat tgagtatcac actcagctct gctgtgttaa     3120
   ctttgtgaaa ctggatggaa caaactttaa cttaccaagc accaagtgtg aaagtgactt     3180
   tcacggttcc ttcataaaac tataataata tccgacactt tgatagaaaa aaattcaaag     3240
   ctgtgccttt gagcctatac tatactgtgt atgtgtggaa ataaaaatgt attgtacttt     3300
   tggagaattt tttgtaggca tttttctgtc agatttgtag taatttgtga ggtttgttag     3360
   agattaatat aggttttctt tctgtattat aaaatgcacc aagcaattat ggtggaccta     3420
   ttaccctatg ggtaagaaat aaatggaaat atgacatcgg atgtttcagc aactgttctg     3480
   taaataaaat ctttgatcac accactcagt gtgataattg tgtctacagc taaaatggaa     3540
   atagttttat ctgtacagtt gtgcaagata tgaatggttt cacactcaaa taaaaaatat     3600
   tgaaacgaaa aaaaaaaaaa aaa                                              3623
<210> 21
   <211> 1099
   <212> DNA
   <213> Homo sapiens
<400> 21
   gctgcattac agacacagac ctgcaaacat ctatggttgt gacagagttt ctttctgaca      60
   cctgagtctt tctcctgctg cacggaaagc ttgctgggag gggcttggaa tctggcatga     120
   agccaaaggg catctctgag ttgcagcatt taaatgatcc cactcagaga ttcacacaga     180
   agactggaca caattccgaa gagctgccca gaaggagaga acaatgtcat cactacccag     240
   tggcagacac ctttcacccc agctacacaa gagggggcag atgtgtgagg atcactgcag     300
   tccaggagtt cgatgtttca gtgagctgtg attgcaccac tgcatatcag cctgggtgac     360
   agagcaagac cctatctcaa aaatacagaa aaatcatcaa ccacttgcag tcgtcgtaga     420
   aatcaatcat tccctccagt tatgtccctg acccacaggc ttcatttgtg caagtactgg     480
   ggctgtgctg tcagtaatgt gtgccgcttc tgggaaggac gtccattgcc cttgatgatt     540
   gtggtaccat acacactgcc tgtttccttg cctgttggtt cgtgcgtgat aatcacaggg     600
   acaccgatcc tcacttttgt caaggaccca cagctggagg tgaatttcta cactgggatg     660
   gatgaggact cagatattgc tttccaattc cgactgcact ttggtcatcc tgcaatcatg     720
   aacagttgtg tgtttggcat atggagatat gaggagaaat gctactattt accctttgaa     780
   gatggcaaac catttgagct gtgcatctat gtgcgtcaca aggaatacaa ggtaatggta     840
   aatggccaac gcatttacaa ctttgcccat cgattcccgc cagcatctgt gaagatgctg     900
   caagtcttca gagatatctc cctgaccaga gtgcttatca gcgattgagg gagatgatca     960
   gactcctcat tgttgaggaa tccctctttc tacctgacca tgggattccc agagcctact    1020
   aacagaataa tccctcctca ccccttcccc tacacttgat cattaaaaca gcaccaaact    1080
   tcaaaaaaaa aaaaaaaaa                                                 1099
<210> 22
   <211> 1660
   <212> DNA
   <213> Homo sapiens
<400> 22
   ggtgcactag caaaacaaac ttattttgaa cactcagctc ctagcgtgcg gcgctgccaa        60
   tcattaacct cctggtgcaa gtggcgcggc ctgtgccctt tataaggtgc gcgctgtgtc       120
   cagcgagcat cggccaccgc catcccatcc agcgagcatc tgccgccgcg ccgccgccac       180
   cctcccagag agcactggcc accgctccac catcacttgc ccagagtttg ggccaccgcc       240
   cgccgccacc agcccagaga gcatcggccc ctgtctgctg ctcgcgcctg gagatgtcag       300
   aggtccccgt tgctcgcgtc tggctggtac tgctcctgct gactgtccag gtcggcgtga       360
   cagccggcgc tccgtggcag tgcgcgccct gctccgccga gaagctcgcg ctctgcccgc       420
   cggtgtccgc ctcgtgctcg gaggtcaccc ggtccgccgg ctgcggctgt tgcccgatgt       480
   gcgccctgcc tctgggcgcc gcgtgcggcg tggcgactgc acgctgcgcc cggggactca       540
   gttgccgcgc gctgccgggg gagcagcaac ctctgcacgc cctcacccgc ggccaaggcg       600
   cctgcgtgca ggagtctgac gcctccgctc cccatgctgc agaggcaggg agccctgaaa       660
   gcccagagag cacggagata actgaggagg agctcctgga taatttccat ctgatggccc       720
   cttctgaaga ggatcattcc atcctttggg acgccatcag tacctatgat ggctcgaagg       780
   ctctccatgt caccaacatc aaaaaatgga aggagccctg ccgaatagaa ctctacagag       840
   tcgtagagag tttagccaag gcacaggaga catcaggaga agaaatttcc aaattttacc       900
   tgccaaactg caacaagaat ggattttatc acagcagaca gtgtgagaca tccatggatg       960
   gagaggcggg actctgctgg tgcgtctacc cttggaatgg gaagaggatc cctgggtctc      1020
   cagagatcag gggagacccc aactgccaga tatattttaa tgtacaaaac tgaaaccaga      1080
   tgaaataatg ttctgtcacg tgaaatattt aagtatatag tatatttata ctctagaaca      1140
   tgcacattta tatatatatg tatatgtata tatatatagt aactactttt tatactccat      1200
   acataacttg atatagaaag ctgtttattt attcactgta agtttatttt ttctacacag      1260
   taaaaacttg tactatgtta ataacttgtc ctatgtcaat ttgtatatca tgaaacactt      1320
   ctcatcatat tgtatgtaag taattgcatt tctgctcttc caaagctcct gcgtctgttt      1380
   ttaaagagca tggaaaaata ctgcctagaa aatgcaaaat gaaataagag agagtagttt      1440
   ttcagctagt ttgaaggagg acggttaact tgtatattcc accattcaca tttgatgtac      1500
   atgtgtaggg aaagttaaaa gtgttgatta cataatcaaa gctacctgtg gtgatgttgc      1560
   cacctgttaa aatgtacact ggatatgttg ttaaacacgt gtctataatg gaaacattta      1620
   caataaatat tctgcatgga aatactgtta aaaaaaaaaa                            1660
<210> 23
   <211> 1504
   <212> DNA
   <213> Homo sapiens
<400> 23
   ggttgaggtc aagtagtagc gttgggctgc ggcagcggag gagctcaaca tgcgtgagtg      60
   tatctctatc cacgtggggc aggcaggagt ccagatcggc aatgcctgct gggaactgta     120
   ctgcctggaa catggaattc agcccgatgg tcagatgcca agtgataaaa ccattggtgg     180
   tggggacgac tccttcaaca cgttcttcag tgagactgga gctggcaagc acgtgcccag     240
   agcagtgttt gtggacctgg agcccactgt ggtcgatgaa gtgcgcacag gaacctatag     300
   gcagctcttc cacccagagc agctgatcac cgggaaggaa gatgcggcca ataattacgc     360
   cagaggccat tacaccatcg gcaaggagat cgtcgacctg gtcctggacc ggatccgcaa     420
   actggcggat ctgtgcacgg gactgcaggg cttcctcatc ttccacagtt ttgggggtgg     480
   cactggctct gggttcgcat ctctgctcat ggagcggctc tcagtggatt acggcaagaa     540
   gtccaagcta gaatttgcca tttacccagc cccccaggtc tccacggccg tggtggagcc     600
   ctacaactcc atcctgacca cccacacgac cctggaacat tctgactgtg ccttcatggt     660
   cgacaatgaa gccatctatg acatatgtcg gcgcaacctg gacatcgagc gtcccacgta     720
   caccaacctc aatcgcctga ttgggcagat cgtgtcctcc atcacggcct ccctgcgatt     780
   tgacggggcc ctgaatgtgg acttgacgga attccagacc aacctagtgc cgtacccccg     840
   catccacttc cccctggcca cctacgcccc ggtcatctca gccgagaagg cctaccacga     900
   gcagctgtcc gtggctgaga tcaccaatgc ctgcttcgag ccagccaatc agatggtcaa     960
   gtgtgaccct cgccacggca agtacatggc ctgctgcatg ttgtacaggg gggatgtggt    1020
   cccgaaagat gtcaacgcgg ccatcgccac catcaagacc aagcgcacca tccagtttgt    1080
   agattggtgc ccaactggat ttaaggtggg cattaactac cagcccccca cggtggtccc    1140
   tgggggagac ctggccaagg tgcagcgggc tgtgtgcatg ctgagcaaca ccacggccat    1200
   cgcggaggcc tgggctcgcc tggaccataa gttcgatctc atgtatgcca agcgggcctt    1260
   tgtgcactgg tacgtgggag aaggcatgga ggagggggag ttctctgagg cccgcgagga    1320
   cctggcagct ctggagaagg attatgaaga ggtgggcgtg gattccgtgg aagccgaggc    1380
   tgaagaaggt gaagaatact gaggggaggg tgtggtgggt tctccactcc actgccaccc    1440
   ccagcgtggc tgctttcaag ttctttgcaa ttaaaggttc tgtataaaaa aaaaaaaaaa    1500
   aaaa                                                                1504
<210> 24
   <211> 753
   <212> PRT
   <213> Homo sapiens
<400> 24
<210> 25
   <211> 363
   <212> PRT
   <213> Homo sapiens
<400> 25
   Met Lys Arg Ser Leu Asn Glu Asn Ser Ala Arg Ser Thr Ala Gly Cys
   1               5                   10                  15
   Leu Pro Val Pro Leu Phe Asn Gln Lys Lys Arg Asn Arg Gln Pro Leu
               20                  25                  30
   Thr Ser Asn Pro Leu Lys Asp Asp Ser Gly Ile Ser Thr Pro Ser Asp
           35                  40                  45
   Asn Tyr Asp Phe Pro Pro Leu Pro Thr Asp Trp Ala Trp Glu Ala Val
       50                  55                  60
   Asn Pro Glu Leu Ala Pro Val Met Lys Thr Val Asp Thr Gly Gln Ile
   65                  70                  75                  80
   Pro His Ser Val Ser Arg Pro Leu Arg Ser Gln Asp Ser Val Phe Asn
                   85                  90                  95
   Ser Ile Gln Ser Asn Thr Gly Arg Ser Gln Gly Gly Trp Ser Tyr Arg
               100                 105                 110
   Asp Gly Asn Lys Asn Thr Ser Leu Lys Thr Trp Asn Lys Asn Asp Phe
           115                 120                 125
   Lys Pro Gln Cys Lys Arg Thr Asn Leu Val Ala Asn Asp Gly Lys Asn
       130                 135                 140
   Ser Cys Pro Val Ser Ser Gly Ala Gln Gln Gln Lys Gln Leu Arg Ile
   145                 150                 155                 160
   Pro Glu Pro Pro Asn Leu Ser Arg Asn Lys Glu Thr Glu Leu Leu Arg
                   165                 170                 175
   Gln Thr His Ser Ser Lys Ile Ser Gly Cys Thr Met Arg Gly Leu Asp
               180                 185                 190
   Lys Asn Ser Ala Leu Gln Thr Leu Lys Pro Asn Phe Gln Gln Asn Gln
           195                 200                 205
   Tyr Lys Lys Gln Met Leu Asp Asp Ile Pro Glu Asp Asn Thr Leu Lys
       210                 215                 220
   Glu Thr Ser Leu Tyr Gln Leu Gln Phe Lys Glu Lys Ala Ser Ser Leu
   225                 230                 235                 240
   Arg Ile Ile Ser Ala Val Ile Glu Ser Met Lys Tyr Trp Arg Glu His
                   245                 250                 255
   Ala Gln Lys Thr Val Leu Leu Phe Glu Val Leu Ala Val Leu Asp Ser
               260                 265                 270
   Ala Val Thr Pro Gly Pro Tyr Tyr Ser Lys Thr Phe Leu Met Arg Asp
           275                 280                 285
   Gly Lys Asn Thr Leu Pro Cys Val Phe Tyr Glu Ile Asp Arg Glu Leu
       290                 295                 300
   Pro Arg Leu Ile Arg Gly Arg Val His Arg Cys Val Gly Asn Tyr Asp
   305                 310                 315                 320
   Gln Lys Lys Asn Ile Phe Gln Cys Val Ser Val Arg Pro Ala Ser Val
   325 330 335
   Ser Glu Gln Lys Thr Phe Gln Ala Phe Val Lys Ile Ala Asp Val Glu
   340 345 350
   Met Gln Tyr Tyr Ile Asn Val Met Asn Glu Thr
   355 360
<210> 26
   <211> 434
   <212> PRT
   <213> Homo sapiens
<400> 26
   Met Pro Asn Arg Lys Ala Ser Arg Asn Ala Tyr Tyr Phe Phe Val Gln
   1 5 10 15
   Glu Lys Ile Pro Glu Leu Arg Arg Arg Gly Leu Pro Val Ala Arg Val
   20 25 30
   Ala Asp Ala Ile Pro Tyr Cys Ser Ser Asp Trp Ala Leu Leu Arg Glu
   35 40 45
   Glu Glu Lys Glu Lys Tyr Ala Glu Met Ala Arg Glu Trp Arg Ala Ala
   50 55 60
   Gln Gly Lys Asp Pro Gly Pro Ser Glu Lys Gln Lys Pro Val Phe Thr
   65 70 75 80
   Pro Leu Arg Arg Pro Gly Met Leu Val Pro Lys Gln Asn Val Ser Pro
   85 90 95
   Pro Asp Met Ser Ala Leu Ser Leu Lys Gly Asp Gln Ala Leu Leu Gly
   100 105 110
   Gly Ile Phe Tyr Phe Leu Asn Ile Phe Ser His Gly Glu Leu Pro Pro
   115 120 125
   His Cys Glu Gln Arg Phe Leu Pro Cys Glu Ile Gly Cys Val Lys Tyr
   130 135 140
   Ser Leu Gln Glu Gly Ile Met Ala Asp Phe His Ser Phe Ile Asn Pro
   145 150 155 160
   Gly Glu Ile Pro Arg Gly Phe Arg Phe His Cys Gln Ala Ala Ser Asp
   165 170 175
   Ser Ser His Lys Ile Pro Ile Ser Asn Phe Glu Arg Gly His Asn Gln
   180 185 190
   Ala Thr Val Leu Gln Asn Leu Tyr Arg Phe Ile His Pro Asn Pro Gly
   195 200 205
   Asn Trp Pro Pro Ile Tyr Cys Lys Ser Asp Asp Arg Thr Arg Val Asn
   210 215 220
   Trp Cys Leu Lys His Met Ala Lys Ala Ser Glu Ile Arg Gln Asp Leu
   225 230 235 240
   Gln Leu Leu Thr Val Glu Asp Leu Val Val Gly Ile Tyr Gln Gln Lys
   245 250 255
   Phe Leu Lys Glu Pro Ser Lys Thr Trp Ile Arg Ser Leu Leu Asp Val
   260 265 270
   Ala Met Trp Asp Tyr Ser Ser Asn Thr Arg Cys Lys Trp His Glu Glu
   275 280 285
   Asn Asp Ile Leu Phe Cys Ala Leu Ala Val Cys Lys Lys Ile Ala Tyr
   290 295 300
   Cys Ile Ser Asn Ser Leu Ala Thr Leu Phe Gly Ile Gln Leu Thr Glu
   305 310 315 320
   Ala His Val Pro Leu Gln Asp Tyr Glu Ala Ser Asn Ser Val Thr Pro
   325 330 335
   Lys Met Val Val Leu Asp Ala Gly Arg Tyr Gln Lys Leu Arg Val Gly
   340 345 350
   Ser Ser Gly Phe Ser His Phe Asn Ser Ser Asn Glu Glu Gln Arg Ser
   355 360 365
   Asn Thr Pro Ile Gly Asp Tyr Pro Ser Arg Ala Lys Ile Ser Gly Gln
   370 375 380
   Asn Ser Ser Val Arg Gly Arg Gly Ile Thr Arg Leu Leu Glu Ser Ile
   385 390 395 400
   Ser Asn Ser Ser Ser Asn Ile His Lys Phe Ser Asn Cys Asp Thr Ser
   405 410 415
   Leu Ser Pro Tyr Met Ser Gln Lys Asp Gly Tyr Lys Ser Phe Ser Ser
               420                 425                 430
   Leu Ser
<210> 27
   <211> 72
   <212> PRT
   <213> Homo sapiens
<400> 27
   Met Pro Leu Leu Arg Gly Arg Cys Pro Ala Arg Arg His Tyr Arg Arg
   1               5                   10                  15
   Leu Ala Leu Leu Gly Leu Gln Pro Ala Pro Arg Phe Ala His Ser Gly
               20                  25                  30
   Pro Pro Arg Gln Arg Pro Leu Ser Ala Ala Glu Met Ala Val Gly Leu
           35                  40                  45
   Val Val Phe Phe Thr Thr Phe Leu Thr Pro Ala Ala Tyr Val Leu Gly
       50                  55                  60
   Asn Leu Lys Gln Phe Arg Arg Asn
   65                  70
<210> 28
   <211> 596
   <212> PRT
   <213> Homo sapiens
<400> 28
   Met Ala Asp Ala Glu Ala Arg Ala Glu Phe Pro Glu Glu Ala Arg Pro
   1               5                   10                  15
   Asp Arg Gly Thr Leu Gln Val Leu Gln Asp Met Ala Ser Arg Leu Arg
               20                  25                  30
   Ile His Ser Ile Arg Ala Thr Cys Ser Thr Ser Ser Gly His Pro Thr
           35                  40                  45
   Ser Cys Ser Ser Ser Ser Glu Ile Met Ser Val Leu Phe Phe Tyr Ile
       50                  55                  60
   Met Arg Tyr Lys Gln Ser Asp Pro Glu Asn Pro Asp Asn Asp Arg Phe
   65                  70                  75                  80
   Val Leu Ala Lys Arg Leu Ser Phe Val Asp Val Ala Thr Gly Trp Leu
                   85                  90                  95
   Gly Gln Gly Leu Gly Val Ala Cys Gly Met Ala Tyr Thr Gly Lys Tyr
               100                 105                 110
   Phe Asp Arg Ala Ser Tyr Arg Val Phe Cys Leu Met Ser Asp Gly Glu
           115                 120                 125
   Ser Ser Glu Gly Ser Val Trp Glu Ala Met Ala Phe Ala Ser Tyr Tyr
       130                 135                 140
   Ser Leu Asp Asn Leu Val Ala Ile Phe Asp Val Asn Arg Leu Gly His
   145                 150                 155                 160
   Ser Gly Ala Leu Pro Ala Glu His Cys Ile Asn Ile Tyr Gln Arg Arg
                   165                 170                 175
   Cys Glu Ala Phe Gly Trp Asn Thr Tyr Val Val Asp Gly Arg Asp Val
               180                 185                 190
   Glu Ala Leu Cys Gln Val Phe Trp Gln Ala Ser Gln Val Lys His Lys
           195                 200                 205
   Pro Thr Ala Val Val Ala Lys Thr Phe Lys Gly Arg Gly Thr Pro Ser
       210                 215                 220
   Ile Glu Asp Ala Glu Ser Trp His Ala Lys Pro Met Pro Arg Glu Arg
   225                 230                 235                 240
   Ala Asp Ala Ile Ile Lys Leu Ile Glu Ser Gln Ile Gln Thr Ser Arg
                   245                 250                 255
   Asn Leu Asp Pro Gln Pro Pro Ile Glu Asp Ser Pro Glu Val Asn Ile
               260                 265                 270
   Thr Asp Val Arg Met Thr Ser Pro Pro Asp Tyr Arg Val Gly Asp Lys
           275                 280                 285
   Ile Ala Thr Arg Lys Ala Cys Gly Leu Ala Leu Ala Lys Leu Gly Tyr
       290                 295                 300
   Ala Asn Asn Arg Val Val Val Leu Asp Gly Asp Thr Arg Tyr Ser Thr
   305                 310                 315                 320
   Phe Ser Glu Ile Phe Asn Lys Glu Tyr Pro Glu Arg Phe Ile Glu Cys
                   325                 330                 335
   Phe Met Ala Glu Gln Asn Met Val Ser Val Ala Leu Gly Cys Ala Ser
               340                 345                 350
   Arg Gly Arg Thr Ile Ala Phe Ala Ser Thr Phe Ala Ala Phe Leu Thr
           355                 360                 365
   Arg Ala Phe Asp His Ile Arg Ile Gly Gly Leu Ala Glu Ser Asn Ile
       370                 375                 380
   Asn Ile Ile Gly Ser His Cys Gly Val Ser Val Gly Asp Asp Gly Ala
   385                 390                 395                 400
   Ser Gln Met Ala Leu Glu Asp Ile Ala Met Phe Arg Thr Ile Pro Lys
                   405                 410                 415
   Cys Thr Ile Phe Tyr Pro Thr Asp Ala Val Ser Thr Glu His Ala Val
               420                 425                 430
   Ala Leu Ala Ala Asn Ala Lys Gly Met Cys Phe Ile Arg Thr Thr Arg
           435                 440                 445
   Pro Glu Thr Met Val Ile Tyr Thr Pro Gln Glu Arg Phe Glu Ile Gly
       450                 455                 460
   Gln Ala Lys Val Leu Arg His Cys Val Ser Asp Lys Val Thr Val Ile
   465                 470                 475                 480
   Gly Ala Gly Ile Thr Val Tyr Glu Ala Leu Ala Ala Ala Asp Glu Leu
                   485                 490                 495
   Ser Lys Gln Asp Ile Phe Ile Arg Val Ile Asp Leu Phe Thr Ile Lys
               500                 505                 510
   Pro Leu Asp Val Ala Thr Ile Val Ser Ser Ala Lys Ala Thr Glu Gly
           515                 520                 525
   Arg Ile Ile Thr Val Glu Asp His Tyr Pro Gln Gly Gly Ile Gly Glu
       530                 535                 540
   Ala Val Cys Ala Ala Val Ser Met Asp Pro Asp Ile Gln Val His Ser
   545                 550                 555                 560
   Leu Ala Val Ser Gly Val Pro Gln Ser Gly Lys Ser Glu Glu Leu Leu
   565 570 575
   Asp Met Tyr Gly Ile Ser Ala Arg His Ile Ile Val Ala Val Lys Cys
   580 585 590
   Met Leu Leu Asn
   595
<210> 29
   <211> 533
   <212> PRT
   <213> Homo sapiens
<400> 29
   Met Asn Glu Glu Asn Ile Asp Gly Thr Asn Gly Cys Ser Lys Val Arg
   1 5 10 15
   Thr Gly Ile Gln Asn Glu Ala Ala Leu Leu Ala Leu Met Glu Lys Thr
   20 25 30
   Gly Tyr Asn Met Val Gln Glu Asn Gly Gln Arg Lys Phe Gly Gly Pro
   35 40 45
   Pro Pro Gly Trp Glu Gly Pro Pro Pro Pro Arg Gly Cys Glu Val Phe
   50 55 60
   Val Gly Lys Ile Pro Arg Asp Met Tyr Glu Asp Glu Leu Val Pro Val
   65 70 75 80
   Phe Glu Arg Ala Gly Lys Ile Tyr Glu Phe Arg Leu Met Met Glu Phe
   85 90 95
   Ser Gly Glu Asn Arg Gly Tyr Ala Phe Val Met Tyr Thr Thr Lys Glu
   100 105 110
   Glu Ala Gln Leu Ala Ile Arg Ile Leu Asn Asn Tyr Glu Ile Arg Pro
   115 120 125
   Gly Lys Phe Ile Gly Val Cys Val Ser Leu Asp Asn Cys Arg Leu Phe
   130 135 140
   Ile Gly Ala Ile Pro Lys Glu Lys Lys Lys Glu Glu Ile Leu Asp Glu
   145 150 155 160
   Met Lys Lys Val Thr Glu Gly Val Val Asp Val Ile Val Tyr Pro Ser
                   165                 170                 175
   Ala Thr Asp Lys Thr Lys Asn Arg Gly Phe Ala Phe Val Glu Tyr Glu
               180                 185                 190
   Ser His Arg Ala Ala Ala Met Ala Arg Arg Lys Leu Ile Pro Gly Thr
           195                 200                 205
   Phe Gln Leu Trp Gly His Thr Ile Gln Val Asp Trp Ala Asp Pro Glu
       210                 215                 220
   Lys Glu Val Asp Glu Glu Thr Met Gln Arg Val Lys Val Leu Tyr Val
   225                 230                 235                 240
   Arg Asn Leu Met Ile Ser Thr Thr Glu Glu Thr Ile Lys Ala Glu Phe
                   245                 250                 255
   Asn Lys Phe Lys Pro Gly Ala Val Glu Arg Val Lys Lys Leu Arg Asp
               260                 265                 270
   Tyr Ala Phe Val His Phe Phe Asn Arg Glu Asp Ala Val Ala Ala Met
           275                 280                 285
   Ser Val Met Asn Gly Lys Cys Ile Asp Gly Ala Ser Ile Glu Val Thr
       290                 295                 300
   Leu Ala Lys Pro Val Asn Lys Glu Asn Thr Trp Arg Gln His Leu Asn
   305                 310                 315                 320
   Gly Gln Ile Ser Pro Asn Ser Glu Asn Leu Ile Val Phe Ala Asn Lys
                   325                 330                 335
   Glu Glu Ser His Pro Lys Thr Leu Gly Lys Leu Pro Thr Leu Pro Ala
               340                 345                 350
   Arg Leu Asn Gly Gln His Ser Pro Ser Pro Pro Glu Val Glu Arg Cys
           355                 360                 365
   Thr Tyr Pro Phe Tyr Pro Gly Thr Lys Leu Thr Pro Ile Ser Met Tyr
       370                 375                 380
   Ser Leu Lys Ser Asn His Phe Asn Ser Ala Val Met His Leu Asp Tyr
   385                 390                 395                 400
   Tyr Cys Asn Lys Asn Asn Trp Ala Pro Pro Glu Tyr Tyr Leu Tyr Ser
                   405                 410                 415
   Thr Thr Ser Gln Asp Gly Lys Val Leu Leu Val Tyr Lys Ile Val Ile
   420 425 430
   Pro Ala Ile Ala Asn Gly Ser Gln Ser Tyr Phe Met Pro Asp Lys Leu
   435 440 445
   Cys Thr Thr Leu Glu Asp Ala Lys Glu Leu Ala Ala Gln Phe Thr Leu
   450 455 460
   Leu His Leu Asp Tyr Asn Phe His Arg Ser Ser Ile Asn Ser Leu Ser
   465 470 475 480
   Pro Val Ser Ala Thr Leu Ser Ser Gly Thr Pro Ser Val Leu Pro Tyr
   485 490 495
   Thr Ser Arg Pro Tyr Ser Tyr Pro Gly Tyr Pro Leu Ser Pro Thr Ile
   500 505 510
   Ser Leu Ala Asn Gly Ser His Val Gly Gln Arg Leu Cys Ile Ser Asn
   515 520 525
   Gln Ala Ser Phe Phe
   530
<210> 30
   <211> 407
   <212> PRT
   <213> Homo sapiens
<400> 30
   Met Pro Arg Gly His Lys Ser Lys Leu Arg Thr Cys Glu Lys Arg Gln
   1 5 10 15
   Glu Thr Asn Gly Gln Pro Gln Gly Leu Thr Gly Pro Gln Ala Thr Ala
   20 25 30
   Glu Lys Gln Glu Glu Ser His Ser Ser Ser Ser Ser Ser Arg Ala Cys
   35 40 45
   Leu Gly Asp Cys Arg Arg Ser Ser Asp Ala Ser Ile Pro Gln Glu Ser
   50 55 60
   Gln Gly Val Ser Pro Thr Gly Ser Pro Asp Ala Val Val Ser Tyr Ser
   65 70 75 80
   Lys Ser Asp Val Ala Ala Asn Gly Gln Asp Glu Lys Ser Pro Ser Thr
                   85                  90                  95
   Ser Arg Asp Ala Ser Val Pro Gln Glu Ser Gln Gly Ala Ser Pro Thr
               100                 105                 110
   Gly Ser Pro Asp Ala Gly Val Ser Gly Ser Lys Tyr Asp Val Ala Ala
           115                 120                 125
   Asn Gly Gln Asp Glu Lys Ser Pro Ser Thr Ser His Asp Val Ser Val
       130                 135                 140
   Pro Gln Glu Ser Gln Gly Ala Ser Pro Thr Gly Ser Pro Asp Ala Gly
   145                 150                 155                 160
   Val Ser Gly Ser Lys Tyr Asp Val Ala Ala Glu Gly Glu Asp Glu Glu
                   165                 170                 175
   Ser Val Ser Ala Ser Gln Lys Ala Ile Ile Phe Lys Arg Leu Ser Lys
               180                 185                 190
   Asp Ala Val Lys Lys Lys Ala Cys Thr Leu Ala Gln Phe Leu Gln Lys
           195                 200                 205
   Lys Phe Glu Lys Lys Glu Ser Ile Leu Lys Ala Asp Met Leu Lys Cys
       210                 215                 220
   Val Arg Arg Glu Tyr Lys Pro Tyr Phe Pro Gln Ile Leu Asn Arg Thr
   225                 230                 235                 240
   Ser Gln His Leu Val Val Ala Phe Gly Val Glu Leu Lys Glu Met Asp
                   245                 250                 255
   Ser Ser Gly Glu Ser Tyr Thr Leu Val Ser Lys Leu Gly Leu Pro Ser
               260                 265                 270
   Glu Gly Ile Leu Ser Gly Asp Asn Ala Leu Pro Lys Ser Gly Leu Leu
           275                 280                 285
   Met Ser Leu Leu Val Val Ile Phe Met Asn Gly Asn Cys Ala Thr Glu
       290                 295                 300
   Glu Glu Val Trp Glu Phe Leu Gly Leu Leu Gly Ile Tyr Asp Gly Ile
   305                 310                 315                 320
   Leu His Ser Ile Tyr Gly Asp Ala Arg Lys Ile Ile Thr Glu Asp Leu
                   325                 330                 335
   Val Gln Asp Lys Tyr Val Val Tyr Arg Gln Val Cys Asn Ser Asp Pro
               340                 345                 350
   Pro Cys Tyr Glu Phe Leu Trp Gly Pro Arg Ala Tyr Ala Glu Thr Thr
           355                 360                 365
   Lys Met Arg Val Leu Arg Val Leu Ala Asp Ser Ser Asn Thr Ser Pro
       370                 375                 380
   Gly Leu Tyr Pro His Leu Tyr Glu Asp Ala Leu Ile Asp Glu Val Glu
   385                 390                 395                 400
   Arg Ala Leu Arg Leu Arg Ala
                   405
<210> 31
   <211> 638
   <212> PRT
   <213> Homo sapiens
<400> 31
   Met Val Val Ser Ala Asp Pro Leu Ser Ser Glu Arg Ala Glu Met Asn
   1               5                   10                  15
   Ile Leu Glu Ile Asn Gln Glu Leu Arg Ser Gln Leu Ala Glu Ser Asn
               20                  25                  30
   Gln Gln Phe Arg Asp Leu Lys Glu Lys Phe Leu Ile Thr Gln Ala Thr
           35                  40                  45
   Ala Tyr Ser Leu Ala Asn Gln Leu Lys Lys Tyr Lys Cys Glu Glu Tyr
       50                  55                  60
   Lys Asp Ile Ile Asp Ser Val Leu Arg Asp Glu Leu Gln Ser Met Glu
   65                  70                  75                  80
   Lys Leu Ala Glu Lys Leu Arg Gln Ala Glu Glu Leu Arg Gln Tyr Lys
                   85                  90                  95
   Ala Leu Val His Ser Gln Ala Lys Glu Leu Thr Gln Leu Arg Glu Lys
               100                 105                 110
   Leu Arg Glu Gly Arg Asp Ala Ser Arg Trp Leu Asn Lys His Leu Lys
           115                 120                 125
   Thr Leu Leu Thr Pro Asp Asp Pro Asp Lys Ser Gln Gly Gln Asp Leu
       130                 135                 140
   Arg Glu Gln Leu Ala Glu Gly His Arg Leu Ala Glu His Leu Val His
   145                 150                 155                 160
   Lys Leu Ser Pro Glu Asn Asp Glu Asp Glu Asp Glu Asp Glu Asp Asp
                   165                 170                 175
   Lys Asp Glu Glu Val Glu Lys Val Gln Glu Ser Pro Ala Pro Arg Glu
               180                 185                 190
   Val Gln Lys Thr Glu Glu Lys Glu Val Pro Gln Asp Ser Leu Glu Glu
           195                 200                 205
   Cys Ala Val Thr Cys Ser Asn Ser His Asn Pro Ser Asn Ser Asn Gln
       210                 215                 220
   Pro His Arg Ser Thr Lys Ile Thr Phe Lys Glu His Glu Val Asp Ser
   225                 230                 235                 240
   Ala Leu Val Val Glu Ser Glu His Pro His Asp Glu Glu Glu Glu Ala
                   245                 250                 255
   Leu Asn Ile Pro Pro Glu Asn Gln Asn Asp His Glu Glu Glu Glu Gly
               260                 265                 270
   Lys Ala Pro Val Pro Pro Arg His His Asp Lys Ser Asn Ser Tyr Arg
           275                 280                 285
   His Arg Glu Val Ser Phe Leu Ala Leu Asp Glu Gln Lys Val Cys Ser
       290                 295                 300
   Ala Gln Asp Val Ala Arg Asp Tyr Ser Asn Pro Lys Trp Asp Glu Thr
   305                 310                 315                 320
   Ser Leu Gly Phe Leu Glu Lys Gln Ser Asp Leu Glu Glu Val Lys Gly
                   325                 330                 335
   Gln Glu Thr Val Ala Pro Arg Leu Ser Arg Gly Pro Leu Arg Val Asp
               340                 345                 350
   Lys His Glu Ile Pro Gln Glu Ser Leu Asp Gly Cys Cys Leu Thr Pro
           355                 360                 365
   Ser Ile Leu Pro Asp Leu Thr Pro Ser Tyr His Pro Tyr Trp Ser Thr
       370                 375                 380
   Leu Tyr Ser Phe Glu Asp Lys Gln Val Ser Leu Ala Leu Val Asp Lys
   385                 390                 395                 400
   Ile Lys Lys Asp Gln Glu Glu Ile Glu Asp Gln Ser Pro Pro Cys Pro
                   405                 410                 415
   Arg Leu Ser Gln Glu Leu Pro Glu Val Lys Glu Gln Glu Val Pro Glu
               420                 425                 430
   Asp Ser Val Asn Glu Val Tyr Leu Thr Pro Ser Val His His Asp Val
           435                 440                 445
   Ser Asp Cys His Gln Pro Tyr Ser Ser Thr Leu Ser Ser Leu Glu Asp
       450                 455                 460
   Gln Leu Ala Cys Ser Ala Leu Asp Val Ala Ser Pro Thr Glu Ala Ala
   465                 470                 475                 480
   Cys Pro Gln Gly Thr Trp Ser Gly Asp Leu Ser His His Gln Ser Glu
                   485                 490                 495
   Val Gln Val Ser Gln Ala Gln Leu Glu Pro Ser Thr Leu Val Pro Ser
               500                 505                 510
   Cys Leu Arg Leu Gln Leu Asp Gln Gly Phe His Cys Gly Asn Gly Leu
           515                 520                 525
   Ala Gln Arg Gly Leu Ser Ser Thr Thr Cys Ser Phe Ser Ala Asn Ala
       530                 535                 540
   Asp Ser Gly Asn Gln Trp Pro Phe Gln Glu Leu Val Leu Glu Pro Ser
   545                 550                 555                 560
   Leu Gly Met Lys Asn Pro Pro Gln Leu Glu Asp Asp Ala Leu Glu Gly
                   565                 570                 575
   Ser Ala Ser Asn Thr Gln Gly Arg Gln Val Thr Gly Arg Ile Arg Ala
               580                 585                 590
   Ser Leu Val Leu Ile Leu Lys Thr Ile Arg Arg Arg Leu Pro Phe Ser
           595                 600                 605
   Lys Trp Arg Leu Ala Phe Arg Phe Ala Gly Pro His Ala Glu Ser Ala
   610 615 620
   Glu Ile Pro Asn Thr Ala Gly Arg Thr Gln Arg Met Ala Gly
   625 630 635
<210> 32
   <211> 70
   <212> PRT
   <213> Homo sapiens
<400> 32
   Met Lys Gln Lys Gln Glu Val Met Phe Gln Ser Arg Gly Arg Leu Ser
   1 5 10 15
   Leu Tyr Ile Gln Met Ser Ser Val Tyr Ser Ala Lys Leu Gly Pro Val
   20 25 30
   Gly Gly Ile Cys Gly Gln Lys Gln Lys Pro Ser Phe Phe Phe Phe Lys
   35 40 45
   Ala Gln Ser Gln Asp Ala Arg Pro Leu Ala Pro Ala Ala Cys Ile Ser
   50 55 60
   Lys Ile Ala Lys Ala Gly
   65 70
<210> 33
   <211> 522
   <212> PRT
   <213> Homo sapiens
<400> 33
<210> 34
   <211> 513
   <212> PRT
   <213> Homo sapiens
<400> 34
   Met Ile Arg Thr Pro Leu Ser Ala Ser Ala His Arg Leu Leu Leu Pro
   1               5                   10                  15
   Gly Ser Arg Gly Arg Pro Pro Arg Asn Met Gln Pro Thr Gly Arg Glu
               20                  25                  30
   Gly Ser Arg Ala Leu Ser Arg Arg Tyr Leu Arg Arg Leu Leu Leu Leu
           35                  40                  45
   Leu Leu Leu Leu Leu Leu Arg Gln Pro Val Thr Arg Ala Glu Thr Thr
       50                  55                  60
   Pro Gly Ala Pro Arg Ala Leu Ser Thr Leu Gly Ser Pro Ser Leu Phe
   65                  70                  75                  80
   Thr Thr Pro Gly Val Pro Ser Ala Leu Thr Thr Pro Gly Leu Thr Thr
                   85                  90                  95
   Pro Gly Thr Pro Lys Thr Leu Asp Leu Arg Gly Arg Ala Gln Ala Leu
               100                 105                 110
   Met Arg Ser Phe Pro Leu Val Asp Gly His Asn Asp Leu Pro Gln Val
           115                 120                 125
   Leu Arg Gln Arg Tyr Lys Asn Val Leu Gln Asp Val Asn Leu Arg Asn
       130                 135                 140
   Phe Ser His Gly Gln Thr Ser Leu Asp Arg Leu Arg Asp Gly Leu Val
   145                 150                 155                 160
   Gly Ala Gln Phe Trp Ser Ala Ser Val Ser Cys Gln Ser Gln Asp Gln
                   165                 170                 175
   Thr Ala Val Arg Leu Ala Leu Glu Gln Ile Asp Leu Ile His Arg Met
               180                 185                 190
   Cys Ala Ser Tyr Ser Glu Leu Glu Leu Val Thr Ser Ala Glu Gly Leu
           195                 200                 205
   Asn Ser Ser Gln Lys Leu Ala Cys Leu Ile Gly Val Glu Gly Gly His
       210                 215                 220
   Ser Leu Asp Ser Ser Leu Ser Val Leu Arg Ser Phe Tyr Val Leu Gly
   225                 230                 235                 240
   Val Arg Tyr Leu Thr Leu Thr Phe Thr Cys Ser Thr Pro Trp Ala Glu
                   245                 250                 255
   Ser Ser Thr Lys Phe Arg His His Met Tyr Thr Asn Val Ser Gly Leu
               260                 265                 270
   Thr Ser Phe Gly Glu Lys Val Val Glu Glu Leu Asn Arg Leu Gly Met
           275                 280                 285
   Met Ile Asp Leu Ser Tyr Ala Ser Asp Thr Leu Ile Arg Arg Val Leu
       290                 295                 300
   Glu Val Ser Gln Ala Pro Val Ile Phe Ser His Ser Ala Ala Arg Ala
   305                 310                 315                 320
   Val Cys Asp Asn Leu Leu Asn Val Pro Asp Asp Ile Leu Gln Leu Leu
                   325                 330                 335
   Lys Lys Asn Gly Gly Ile Val Met Val Thr Leu Ser Met Gly Val Leu
               340                 345                 350
   Gln Cys Asn Leu Leu Ala Asn Val Ser Thr Val Ala Asp His Phe Asp
           355                 360                 365
   His Ile Arg Ala Val Ile Gly Ser Glu Phe Ile Gly Ile Gly Gly Asn
       370                 375                 380
   Tyr Asp Gly Thr Gly Arg Phe Pro Gln Gly Leu Glu Asp Val Ser Thr
   385                 390                 395                 400
   Tyr Pro Val Leu Ile Glu Glu Leu Leu Ser Arg Ser Trp Ser Glu Glu
                   405                 410                 415
   Glu Leu Gln Gly Val Leu Arg Gly Asn Leu Leu Arg Val Phe Arg Gln
               420                 425                 430
   Val Glu Lys Val Arg Glu Glu Ser Arg Ala Gln Ser Pro Val Glu Ala
           435                 440                 445
   Glu Phe Pro Tyr Gly Gln Leu Ser Thr Ser Cys His Ser His Leu Val
       450                 455                 460
   Pro Gln Asn Gly His Gln Ala Thr His Leu Glu Val Thr Lys Gln Pro
   465                 470                 475                 480
   Thr Asn Arg Val Pro Trp Arg Ser Ser Asn Ala Ser Pro Tyr Leu Val
                   485                 490                 495
   Pro Gly Leu Val Ala Ala Ala Thr Ile Pro Thr Phe Thr Gln Trp Leu
   500 505 510
   Cys
<210> 35
   <211> 154
   <212> PRT
   <213> Homo sapiens
<400> 35
   Met Glu Pro Ser Lys Thr Phe Met Arg Asn Leu Pro Ile Thr Pro Gly
   1 5 10 15
   Tyr Ser Gly Phe Val Pro Phe Leu Ser Cys Gln Gly Met Ser Lys Glu
   20 25 30
   Asp Asp Met Asn His Cys Val Lys Thr Phe Gln Glu Lys Thr Gln Arg
   35 40 45
   Tyr Lys Glu Gln Leu Arg Glu Leu Cys Cys Ala Val Ala Thr Ala Pro
   50 55 60
   Lys Leu Lys Pro Val Asn Ser Glu Glu Thr Val Leu Gln Ala Leu His
   65 70 75 80
   Gln Tyr Asn Leu Gln Tyr His Pro Leu Ile Leu Glu Cys Lys Tyr Val
   85 90 95
   Lys Lys Pro Leu Gln Glu Pro Pro Ile Pro Gly Trp Ala Gly Tyr Leu
   100 105 110
   Pro Arg Ala Lys Val Thr Glu Phe Gly Cys Gly Thr Arg Tyr Thr Val
   115 120 125
   Met Ala Lys Asn Cys Tyr Lys Asp Phe Leu Glu Ile Thr Glu Arg Ala
   130 135 140
   Lys Lys Ala His Leu Lys Pro Tyr Glu Glu
   145 150
<210> 36
   <211> 120
   <212> PRT
   <213> Homo sapiens
<400> 36
   Met Gly Val Leu Arg Ala Arg Gly Glu Val Gly Leu Ala Leu Ser Pro
   1               5                   10                  15
   Arg Leu Val Gly Gly Ala Ser Pro Pro Cys Asp Gly Gly Pro Glu Ser
               20                  25                  30
   Arg Gly Arg Lys Arg Gly Cys Leu Leu Ser Pro Cys Leu Val Gly Val
           35                  40                  45
   Ala Ser Pro Ser Cys Asp Asp Gly Pro Lys Ser Gln Arg Gly Lys Arg
       50                  55                  60
   Gly Trp Leu Ser Val Pro Ala Ser Leu Gly Val Pro Pro Pro Pro Ala
   65                  70                  75                  80
   Ile Gly Val Leu Arg Ser Arg Gly Gly Arg Gly Ala Gly Ser Gln Ser
                   85                  90                  95
   Pro Pro Arg Gly Gly Cys Leu Pro Pro Cys Asp Gly Gly Pro Glu Ser
               100                 105                 110
   Arg Glu Arg Lys Arg Gly Cys Leu
           115                 120
<210> 37
   <211> 0
   <212> PRT
   <213> Homo sapiens
<400> 37
   000
<210> 38
   <211> 70
   <212> PRT
   <213> Homo sapiens
<400> 38
   Met Ala Arg Thr Lys Gln Thr Ala Arg Lys Ser Thr Gly Gly Lys Ala
   1               5                   10                  15
   Pro Arg Lys Gln Leu Ala Thr Lys Ala Ala Arg Lys Ser Ala Pro Ala
               20                  25                  30
   Thr Gly Gly Val Lys Lys Pro His Arg Tyr Arg Pro Gly Thr Val Ala
           35                  40                  45
   Leu Arg Glu Ile Arg Arg Tyr Gln Lys Ser Thr Glu Leu Leu Ile Arg
       50                  55                  60
   Lys Leu Pro Phe Gln Arg
   65                  70
<210> 39
   <211> 861
   <212> PRT
   <213> Homo sapiens
<400> 39
   Met Thr Gly Arg Ala Arg Ala Arg Ala Arg Gly Arg Ala Arg Gly Gln
   1               5                   10                  15
   Glu Thr Ala Gln Leu Val Gly Ser Thr Ala Ser Gln Gln Pro Gly Tyr
               20                  25                  30
   Ile Gln Pro Arg Pro Gln Pro Pro Pro Ala Glu Gly Glu Leu Phe Gly
           35                  40                  45
   Arg Gly Arg Gln Arg Gly Thr Ala Gly Gly Thr Ala Lys Ser Gln Gly
       50                  55                  60
   Leu Gln Ile Ser Ala Gly Phe Gln Glu Leu Ser Leu Ala Glu Arg Gly
   65                  70                  75                  80
   Gly Arg Arg Arg Asp Phe His Asp Leu Gly Val Asn Thr Arg Gln Asn
                   85                  90                  95
   Leu Asp His Val Lys Glu Ser Lys Thr Gly Ser Ser Gly Ile Ile Val
               100                 105                 110
   Arg Leu Ser Thr Asn His Phe Arg Leu Thr Ser Arg Pro Gln Trp Ala
           115                 120                 125
   Leu Tyr Gln Tyr His Ile Asp Tyr Asn Pro Leu Met Glu Ala Arg Arg
       130                 135                 140
   Leu Arg Ser Ala Leu Leu Phe Gln His Glu Asp Leu Ile Gly Lys Cys
   145                 150                 155                 160
   His Ala Phe Asp Gly Thr Ile Leu Phe Leu Pro Lys Arg Leu Gln Gln
                   165                 170                 175
   Lys Val Thr Glu Val Phe Ser Lys Thr Arg Asn Gly Glu Asp Val Arg
               180                 185                 190
   Ile Thr Ile Thr Leu Thr Asn Glu Leu Pro Pro Thr Ser Pro Thr Cys
             195                 200                 205
   Leu Gln Phe Tyr Asn Ile Ile Phe Arg Arg Leu Leu Lys Ile Met Asn
       210                 215                 220
   Leu Gln Gln Ile Gly Arg Asn Tyr Tyr Asn Pro Asn Asp Pro Ile Asp
   225                 230                 235                 240
   Ile Pro Ser His Arg Leu Val Ile Trp Pro Gly Phe Thr Thr Ser Ile
                   245                 250                 255
   Leu Gln Tyr Glu Asn Ser Ile Met Leu Cys Thr Asp Val Ser His Lys
               260                 265                 270
   Val Leu Arg Ser Glu Thr Val Leu Asp Phe Met Phe Asn Phe Tyr His
           275                 280                 285
   Gln Thr Glu Glu His Lys Phe Gln Glu Gln Val Ser Lys Glu Leu Ile
       290                 295                 300
   Gly Leu Val Val Leu Thr Lys Tyr Asn Asn Lys Thr Tyr Arg Val Asp
   305                 310                 315                 320
   Asp Ile Asp Trp Asp Gln Asn Pro Lys Ser Thr Phe Lys Lys Ala Asp
                   325                 330                 335
   Gly Ser Glu Val Ser Phe Leu Glu Tyr Tyr Arg Lys Gln Tyr Asn Gln
               340                 345                 350
   Glu Ile Thr Asp Leu Lys Gln Pro Val Leu Val Ser Gln Pro Lys Arg
           355                 360                 365
   Arg Arg Gly Pro Gly Gly Thr Leu Pro Gly Pro Ala Met Leu Ile Pro
       370                 375                 380
   Glu Leu Cys Tyr Leu Thr Gly Leu Thr Asp Lys Met Arg Asn Asp Phe
   385                 390                 395                 400
   Asn Val Met Lys Asp Leu Ala Val His Thr Arg Leu Thr Pro Glu Gln
                   405                 410                 415
   Arg Gln Arg Glu Val Gly Arg Leu Ile Asp Tyr Ile His Lys Asn Asp
               420                 425                 430
   Asn Val Gln Arg Glu Leu Arg Asp Trp Gly Leu Ser Phe Asp Ser Asn
           435                 440                 445
   Leu Leu Ser Phe Ser Gly Arg Ile Leu Gln Thr Glu Lys Ile His Gln
       450                 455                 460
   Gly Gly Lys Thr Phe Asp Tyr Asn Pro Gln Phe Ala Asp Trp Ser Lys
   465                 470                 475                 480
   Glu Thr Arg Gly Ala Pro Leu Ile Ser Val Lys Pro Leu Asp Asn Trp
                   485                 490                 495
   Leu Leu Ile Tyr Thr Arg Arg Asn Tyr Glu Ala Ala Asn Ser Leu Ile
               500                 505                 510
   Gln Asn Leu Phe Lys Val Thr Pro Ala Met Gly Met Gln Met Arg Lys
           515                 520                 525
   Ala Ile Met Ile Glu Val Asp Asp Arg Thr Glu Ala Tyr Leu Arg Val
       530                 535                 540
   Leu Gln Gln Lys Val Thr Ala Asp Thr Gln Ile Val Val Cys Leu Leu
   545                 550                 555                 560
   Ser Ser Asn Arg Lys Asp Lys Tyr Asp Ala Ile Lys Lys Tyr Leu Cys
                   565                 570                 575
   Thr Asp Cys Pro Thr Pro Ser Gln Cys Val Val Ala Arg Thr Leu Gly
               580                 585                 590
   Lys Gln Gln Thr Val Met Ala Ile Ala Thr Lys Ile Ala Leu Gln Met
           595                 600                 605
   Asn Cys Lys Met Gly Gly Glu Leu Trp Arg Val Asp Ile Pro Leu Lys
       610                 615                 620
   Leu Val Met Ile Val Gly Ile Asp Cys Tyr His Asp Met Thr Ala Gly
   625                 630                 635                 640
   Arg Arg Ser Ile Ala Gly Phe Val Ala Ser Ile Asn Glu Gly Met Thr
                   645                 650                 655
   Arg Trp Phe Ser Arg Cys Ile Phe Gln Asp Arg Gly Gln Glu Leu Val
               660                 665                 670
   Asp Gly Leu Lys Val Cys Leu Gln Ala Ala Leu Arg Ala Trp Asn Ser
           675                 680                 685
   Cys Asn Glu Tyr Met Pro Ser Arg Ile Ile Val Tyr Arg Asp Gly Val
       690                 695                 700
   Gly Asp Gly Gln Leu Lys Thr Leu Val Asn Tyr Glu Val Pro Gln Phe
   705                 710                 715                 720
   Leu Asp Cys Leu Lys Ser Ile Gly Arg Gly Tyr Asn Pro Arg Leu Thr
                   725                 730                 735
   Val Ile Val Val Lys Lys Arg Val Asn Thr Arg Phe Phe Ala Gln Ser
               740                 745                 750
   Gly Gly Arg Leu Gln Asn Pro Leu Pro Gly Thr Val Ile Asp Val Glu
           755                 760                 765
   Val Thr Arg Pro Glu Trp Tyr Asp Phe Phe Ile Val Ser Gln Ala Val
       770                 775                 780
   Arg Ser Gly Ser Val Ser Pro Thr His Tyr Asn Val Ile Tyr Asp Asn
   785                 790                 795                 800
   Ser Gly Leu Lys Pro Asp His Ile Gln Arg Leu Thr Tyr Lys Leu Cys
                   805                 810                 815
   His Ile Tyr Tyr Asn Trp Pro Gly Val Ile Arg Val Pro Ala Pro Cys
               820                 825                 830
   Gln Tyr Ala His Lys Leu Ala Phe Leu Val Gly Gln Ser Ile His Arg
           835                 840                 845
   Glu Pro Asn Leu Ser Leu Ser Asn Arg Leu Tyr Tyr Leu
       850                 855                 860
<210> 40
   <211> 221
   <212> PRT
   <213> Homo sapiens
<400> 40
   Met Pro Leu Ala Leu Thr Leu Leu Leu Leu Ser Gly Leu Gly Ala Pro
   1               5                   10                  15
   Gly Gly Trp Gly Cys Leu Gln Cys Asp Pro Leu Val Leu Glu Ala Leu
               20                  25                  30
   Gly His Leu Arg Ser Ala Leu Ile Pro Ser Arg Phe Gln Leu Glu Gln
   35 40 45
   Leu Gln Ala Arg Ala Gly Ala Val Leu Met Gly Met Glu Gly Pro Phe
   50 55 60
   Phe Arg Asp Tyr Ala Leu Asn Val Phe Val Gly Lys Val Glu Thr Asn
   65 70 75 80
   Gln Leu Asp Leu Val Ala Ser Phe Val Lys Asn Gln Thr Gln His Leu
   85 90 95
   Met Gly Asn Ser Leu Lys Asp Glu Pro Leu Leu Glu Glu Leu Val Thr
   100 105 110
   Leu Arg Ala Asn Val Ile Lys Glu Phe Lys Lys Val Leu Ile Ser Tyr
   115 120 125
   Glu Leu Lys Ala Cys Asn Pro Lys Leu Cys Arg Leu Leu Lys Glu Glu
   130 135 140
   Val Leu Asp Cys Leu His Cys Gln Arg Ile Thr Pro Lys Cys Ile His
   145 150 155 160
   Lys Lys Tyr Cys Phe Val Asp Arg Gln Pro Arg Val Ala Leu Gln Tyr
   165 170 175
   Gln Met Asp Ser Lys Tyr Pro Arg Asn Gln Ala Leu Leu Gly Ile Leu
   180 185 190
   Ile Ser Val Ser Leu Ala Val Phe Val Phe Val Val Ile Val Val Ser
   195 200 205
   Ala Cys Thr Tyr Arg Gln Asn Arg Lys Leu Leu Leu Gln
   210 215 220
<210> 41
   <211> 1623
   <212> PRT
   <213> Homo sapiens
<400> 41
   Met Ala Ala Glu Ala Ser Lys Thr Gly Pro Ser Arg Ser Ser Tyr Gln
   1 5 10 15
   Arg Met Gly Arg Lys Ser Gln Pro Trp Gly Ala Ala Glu Ile Gln Cys
   20 25 30
   Thr Arg Cys Gly Arg Arg Val Ser Arg Ser Ser Gly His His Cys Glu
           35                  40                  45
   Leu Gln Cys Gly His Ala Phe Cys Glu Leu Cys Leu Leu Met Thr Glu
       50                  55                  60
   Glu Cys Thr Thr Ile Ile Cys Pro Asp Cys Glu Val Ala Thr Ala Val
   65                  70                  75                  80
   Asn Thr Arg Gln Arg Tyr Tyr Pro Met Ala Gly Tyr Ile Lys Glu Asp
                   85                  90                  95
   Ser Ile Met Glu Lys Leu Gln Pro Lys Thr Ile Lys Asn Cys Ser Gln
               100                 105                 110
   Asp Phe Lys Lys Thr Ala Asp Gln Leu Thr Thr Gly Leu Glu Arg Ser
           115                 120                 125
   Ala Ser Thr Asp Lys Thr Leu Leu Asn Ser Ser Ala Val Met Leu Asp
       130                 135                 140
   Thr Asn Thr Ala Glu Glu Ile Asp Glu Ala Leu Asn Thr Ala His His
   145                 150                 155                 160
   Ser Phe Glu Gln Leu Ser Ile Ala Gly Lys Ala Leu Glu His Met Gln
                   165                 170                 175
   Lys Gln Thr Ile Glu Glu Arg Glu Arg Val Ile Glu Val Val Glu Lys
               180                 185                 190
   Gln Phe Asp Gln Leu Leu Ala Phe Phe Asp Ser Arg Lys Lys Asn Leu
           195                 200                 205
   Cys Glu Glu Phe Ala Arg Thr Thr Asp Asp Tyr Leu Ser Asn Leu Ile
       210                 215                 220
   Lys Ala Lys Ser Tyr Ile Glu Glu Lys Lys Asn Asn Leu Asn Ala Ala
   225                 230                 235                 240
   Met Asn Ile Ala Arg Ala Leu Gln Leu Ser Pro Ser Leu Arg Thr Tyr
                   245                 250                 255
   Cys Asp Leu Asn Gln Ile Ile Arg Thr Leu Gln Leu Thr Ser Asp Ser
               260                 265                 270
   Glu Leu Ala Gln Val Ser Ser Pro Gln Leu Arg Asn Pro Pro Arg Leu
           275                 280                 285
   Ser Val Asn Cys Ser Glu Ile Ile Cys Met Phe Asn Asn Met Gly Lys
       290                 295                 300
   Ile Glu Phe Arg Asp Ser Thr Lys Cys Tyr Pro Gln Glu Asn Glu Ile
   305                 310                 315                 320
   Arg Gln Asn Val Gln Lys Lys Tyr Asn Asn Lys Lys Glu Leu Ser Cys
                   325                 330                 335
   Tyr Asp Thr Tyr Pro Pro Leu Glu Lys Lys Lys Val Asp Met Ser Val
               340                 345                 350
   Leu Thr Ser Glu Ala Pro Pro Pro Pro Leu Gln Pro Glu Thr Asn Asp
           355                 360                 365
   Val His Leu Glu Ala Lys Asn Phe Gln Pro Gln Lys Asp Val Ala Thr
       370                 375                 380
   Ala Ser Pro Lys Thr Ile Ala Val Leu Pro Gln Met Gly Ser Ser Pro
   385                 390                 395                 400
   Asp Val Ile Ile Glu Glu Ile Ile Glu Asp Asn Val Glu Ser Ser Ala
                   405                 410                 415
   Glu Leu Val Phe Val Ser His Val Ile Asp Pro Cys His Phe Tyr Ile
               420                 425                 430
   Arg Lys Tyr Ser Gln Ile Lys Asp Ala Lys Val Leu Glu Lys Lys Val
           435                 440                 445
   Asn Glu Phe Cys Asn Arg Ser Ser His Leu Asp Pro Ser Asp Ile Leu
       450                 455                 460
   Glu Leu Gly Ala Arg Ile Phe Val Ser Ser Ile Lys Asn Gly Met Trp
   465                 470                 475                 480
   Cys Arg Gly Thr Ile Thr Glu Leu Ile Pro Ile Glu Gly Arg Asn Thr
                   485                 490                 495
   Arg Lys Pro Cys Ser Pro Thr Arg Leu Phe Val His Glu Val Ala Leu
               500                 505                 510
   Ile Gln Ile Phe Met Val Asp Phe Gly Asn Ser Glu Val Leu Ile Val
           515                 520                 525
   Thr Gly Val Val Asp Thr His Val Arg Pro Glu His Ser Ala Lys Gln
       530                 535                 540
   His Ile Ala Leu Asn Asp Leu Cys Leu Val Leu Arg Lys Ser Glu Pro
   545                 550                 555                 560
   Tyr Thr Glu Gly Leu Leu Lys Asp Ile Gln Pro Leu Ala Gln Pro Cys
                   565                 570                 575
   Ser Leu Lys Asp Ile Val Pro Gln Asn Ser Asn Glu Gly Trp Glu Glu
               580                 585                 590
   Glu Ala Lys Val Glu Phe Leu Lys Met Val Asn Asn Lys Ala Val Ser
           595                 600                 605
   Met Lys Val Phe Arg Glu Glu Asp Gly Val Leu Ile Val Asp Leu Gln
       610                 615                 620
   Lys Pro Pro Pro Asn Lys Ile Ser Ser Asp Met Pro Val Ser Leu Arg
   625                 630                 635                 640
   Asp Ala Leu Val Phe Met Glu Leu Ala Lys Phe Lys Ser Gln Ser Leu
                   645                 650                 655
   Arg Ser His Phe Glu Lys Asn Thr Thr Leu His Tyr His Pro Pro Ile
               660                 665                 670
   Leu Pro Lys Glu Met Thr Asp Val Ser Val Thr Val Cys His Ile Asn
           675                 680                 685
   Ser Pro Gly Asp Phe Tyr Leu Gln Leu Ile Glu Gly Leu Asp Ile Leu
       690                 695                 700
   Phe Leu Leu Lys Thr Ile Glu Glu Phe Tyr Lys Ser Glu Asp Gly Glu
   705                 710                 715                 720
   Asn Leu Glu Ile Leu Cys Pro Val Gln Asp Gln Ala Cys Val Ala Lys
                   725                 730                 735
   Phe Glu Asp Gly Ile Trp Tyr Arg Ala Lys Val Ile Gly Leu Pro Gly
               740                 745                 750
   His Gln Glu Val Glu Val Lys Tyr Val Asp Phe Gly Asn Thr Ala Lys
           755                 760                 765
   Ile Thr Ile Lys Asp Val Arg Lys Ile Lys Asp Glu Phe Leu Asn Ala
       770                 775                 780
   Pro Glu Lys Ala Ile Lys Cys Lys Leu Ala Tyr Ile Glu Pro Tyr Lys
   785                 790                 795                 800
   Arg Thr Met Gln Trp Ser Lys Glu Ala Lys Glu Lys Phe Glu Glu Lys
                   805                 810                 815
   Ala Gln Asp Lys Phe Met Thr Cys Ser Val Ile Lys Ile Leu Glu Asp
               820                 825                 830
   Asn Val Leu Leu Val Glu Leu Phe Asp Ser Leu Gly Ala Pro Glu Met
           835                 840                 845
   Thr Thr Thr Ser Ile Asn Asp Gln Leu Val Lys Glu Gly Leu Ala Ser
       850                 855                 860
   Tyr Glu Ile Gly Tyr Ile Leu Lys Asp Asn Ser Gln Lys His Ile Glu
   865                 870                 875                 880
   Val Trp Asp Pro Ser Pro Glu Glu Ile Ile Ser Asn Glu Val His Asn
                   885                 890                 895
   Leu Asn Pro Val Ser Ala Lys Ser Leu Pro Asn Glu Asn Phe Gln Ser
               900                 905                 910
   Leu Tyr Asn Lys Glu Leu Pro Val His Ile Cys Asn Val Ile Ser Pro
           915                 920                 925
   Glu Lys Ile Tyr Val Gln Trp Leu Leu Thr Glu Asn Leu Leu Asn Ser
       930                 935                 940
   Leu Glu Glu Lys Met Ile Ala Ala Tyr Glu Asn Ser Lys Trp Glu Pro
   945                 950                 955                 960
   Val Lys Trp Glu Asn Asp Met His Cys Ala Val Lys Ile Gln Asp Lys
                   965                 970                 975
   Asn Gln Trp Arg Arg Gly Gln Ile Ile Arg Met Val Thr Asp Thr Leu
               980                 985                 990
   Val Glu Val Leu Leu Tyr Asp Val  Gly Val Glu Leu Val  Val Asn Val
           995                 1000                1005
   Asp Cys  Leu Arg Lys Leu Glu  Glu Asn Leu Lys Thr  Met Gly Arg
       1010                 1015                 1020
   Leu Ser  Leu Glu Cys Ser Leu  Val Asp Ile Arg Pro  Ala Gly Gly
       1025                 1030                 1035
   Ser Asp  Lys Trp Thr Ala Thr  Ala Cys Asp Cys Leu  Ser Leu Tyr
       1040                 1045                 1050
   Leu Thr  Gly Ala Val Ala Thr  Ile Ile Leu Gln Val  Asp Ser Glu
       1055                 1060                 1065
   Glu Asn  Asn Thr Thr Trp Pro  Leu Pro Val Lys Ile  Phe Cys Arg
       1070                 1075                 1080
   Asp Glu  Lys Gly Glu Arg Val  Asp Val Ser Lys Tyr  Leu Ile Lys
       1085                 1090                 1095
   Lys Gly  Leu Ala Leu Arg Glu  Arg Arg Ile Asn Asn  Leu Asp Asn
       1100                 1105                 1110
   Ser His  Ser Leu Ser Glu Lys  Ser Leu Glu Val Pro  Leu Glu Gln
       1115                 1120                 1125
   Glu Asp  Ser Val Val Thr Asn  Cys Ile Lys Thr Asn  Phe Asp Pro
       1130                 1135                 1140
   Asp Lys  Lys Thr Ala Asp Ile  Ile Ser Glu Gln Lys  Val Ser Glu
       1145                 1150                 1155
   Phe Gln  Glu Lys Ile Leu Glu  Pro Arg Thr Thr Arg  Gly Tyr Lys
       1160                 1165                 1170
   Pro Pro  Ala Ile Pro Asn Met  Asn Val Phe Glu Ala  Thr Val Ser
       1175                 1180                 1185
   Cys Val  Gly Asp Asp Gly Thr  Ile Phe Val Val Pro  Lys Leu Ser
       1190                 1195                 1200
   Glu Phe  Glu Leu Ile Lys Met  Thr Asn Glu Ile Gln  Ser Asn Leu
       1205                 1210                 1215
   Lys Cys  Leu Gly Leu Leu Glu  Pro Tyr Phe Trp Lys  Lys Gly Glu
       1220                 1225                 1230
   Ala Cys  Ala Val Arg Gly Ser  Asp Thr Leu Trp Tyr  Arg Gly Lys
       1235                 1240                 1245
   Val Met  Glu Val Val Gly Gly  Ala Val Arg Val Gln  Tyr Leu Asp
       1250                 1255                 1260
   His Gly  Phe Thr Glu Lys Ile  Pro Gln Cys His Leu  Tyr Pro Ile
       1265                 1270                 1275
   Leu Leu  Tyr Pro Asp Ile Pro  Gln Phe Cys Ile Pro  Cys Gln Leu
       1280                 1285                 1290
   His Asn  Thr Thr Pro Val Gly  Asn Val Trp Gln Pro  Asp Ala Ile
       1295                 1300                 1305
   Glu Val  Leu Gln Gln Leu Leu  Ser Lys Arg Gln Val  Asp Ile His
       1310                 1315                 1320
   Ile Met  Glu Leu Pro Lys Asn  Pro Trp Glu Lys Leu  Ser Ile His
       1325                 1330                 1335
   Leu Tyr  Phe Asp Gly Met Ser  Leu Ser Tyr Phe Met  Ala Tyr Tyr
       1340                 1345                 1350
   Lys Tyr  Cys Thr Ser Glu His  Thr Glu Glu Met Leu  Lys Glu Lys
       1355                 1360                 1365
   Pro Arg  Ser Asp His Asp Lys  Lys Tyr Glu Glu Glu  Gln Trp Glu
       1370                 1375                 1380
   Ile Arg  Phe Glu Glu Leu Leu  Ser Ala Glu Thr Asp  Thr Pro Leu
       1385                 1390                 1395
   Leu Pro  Pro Tyr Leu Ser Ser  Ser Leu Pro Ser Pro  Gly Glu Leu
       1400                 1405                 1410
   Tyr Ala  Val Gln Val Lys His  Val Val Ser Pro Asn  Glu Val Tyr
       1415                 1420                 1425
   Ile Cys  Leu Asp Ser Ile Glu  Thr Ser Asn Gln Ser  Asn Gln His
       1430                 1435                 1440
   Ser Asp  Thr Asp Asp Ser Gly  Val Ser Gly Glu Ser  Glu Ser Glu
       1445                 1450                 1455
   Ser Leu  Asp Glu Ala Leu Gln  Arg Val Asn Lys Lys  Val Glu Ala
   1460 1465 1470
   Leu Pro Pro Leu Thr Asp Phe Arg Thr Glu Met Pro Cys Leu Ala
   1475 1480 1485
   Glu Tyr Asp Asp Gly Leu Trp Tyr Arg Ala Lys Ile Val Ala Ile
   1490 1495 1500
   Lys Glu Phe Asn Pro Leu Ser Ile Leu Val Gln Phe Val Asp Tyr
   1505 1510 1515
   Gly Ser Thr Ala Lys Leu Thr Leu Asn Arg Leu Cys Gln Ile Pro
   1520 1525 1530
   Ser His Leu Met Arg Tyr Pro Ala Arg Ala Ile Lys Val Leu Leu
   1535 1540 1545
   Ala Gly Phe Lys Pro Pro Leu Arg Asp Leu Gly Glu Thr Arg Ile
   1550 1555 1560
   Pro Tyr Cys Pro Lys Trp Ser Met Glu Ala Leu Trp Ala Met Ile
   1565 1570 1575
   Asp Cys Leu Gln Gly Lys Gln Leu Tyr Ala Val Ser Met Ala Pro
   1580 1585 1590
   Ala Pro Glu Gln Ile Val Thr Leu Tyr Asp Asp Glu Gln His Pro
   1595 1600 1605
   Val His Met Pro Leu Val Glu Met Gly Leu Ala Asp Lys Asp Glu
   1610 1615 1620
<210> 42
   <211> 443
   <212> PRT
   <213> Homo sapiens
<400> 42
   Met Arg Asn Ala Ile Ile Gln Gly Leu Phe Tyr Gly Ser Leu Thr Phe
   1 5 10 15
   Gly Ile Trp Thr Ala Leu Leu Phe Ile Tyr Leu His His Asn His Val
   20 25 30
   Ser Ser Trp Gln Lys Lys Ser Gln Glu Pro Leu Ser Ala Trp Ser Pro
   35 40 45
   Gly Lys Lys Val His Gln Gln Ile Ile Tyr Gly Ser Glu Gln Ile Pro
       50                  55                  60
   Lys Pro His Val Ile Val Lys Arg Thr Asp Glu Asp Lys Ala Lys Ser
   65                  70                  75                  80
   Met Leu Gly Thr Asp Phe Asn His Thr Asn Pro Glu Leu His Lys Glu
                   85                  90                  95
   Leu Leu Lys Tyr Gly Phe Asn Val Ile Ile Ser Arg Ser Leu Gly Ile
               100                 105                 110
   Glu Arg Glu Val Pro Asp Thr Arg Ser Lys Met Cys Leu Gln Lys His
           115                 120                 125
   Tyr Pro Ala Arg Leu Pro Thr Ala Ser Ile Val Ile Cys Phe Tyr Asn
       130                 135                 140
   Glu Glu Cys Asn Ala Leu Phe Gln Thr Met Ser Ser Val Thr Asn Leu
   145                 150                 155                 160
   Thr Pro His Tyr Phe Leu Glu Glu Ile Ile Leu Val Asp Asp Met Ser
                   165                 170                 175
   Lys Val Asp Asp Leu Lys Glu Lys Leu Asp Tyr His Leu Glu Thr Phe
               180                 185                 190
   Arg Gly Lys Val Lys Ile Ile Arg Asn Lys Lys Arg Glu Gly Leu Ile
           195                 200                 205
   Arg Ala Arg Leu Ile Gly Ala Ser His Ala Ser Gly Asp Val Leu Val
       210                 215                 220
   Phe Leu Asp Ser His Cys Glu Val Asn Arg Val Trp Leu Glu Pro Leu
   225                 230                 235                 240
   Leu His Ala Ile Ala Lys Asp Pro Lys Met Val Val Cys Pro Leu Ile
                   245                 250                 255
   Asp Val Ile Asp Asp Arg Thr Leu Glu Tyr Lys Pro Ser Pro Leu Val
               260                 265                 270
   Arg Gly Thr Phe Asp Trp Asn Leu Gln Phe Lys Trp Asp Asn Val Phe
           275                 280                 285
   Ser Tyr Glu Met Asp Gly Pro Glu Gly Ser Thr Lys Pro Ile Arg Ser
       290                 295                 300
   Pro Ala Met Ser Gly Gly Ile Phe Ala Ile Arg Arg His Tyr Phe Asn
   305                 310                 315                 320
   Glu Ile Gly Gln Tyr Asp Lys Asp Met Asp Phe Trp Gly Arg Glu Asn
                   325                 330                 335
   Leu Glu Leu Ser Leu Arg Ile Trp Met Cys Gly Gly Gln Leu Phe Ile
               340                 345                 350
   Ile Pro Cys Ser Arg Val Gly His Ile Ser Lys Lys Gln Thr Gly Lys
           355                 360                 365
   Pro Ser Thr Ile Ile Ser Ala Met Thr His Asn Tyr Leu Arg Leu Val
       370                 375                 380
   His Val Trp Leu Asp Glu Tyr Lys Glu Gln Phe Phe Leu Arg Lys Pro
   385                 390                 395                 400
   Gly Leu Lys Tyr Val Thr Tyr Gly Asn Ile Arg Glu Arg Val Glu Leu
                   405                 410                 415
   Arg Lys Arg Leu Gly Cys Lys Ser Phe Gln Trp Tyr Leu Asp Asn Val
               420                 425                 430
   Phe Pro Glu Leu Glu Ala Ser Val Asn Ser Leu
           435                 440
<210> 43
   <211> 735
   <212> PRT
   <213> Homo sapiens
<400> 43
   Met His Cys Gly Leu Leu Glu Glu Pro Asp Met Asp Ser Thr Glu Ser
   1               5                   10                  15
   Trp Ile Glu Arg Cys Leu Asn Glu Ser Glu Asn Lys Arg Tyr Ser Ser
               20                  25                  30
   His Thr Ser Leu Gly Asn Val Ser Asn Asp Glu Asn Glu Glu Lys Glu
           35                  40                  45
   Asn Asn Arg Ala Ser Lys Pro His Ser Thr Pro Ala Thr Leu Gln Trp
       50                  55                  60
   Leu Glu Glu Asn Tyr Glu Ile Ala Glu Gly Val Cys Ile Pro Arg Ser
   65                  70                  75                  80
   Ala Leu Tyr Met His Tyr Leu Asp Phe Cys Glu Lys Asn Asp Thr Gln
                   85                  90                  95
   Pro Val Asn Ala Ala Ser Phe Gly Lys Ile Ile Arg Gln Gln Phe Pro
               100                 105                 110
   Gln Leu Thr Thr Arg Arg Leu Gly Thr Arg Gly Gln Ser Lys Tyr His
           115                 120                 125
   Tyr Tyr Gly Ile Ala Val Lys Glu Ser Ser Gln Tyr Tyr Asp Val Met
       130                 135                 140
   Tyr Ser Lys Lys Gly Ala Ala Trp Val Ser Glu Thr Gly Lys Lys Glu
   145                 150                 155                 160
   Val Ser Lys Gln Thr Val Ala Tyr Ser Pro Arg Ser Lys Leu Gly Thr
                   165                 170                 175
   Leu Leu Pro Glu Phe Pro Asn Val Lys Asp Leu Asn Leu Pro Ala Ser
               180                 185                 190
   Leu Pro Glu Glu Lys Val Ser Thr Phe Ile Met Met Tyr Arg Thr His
           195                 200                 205
   Cys Gln Arg Ile Leu Asp Thr Val Ile Arg Ala Asn Phe Asp Glu Val
       210                 215                 220
   Gln Ser Phe Leu Leu His Phe Trp Gln Gly Met Pro Pro His Met Leu
   225                 230                 235                 240
   Pro Val Leu Gly Ser Ser Thr Val Val Asn Ile Val Gly Val Cys Asp
                   245                 250                 255
   Ser Ile Leu Tyr Lys Ala Ile Ser Gly Val Leu Met Pro Thr Val Leu
               260                 265                 270
   Gln Ala Leu Pro Asp Ser Leu Thr Gln Val Ile Arg Lys Phe Ala Lys
           275                 280                 285
   Gln Leu Asp Glu Trp Leu Lys Val Ala Leu His Asp Leu Pro Glu Asn
       290                 295                 300
   Leu Arg Asn Ile Lys Phe Glu Leu Ser Arg Arg Phe Ser Gln Ile Leu
   305                 310                 315                 320
   Arg Arg Gln Thr Ser Leu Asn His Leu Cys Gln Ala Ser Arg Thr Val
                   325                 330                 335
   Ile His Ser Ala Asp Ile Thr Phe Gln Met Leu Glu Asp Trp Arg Asn
               340                 345                 350
   Val Asp Leu Asn Ser Ile Thr Lys Gln Thr Leu Tyr Thr Met Glu Asp
           355                 360                 365
   Ser Arg Asp Glu His Arg Lys Leu Ile Thr Gln Leu Tyr Gln Glu Phe
       370                 375                 380
   Asp His Leu Leu Glu Glu Gln Ser Pro Ile Glu Ser Tyr Ile Glu Trp
   385                 390                 395                 400
   Leu Asp Thr Met Val Asp Arg Cys Val Val Lys Val Ala Ala Lys Arg
                   405                 410                 415
   Gln Gly Ser Leu Lys Lys Val Ala Gln Gln Phe Leu Leu Met Trp Ser
               420                 425                 430
   Cys Phe Gly Thr Arg Val Ile Arg Asp Met Thr Leu His Ser Ala Pro
           435                 440                 445
   Ser Phe Gly Ser Phe His Leu Ile His Leu Met Phe Asp Asp Tyr Val
       450                 455                 460
   Leu Tyr Leu Leu Glu Ser Leu His Cys Gln Glu Arg Ala Asn Glu Leu
   465                 470                 475                 480
   Met Arg Ala Met Lys Gly Glu Gly Ser Thr Ala Glu Val Arg Glu Glu
                   485                 490                 495
   Ile Ile Leu Thr Glu Ala Ala Ala Pro Thr Pro Ser Pro Val Pro Ser
               500                 505                 510
   Phe Ser Pro Ala Lys Ser Ala Thr Ser Val Glu Val Pro Pro Pro Ser
           515                 520                 525
   Ser Pro Val Ser Asn Pro Ser Pro Glu Tyr Thr Gly Leu Ser Thr Thr
       530                 535                 540
   Gly Ala Met Gln Ser Tyr Thr Trp Ser Leu Thr Tyr Thr Val Thr Thr
   545                 550                 555                 560
   Ala Ala Gly Ser Pro Ala Glu Asn Ser Gln Gln Leu Pro Cys Met Arg
                   565                 570                 575
   Asn Thr His Val Pro Ser Ser Ser Val Thr His Arg Ile Pro Val Tyr
               580                 585                 590
   Pro His Arg Glu Glu His Gly Tyr Thr Gly Ser Tyr Asn Tyr Gly Ser
           595                 600                 605
   Tyr Gly Asn Gln His Pro His Pro Met Gln Ser Gln Tyr Pro Ala Leu
       610                 615                 620
   Pro His Asp Thr Ala Ile Ser Gly Pro Leu His Tyr Ala Pro Tyr His
   625                 630                 635                 640
   Arg Ser Ser Ala Gln Tyr Pro Phe Asn Ser Pro Thr Ser Arg Met Glu
                   645                 650                 655
   Pro Cys Leu Met Ser Ser Thr Pro Arg Leu His Pro Thr Pro Val Thr
               660                 665                 670
   Pro Arg Trp Pro Glu Val Pro Ser Ala Asn Thr Cys Tyr Thr Ser Pro
           675                 680                 685
   Ser Val His Ser Ala Arg Tyr Gly Asn Ser Ser Asp Met Tyr Thr Pro
       690                 695                 700
   Leu Thr Thr Arg Arg Asn Ser Glu Tyr Glu His Met Gln His Phe Pro
   705                 710                 715                 720
   Gly Phe Ala Tyr Ile Asn Gly Glu Ala Ser Thr Gly Trp Ala Lys
                   725                 730                 735
<210> 44
   <211> 168
   <212> PRT
   <213> Homo sapiens
<400> 44
   Met Ser Leu Thr His Arg Leu His Leu Cys Lys Tyr Trp Gly Cys Ala
   1               5                   10                  15
   Val Ser Asn Val Cys Arg Phe Trp Glu Gly Arg Pro Leu Pro Leu Met
               20                  25                  30
   Ile Val Val Pro Tyr Thr Leu Pro Val Ser Leu Pro Val Gly Ser Cys
           35                  40                  45
   Val Ile Ile Thr Gly Thr Pro Ile Leu Thr Phe Val Lys Asp Pro Gln
       50                  55                  60
   Leu Glu Val Asn Phe Tyr Thr Gly Met Asp Glu Asp Ser Asp Ile Ala
   65                  70                  75                  80
   Phe Gln Phe Arg Leu His Phe Gly His Pro Ala Ile Met Asn Ser Cys
                   85                  90                  95
   Val Phe Gly Ile Trp Arg Tyr Glu Glu Lys Cys Tyr Tyr Leu Pro Phe
               100                 105                 110
   Glu Asp Gly Lys Pro Phe Glu Leu Cys Ile Tyr Val Arg His Lys Glu
           115                 120                 125
   Tyr Lys Val Met Val Asn Gly Gln Arg Ile Tyr Asn Phe Ala His Arg
       130                 135                 140
   Phe Pro Pro Ala Ser Val Lys Met Leu Gln Val Phe Arg Asp Ile Ser
   145                 150                 155                 160
   Leu Thr Arg Val Leu Ile Ser Asp
                   165
<210> 45
   <211> 259
   <212> PRT
   <213> Homo sapiens
<400> 45
   Met Ser Glu Val Pro Val Ala Arg Val Trp Leu Val Leu Leu Leu Leu
   1               5                   10                  15
   Thr Val Gln Val Gly Val Thr Ala Gly Ala Pro Trp Gln Cys Ala Pro
               20                  25                  30
   Cys Ser Ala Glu Lys Leu Ala Leu Cys Pro Pro Val Ser Ala Ser Cys
           35                  40                  45
   Ser Glu Val Thr Arg Ser Ala Gly Cys Gly Cys Cys Pro Met Cys Ala
       50                  55                  60
   Leu Pro Leu Gly Ala Ala Cys Gly Val Ala Thr Ala Arg Cys Ala Arg
   65                  70                  75                  80
   Gly Leu Ser Cys Arg Ala Leu Pro Gly Glu Gln Gln Pro Leu His Ala
                   85                  90                  95
   Leu Thr Arg Gly Gln Gly Ala Cys Val Gln Glu Ser Asp Ala Ser Ala
               100                 105                 110
   Pro His Ala Ala Glu Ala Gly Ser Pro Glu Ser Pro Glu Ser Thr Glu
           115                 120                 125
   Ile Thr Glu Glu Glu Leu Leu Asp Asn Phe His Leu Met Ala Pro Ser
       130                 135                 140
   Glu Glu Asp His Ser Ile Leu Trp Asp Ala Ile Ser Thr Tyr Asp Gly
   145                 150                 155                 160
   Ser Lys Ala Leu His Val Thr Asn Ile Lys Lys Trp Lys Glu Pro Cys
                   165                 170                 175
   Arg Ile Glu Leu Tyr Arg Val Val Glu Ser Leu Ala Lys Ala Gln Glu
               180                 185                 190
   Thr Ser Gly Glu Glu Ile Ser Lys Phe Tyr Leu Pro Asn Cys Asn Lys
           195                 200                 205
   Asn Gly Phe Tyr His Ser Arg Gln Cys Glu Thr Ser Met Asp Gly Glu
       210                 215                 220
   Ala Gly Leu Cys Trp Cys Val Tyr Pro Trp Asn Gly Lys Arg Ile Pro
   225                 230                 235                 240
   Gly Ser Pro Glu Ile Arg Gly Asp Pro Asn Cys Gln Ile Tyr Phe Asn
                   245                 250                 255
   Val Gln Asn
<210> 46
   <211> 450
   <212> PRT
   <213> Homo sapiens
<400> 46
   Met Arg Glu Cys Ile Ser Ile His Val Gly Gln Ala Gly Val Gln Ile
   1               5                   10                  15
   Gly Asn Ala Cys Trp Glu Leu Tyr Cys Leu Glu His Gly Ile Gln Pro
               20                  25                  30
   Asp Gly Gln Met Pro Ser Asp Lys Thr Ile Gly Gly Gly Asp Asp Ser
           35                  40                  45
   Phe Asn Thr Phe Phe Ser Glu Thr Gly Ala Gly Lys His Val Pro Arg
       50                  55                  60
   Ala Val Phe Val Asp Leu Glu Pro Thr Val Val Asp Glu Val Arg Thr
   65                  70                  75                  80
   Gly Thr Tyr Arg Gln Leu Phe His Pro Glu Gln Leu Ile Thr Gly Lys
                   85                  90                  95
   Glu Asp Ala Ala Asn Asn Tyr Ala Arg Gly His Tyr Thr Ile Gly Lys
               100                 105                 110
   Glu Ile Val Asp Leu Val Leu Asp Arg Ile Arg Lys Leu Ala Asp Leu
           115                 120                 125
   Cys Thr Gly Leu Gln Gly Phe Leu Ile Phe His Ser Phe Gly Gly Gly
       130                 135                 140
   Thr Gly Ser Gly Phe Ala Ser Leu Leu Met Glu Arg Leu Ser Val Asp
   145                 150                 155                 160
   Tyr Gly Lys Lys Ser Lys Leu Glu Phe Ala Ile Tyr Pro Ala Pro Gln
                   165                 170                 175
   Val Ser Thr Ala Val Val Glu Pro Tyr Asn Ser Ile Leu Thr Thr His
               180                 185                 190
   Thr Thr Leu Glu His Ser Asp Cys Ala Phe Met Val Asp Asn Glu Ala
           195                 200                 205
   Ile Tyr Asp Ile Cys Arg Arg Asn Leu Asp Ile Glu Arg Pro Thr Tyr
       210                 215                 220
   Thr Asn Leu Asn Arg Leu Ile Gly Gln Ile Val Ser Ser Ile Thr Ala
   225                 230                 235                 240
   Ser Leu Arg Phe Asp Gly Ala Leu Asn Val Asp Leu Thr Glu Phe Gln
                   245                 250                 255
   Thr Asn Leu Val Pro Tyr Pro Arg Ile His Phe Pro Leu Ala Thr Tyr
               260                 265                 270
   Ala Pro Val Ile Ser Ala Glu Lys Ala Tyr His Glu Gln Leu Ser Val
           275                 280                 285
   Ala Glu Ile Thr Asn Ala Cys Phe Glu Pro Ala Asn Gln Met Val Lys
       290                 295                 300
   Cys Asp Pro Arg His Gly Lys Tyr Met Ala Cys Cys Met Leu Tyr Arg
   305                 310                 315                 320
   Gly Asp Val Val Pro Lys Asp Val Asn Ala Ala Ile Ala Thr Ile Lys
                   325                 330                 335
   Thr Lys Arg Thr Ile Gln Phe Val Asp Trp Cys Pro Thr Gly Phe Lys
               340                 345                 350
   Val Gly Ile Asn Tyr Gln Pro Pro Thr Val Val Pro Gly Gly Asp Leu
           355                 360                 365
   Ala Lys Val Gln Arg Ala Val Cys Met Leu Ser Asn Thr Thr Ala Ile
       370                 375                 380
   Ala Glu Ala Trp Ala Arg Leu Asp His Lys Phe Asp Leu Met Tyr Ala
   385                 390                 395                 400
   Lys Arg Ala Phe Val His Trp Tyr Val Gly Glu Gly Met Glu Glu Gly
                   405                 410                 415
   Glu Phe Ser Glu Ala Arg Glu Asp Leu Ala Ala Leu Glu Lys Asp Tyr
               420                 425                 430
   Glu Glu Val Gly Val Asp Ser Val Glu Ala Glu Ala Glu Glu Gly Glu
           435                 440                 445
   Glu Tyr
       450
<210> 47
   <211> 544
   <212> DNA
   <213> Artificial Sequence
<220>
   <223> probe derived from SOX30
<400> 47
   gaaagttttc agtcgtgatt tgaggagtta aaaccaaatg caatttatgt cttcataaaa      60
   ttttgattag tgaaactaga gtctggatgt ttcattgtag gaatatttaa gttattaagt     120
   agtttaattt taatggctga aatttgcatc aacatgtatt attattactt tatcctggaa     180
   catgcaaaat actgaagcct cacagttgta tgtgagggga aaggggaaat aaatctagca     240
   tagtgtgatt tttattttat ctcaggatac attttttaaa tgattttttg tttgcttttt     300
   atgtaatact tatggatgtt gtcaattttt gatgtaacat tttgaaagta ttttgacaac     360
   tcctagtgaa cttggacttg gttgctaaat ttaacttaca ctaataacca attataagtt     420
   ccaaatgtgt tttaatggca cctgggtgat tcttcagcta aatttagtca tttctgtttc     480
   taaatatttt tatcatttta aaatattttt tttccatttg gcatacatcg ttctttgttg     540
   taat                                                                  544
<210> 48
   <211> 289
   <212> DNA
   <213> Artificial Sequence
<220>
   <223> probe derived from SPATA22
<400> 48
   gatcgtgaac ttccgagact gattagaggc cgagttcata gatgtgttgg caactatgac      60
   cagaaaaaga acattttcca atgtgtttct gtcagaccgg cgtctgtttc tgaacaaaaa     120
   actttccagg catttgtcaa aattgcagat gttgagatgc agtattatat taatgtgatg     180
   aatgaaactt aagtagtgat aaaaggaagt ttagcataaa ttatagcagt tttctgttat     240
   tgcttaattt accatctcca tagttttata gctactattg tatttcact                 289
<210> 49
   <211> 386
   <212> DNA
   <213> Artificial Sequence
<220>
   <223> probe derived from MAEL
<400> 49
   gaggccagca atagtgtgac acccaaaatg gttgtattgg atgcagggcg ttaccagaag        60
   ctaagggttg ggagttcagg attctctcat ttcaactctt ctaatgagga acaaagatca       120
   aacacaccca ttggtgacta cccatctagg gcaaaaattt ctggccaaaa cagcagcgtt       180
   cggggaagag gaattacccg cttactagag agcatttcca attcttccag caatatccac       240
   aaattctcca actgtgacac ttcactctca ccttacatgt cccaaaaaga tggatacaaa       300
   tctttctctt ccttatctta atgatggtac tcttttcaat ttctgaaaac agtaacaggc       360
   ccaacttcct tcttactaca gtcata                                            386
<210> 50
   <211> 313
   <212> DNA
   <213> Artificial Sequence
<220>
   <223> probe derived from COX8C
<220>
   <221> misc_feature
   <222> (47)..(47)
   <223> n is a, c, g, or t
<400> 50
   ccctgtctgc cgcggaaatg gctgttggac ttgtggtgtt ttttacnacc ttcttaacac        60
   cagctgcata tgtgctaggc aacctgaagc agttcagaag gaattagatg gaagatgatg       120
   ttgaacagct gttaacgtcc aaaaaacttt cagaaaaagc tgtgtttttg ttaacgagca       180
   aaattgccta gttgagttga tgcaaccatt gtggtattca ctttcctcat gtttatgatg       240
   aatattttgc acttttttag tactgtgcat tatatagatg tatagtcaaa aatgttctgc       300
   ttaagtgtta aat                                                          313
<210> 51
   <211> 521
   <212> DNA
   <213> Artificial Sequence
<220>
   <223> probe derived from TKTL1
<400> 51
   ccttcattca tccctagttc ggaaattcaa gctaactact taccctttaa actgtcactg 60
   catatgcaag taccgctcta atttttggat cattaaaggg agttacacaa cttttaagtg 120
   aaaaaaatag gtaacaaaac aaccacctga tagtaagttt tctgataaga ctatagataa 180
   gtggtagagg taatcaattc ttccgaagtg tttccttcgt gaataactgg tagaggtaat 240
   agttttttca atgtatttcc ttcatgagta aagaaaatgt ggattgaagt atagattcca 300
   gtagcctagt ttccacagca cgataacacc atgacgccta ctgctgttcc caccttggga 360
   ttctgtgtgc tgccatccca cctgcagctg ccctggaatt cccttcgctg tttgccttca 420
   tctccctcca cgtttgagag gctgtcaggc agcagcgaaa gcttgttagg atgtcctgtg 480
   ctgcttgtga tgagagcctc cacactgtac tgttcaagtc a 521
<210> 52
   <211> 355
   <212> DNA
   <213> Artificial Sequence
<220>
   <223> probe derived from RBM46
<220>
   <221> misc feature
   <222> (41)..(41)
   <223> n is a, c, g, or t
<220>
   <221> misc feature
   <222> (56)..(56)
   <223> n is a, c, g, or t
<400> 52
   agcacttgaa gacaatggtt atagtagatt tgattaccaa ngatcactat ctgtanctgg 60
   agattagaac aattatatga ccagaagcat ctaaccatta tgtaaaaaga aatgatgaga 120
   caaaaagatt aagatacaaa ttttgtgcag tactaaagaa aaagcagtct accattgtgg 180
   tccttgaaaa taactataga tatttttgtt atttgttaga cacaaattat aattttgttg 240
   ttaatgtatt taagcatttt atagttatgc tttgtgtttt tgatattctt tgtattgtta 300
   ataacaagtg ttatgggttt ttaatgttga aatcatgtgt taatttttgt acttg 355
<210> 53
   <211> 279
   <212> DNA
   <213> Artificial Sequence
<220>
   <223> probe derived from MAGEB6
<220>
   <221> misc feature
   <222> (138)..(138)
   <223> n is a, c, g, or t
<220>
   <221> misc feature
   <222> (167)..(167)
   <223> n is a, c, g, or t
<400> 53
   tcacattcat ggctgtttaa ccaatctgaa agttacggtt tgggaattaa taaaacaaag      60
   tcatacaaca cattttcttt gtaattgaga actagataac atggtaacag agaattgatt     120
   ttcatatgaa tcttaacncc acagtaaaat agttgacatc ataatangaa gagaaagaaa     180
   aggaaaaaca gaaatgtaaa agttgtttaa ttcttggttt gcctaattcg ttttcctatt     240
   tcttttcata caaataaagg atacctggat ttatttagg                            279
<210> 54
   <211> 497
   <212> DNA
   <213> Artificial Sequence
<220>
   <223> probe derived from NBPF4
<220>
   <221> misc feature
   <222> (284)..(284)
   <223> n is a, c, g, or t
<220>
   <221> misc feature
   <222> (393)..(393)
   <223> n is a, c, g, or t
<220>
   <221> misc feature
   <222> (438)..(438)
   <223> n is a, c, g, or t
<400> 54
   gcagagaagg cccagtgtgt ccatccccag tgcggtgata ctaggatggt cacttggtta 60
   aggaggggtc taggagctct gtcccttgta aagacatctt atttgtaagt aatttggaaa 120
   gtggtttgaa atagtataaa tatcctgtat tctagtgatc ttcttcagaa cattttatca 180
   ccaattaatc accccgtctg tgtcagttat tatatttaag tttgtacatt gaaaattgtc 240
   tatctcaaaa tcttacctta tacttgcttt tgctggcatt cttngtaaaa aagatcattc 300
   cctgcccaaa ttttaacttt catccaaaat taattttaat ttctttttgc tggcattctg 360
   ttgtgaaaaa gaatattctc tgccccaatt atnactttca tccaaaatta attttagtcc 420
   atcagttaaa attttaantt ttaaatctgt ttaattaaaa catttcttgc ctctcactct 480
   ggactattgg atttttt 497
<210> 55
   <211> 417
   <212> DNA
   <213> Artificial Sequence
<220>
   <223> probe derived from C12orf37
<400> 55
   ttcctcctga ccacaaaaag cacttatact ctaggatgac tgattccagc ccagtggcct 60
   ggcaagggtg aattacacct tgcatatcac actcttgaca tttgtgtgcg ctagcataag 120
   aattataatt gaaacaggga tttaagtatc tcctctctag gtgcctaccc tccttggact 180
   caggtcaaat ttattaaagg aagttttgtt tctagatagg ttgtttgaaa taaaataaca 240
   gaatgttcaa gtaacacagt gtacctacag cttttaacaa aattgaggac ttgggtctcg 300
   aaacaatttc ctttgatttt caggtatttt atctataaaa agggagataa agcattagtt 360
   cataggacag ttatatgttt aaatgtgata atgtatatta accaccttgc atgtatt 417
<210> 56
   <211> 364
   <212> DNA
   <213> Artificial Sequence
<220>
   <223> probe derived from TPTE2P2
<220>
   <221> misc_feature
   <222> (37)..(37)
   <223> n is a, c, g, or t
<220>
   <221> misc_feature
   <222> (90)..(90)
   <223> n is a, c, g, or t
<220>
   <221> misc_feature
   <222> (92)..(92)
   <223> n is a, c, g, or t
<220>
   <221> misc_feature
   <222> (102)..(102)
   <223> n is a, c, g, or t
<220>
   <221> misc_feature
   <222> (107)..(107)
   <223> n is a, c, g, or t
<220>
   <221> misc_feature
   <222> (120)..(120)
   <223> n is a, c, g, or t
<220>
   <221> misc_feature
   <222> (179)..(179)
   <223> n is a, c, g, or t
<220>
   <221> misc_feature
   <222> (214)..(214)
   <223> n is a, c, g, or t
<220>
   <221> misc_feature
   <222> (222)..(222)
   <223> n is a, c, g, or t
<220>
   <221> misc_feature
   <222> (277)..(277)
   <223> n is a, c, g, or t
<400> 56
   ctgaggttct attcatggtg agcaagtctt ttttttngtt tgtttcttca agctctaaca      60
   agggtgccta ctacatggct tttcagttan cnccaaaata anatgtnaca attttttttn     120
   ctattcttag gctttatcta caaagaaatg aattggataa tcttcataaa caaaaaacnt     180
   ggaaaattta tcaaccagaa tatgcagtag aganatattt tnatgagaaa tgacttaagt     240
   tatgttgtaa ctggtagctg attaagtata gttcccngca ccccttctgg gaaagaatta     300
   tgttctttct aaccctgcca catagttata tgttctaaat cttccttgct ggtacatcta     360
   tatt                                                                  364
<210> 57
   <211> 504
   <212> DNA
   <213> Artificial Sequence
<220>
   <223> probe derived from DPEP3
<400> 57
   gggcagtcat tggatctgag ttcatcggga ttggtggaaa ttatgacggg actggccggt 60
   tccctcaggg gctggaggat gtgtccacat acccagtcct gatagaggag ttgctgagtc 120
   gtagctggag cgaggaagag cttcaaggtg tccttcgtgg aaacctgctg cgggtcttca 180
   gacaagtgga aaaggtgaga gaggagagca gggcgcagag ccccgtggag gctgagtttc 240
   catatgggca actgagcaca tcctgccact cccacctcgt gcctcagaat ggacaccagg 300
   ctactcatct ggaggtgacc aagcagccaa ccaatcgggt cccctggagg tcctcaaatg 360
   cctccccata ccttgttcca ggccttgtgg ctgctgccac catcccaacc ttcacccagt 420
   ggctctgctg acacagtcgg tccccgcaga ggtcactgtg gcaaagcctc acaaagcccc 480
   ctctcctagt tcattcacaa gcat 504
<210> 58
   <211> 446
   <212> DNA
   <213> Artificial Sequence
<220>
   <223> probe derived from C10orf82
<400> 58
   aggtcactga atttggctgt ggcacgagat acactgtcat ggccaaaaac tgctacaagg 60
   acttcctgga gatcacggag agggccaaga aggcacatct gaaaccatat gaagaaatat 120
   atggagttag ctccacaaaa acttctgctc cgtctccaaa agttttgcag catgaagagc 180
   tgctgccaaa atatcccgat ttttctattc cagatggaag ctgccctgcc cttggaaggc 240
   ccctgagaga ggaccccaaa actccgctga catgtggctg tgctcagagg ccaagtatac 300
   catgcagtgg gaagatgtat ctagagccac tgtcctccgc aaagtatgca gaaggctaga 360
   agcgcagagt ctcccaagga ggtgaacttt aagtggggct tccaaaacct gccattctca 420
   tgttggaatc acgcccagtg agcaat 446
<210> 59
   <211> 262
   <212> DNA
   <213> Artificial Sequence
<220>
   <223> probe derived from LOC440896
<220>
   <221> misc_feature
   <222> (234)..(234)
   <223> n is a, c, g, or t
<400> 59
   agctgagtgc cacgtgctga cgtcactaag atcaatacag caaactctga aagatggaca      60
   gagagacagg agatggtcct ttataatgca gtgtgatctg tgctgcaata gagatggaag     120
   agtgttctga tcggctggaa aacaacgcac atgggaagct gtacagaaat gagtggggaa     180
   agttttggaa gctagaagtt caaaacgagg tgttggcagc accatgctct ctcngaagat     240
   gctaggaaga atctgctcca tg                                              262
<210> 60
   <211> 503
   <212> DNA
   <213> Artificial Sequence
<220>
   <223> probe derived from CDNA clone IMAGE:5265646
<220>
   <221> misc feature
   <222> (189)..(196)
   <223> n is a, c, g, or t
<220>
   <221> misc_feature
   <222> (215)..(215)
   <223> n is a, c, g, or t
<400> 60
   gataccaaca tctaatccgc caagaaagaa tttcagaatt aaaatttggg tgtgttcttg      60
   cgctggggat ttctctgacc taccttctga tagaactttg aacccgagtc aatagtcaat     120
   atactagttt tttacttaaa actgtaacat tttaatctaa ttttggggac gtgaaataaa     180
   actaatggnn nnnnnnagaa atttttatca ctggnaataa cctaattttg aaaacactga     240
   tggttagttt cttgaaatat taatattact acaagtcata agtaaaagca ttctatctta     300
   agtgagaaac tacaaagttg gataattact atttgagttt gtggcttggt ttgaataaac     360
   acttgcttgt tttaagtaaa agttcagctg aagtgacaat caacctttaa tcttgtaaag     420
   cttctgtgtt agatattttc tatctctaac atgccaaaca tgcatattaa actgagtttt     480
   tttgcatgca atttctgtgc cca                                             503
<210> 61
   <211> 361
   <212> DNA
   <213> Artificial Sequence
<220>
   <223> probe derived from HIST1H3C
<400> 61
   tccgcgcaag cagcttgcta ctaaagcagc ccgtaagagc gctccggcca ccggtggcgt     60
   gaagaaacct catcgctacc gcccgggcac cgtggccttg cgcgaaatcc gtcgctacca    120
   gaagtccacc gagctgctga tccggaagct gccgttccag cgcctggtgc gagaaatcgc    180
   ccaggacttc aaaaccgacc tgcgtttcca gagctctgcg gtgatggcgc tgcaggaggc    240
   ttgcgaggcc tacctggtgg gactcttcga agacaccaat ctgtgcgcta ttcacgctaa    300
   acgcgtcacc atcatgccca aagatatcca gctggcacgt cgcatccgtg gggaaagggc    360
   a                                                                    361
<210> 62
   <211> 546
   <212> DNA
   <213> Artificial Sequence
<220>
   <223> probe derived from PIWIL1
<220>
   <221> misc_feature
   <222> (148)..(148)
   <223> n is a, c, g, or t
<400> 62
   attcaccggc ttccttattt tatatgtaaa aattaagatt ttatatttta tcttcttgtt     60
   tctcatagat attttgtgag catttttttg tttattttga agaaatgtgg ataagatact      120
   tggtagtata aaacagactc tctgagangt atttgaaatg tgtttggaga tttacttaaa      180
   cgtactttca ggagtgagca agtcctactt ataaacctat attaacttta tttttgagat      240
   acctgttttg aatttaaagg agataagagg cgtaaagtag gatgctcact acaaccatag      300
   gtggggtttc agctcatatc ttaaagataa aaggtactat tatataacct atacacaaga      360
   tacaggagaa aatatgcttg atttttattt ggcagggggg ctaggttgta tgggagtaaa      420
   aaaaacattg aaaattttta aattgtccaa agaaacattt taagactctt taacaaaaaa      480
   ggccatgagt aaatctctat attaacatta ctatttattt tgttttggaa ctgggacatg      540
   attcta                                                                  546
<210> 63
   <211> 521
   <212> DNA
   <213> Artificial Sequence
<220>
   <223> probe derived from C19orf41
<400> 63
   ggccgtgcgt gatgggcatg gaggggcctt tcttccggga ctacgcgctg aacgtgtttg       60
   tggggaaagt ggagacaaat caactggacc ttgtggcgtc ctttgtcaag aaccaaacgc      120
   agcacttaat gggtaactct ctgaaagatg agcctctgct ggaagagctg gtgaccctca      180
   gggcgaatgt gatcaaggaa ttcaagaaag ttttaatttc atatgaatta aaagcctgca      240
   accccaaact ttgccgcttg ctaaaagaag aggtgttgga ctgtttacat tgccagagga      300
   tcactcccaa gtgtatccac aaaaagtact gctttgtcga ccggcaaccc cgcgtggccc      360
   tgcagtacca gatggacagc aaatacccga ggaaccaggc gctgttgggc atcctcattt      420
   ctgtgtctct ggctgtcttt gtcttcgtgg tcatcgtggt ctcggcttgt acatacagac      480
   aaaaccgaaa actcctgctg cagtaggacg gtggtttggg g                          521
<210> 64
   <211> 486
   <212> DNA
   <213> Artificial Sequence
<220>
   <223> probe derived from RNF17
<400> 64
   gaatttaatc ctttatctat cttagtacaa tttgttgatt atggatcaac tgcaaagctg 60
   acattaaaca gactgtgcca aattccttct catcttatgc ggtatccagc tcgagccata 120
   aaggttctct tggcagggtt taaacctccc ttaagggatc taggggagac aagaatacca 180
   tattgtccca aatggagcat ggaggcactg tgggctatga tagactgtct tcaaggaaaa 240
   caactctatg ctgtgtccat ggctccagca ccagaacaga tagtgacatt atatgacgat 300
   gaacagcatc cagttcatat gccgttggta gaaatggggc ttgcagataa agatgaataa 360
   gtgcctaagt gtatacagtg agagcatcta tagaagccta gaagaattct gttatgttta 420
   gactatgtct tatctttaga ctatttcagg cttaattttc ctaacttgtt cagccctagt 480
   gcttta 486
<210> 65
   <211> 306
   <212> DNA
   <213> Artificial Sequence
<220>
   <223> probe derived from GALNTL5
<220>
   <221> misc_feature
   <222> (44)..(44)
   <223> n is a, c, g, or t
<400> 65
   ggaactttca ctaaggatct ggatgtgtgg aggccaactc tttnataatc ccctgctctc 60
   gagtaggaca tatcagtaag aaacaaactg gaaaaccttc tacaatcatc agtgctatga 120
   cacataacta cctaagactg gtgcacgttt ggctggatga atataaggag cagttttttc 180
   ttcgaaagcc tggtctgaaa tatgtcacct acggaaatat tcgcgagcgt gttgagttaa 240
   ggaaacgact gggttgcaag tcatttcagt ggtatttgga taatgtcttc ccagagttgg 300
   aggcat 306
<210> 66
   <211> 454
   <212> DNA
   <213> Artificial Sequence
<220>
   <223> probe derived from RFX4
<400> 66
   gatttatggc attgagtatc acactcagct ctgctgtgtt aactttgtga aactggatgg 60
   aacaaacttt aacttaccaa gcaccaagtg tgaaagtgac tttcacggtt ccttcataaa 120
   actataataa tatccgacac tttgatagaa aaaaattcaa agctgtgcct ttgagcctat 180
   actatactgt gtatgtgtgg aaataaaaat gtattgtact tttggagaat tttttgtagg 240
   catttttctg tcagatttgt agtaatttgt gaggtttgtt agagattaat ataggttttc 300
   tttctgtatt ataaaatgca ccaagcaatt atggtggacc tattacccta tgggtaagaa 360
   ataaatggaa atatgacatc ggatgtttca gcaactgttc tgtaaataaa atctttgatc 420
   acaccactca gtgtgataat tgtgtctaca gcta 454
<210> 67
   <211> 505
   <212> DNA
   <213> Artificial Sequence
<220>
   <223> probe derived from LGALS14
<400> 67
   agaacaatgt catcactacc cgtaccatac acactgcctg tttccttgcc tgttggttcg 60
   tgcgtgataa tcacagggac accgatcctc acttttgtca aggacccaca gctggaggtg 120
   aatttctaca ctgggatgga tgaggactca gatattgctt tccaattccg actgcacttt 180
   ggtcatcctg caatcatgaa cagttgtgtg tttggcatat ggagatatga ggagaaatgc 240
   tactatttac cctttgaaga tggcaaacca tttgagctgt gcatctatgt gcgtcacaag 300
   gaatacaagg taatggtaaa tggccaacgc atttacaact ttgcccatcg attcccgcca 360
   gcatctgtga agatgctgca agtcttcaga gatatctccc tgaccagagt gcttatcagc 420
   gattgaggga gatgatcaga ctcctcattg ttgaggaatc cctctttcta cctgaccatg 480
   ggattcccag agcctactaa cagaa 505
<210> 68
   <211> 526
   <212> DNA
   <213> Artificial Sequence
<220>
   <223> probe derived from IGFBP1
<400> 68
   aataatgttc tgtcacgtga aatatttaag tatatagtat atttatactc tagaacatgc 60
   acatttatat atatatgtat atgtatatat atatagtaac tactttttat actccataca 120
   taacttgata tagaaagctg tttatttatt cactgtaagt ttattttttc tacacagtaa 180
   aaacttgtac tatgttaata acttgtccta tgtcaatttg tatatcatga aacacttctc 240
   atcatattgt atgtaagtaa ttgcatttct gctcttccaa agctcctgcg tctgttttta 300
   aagagcatgg aaaaatactg cctagaaaat gcaaaatgaa ataagagaga gtagtttttc 360
   agctagtttg aaggaggacg gttaacttgt atattccacc attcacattt gatgtacatg 420
   tgtagggaaa gttaaaagtg ttgattacat aatcaaagct acctgtggtg atgttgccac 480
   ctgttaaaat gtacactgga tatgttgtta aacacgtgtc gataat 526
<210> 69
   <211> 287
   <212> DNA
   <213> Artificial Sequence
<220>
   <223> probe derived from TUBA3C
<400> 69
   tctccggcag gtgggcatta actaccagcc ccccacggtg gtccctgggg gagacctggc 60
   caaggtgcag cgggctgtgt gcatgctgag caacaccacg gccatcgcgg aggcctgggc 120
   tcgcctggac cataagttcg atctcatgta tgccaagcgg gcctttgtgc actggtacgt 180
   gggagaaggc atggaggagg gggagttctc tgaggcccgc gaggacctgg cagctctgga 240
   gaaggattat gaagaggtgg gcgtggattc cgtggaagcc gaggctg 287